# EUROPEAN PATENT APPLICATION

(11) **EP 1 728 792 A1**
(43) Date of publication of application: **06.12.2006**
(21) Application number: 05727145.4
(22) Date of filing: 25.03.2005
(51) Int. Cl.: C07D 473/16, A61K 31/522, A61K 31/5377, A61P 31/12, A61P 35/00, A61P 37/02, A61P 37/08, C07D 473/18, C07D 473/24, C07D 473/34

(54) **8-OXOADENINE COMPOUND**

(30) Priority: 26.03.2004 JP 2004093775
(71) Applicant: Dainippon Sumitomo Pharma Co., Ltd., Osaka 541-8524 (JP); AstraZeneca AB, S-151 85 Södertälje (SE)
(72) Inventor: OGITA, Haruhisa, 3310802 (JP); NAKAMURA, T. Dainippon Sumitomo Pharma Co., Ltd, Osaka 554-0022 (JP); ISOBE, Y. Dainippon Sumitomo Pharma Co., Ltd, Osaka 5540022 (JP); HASHIMOTO, K. Dainippon Sumitomo Pharma Co., Ltd, Osaka 5540022 (JP); KURIMOTO, A. Dainippon Sumitomo Pharma Co., Ltd, Osaka 5540022 (JP)
(74) Representative: Vossius & Partner
(86) International application number: PCT/JP2005/005514
(87) International publication number: WO 2005/092892

(57) **Abstract**

An 8-oxoadenine compound useful as an immuno-modulator having specific activity against Th1/Th2, specifically a prophylactic and therapeutic agent for a topical application for allergic diseases, viral siseases and cancers, which is represented by the following formula (1): 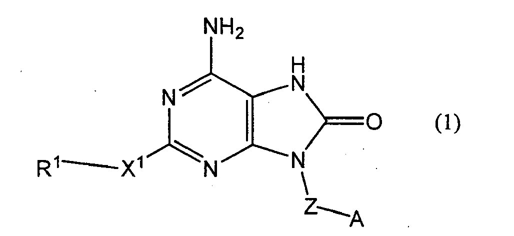 , wherein A is a group of a formula represented by the formula (2): 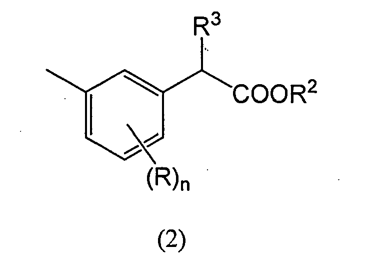 , wherein R² is a substituted or unsubstituted alkyl group and so on, R³ is hydrogen atom or an alkyl group, R is a halogen atom and so on,
n is 0-2,
X¹ is oxygen atom, Z is straight or branched chain alkylene, and R¹ is an alkyl group which is optionally substituted by hydroxy group, an alkoxy group, alkoxycarbonyl group and so on,
or its pharmaceutically acceptable salt.

## Description

### TECHNICAL FIELD

The present invention relates to a novel 8-oxoadenine compound useful as a prophylactic or therapeutic agent for allergic diseases, viral diseases or cancers.

### BACKGROUND ART

Interferon is an endogenous protein having an important role in an immune system in mammals, and not only takes a partial role in a nonspecific defense mechanism in a living body but also strongly participates in a specific defense mechanism thereof. Actually, interferon has been used as an agent for treating viral diseases such as hepatitis B and hepatitis C in a clinical field. A low molecular weight organic compound (an interferon-inducing agent) which induces a biosynthesis of the said interferon has been developed as the next generation interferon therapy, including an imidazoquinoline derivative (refer to the patent document 1) and an adenine derivative (refer to the patent documents 2 and 3), and an imidazoquinoline derivative, Imiquimod has been used as an external antiviral agent for genital wart in a clinical field.

On the other hand, T-cell taking a central role in an immune response in a living body is classified into two groups, Th1-cell and Th2-cell, and in a living body of a patient suffering from an allergic disease, an excess amount of cytokines such as interleukin-4 (IL-4) and interleukin-5 (IL-5) is excreted from Th-2 cell, and thus a compound suppressing an immune response of Th2 cell can be expected as an agent for treating allergic diseases.

The above imidazoquinoline derivative and adenine derivative have been known as showing a suppressing activity of production of interleukin-4 (IL-4) and interleukin-5 (IL-5) as well as an inducing activity of interferon, and have been actually known to be effective to an allergic disease also in a model animal.

However, there is such a fear that systemic adverse effects based on the interferon inducing activity would be problem upon using such derivatives as an anti-allergic agent.
[Patent Document 1] US Patent 4,689,338
[Patent Document 2] WO 98/01448
[Patent Document 3] WO 99/28321

### DISCLOSURE OF INVENTION

The problem to be solved by the present invention is to provide a novel 8-oxoadenine compound useful as an immuno-modulator and a medicament for allergic disease such as ashma comprising said compound as an active ingredient.

The present invention is to provide an immuno-modulator having specific activity against Th1/Th2, preferably an immuno-modulator having an interferon inducing activity and having a suppressing activity of production of a cytokine due to IL-4 and IL-5 originated from Th2-cell, and to provide a medicament for topical application which is characterized by preventing the systemic adverse effects based on the interferon inducing activity. That is, the present invention is to provide a novel 8-oxoadenine compound which is quickly metabolized to convert into a reduced active compound when topically administered, and a medicament for topical application as a therapeutic or prophylactic agent showing the reduced systemic pharmacological activity for allergic diseases, viral diseases and cancers comprising the said compound as an effective ingredient.

The present inventors have made extensive study for obtaining an immuno-modulator useful as a therapeutic or a prophylactic agent for allergic diseases such as asthma, viral diseases and cancers which shows potent effect at the administered region and does not show the systemic adverse effects when externally administered in a form of aerosols, etc., to find the 8-oxoadenine compound of the present invention. Namely, the compound of the present invention is useful as a therapeutic or prophylactic agent for allergic diseases, viral diseases and cancers with the reduced systemic pharmacological activity.

The prevent invention has been completed on the basis of the above finding.

### BEST MODE FOR CARRYING OUT THE INVENTION

The present invention is as follows:
[1] An 8-oxoadenine compound represented by the following formula (1): 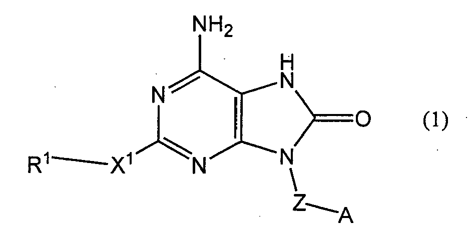 , wherein A is a group selected from the group consisting of the following formulas (2) to (8): 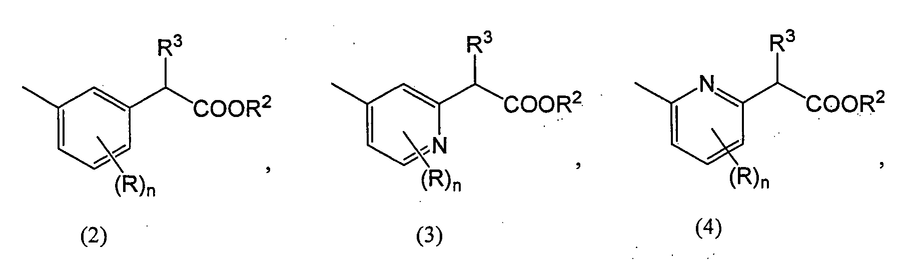 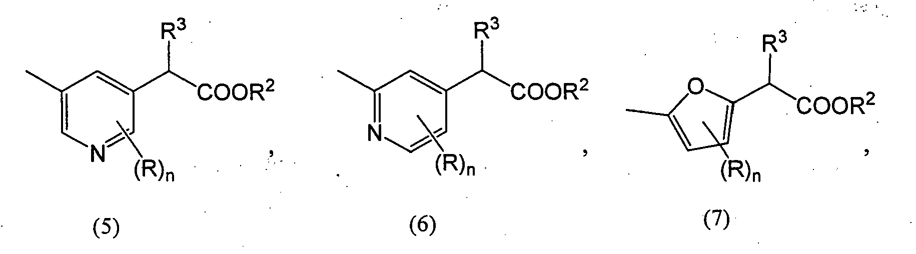 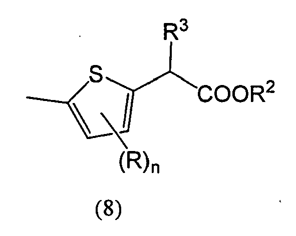 , wherein R² is hydrogen atom, or a substituted or unsubstituted alkyl group;
R³ is hydrogen atom or an alkyl group;
R is a halogen atom, a haloalkyl group, a haloalkoxy group, an alkyl group, an alkoxy group, amino group, an alkylamino group or a dialkylamino group;
n is an integer of 0 to 2, and when n is 2, R_{S} may be the same or different;
X¹ is oxygen atom, sulfur atom, SO₂, NR⁴ (wherein R⁴ is hydrogen atom or an alkyl group.), or a single bond;
Z is a straight or branched chain alkylene;
R¹ is hydrogen atom, a halogen atom, a substituted or unsubstituted alkyl group, a substituted or unsubstituted alkenyl group, a substituted or unsubstituted alkynyl group, a substituted or unsubstituted aryl group, a substituted or unsubstituted heteroaryl group or a substituted or unsubstituted cycloalkyl group,
or a pharmaceutically acceptable salt thereof.
[2] The 8-oxoadenine compound as described in the above [1], wherein R² is a substituted or unsubstituted C₁₋₈ alkyl group, wherein said alkyl group may be substituted by one or plural substituents which may be the same or different,
and the substituents on said alkyl group are selected from the group consisting of a halogen atom, hydroxy group, carboxy group, C₃₋₈ cycloalkyl group, an C₁₋₆ alkoxy group, an C₁₋₆ alkylthio group, a C₃₋₈ cycloalkoxy group, an C₂₋₁₀ acyloxy group, an C₁₋₆ alkylsulfonyl group, an C₁₋₆ alkylsulfinyl group, a substituted or unsubstituted carbamoyl group, a substituted or unsubstituted sulfamoyl group, a substituted or unsubstituted amino group, a substituted or unsubstituted 6 to 10 membered aryl group, a substituted or unsubstituted 5 to 10 membered heteroaryl group which contains 1 to 4 hetero atoms consisting of 0 to 2 nitrogen atoms, 0 to 1 oxygen atom and 0 to 1 sulfur atom, and a substituted or unsubstituted 4 to 7 membered saturated heterocyclic group which contains 1 to 4 hetero atoms consisting of 0 to 2 nitrogen atoms, 0 to 2 oxygen atoms and 0 to 2 sulfur atoms;
R³ is hydrogen atom or an alkyl group;
R is a halogen atom, a C₁₋₆ haloalkyl group, a C₁₋₆ haloalkoxy group, an C₁₋₆ alkyl group, an C₁₋₆ alkoxy group, amino group, an C₁₋₆ alkylamino group, or a di C₁₋₆ alkyl amino group;
n is an integer of 0 to 2, and when n is 2, Rs may be the same or different;
X¹ is oxygen atom, sulfur atom, SO₂, NR⁴ (wherein R⁴ is hydrogen atom or an C₁₋₆ alkyl group.), or a single bond;
Z is a straight or branched chain C₁₋₈ alkylene;
R¹ is hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group;
and the substituents of the said alkyl group, alkenyl group and alkynyl group are selected from the group consisting of a halogen atom, hydroxy group, carboxy group, an C₁₋₆ alkoxy group, a C₁₋₆ haloalkoxy group, an C₁₋₆
alkylthio group, an C₁₋₆ alkylsulfonyl group, an C₁₋₆ alkylsulfinyl group, an C₂₋₅ alkoxycarbonyl group, an C₂₋₁₀ acyloxy group, a substituted or unsubstituted amino group, a substituted or unsubstituted carbamoyl group, a substituted or unsubstituted sulfamoyl group, an ureido group which may be substituted by the same or different one or two alkyl groups, a substituted or unsubstituted 6 to 10 membered aryl group, a substituted or unsubstituted aryloxy group, a substituted or unsubstituted arylthio group, a substituted or unsubstituted 5 to 10 membered heteroaryl group which contains 1 to 4 hetero atom selected from 0 to 2 nitrogen atoms, 0 to 1 oxygen atom and 0 to 1 sulfur atom, a substituted or unsubstituted heteroaryloxy group, a substituted or unsubstituted heteroarylthio group, a substituted or unsubstituted C₃₋₈ cycloalkyl group, a substituted or unsubstituted C₃₋₈ cycloalkoxy group, a substituted or unsubstituted cycloalkylthio group, a substituted or unsubstituted 4 to 7 membered saturated heterocyclic group which contains 1 to 4 hetero atoms selected from 0 to 2 nitrogen atoms, 0 to 2 oxygen atoms and 0 to 2 sulfur atoms, a substituted or unsubstituted saturated heterocycle-oxy group, and a substituted or unsubstituted saturated heterocycle-thio group; and the substituents of said amino group, carbamoyl group and sulfamoyl group are selected from the group consisting an C₁₋₆ alkyl group, an C₂₋₆ alkenyl group, an C₂₋₆ alkynyl group, C₃₋₈ acycloalkyl group, an C₂₋₅ alkylcarbonyl group, an C₂₋₅ alkoxycarbonyl group and an C₁₋₆ alkylsulfonyl group (the above seven groups may be substituted by a halogen atom, hydroxy group or an alkoxy group, respectively.), or the two substituents may be combined together to form a a substituted or unsubstituted 4 to 7 membered saturated heterocyclic group containing 1 to 4 hetero atoms selecting from 1 to 2 nitrogen atoms, 0 to 1 oxygen atom and 0 to 1 sulfur atom;
the substituents of said aryl group, aryloxy group, arylthio group, heteroaryl group, heteroaryloxy group, heteroarylthio group, cycloalkyl group, cycloalkoxy group, cycloalkylthio group, saturated heterocyclic group, saturated heterocycle-oxy group, saturated heterocycle-thio group and saturated nitrogen containing heterocyclic group are selected from the group consisting of a halogen atom, hydroxy group, carboxy group, an C₁₋₆ alkyl group, an C₁₋₆ alkoxy group, an C₂₋₅ alkylcarbonyl group, an C₂₋₅ alkoxycarbonyl group (the above four groups may be substituted by a halogen atom, hydroxy group or an alkoxy group, respectively.), a C₁₋₆ haloalkyl group, a C₁₋₆ haloalkoxy group, amino group, an C₁₋₆ alkylamino group, and a di C₁₋₆ alkyl amino group, in the formula (1),
or its pharmaceutically acceptable salt.
[3] The 8-oxoadenine compound or a pharmaceutically acceptable salt thereof as described in [1] or [2] wherein R² in the formula (1) is methyl group.
[4] The 8-oxoadenine compound or a pharmaceutically acceptable salt thereof as described in [1] or [2] wherein R² in the formula (1) is a substituted C₂₋₆ alkyl group.
[5] The 8-oxoadenine compound or a pharmaceutically acceptable salt thereof as described in [4] wherein R² in the formula (1) is an C₂₋₁₀ alkyl group substituted by a substituted or unsubstituted amino group.
[6] The 8-oxoadenine compound or a pharmaceutically acceptable salt thereof as described in any one of [1] to [5] wherein R³ in the formula (1) is hydrogen atom.
[7] The 8-oxoadenine compound or a pharmaceutically acceptable salt thereof as described in any one of [1] to [6] wherein Z in the formula (1) is a straight chain C₁₋₆ alkylene group.
[8] The 8-oxoadenine compound or a pharmaceutically acceptable salt thereof as described in any one of [1] to [7] wherein X¹ in the formula (1) is a single bond, oxygen atom or sulfur atom.
[9] The 8-oxoadenine compound or a pharmaceutically acceptable salt thereof as described in any one of [1] to [8] wherein R¹ in the formula (1) is an C₁₋₆ alkyl group which is optionally substituted by an alkoxycarbonyl group, hydroxy group or an alkoxy group.
[10] The 8-oxoadenine compound or a pharmaceutically acceptable salt thereof as described in [1] wherein X¹ in the formula (1) is a single bond, R¹ is an C₁₋₆ alkyl group which is substituted by methoxycarbonyl group.
[11] The 8-oxoadenine compound or a pharmaceutically acceptable salt thereof as described in any one of [1] to [10], provided that the compounds mentioned in the following Table 1 are excluded.

**Table 1**

| Compound | Structure |
|---|---|
| 2-Butoxy-9-(5-methoxycarbonylmethyl furfuryl)-8-oxoadenine | 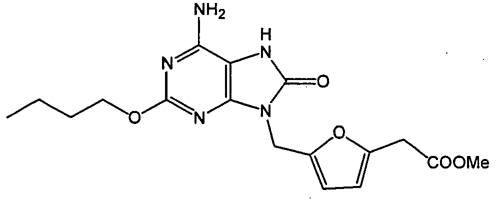 |
| 2-Butoxy-9-(3-methoxycarbonylmethyl benzyl)-8-oxoadenine | 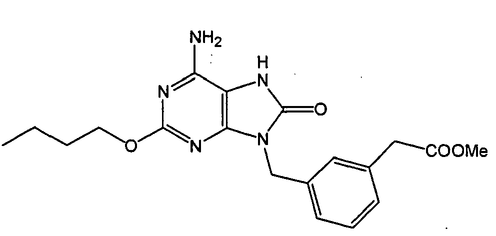 |
| 2-Butoxy-9-(3-ethoxycarbonylmethylbenzyl)-8-oxoadenine | 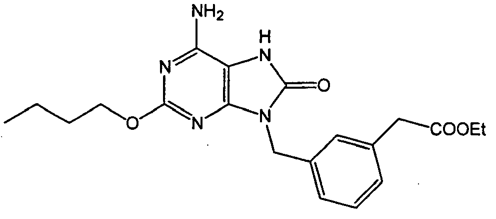 |
| 9-(3-Methoxycarbonylmethyl benzyl)-2-(2-methoxyethoxy)-8-oxoadenine | 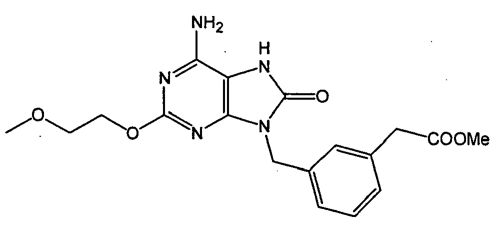 |
| 2-(2-Hydroxyethylthio)-9-(3-methoxycarbonylmethyl benzyl)-8-oxoadenine | 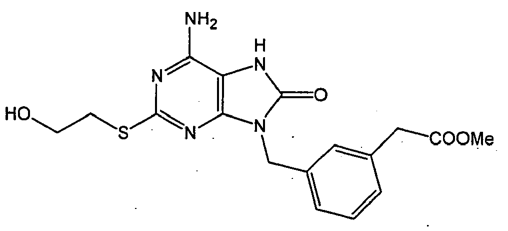 |
| 2-Butylamino-9-(3-methoxycarbonylmethyl benzyl)-8-oxoadenine | 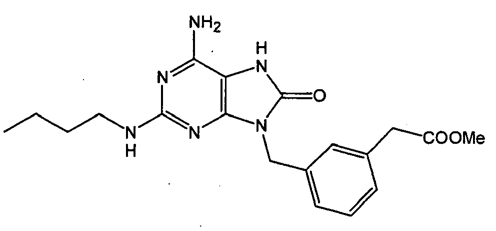 |
| 2-Butoxy-9-{3-((1R,S)-1-methoxycarbonylethyl)benzyl}-8-oxoadenine | 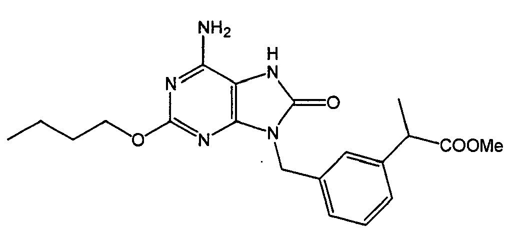 |
| 2-Butoxy-9-{(5-methoxycarbonylmethyl-3-pyridyl)methyl}-8-oxoadenine | 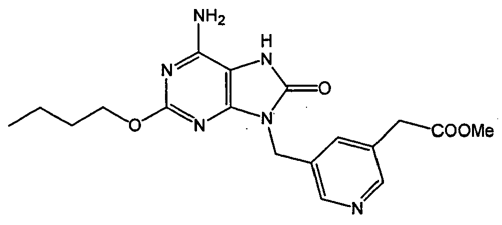 |
| 2-Butoxy-9-{3-(2-fluoroethoxycarbonylmethyl) benzyl}-8-oxoadenine | 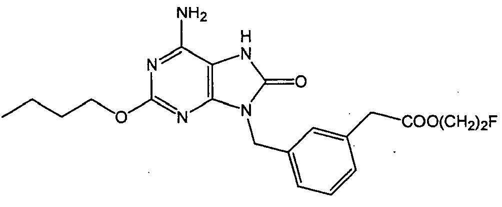 |
| 2-Butoxy-9-{2-fluoro-3-(hydroxymethyloxycarbonyl methyl)benzyl}-8-oxoadenine | 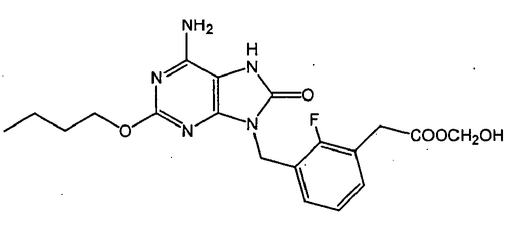 |
| 2-Butoxy-9-{3-(methoxycarbonylmethyl)-5-methylbenzyl}-8-oxoadenine | 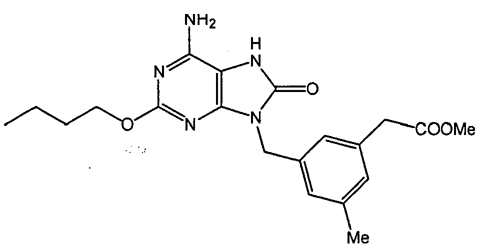 |
| 2-Butoxy-9-[2-{5-(2-fluoroethoxycarbonylmethyl)-3-pyridyl}ethyl]-8-oxoadenine | 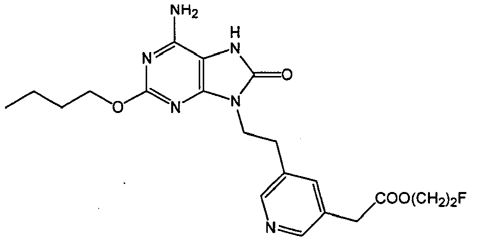 |
| 2-Butoxy-9-(6-methyl-2-(1-methoxycarbonyl)ethyl-4-pyridylmethyl)-8-oxoadenine | 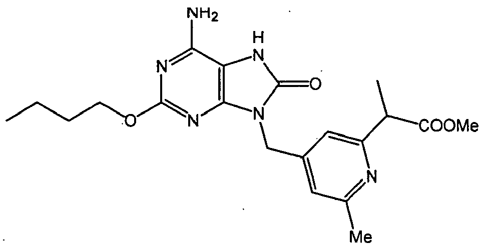 |
| 2-Methoxyethyl-9-(5-methyl-3-methoxycarbonylmethyl benzyl)-8-oxoadenine | 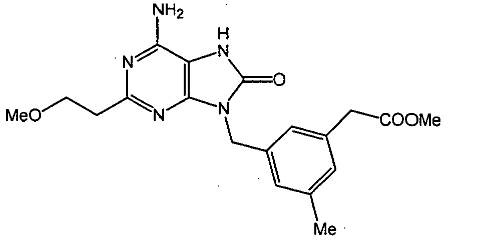 |
| 2-Methoxymethyl-9-[2-{5-(2-fluoroethoxycarbonylmethyl)-3-pyridyl}ethyl]-8-oxoadenine | 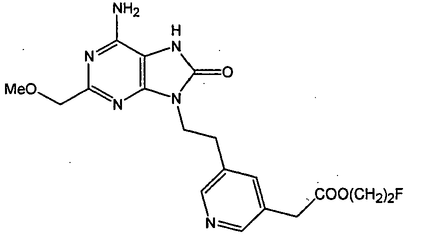 |
| 2-Methoxymethylamino-9-(5-methyl-3-methoxycarbonylmethyl benzyl)-8-oxoadenine | 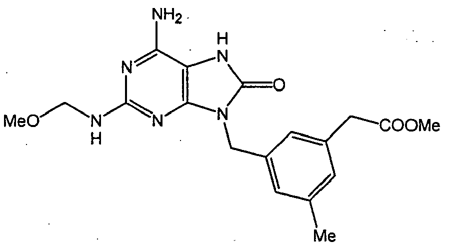 |
| 2-Butylamino-9-[2-{5-(2-fluoroethoxycarbonylmethyl)-3-pyridyl}ethyl]-8-oxoadenine | 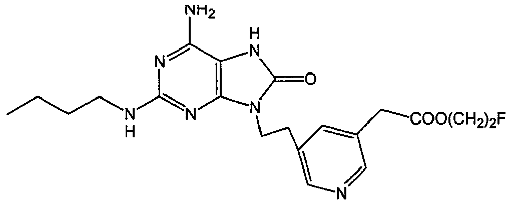 |
| 2-(3-Ethoxypropylthio)-9-{3-(2-fluoroethoxycarbonylmethyl) benzyl}-8-oxoadenine | 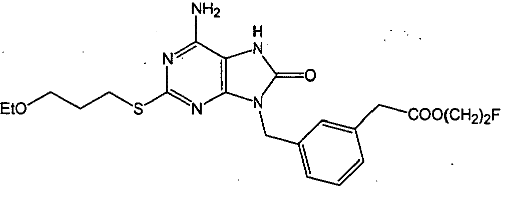 |
| 2-Butylthio-9-(2-fluoro-3-hydroxymethyloxycarbonyl methylbenzyl)-8-oxoadenine | 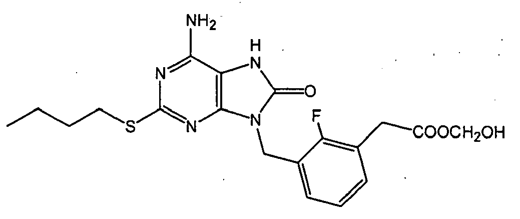 |
| 2-(2-Hydroxyethoxy)-9-(6-methyl-2-methoxycarbonylmethyl-4-pyridylmethyl)-8-oxoadenine | 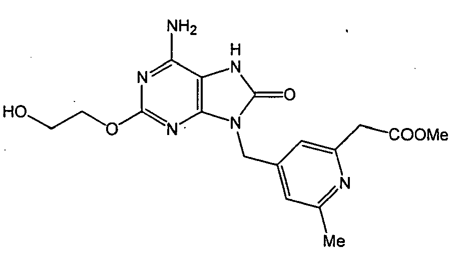 |
| 2-(2-Ethoxycarbonyl)ethyl-9-{3-(2-fluoroethoxycarbonylmethyl) benzyl}-8-oxoadenine | 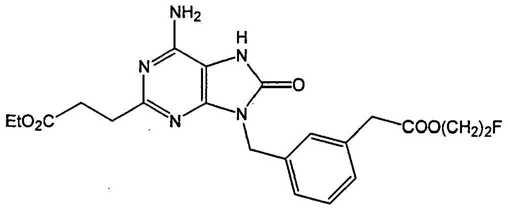 |
| 2-(2-Ethoxycarbonylethylthio)-9-{3-(2-fluoroethoxycarbonylmethyl) benzyl}-8-oxoadenine | 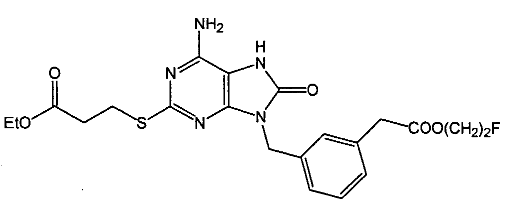 |
| 2-(2-Ethoxycarbonylethoxy)-9-{3-(2-fluoroethoxycarbonylmethyl) benzyl}-8-oxoadenine | 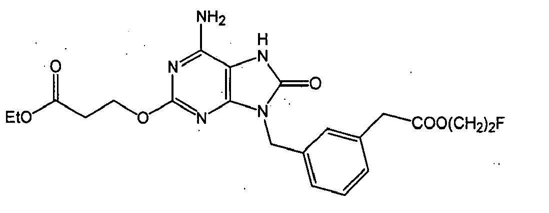 |

[12] The 8-oxoadenine compound or a pharmaceutically acceptable salt thereof as described in [1] wherein A in the formula (1) is a formula (4), a formula (6) or a formula (8): 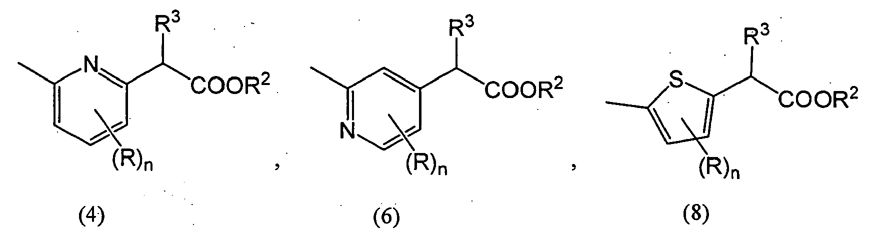 , wherein n, R² and R³ are the same meaning as defined above.
[13] The 8-oxoadenine compound or a pharmaceutically acceptable salt thereof as described in [1] wherein Z in the formula (1) is a straight chain C₂₋₅ alkylene, provided that A is not a group represented by the formula (9): 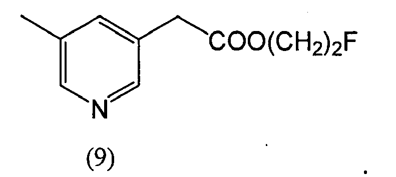
[14] The 8-oxoadenine compound or a pharmaceutically acceptable salt thereof as described in [1] wherein A in the formula (1) is a group represented by the formula (10): 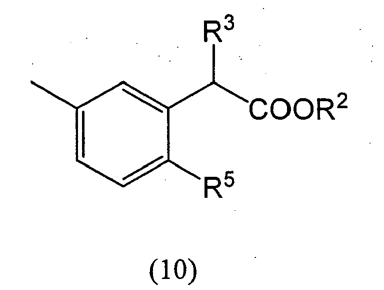 , wherein, R² and R³ are the same as defined above and R⁵ is a halogen atom or an alkoxy group.
[15] The 8-oxoadenine compound or a pharmaceutically acceptable salt thereof as described in [1] wherein A in the formula (1) is a group represented by the formula (11): 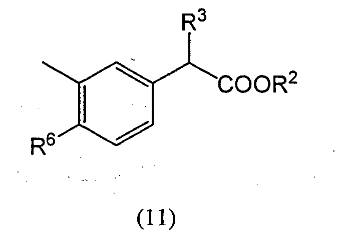 , wherein, R² and R³ are the same as defined above and R⁶ is a halogen atom or an alkoxy group.
[16] The 8-oxoadenine compound or a pharmaceutically acceptable salt thereof as described in [1] wherein A in the formula (1) is a group represented by the formula (12): 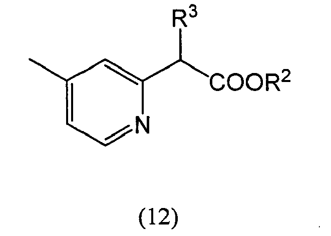 , wherein R² and R³ are the same as defined above.
[17] The 8-oxoadenine compound or a pharmaceutically acceptable salt thereof as described in [1] wherein R¹ in the formula (1) is an alkoxycarbonyl group, provided that R² is not 2-fluoroethyl group, Y¹ is not ethylene group and R¹ is not ethoxycarbonyl group.
[18] The 8-oxoadenine compound or a pharmaceutically acceptable salt thereof as described in [17] wherein X¹ is a single bond.
[19] A pharmaceutical composition containing the 8-oxoadenine compound or a pharmaceutically acceptable salt thereof as described in any one of [1] to [18] as an active ingredient.
[20] A medicament for topical administration containing the 8-oxoadenine compound or a pharmaceutically acceptable salt thereof as described in any one of [1] to [18] as an active ingredient.
[21] An immuno-modulator containing the 8-oxoadenine compound or a pharmaceutically acceptable salt thereof as described in any one of [1] to [18] as an active ingredient.
[22] A therapeutic or prophylactic agent for viral diseases, cancers or allergic diseases containing the 8-oxoadenine compound or a pharmaceutically acceptable salt thereof as described in any one of [1] to [18] as an active ingredient.
[23] Use of the 8-oxoadenine compound, or a pharmaceutically acceptable salt thereof as described in any of [1] to [18], as a medicament.
[24] Use of the 8-oxoadenine compound, or a pharmaceutically acceptable salt thereof as described in any of [1] to [18] for manufacturing an immuno-modulator.
[25] Use of the 8-oxoadenine compound, or a pharmaceutically acceptable salt thereof as described in any of [1] to [18] for manufacturing a therapeutic or prophylactic agent for viral diseases, cancers or allergic diseases.
[26] A method for modulating immune response which comprises administering, to a patient an effective amount of the 8-oxoadenine compound, or a pharmaceutically acceptable salt thereof as described in any of [1] to [18].
[27] A method for treating or preventing viral diseases, cancers or allergic diseases which comprises administering, to a patient an effective amount of the 8-oxoadenine compound, or a pharmaceutically acceptable salt thereof as described in any of [1] to [18].
[28] A process for preparing the 8-oxoadenine compound as described in any of [1] to [18], which comprises brominating a compound represented by the formula (9): 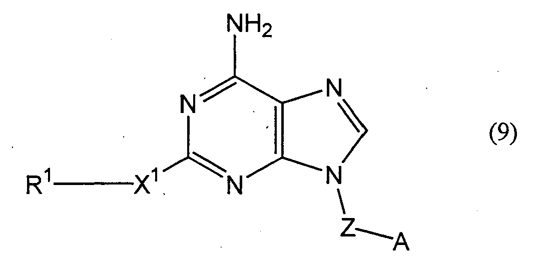 , wherein A, Z, R¹ and X¹ are the same as defined above, , reacting the resultant with a metal alkoxide and then hydrolyzing, or hydrolyzing the resultant.
[29] A compound represented by the formula (9): 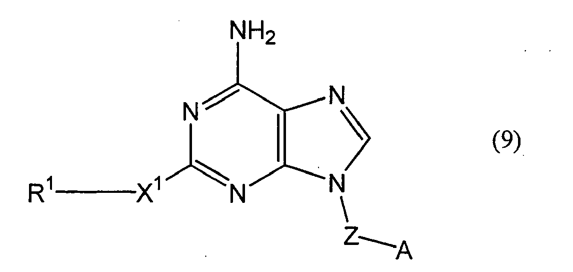 , wherein A, Z, R¹ and X¹ are the same as defined above.

### DETAILED DESCRIPTION OF THE PREFERABLE EMBODIMENTS

"A halogen atom" in the present specification is exemplified by fluorine, chlorine, bromine or iodine, preferably fluorine or chlorine.

"Alkyl group" is exemplified by a straight or branched chain C₁₋₁₀ alkyl group, including specifically methyl group, ethyl group, propyl group, 1-methylethyl group, butyl group, 2-methylpropyl group, 1-methylpropyl group, 1,1-dimethylethyl group, pentyl group, 3-methylbutyl group, 2-methylbutyl group, 2,2-dimethylpropyl group, 1-ethylpropyl group, 1,1-dimethylpropyl group, hexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, heptyl group, 1-methylhexyl group, 1-ethylpentyl group, octyl group, 1-methylheptyl group, 2-ethylhexyl group, nonyl group, and decyl group, and preferably an C₁-C₆ alkyl group, more preferably an C₁-C₄ alkyl group.

"Alkenyl group" is exemplified by a straight or branched chain C₂-C₁₀ alkenyl group, including specifically ethenyl group, propenyl group, 1-methylethenyl group, butenyl group, 2-methylpropenyl group, 1-methylpropenyl group, pentenyl group, 3-methylbutenyl group, 2-methylbutenyl group, 1-ethylpropenyl group, hexenyl group, 4-methylpentenyl group, 3-methylpentenyl group, 2-methylpentenyl group,1-methylpentenyl group, 3,3-dimethylbutenyl group, 1,2-dimethylbutenyl group, heptenyl group,1-methylhexenyl group, 1-ethylpentenyl group, octenyl group, 1-methylheptenyl group, 2-ethylhexenyl group, nonenyl group, and decenyl group, and preferably an C₁-C₆ alkenyl group, more preferably an C₁-C₄ alkenyl group.

"Alkynyl group" is exemplified by a straight or branched chain C₁-C₁₀ alkynyl group including specifically ethinyl group, propynyl group, butynyl group, pentynyl group, 3-methylbutynyl group, hexynyl group, 4-methylpentynyl, 3-methylpentynyl, 3,3-dimethylbutynyl, heptynyl, octynyl, 3-methylheptynyl, 3-ethylhexynyl, nonynyl and decynyl, and preferably C₁-C₆ alkynyl group, more preferably an C₁-C₄ alkynyl group.

"Cycloalkyl group" is exemplified by a 3 to 8 membered monocyclic cycloalkyl group, including specifically cyclopropyl group, cyclobutyl group, cyclopentyl group, cyclohexyl group, cycloheptyl group, and cyclooctyl group.

"Cycloalkoxy group" is exemplified by a 3 to 8 membered monocyclic cycloalkoxy group, including specifically cyclopropoxy group, cyclobutoxy group, cyclopentyloxy group, cyclohexyloxy group, cycloheptyloxy group, and cyclooctyloxy group.

Cycloalkyl moiety in "cycloalkylthio group" is the same as the above mentioned cycloalkyl group.

"Alkylene" is exemplified by a straight or branched chain C₁-C₆ alkylene, including specifically methylene, ethylene, trimethylene, tetramethylene, pentamethylene, hexamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, and 3-methylpentamethylene, and preferably an C₁-C₄ alkylene.

"Alkoxy group" is exemplified by a straight or branched chain C₁-C₁₀ alkoxy group, including specifically methoxy group, ethoxy group, propoxy group, 1-methylethoxy group, butoxy group, 2-methylpropoxy group, 1-methylpropoxy group, 1,1-dimethylethoxy group, pentoxy group, 3-methylbutoxy group, 2-methylbutoxy group, 2,2-dimethylpropoxy group, 1-ethylpropoxy group, 1,1-dimethylpropoxy group, hexyloxy group, 4-methylpentyloxy group, 3-methylpentyloxy group, 2-methylpentyloxy group, 1-methylpentyloxy group, 3,3-dimethylbutoxy group, 2,2-dimethylbutoxy group, 1,1-dimethylbutoxy group, 1,2-dimethylbutoxy group, heptyloxy group, 1-methylhexyloxy group, 1-ethylpentyloxy group, octyloxy group, 1-methylheptyloxy group, 2-ethylhexyloxy group, nonyloxy group, and decyloxy group, and preferably an C₁-C₆ alkoxy group, more preferably an C₁-C₄ alkoxy group.

"Alkylthio group" is exemplified by a straight or branched chain C₁-C₁₀ alkylthio group, including specifically methylthio group, ethylthio group, propylthio group, 1-methylethylthio group, butylthio group, 2-methylpropylthio group, 1-methylpropylthio group, 1,1-dimethylethylthio group, pentylthio group, 3-methylbutylthio group, 2-methylbutylthio group, 2,2-dimethylpropylthio group, 1-ethylpropylthio group, 1,1-dimethylpropylthio group, hexylthio group, 4-methylpentylthio group, 3-methylpentylthio group, 2-methylpentylthio group, 1-methylpentylthio group, 3,3-dimethylbutylthio group, 2,2-dimethylbutylthio group, 1,1-dimethylbutylthio group, 1,2-dimethylbutylthio group, heptylthio group, 1-methylhexylthio group, 1-ethylpentylthio group, octylthio group, 1-methylheptylthio group, 2-ethylhexylthio group, nonylthio group, or decylthio group, preferably an C₁-C₆ alkylthio group, more preferably an C₁-C₄ alkylthio group.

"Alkoxy moiety" in "Alkoxycarbonyl group" is the same as the above mentioned alkoxy group. The alkoxycarbonyl group is exemplified by a straight or branched chain C₂-C₅ alkoxycarbonyl group, including specifically methoxycarbonyl group, ethoxycarbonyl group, propoxycarbonyl group, 2-methylethoxycarbonyl group, butoxycarbonyl group and 2-methylpropoxycarbonyl group.

"Alkyl moiety" in "alkylcarbonyl group", "alkylsulfonyl group" or "alkylsulfinyl group" is the same as the above mentioned alkyl group. The alkylsulfonyl group is preferably exemplified by a straight or branched chain C₁₋₄ alkylamino group, including specifically methanesulfonyl group, ethanesulfonyl group, propylsulfonyl group, 2-methylethylsulfonyl group and butylsulfonyl group.

"Alkyl moiety" in "alkylamino group" is the same as the above mentioned alkyl group. The alkylamino group is preferably exemplified by a straight or branched chain C₁-C₄ alkylamino group, including specifically methylamino group, ethylamino group, propylamino group, 1-methylethylamino group and butylamino group.

"Two alkyl moieties" in "dialkylamino group" are the same or different and the alkyl moiety is the same as the above mentioned alkyl group. The dialkylamino group is preferably exemplified by a straight or branched chain di-C₁-C₄ alkylamino group, including specifically dimethylamino group, diethylamino group, dipropylamino group, methylethylamino group, methylpropylamino group and ethylpropylamino group.

"Haloalkyl group" is exemplified by an alkyl group substituted by the same or different, 1-5 halogen atoms, including specifically trifluoromethyl group, 2,2,2-trifluoroethyl group, 2,2-difluoroethyl group and pentafluoroethyl group.

"Haloalkoxy group" is exemplified by an alkoxy group substituted by the same or different, 1-5 halogen atoms, including specifically trifluoromethoxy group, 2,2,2-trifluoroethoxy group, 2,2-difluoroethoxy group, 2-fluoroethoxy group and pentafluoroethoxy group.

"Aryl group" is exemplified by an C₆-C₁₀ aryl group, including specifically phenyl group, 1-naphthyl group or 2-naphthyl group.

"Aryl moiety" in "aryloxy group" and "arylthio group" is the same as the above mentioned aryl group.

"Heteroaryl group" is exemplified by a 5-10 membered mono or bicyclic heteroaryl group containing 1-4 hetero atoms selected from 0-2 nitrogen atoms, 0-1 oxygen atom and 0-1 sulfur atom, including specifically furyl group, thienyl group, pyrrolyl group, pyridyl group, indolyl group, isoindolyl group, quinolyl group, isoquinolyl group, pyrazolyl group, imidazolyl group, pyrimidinyl group, pyrazinyl group, pyridazinyl group, thiazolyl group and oxazolyl group.

"Heteroaryl moiety" in "heteroaryloxy group" or "heteroarylthio group" is the same as the above mentioned heteroaryl group.

The substituents wherein alkyl group, alkenyl group and alkynyl group are substituted in the present specification include a halogen atom, hydroxy group, carboxy group, an alkoxy group, a haloalkoxy group, an alkylthio group, an alkylsulfonyl group, an alkylsulfinyl group, an alkoxycarbonyl group, a substituted or unsubstituted acyloxy group, a substituted or unsubstituted amino group, a substituted or unsubstituted carbamoyl group, a substituted or unsubstituted sulfamoyl group, a substituted or unsubstituted ureido group, a substituted or unsubstituted aryl group, a substituted or unsubstituted aryloxy group, a substituted or unsubstituted arylthio group, a substituted or unsubstituted heteroaryl group, a substituted or unsubstituted heteroaryloxy group, a substituted or unsubstituted heteroarylthio group, a substituted or unsubstituted cycloalkyl group, a substituted or unsubstituted cycloalkoxy group, a substituted or unsubstituted cycloalkylthio group, a substituted or unsubstituted saturated heterocyclic group, a substituted or unsubstituted saturated heterocycle-oxy group, and a substituted or unsubstituted saturated heterocycle-thio group.

The above mentioned acyloxy group includes C₂-C₁₀ acyloxy group, such as an C₂-C₆ alkylcarbonyloxy group, an arylcarbonyloxy group, or an heteroarylcarbonyloxy group. "Alkyl moiety" in the above mentioned alkylcarbonyloxy group is the same as the above mentioned alkyl group. "Aryl moiety" in the above mentioned arylcarbonyloxy group is the same as the above mentioned aryl group. "Heteroaryl moiety" in above mentioned heteroarylcarbonyloxy group is the same as the above mentioned heteroaryl group.

"Saturated heterocyclic group" is exemplified by a 4-7 membered saturated heterocyclic group containing 1-4 hetero atoms selected from 0-2 nitrogen atoms, 0-2 oxygen atom and 0-2 sulfur atom, including specifically, tetrahydropyranyl group, pyrrolidinyl group, tetrahydrofuranyl group, piperidinyl group, piperazinyl group, morpholinyl group and thiomorpholinyl group.

The substituents in a substituted or unsubstituted "amino group", a substituted or unsubstituted "carbamoyl group" and a substituted or unsubstituted "sulfamoyl group" include an alkyl group, an alkenyl group, an alkynyl group, a cycloalkyl group, an alkylcarbonyl group, an alkoxycarbonyl group and an alkylsulfonyl group. The above seven groups may be substituted by a halogen atom, hydroxy group or a C₁₋₄ alkoxy group, respectively.

Two substituents on the amino group, carbamoyl group and sulfamoyl group may be combined together to form a saturated or unsaturated 4-7 membered heterocycle containing 1-4 hetero atoms selected from 1-2 nitrogen atoms, 0-1 oxygen atom and 0-1 sulfur atom, including specifically, azetidine, pyrrolidine, piperidine, piperazine, morpholine, thiomorpholine and perhydroazepine.

When the aryl group, aryloxy group, arylthio group, heteroaryl group, heteroaryloxy group, heteroarylthio group, cycloalkyl group, cycloalkoxy group, saturated heterocyclic group, saturated heterocycle-oxy group, saturated heterocycle-thio group and a saturated nitrogen containing heterocyclic group are substituted, the substituents include a halogen atom, hydroxy group, carboxy group, an alkyl group, an alkoxy group, an alkylcarbonyl group, an alkoxycarbonyl group (the alkyl group, alkoxy group, alkylcarbonyl group and alkoxycarbonyl group may be substituted by a halogen atom, hydroxy group or an alkoxy group, respectively.), a haloalkyl group, a haloalkoxy group, amino group, an alkylamino group, and a dialkylamino group.

A in the formula (1) is preferably the group represented by the formula (2), the formula (3), the formula (4), the formula (7) or the formula (8), more preferably the group represented by the formula (2), the formula (7) or the formula (8).

R in the formulas (2) to (8) is preferably, fluorine, chlorine, methyl group, ethyl group, methoxy group, ethoxy group, trifluoromethyl group, trifluoromethoxy group and dimethylamino group.

n in the formulas (2) to (8) is preferably 0 or 1.

R² in the formula (2) to (8) is preferably C₁-C₄ alkyl group, a substituted C₂-C₈ alkyl group or a C₃-C₈ acyloxy alkyl group. The substituents on the substituted C₂-C₆ alkyl group are preferably a substituted or unsubstituted amino group. The acyloxyalkyl group includes acetoxymethyl group; 1-acetoxyethyl group and benzoyloxymethyl.

R² is further preferably methyl group, and a C₂-C₆ alkyl group substituted by a substituted or unsubstituted amino group.

The compound in the formula (1) wherein R² represents hydrogen atom is also useful as the synthetic intermediate of the compound wherein R² represents except hydrogen atom. The compound in the formula (1) wherein R² represents hydrogen atom is also useful as a reagent for testing pharmacokinetics of the compound wherein R² represents except hydrogen atom because the former corresponds to a metabolite of the latter.

R³ in the formulas (2) to (8) is preferably hydrogen atom or methyl group, more preferably hydrogen atom.

Z in the formula (1) is preferably a straight or branched chain C₁-C₆ alkylene, more preferably a straight chain C₁-C₅ alkylene such as methylene, methylmethylene, ethylene, trimethylene, tetramethylene, pentamethylene or hexamethylene, and further preferably an C₁-C₄ alkylene.

When X¹ is NR⁴ in the formula (1), R⁴ is preferably hydrogen atom or an C₁-C₃ alkyl group, more preferably hydrogen atom or methyl group. X¹ is preferably a single bond, oxygen atom or sulfur atom.

R¹ in the formula (1) is preferably a substituted or unsubstituted straight or branched chain C₁-C₆ alkyl group, including methyl group, ethyl group, propyl group, butyl group, pentyl group, 1-methylethyl group, 1-methylpropyl group and 2-methylbutyl group which are respectively substituted or unsubstituted, and preferably a straight chain C₁-C₄ alkyl group.

When R¹ is a substituted alkyl group, said group may be substituted by one or more, preferably 1 to 3 and the same or different substituents. The substituents on said substituted alkyl group is preferably fluorine, hydroxy group, a straight or branched chain C₁-C₄ alkoxy group, a straight or branched chain C₁-C₄ alkylthio group, a straight or branched chain C₂-C₅ alkoxycarbonyl group, a straight or branched chain C₁-C₄ alkylsulfonyl group (The alkoxy group, the alkylthio group, the alkoxycarbonyl group and the alkylsulfonyl group may be respectively substituted by a halogen atom, hydroxy group, an C₁-C₄ alkoxy group, an C₂-C₅ alkylcarbonyloxy group, benzoyloxy group, phenyl group or pyridyl group.), amino group, a straight or branched chain C₁-C₄ alkylamino group, a straight or branched chain di C₁-C₄ alkylamino group, a haloalkoxy group, morpholino group, 1-piperazynyl group, 1-pyrrolidinyl group, phenyl group, or pyridyl group, and more preferably hydroxy group, a straight or branched chain C₁-C₄ alkoxy group or a straight or branched chain C₂-C₅ alkoxycarbonyl group.

The above mentioned alkoxy group includes methoxy group, ethoxy group, and propoxy group. The above mentioned alkylthio group includes methylthio group, ethylthio group and propylthio group. The above mentioned alkoxycarbonyl group includes methoxycarbonyl group and ethoxycarbonyl group. The above mentioned alkylsulfonyl group includes methanesulfonyl group and ethanesulfonyl group. The above mentioned haloalkyl group includes trifluoromethyl group. The above mentioned haloalkoxy group includes trifluoromethoxy group.

The adenine compound of the present invention includes all tautomers, geometrical isomers and stereoisomers which are formed in accordance with the kind of the substituent, and a mixture thereof.

Namely, in a case where there are one or more asymmetrical carbon atoms in the compound of the formula (1), there exist diastereomers and optical isomers, and mixtures of those diastereomers and optical isomers and separated ones are also included in the present invention.

Additionally, the adenine compound shown by the formula (1) and its tautomer is chemically equivalent, and the adenine compound of the present invention includes such a tautomer. The tautomer is specifically a hydroxy compound shown by the formula (1'): 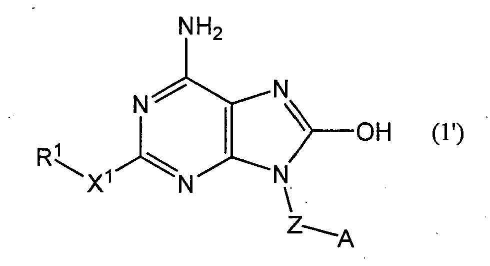 , wherein A, X¹, Z and R¹ are the same as define above.

The pharmaceutically acceptable salt is exemplified by an acid salt and a base addition salt. The acid salt is, for example, an inorganic acid salt such as hydrochloride, hydrobromide, sulfate, hydroiodide, nitrate and phosphate, and an organic acid salt such as citrate, oxalate, acetate, formate, propionate, benzoate, trifluoroacetate, maleate, tartrate, methanesulfonate, benzenesulfonate and p-toluenesulfonate, and the base salt is exemplified by an inorganic base salt such as sodium salt, potassium salt, calcium salt, magnesium salt and ammonium salt, and an organic base salt such as triethylammonium salt, triethanolammonium salt, pyridinium salt and diisopropylammonium salt, and further a basic or acidic amino acid salt such as arginine salt, aspartic acid salt and glutamic acid salt. The compound shown by the formula (1) may be hydrate and a solvate such as ethanolate.

The compound shown by the formula (1) can be prepared by the following methods. The starting compounds not disclosed below can be prepared by a similar method to the following method or by a known method or its similar method.

### Preparation method 1

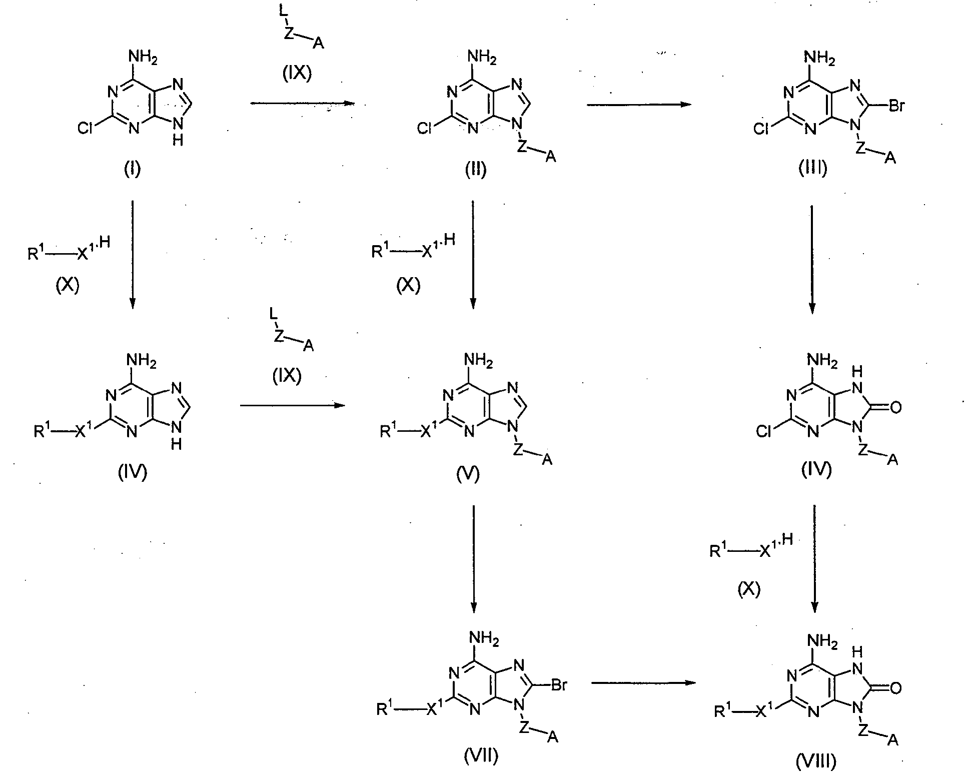

In the above formulas, L is a leaving group, A, R¹, X¹ and Z are same as defined above.

Compound (II) can be prepared by reacting compound (I) and compound (IX) in the presence of a base.

The base includes an alkali metal carbonate such as sodium carbonate or potassium carbonate, alkaline earth metal carbonate such as calcium carbonate, metal hydroxide such as sodium hydroxide, or potassium hydroxide, or a metal hydride such as sodium hydride, or a metal alkoxide such as potassium t-butoxide. The solvent includes a halogenated hydrocarbon such as carbon tetrachloride, chloroform or methylene chloride, an ether such as diethyl ether, tetrahydrofuran, or 1,4-dioxane, an aprotic solvent such as dimethylformamide, dimethyl sulfoxide, or acetonitrile. The reaction is carried out at about 0°C to the boiling point of the solvent.

Compound (III) can be prepared by brominating compound (II). The brominating agent includes bromine, hydroperbromic acid, or N-bromosuccimide. In this reaction a reaction auxiary such as sodium acetate may be added. Can be used the solvents such as a halogenated hydrocarbon, like carbon tetrachloride, ethylene chloride or dichloroethane, an ether like diethyl ether, acetic acid, or carbon disulfide. The reaction temperature is selected from a range of about 0°C to around the boiling point of the solvent.

Compound (VI) can be prepared by reacting compound (III) and a metal alkoxide such as sodium methoxide, followed by treating it under acidic conditions.

The solvents used in reacting with a metal alkoxide include an ether such as diethyl ether, tetrahydrofuran or 1,4-dioxane, an aprotic solvent such as dimethylformamide, or an alcohol such as methanol corresponding to a metal alkoxide used. The reaction temperature is selected from a range of room temperature to around the boiling point of the solvent.

The acid used in acid-treatment includes an inorganic acid such as hydrochloric acid, hydrobromic acid or sulfuric acid, or an organic acid such as trifluoroacetic acid. Can be used the solvent such as water or a mixture of water and an organic solvent. The organic solvent includes an ether such as diethyl ether or tetrahydrofuran, an aprotic solvent such as dimethylformamide or acetonitrile, or an alcohol such as methanol or ethanol. The reaction temperature is selected from a range of room temperature to around the boiling point of the solvent.

Compound (VIII) can be prepared by reacting compound (IV) and compound (X).

When X¹ is NR⁴, the reaction is carried out in the presence or absence of a base. Can be used the base such as an alkali metal carbonate, like sodium carbonate or potassium carbonate, an alkaline earth metal carbonate such as calcium carbonate, a metal hydroxide, sodium hydroxide or potassium hydroxide, or an organic base, like triethylamine, diisopropylethylamine or 4-dimethylaminopyridine. Can be used the solvent such as an ether, like tetrahydrofuran, 1,4-dioxane or diglyme, an alcohol, like propanol or butanol, or an aprotic solvent, like dimethylformamide. The reaction may be carried out in the absence of a solvent. The reaction temperature is selected from a range of about 50°C to 200°C.

When X¹ is oxygen atom or sulfur atom, the reaction is carried out in the presence of a base. Can be used the base such as an alkali metal, like sodium or potassium, an alkali metal hydride, like sodium hydride. Can be used the solvent such as an ether, like tetrahydrofuran, 1,4-dioxane or diglyme, or an aprotic solvent, like dimethylformamide or dimethyl sulfoxide. The reaction may be carried out in the absence of a solvent. The reaction temperature is selected from a range of about 50°C to 200°C.

The compound wherein X¹ is SO₂, can be obtained by oxidizing the intermediate compound wherein the corresponding X¹ is sulfur atom with Oxone or m-chloroperbenzoic acid (m-CPBA).

In the process of preparing the compound (VIII) from the compound (I), the compound (V) can also be synthesized from the compound (II) by the same method as above, or the compound (VIII) can also be obtained by synthesizing the compound (V) from the compound (I) through the compound (IV) and converting the resultant to the compound (VII).

### Preparation method 2

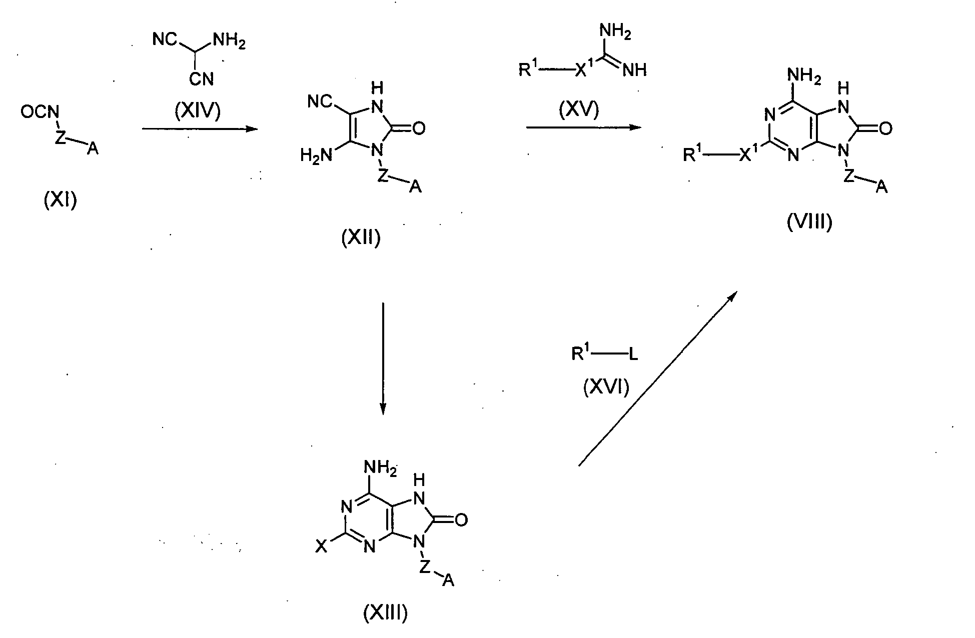

In the above formulas, L is a leaving group, A, R¹, X¹ and Z have the same meaning as above, and X is amino group, hydroxy group or mercapto group.

Compound (XII) can be obtained by reacting compound (XI) and compound (XIV) in the presence of a base.

Can be used the base such as an alkali metal carbonate, like sodium carbonate or potassium carbonate, an alkaline earth metal carbonate, like calcium carbonate, a metal hydroxide, like sodium hydroxide or potassium hydroxide, an organic base, like triethylamine, diisopropylethylamine, pyridine or 4-dimethylaminopyridine, or a metal alkoxide, like sodium methoxide. Can be used the solvent such as a halogenated hydrocarbon, like methylene chloride, an ether, like diethyl ether, tetrahydrofuran or 1,4-dioxane, an alcohol, like methanol or ethanol, or an aprotic solvent, like dimethylformamide, dimethyl sulfoxide or acetonitrile. The reaction temperature is selected from a range of about 0°C to around the boiling point of the solvent.

Compound (VIII) can be prepared by reacting compound (XII) and compound (XV) in the presence or absence of a base.

Can be used the base such as an inorganic base such as alkali metal carbonate, like sodium carbonate or potassium carbonate, an alkaline earth metal carbonate, like calcium carbonate, a metal hydroxide, like sodium hydroxide or potassium hydroxide, or an organic base such as a triethylamine, diisopropylethylamine, pyridine or 4-dimethylaminopyridine, or a metal alkoxide, like sodium methoxide. Can be used the solvent such as an ether, like tetrahydrofuran, 1,4-dioxane or diglyme, an alcohol, like methanol or ethanol, or an aprotic solvent, like toluene, dimethylformamide or dimethyl sulfoxide. The reaction may be carried out in the absence of a solvent. The reaction temperature is selected from a range of room temperature to around the boiling point of the solvent.

In the process of preparing compound (VIII) from compound (XII), compound (VIII) is also obtainable after preparing compound (XIII).

When X is amino group, compound (XIII) can be prepared by reacting compound (XII) with guanidine in the presence or absence of a base.

Can be used the base such as an alkali metal carbonate, like sodium carbonate or potassium carbonate, an alkaline earth metal carbonate, like calcium carbonate, a metal hydroxide, like sodium hydroxide or potassium hydroxide, an organic base, like triethylamine, diisopropylethylamine, pyridine or 4-dimethylaminopyridin, or a metal alkoxide, like sodium methoxide. Can be used the solvent such as an ether, like tetrahydrofuran, 1,4-dioxane or diglyme, an alcohol, like methanol or ethanol, or an aprotic solvent, like toluene, dimethylformamide or dimethyl sulfoxide. The reaction may be carried out in the absence of a solvent. The reaction temperature is selected from a range of room temperature to around the boiling point of the solvent.

When X is hydroxy group, compound (XIII) can be prepared by reacting compound (XII) and urea in the presence or absence of a base. Can be used the base such as an alkali metal carbonate, like sodium carbonate or potassium carbonate, an alkaline earth metal carbonate, like calcium carbonate, a metal hydroxide, like sodium hydroxide or potassium hydroxide, an organic base, like triethylamine, diisopropylethylamine, pyridine or 4-dimethylaminopyridine, or a metal alkoxide, like sodium methoxide. Can be used the solvent such as an ether, like tetrahydrofuran, 1,4-dioxane or diglyme, an alcohol, like methanol or ethanol, or an aprotic solvent, like toluene, dimethylformamide or dimethyl sulfoxide. The reaction may be carried out in the absence of a solvent. The reaction temperature is selected from a range of room temperature to around the boiling point of the solvent.

When X is mercapto group, compound (XIII) can be prepared by reacting compound (XII) and benzoylisothiocyanate in the presence or absence of a base, followed by cyclization.

The base used in reaction with benzoylisothiocyanate includes an alkali metal carbonate such as sodium carbonate or potassium carbonate, an alkaline earth metal carbonate such as calcium carbonate, or an organic base such as triethylamine, diisopropylethylamine, pyridine or 4-dimethylaminopyridine. Can be used the solvent such as a halogenated hydrocarbon, like methylene chloride, an ether, like tetrahydrofuran or 1,4-dioxane or an aprotic solvent, like dimethylformamide or dimethyl sulfoxide. The reaction temperature is selected from a range of about 0°C to around the boiling point of the solvent.

The solvent used in the cyclization reaction includes a metal hydroxide such as sodium hydroxide or potassium hydroxide, a metal alkoxide such as sodium methoxide or potassium t-butoxide. Can be used the solvent such as an ether, like tetrahydrofuran, an alcohol, like ethanol or 2-propanol, or an aprotic solvent, like dimethylformamide or dimethyl sulfoxide. The reaction temperature is selected from a range of room temperature to around the boiling point of the solvent.

Compound (VIII) can be prepared by reacting compound (XIII) and compound (XVI) in the presence of a base. Can be used the base such as an alkali metal hydrogencarbonate, like sodium hydrogencarbonate, an alkali metal carbonate, like sodium carbonate or potassium carbonate, an alkaline earth metal carbonate, like calcium carbonate, a metal hydroxide, like sodium hydroxide or potassium hydroxide, a metal hydride, like sodium hydride, a organic base, like triethylamine, diisopropylethylamine, pyridine, or 4-dimethylaminopyridine, or a metal alkoxide, like potassium t-butoxide. Can be used the solvent such as a halogenated hydrocarbon, like carbon tetrachloride, chloroform or methylene chloride, an ether, like diethyl ether, tetrahydrofuran or 1,4-dioxane, or an aprotic solvent, like dimethylformamide, dimethyl sulfoxide or acetonitrile. The reaction temperature is selected from a range of about 0°C to around the boiling point of the solvent.

The compounds represented by the formulas (XI), (X), (XIV), (XV) or (XVI), which are intermediates of the adenine compound of the present invention are known compounds or can be prepared by conventional methods for the skilled person in the art.

In a case where the compound of the present invention or its intermediate or the starting compound contains a functional group, a reaction for increasing a carbon atom, a reaction for introducing a substituent or a reaction for conversion of the functional group can be conducted optionally according to a manner conventional to the skilled artisan in an appropriate step, namely in an intermittent step in each of the preparation methods described in the preparation method 1 or 2. For this purpose, the methods described in "JIKKEN KAGAKU-KOZA (edited by NIHON KAGAKU-KAI, MARUZEN)", or "Comprehensive Organic Transformation, R. C. Lalock (VCH Publishers, Inc. 1989)" can be used. The reaction for increasing a carbon atom includes a method comprising converting an ester group to hydroxymethyl group using a reducing agent such as aluminum lithium hydride, introducing a leaving group and then introducing a cyano group. The reacting for conversion of a functional group includes a reaction for conducting acylation or sulfonylation using an acid halide, a sulfonyl halide, etc., a reaction for reacting an alkylation agent such as a halogenated alkyl, a hydrolysis reaction, a reaction for C-C bond formation such as Friedel-Crafts reaction and Wittig reaction, and oxidizing or reducing reaction, etc.

In a case where the compound of the present invention or its intermediate contains a functional group such as amino group, carboxy group, hydroxy group and oxo group, a technology of protection and deprotection can optionally be used. A preferable protecting group, a protection method and a deprotection method are described in details in "Protective Groups in Organic Synthesis 2nd Edition (John Wiley & Sons, Inc.; 1990)", etc.

The compound (1) of the present invention and the intermediate compound for production thereof can be purified by a method known to the skilled artisan. For instance, purification can be conducted by column chromatography (e.g. silica gel column chromatography or ion exchange chromatography) or recrystallization. As the recrystallization solvent, for instance, can be used an alcohol such as methanol, ethanol and 2-propanol, an ether such as diethyl ether, an ester such as ethyl acetate, an aromatic hydrocarbon such as benzene and toluene, a ketone such as acetone, a hydrocarbon such as hexane, an aprotic solvent such as dimethylformamide and acetonitrile, water and a mixture of two or more thereof. As other purification method, can be used those described in "JIKKEN KAGAKU-KOZA (edited by NIHON KAGAKU-KAI, MARUZEN) Vol. 1", etc.

In a case where the compound of the formula (1) of the present invention contains one or more asymmetric carbon, its production can be conducted by using the starting material containing those asymmetric carbon or by introducing the asymmetric carbon during the production steps. For instance, in a case of an optical isomer, the object can be obtained by using an optically active starting material or by conducting an optical resolution at a suitable stage of the production steps. The optical resolution method can be conducted by a diastereomer method comprising allowing the compound of the formula (1) or its intermediate to form a salt with an optically active acid (e.g. a monocarboxylic acid such as mandelic acid, N-benzyloxyalanine and lactic acid, a dicarboxylic acid such as tartaric acid, o-diisopropylidene tartrate and malic acid, a sulfonic acid such as camphor sulfonic acid and bromocamphor sulfonic acid) in an inert solvent (e.g. an alcohol such as methanol, ethanol, and 2-propanol, an ether such as diethyl ether, an ester such as ethyl acetate, a hydrocarbon such as toluene, an aprotic solvent such as acetonitrile and a mixture of two or more thereof).

In a case where the compound of the formula (1) or its intermediate contains a functional group such as carboxylic group, the object can be attained also by forming a salt with an optically active amine (e.g. an organic amine such as α-phenethylamine, quinine, quinidine, cinchonidine, cinchonine and strychnine).

The temperature for formation of the salt is selected from room temperature to the boiling point of the solvent. In order to increase optical purity, the temperature is preferably once increased up to the boiling point of the solvent. Upon recovering the salt formed by filtration, the yield can be increased optionally by cooling. An amount of the optical active acid or amine is about 0.5 to about 2.0 equivalent, preferably around 1 equivalent, relative to the substrate. An optically active salt with highly optical purity can be obtained optionally by recrystallization from an inert solvent (e.g. an alcohol such as methanol, ethanol and 2-propanol, an ether such as diethyl ether, an ester such as ethyl acetate, a hydrocarbon such as toluene, an aprotic solvent such as acetonitrile and a mixture of two or more thereof). If necessary, the optically resoluted salt can be converted into a free form by treating with an acid or a base by the conventional method.

The 8-oxoadenine compound and its pharmaceutically acceptable salt of the present invention is useful as an immuno-modulator and thus useful as a therapeutic and prophylactic agent for diseases associated with an abnormal immune response (e.g. autoimmune diseases and allergic diseases) and various infections and cancers which are required for activation of an immune response. For instance, the 8-oxoadenine compound and its pharmaceutically acceptable salt is useful as a therapeutic and prophylactic agent for the diseases mentioned in the following (1)-(8).
(1) Respiratory diseases: asthma, including bronchial, allergic, intrinsic, extrinsic, exercise-induced, drug-induced (including NSAID such as aspirin and indomethacin) and dust-induced asthma; intermittent and persistent and of all severities, and other causes of airway hyperresponsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus.
(2) (Skin) psoriasis, atopic dermatitis, contact dermatitis or other eczematous dermatoses, and delayed-type hypersensitivity reactions; phyto-and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis; cutaneous lymphomas, non-melanoma skin cancer and other dysplastic lesions; drug-induced disorders including fixed drug eruptions.
(3) (Eyes) blepharitis; conjunctivitis including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral, fungal, and bacterial.
(4) (Genitourinary) nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female).
(5) (Allograft rejection) acute and chronic following, for example, transplantation of kidney, heart, liver, lung, bone marrow, skin or cornea or following blood transfusion; or chronic graft versus host disease.
(6) Other auto-immune arid allergic disorders including rheumatoid arthritis, irritable bowel syndrome, systemic lupus erythematosus, multiple sclerosis, Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome, Sazary syndrome.
(7) (Oncology) treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumor recurrences, and paraneoplastic syndromes.
(8) (Infectious diseases): virus diseases such as genital warts, common warts, plantar warts, hepatitis B, hepatitis C, herpes simplex virus, molluscum contagiosum, variola, HIV, CMV, VZV, rhinovirus, adenovirus, coronavirus, influenza, para-influenza; bacterial diseases such as tuberculosis and mycobacterium avium, leprosy; other infectious diseases, such as fungal diseases, chlamydia, candida, aspergillus, cryptococcal meningitis, pneumocystis carnii, cryptosporidiosis, histoplasmosis, toxoplasmosis, trypanosome infection, leishmaniasis.

The 8-oxoadenine compounds or pharmaceutically acceptable salt thereof can also be used as vaccine adjuvant.

The 8-oxoadenine compound of the present invention, or its pharmaceutically acceptable salt shows an interferon inducing activity and/or a suppressing activity of the production of IL-4 and IL-5, and thus shows an effect as a medicament having an immunomodulating activity specific against type 1 helper T-cell (Th1 cell)/type 2 helper T-cell (Th2 cell), namely, preferably useful as a prophylactic or therapeutic agent for asthma caused by Th2 cell, and allergic diseases such as allergic rhinitis, allergic conjunctivitis and atopic dermatosis. Additionally, due to its an immuno activating activity such as interferon α and interferon y inducing activity, it is useful as a prophylactic or therapeutic agent for cancer, a viral disease caused by infection with virus such as hepatitis B virus, hepatitis C virus, HIV and human papilloma virus (HPV), infections by bacteria and dermatosis such proriasis.

The compound of the present invention has no limitation as to its administration formulation and is administered orally or parenterally.
The preparation for oral administration can be exemplified by capsules, powders, tablets, granules, fine-grain, syrups, solutions, suspensions, etc., and the preparation for parenteral administration can be exemplified by injections, drips, eye-drops, intrarectal preparations, inhalations, sprays (e.g. sprays, aerosols, liquids/susperisions for cartridge spray for inhalators or insufflators), lotions, gels, ointments, creams, transdermal preparations, transmucosa preparations, collunariums, ear drops, tapes, transdermal patches, cataplasms, powders for external application, and the like. Those preparations can be prepared by so-far known manners, and acceptable conventional carriers, fillers, binders, lubricants, stabilizers, disintegrants, buffering agents, solubilizing agents, isotonic agents, surfactants, antiseptics, perfumes, and so on can be used. Two or more pharmaceutical carriers can be appropriately used.

The liquid preparation such as emulsions and syrups, among the preparations for oral administration, can be prepared by using additives including water; a sugar such as sucrose, sorbitol and fructose; a glycol such as polyethylene glycol and propylene glycol; an oil such as sesame oil, olive oil and soybean oil; an antiseptic such as p-hydroxybenzoate; a flavor such as strawberry flavor and peppermint flavor.

The solid preparation such as capsules, tablets, powders and granules can be prepared by using a filler such as lactose, glucose, sucrose and mannitol; a disintegrant such as starch and sodium alginate; a lubricant such as magnesium stearate and talc; a binder such as polyvinyl alcohol, hydroxypropyl cellulose and gelatin; a surfactant such as a fatty acid ester; a plasticizer such as glycerin.

The liquid preparation such as injections, drips, eye-drops and ear drops, among the preparations for parenteral administration, can be prepared preferably as a sterilized isotonic liquid preparation. For instance, injections can be prepared by using an aqueous medium such as a salt solution, a glucose solution or a mixture of a salt solution and a glucose solution. The preparation for intrarectal administration can be prepared by using a carrier such as cacao butter usually in the form of suppository.

The ointments, creams and gels contain the compound of the present invention usually in an amount of 0.01-10 w/w%, and there may be incorporated a thickener suitable to an aqueous or oily base and/or a gelling agent and/or a solvent. The base is exemplified by water and/or an oil such as liquid paraffin, a vegetable oil such as arachis oil and castor oil, a solvent such as polyethylene glycol, and so on. The thickener and gelling agent are exemplified by soft paraffin, aluminum stearate, cetostearic alcohol, polyethylene glycol, sheep fat, beeswax, carboxypolymethylene and cellulose derivatives and/or glyceryl monostearate and/or nonionic emulsifiers.

The lotions contain the compound of the present invention usually in an amount of 0.01-10 w/w%, and it may be prepared with the use of an aqueous or oily base, it may contain generally emulsifiers, stabilizers, dispersing agents, precipitation inhibitors and also thickeners.

Powders for external use contain the compound of the present invention usually an amount of 0.01-10 w/w%, and it may be formulated using a suitable powdery base such as talc, lactose and starch.

The drips may be formulated by using an aqueous or non-aqueous base, and may contain dispersing agents, solubilizing agents, precipitation inhibitors or antiseptics.

The sprays may be formulated into an aqueous solution or suspension using a suitable liquid propellant, or into an aerosol distributed from a pressured package such as a metered-dose inhaler.

The aerosols suitable to inhalation may be a suspension or aqueous solution, and they contain generally the compound of the present invention and a suitable propellant such as fluorocarbon, hydrogencontaining chlorofluorocarbon and a mixture thereof, particularly hydrofluoroalkane, specifically 1,1,1,2-tetrafluoroethane, 1,1,1,2,3,3,3-heptafluoro-n-propane or a mixture thereof. The aerosols may contain optionally additional excipients well known in the art such as a surfactant, (e.g., oleic acid or lecithin) and a co-solvent such as ethanol.

The gelatin capsules or cartridges used for inhalator or insufflator may be formulated by using a powdery mixture of the compounds used in the present invention and a powdery base such as lactose and starch. They contain the compound of the present invention usually in an amount of 20µg- 10mg: The compound of the present invention may be administered without using excipients such as lactose as an alternative method.

The 8-oxoadenine compound of the present invention is preferably parenterally administered as a preparation for topical administration. The suitable preparation is exemplified by ointments, lotions, creams, gels, tapes, transdermal patches, cataplasms, sprays, aerosols, aqueous solutions/suspensions for cartridge spray for inhalators or insufflators, eye-drops, ear drops, nasal drops, powders for external administrations and so on.

A ratio of the active compound of the present invention in the preparation for topical administration of the present invention is, though depending upon the formulation, generally 0.001-10 wt%, preferably 0.005-1%. The ratio used in powders for inhalation or insufflation is 0.1-5%.

In a case of aerosols, the compound of the present invention is preferably contained in an amount of 20-2000µg, more preferably about 20µg-500µg per each a measured amount or one sprayed amount. The dosage is once or several times per day, for instance, 2, 3, 4 or 8 times, and one to three units are administered per each time.

The 8-oxoadenine compound of the present invention, preferably the compound (1) wherein R² is except hydrogen atom, its tautomer or its pharmaceutically acceptable salt can show the pharmacological activity at the site administered in a case of topical administration, and further they are useful as a pharmaceutical preparation for topical administration characterized by showing no systemic pharmacological activity because the compounds are converted by an enzyme in vivo into different compounds (degraded compounds) having only a substantially reduced medical effect. The medical effect used here means a pharmacological activity of the compound, including specifically an interferon inducing activity, and a suppressing activity of the production IL-4 and/or IL-5.

The medical effect of the degraded compound is preferably 10 times, more preferably 100 times, still more preferably 1000 times reduced comparing with that of the parent compound.

The pharmacological activity can be measured by any of conventional evaluation methods, preferably by an in vitro evaluation method. Specific examples of the methods are one described in Method in ENZYMOLOGY (Academic Press), a method using commercially available ELISA kits (e.g. AN'ALYSA (immunoassay System)) and a method described in examples of the present specification.

For instance, by measuring interferon inducing activity with bioassay using cells of mouse spleen, the amount of each interferon induction (IU/ml at the same concentration of the parent compound (the compound of the present invention) and the degraded compound can be compared.

As the pharmacological activity, the activity in vivo caused by interferon inducing activity, etc. is illustrated. Said activity in vivo includes immune activating activity, influenza-like symptom, etc. The immune activating activity includes induction of cytotoxic activity such as natural killer (NK) cells, etc. The influenza-like symptom includes fever, etc. The fever means elevation in body temperature of a mammalian, for example, in a case of human, the fever means that the body temperature increases more than normal temperature.

The topical administration is not limited as to the administration method, and the administration is conducted in a case of administration via nasal cavity, alveolus or air way, by aeration or inhalation, in a case of administration to skin, by spreading on the skins, and in a case of administration to eye, by eye dropping, etc. Preferable administration is aeration and inhalation.

It can be confirmed that when the pharmaceutical composition for topical administration of the present invention is administered topically, the compound of the present invention therein is converted to a degraded compound in the blood, etc. in human or animal for example, by its half life in the serum or in lever S9 in vitro. The test method to determine the half life of the compound of the present invention in vitro is known.

In the vitro measuring test, the compound of the present invention is metabolized in liver S9 and its half life is preferably not longer than 60 minutes, more preferably not longer than 30 minutes, and still more preferably not longer than 10 minutes.

Further, the compound of the present invention is metabolized in serum, and its half life is preferably not longer than 60 minutes, more preferably not longer than 30 minutes, and still more preferably not longer than 10 minutes.

As the degraded compound, there is exemplified the compound having a carboxy group obtained by hydrolysing the compound having an ester group in the formula (1).

The method for measuring the half life in liver S9 is as follows. Namely, the compound of the present invention is added to a liver S9 solution and incubated at 37±0.5°C for 5 minutes to 2 hours. By quantitative analyzing at the definite interval the amount of the compound of the present invention remaining in the lever S9 solution with HPLC (high performance liquid chromatograph), etc., the constant of quenching velocity calculated and the half life is calculated. The specific method is described in the Example.

The liver S9 solution used here means product obtained by homogenizing a liver of a mammal in an aqueous solution such as a physiological saline solution, a sucrose solution and a KC1 solution and then by recovering the supernatant upon centrifugation at 9000 xg. The aqueous solution is used usually in an amount of 2 to 4 times as much as the liver. The mammal includes human, dog, rabbit, guinea pig, mouse and rat. The liver S9 can be used optionally after dilution with a buffering solution.

The measuring method for the half life in serum of the present invention is as follows. Namely, the compound of the present invention is a serum solution and incubated at 37±0.5°C for 5 minutes to 2 hours. By quantitative analyzing at the definite interval the amount of the compound of the present invention remaining in the serum solution with HPLC (high performance liquid chromatograph), etc., the constant of quenching velocity calculated and the half life is calculated.

The serum used here means a supernatant fraction obtained by leaving hemocytes and blood coagulation factor from blood by centrifugation, etc. and it may be used after dilution with a buffering solution.

The invention further relates to combination therapies wherein a compound of formula (1) or a pharmaceutically acceptable salt or a pharmaceutical composition or formulation comprising a compound of formula (1) is administered concurrently or sequentially or as a combined preparation with another therapeutic agent or agents, for the treatment of one or more of the conditions listed.

In particular, for the treatment of the inflammatory diseases, COPD, asthma and allergic rhinitis, the compounds of the invention may be combined with agents such as tumour necrosis factor alpha (TNF-a) inhibitors such as anti-TNF monoclonal antibodies (for example Remicade, CDP-870 and adalimumab) and TNF receptor immunoglobulin molecules (such as Enbrel); non-selective cyclo-oxygenase (COX)-1 / COX-2 inhibitors whether applied topically or systemically (such as piroxicam, diclofenac, propionic acids such as naproxen, flubiprofen, fenoprofen, ketoprofen and ibuprofen, fenamates such as mefenamic acid, indomethacin, sulindac, azapropazone, pyrazolones such as phenylbutazone, salicylates such as aspirin), COX-2 inhibitors (such as meloxicam, celecoxib, rofecoxib, valdecoxib, lumarocoxib, parecoxib and etoricoxib); glucocorticosteroids (whether administered by topical, oral, intramuscular, intravenous, or intra-articular routes); methotrexate, lefunomide; hydroxychloroquine, d-penicillamine, auranofin or other parenteral or oral gold preparations.

The present invention still further relates to combination therapies of a compound of the invention together with a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; the compound SB-210661; pyridinyl-substituted 2-cyanonaphthalene compounds such as L-739,010; 2-cyanoquinoline compounds such as L-746,530; indole and quinoline compounds such as MK-591, MK-886, and BAY x 1005.

The present invention still further relates to combination therapies of a compound of the invention together with a receptor antagonist for leukotrienes (LT)B4, LTC4, LTD4 and LTE4 selected from the group consisting of phenothiazin compound such as L-651,392; amidino compounds such as CGS-25019; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to combination therapies of a compound of the invention together with a phosphodiesterase (PDE) inhibitor such as the methylxanthanines including theophylline and aminophylline; and selective PDE isoenzyme inhibitors including PDE4 inhibitors and inhibitors of isoform PDE4D, and inhibitors of PDE5.

The present invention still further relates to combination therapies of a compound of the invention together with histamine type 1 receptor antagonists such as cetirizine, loratadine, desloratadine, fexofenadine, acrivastine, terfenadine, astemizole, azelastine, levocabastine, chlorpheniramine, promethazine, cyclizine, and mizolastine, which is applied orally, topically or parenterally.

The present invention still further relates to combination therapies of a compound of the invention together with a gastroprotective histamine type 2 receptor antagonist.

The present invention still further relates to combination therapies of a compound of the invention with antagonists of the histamine type 4 receptor.

The present invention still further relates to combination therapies of a compound of the invention together with an alpha-1/alpha-2 adrenoceptor agonist, vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, and ethylnorepinephrine hydrochloride.

The present invention still further relates to combination therapies of a compound of the invention together with anticholinergic agents including muscarinic receptor (M1, M2 and M3) antagonists such as atropine, hyoscine, glycopyrrolate, ipratropium bromide; tiotropium bromide; oxitropium bromide; pirenzepine; and telenzepine.

The present invention still further relates to combination therapies of a compound of the invention together with a beta-adrenoceptor agonist (including beta receptor subtypes 1-4) such as isoprenaline, salbutamol, formoterol, salmeterol, terbutaline, orciprenaline, bitolterol mesylate, and pirbuterol.

The present invention still further relates to combination therapies of a compound of the invention together with a chromone, including sodium cromoglycate and nedocromil sodium.

The present invention still further relates to combination therapies of a compound of the invention together with an insulin-like growth factor type I (IGF-1) mimetic.

The present invention still further relates to combination therapies of a compound of the invention together with an inhaled glucocorticoid, such as flunisolide, triamcinolone acetonide, beclomethasone dipropionate, budesonide, fluticasone propionate, ciclesonide, and mometasone furoate.

The present invention still further relates to combination therapies of a compound of the invention together with an inhibitor of matrix metalloproteases (MMPs), i.e., stromelysin, collagenase, gelatinase, aggrecanase; especially collagenase-1 (MMP-1), collagenase-2 (MMP-8), collagenase-3 (MMP-13), stromelysin-1 (MMP-3), stromelysin-2 (MMP-10), stromelysin-3 (MMP-11), MMP-9 and MMP-12.

The present invention still further relates to combination therapies of a compound of the invention together with modulators of chemokine receptor function such as antagonists of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX3CR1 (for the C-X3-C family).

The present invention still further relates to combination therapies of a compound of the invention together with a cytokine or a modulator of cytokine function including agents which act on cytokine signalling pathways, such as alpha-, beta-, and gamma-interferon; interleukins (IL) including IL-1 to IL-15, and interleukin antagonists or inhibitors.

The present invention still further relates to combination therapies of a compound of the invention together with an immunoglobulin (Ig), an Ig preparation, or an antagonist or antibody modulating Ig function such as anti-IgE (omalizumab).

The present invention still further relates to combination therapies of a compound of the invention together with thalidomide and derivatives, or systemic or topically-applied anti-inflammatory agents such as retinoids, dithranol, and calcipotriol.

The present invention still further relates to combination therapies of a compound of the invention together with an antibacterial agent including penicillin derivatives, tetracyclines, macrolides, beta-lactams, flouroquinolones, and inhaled aminoglycosides; and antiviral agents including acyclovir, famciclovir, valaciclovir, ganciclovir, cidofovir, amantadine, rimantadine, ribavirin; zanamavir and oseltamavir; protease inhibitors such as indinavir, nelfinavir, ritonavir, and saquinavir; nucleoside reverse transcriptase inhibitors such as didanosine, lamivudine, stavudine, zalcitabine and zidovudine; non-nucleoside reverse transcriptase inhibitors such as nevirapine and efavirenz.

The present invention still further relates to combination therapies of a compound of the invention together with agents used for treatment of cancer. Suitable agents to be used in the combination therapies include:
(i) antiproliferative/antineoplastic drugs and combinations thereof, which are used as an anticancer agent, such as alkylating agents (for example cis platin, carboplatin, cyclophosphamide, nitrogen mustard, melphalan, chlorambucil, busulphan and nitrosoureas); antimetabolites (for example fluoropyrimidines, like 5-fluorouracil and tegafur, antifolates such as raltitrexed, methotrexate, cytosine arabinoside, hydroxyurea, gemcitabine and paclitaxel; antitumour antibiotics (for example anthracyclines, like adriamycin, bleomycin, doxorubicin, daunomycin, epirubicin, idarubicin, mitomycin-C, dactinomycin and mithramycin); antimitotic agents (for example vinca alkaloids, like vincristine, vinblastine, vindesine and vinorelbine and taxoids, like taxol and taxotere); and topoisomerase inhibitors (for example epipodophyllotoxins, like etoposide and teniposide, amsacrine, topotecan and camptothecins);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors, like marimastat and inhibitors of urokinase plasminogen activator receptor function);
(iv) inhibitors of growth factor function, for example such inhibitors include growth factor antibodies, growth factor receptor antibodies (for example the anti erbb2 antibody trastuzumab and the anti erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI 774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti vascular endothelial cell growth factor antibody bevacizumab, compounds disclosed in WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as combretastatin A4 and compounds disclosed in WO 99/02166, WO00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene directed enzyme pro drug therapy) approaches such as those using cytosine deaminase, thymidine kinase or a bacterial nitroreductase enzyme and approaches to increase patient tolerance to chemotherapy or radiotherapy such as multi drug resistance gene therapy; and
(ix) immunotherapy approaches, including for example ex vivo and in vivo approaches to increase the immunogenicity of patient tumour cells, such as transfection with cytokines such as interleukin 2, interleukin 4 or granulocyte macrophage colony stimulating factor, approaches to decrease T cell anergy, approaches using transfected immune cells such as cytokine transfected dendritic cells, approaches using cytokine transfected tumour cell lines and approaches using anti idiotypic antibodies.

The compounds of the present invention are illustrated in the following Tables 2 to 56, but should not be limited to these compounds. In these Tables, the compounds of the present invention are shown in a form of 8-hydroxy type for convenience and it is not different from 8-oxo type.

**Table 2**

| | | | | |
|---|---|---|---|---|
| 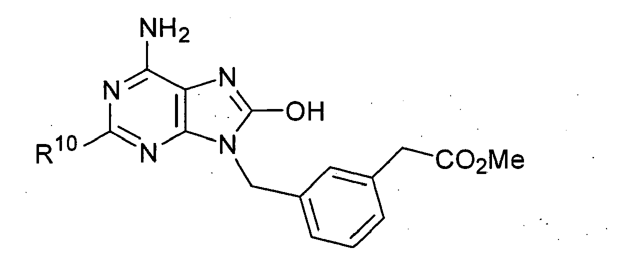 | | | | |

| -R¹⁰ | | -R¹⁰ | | -R¹⁰ |
|---|---|---|---|---|
| -O(CH₂)₅OH | | -O(CH₂)₂NMe₂ | | -NH(CH₂)₃OMe |
| -O(CH₂)₂OPr | | -O(CH₂)₃NMe₂ | | -NH(CH₂)₂SMe |
| -O(CH₂)₃OMe | | -O(CH₂)₂CF₂CF₃ | | -NH(CH₂)₃SMe |
| -O(CH₂)₃OEt | | -S(CH₂)₃OMe | | -NH(CH₂)₃CF₃ |
| -O(CH₂)₄OMe | | -S(CH₂)₂SMe | | -NMe(CH₂)₃OMe |
| -O(CH₂)₂SMe | | -S(CH₂)₃SMe | | -NMe(CH₂)₃CH₃ |
| -O(CH₂)₂SEt | | -S(CH₂)₃Me | | -CH₂COOMe |
| -O(CH₂)₃SMe | | -S(CH₂)₃CF₃ | | -CF₃ |
| -O(CH₂)₂SO₂Me | | -NH(CH₂)₂OH | | |
| -O(CH₂)₂SO₂Et | | -NH(CH₂)₃OH | | |
| -O(CH₂)₃SO₂Me | | -NH(CH₂)₄OH | | |

**Table 3**

| | | |
|---|---|---|
| 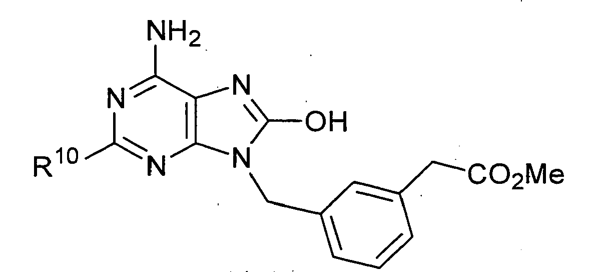 | | |

| -R¹⁰ | | -R¹⁰ |
|---|---|---|
| 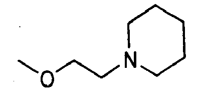 | | 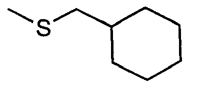 |
| 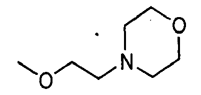 | | 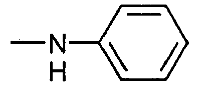 |
| 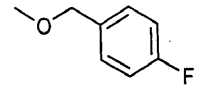 | | 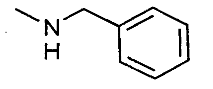 |
| 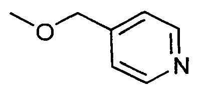 | | 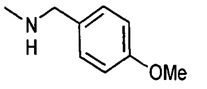 |
| 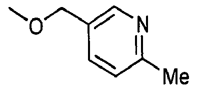 | | 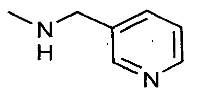 |
| 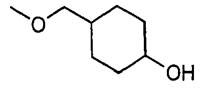 | | 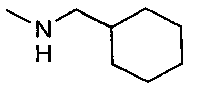 |
| 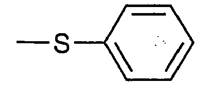 | | 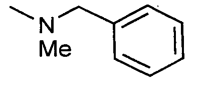 |
| 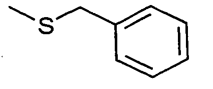 | | |

**Table 4**

| | | | | |
|---|---|---|---|---|
| 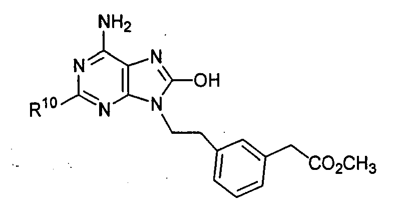 | | | | |

| -R¹⁰ | | -R¹⁰ | | -R¹⁰ |
|---|---|---|---|---|
| -O(CH₂)₂OH | | -O(CH₂)₂SO₂Et | | -S(CH₂)₃CF₃ |
| -O(CH₂)₃OH | | -O(CH₂)₃SO₂Me | | -NH(CH₂)₂OH |
| -O(CH₂)₄OH | | -O(CH₂)₂NMe₂ | | -NH(CH₂)₃OH |
| -O(CH₂)₅OH | | -O(CH₂)₃NMe₂ | | -NH(CH₂)₄OH |
| -O(CH₂)₂OMe | | -O(CH₂)₃CF₃ | | -N(CH₂)₂OMe |
| -O(CH₂)₂OEt | | -O(CH₂)₂CF₂CF₃ | | -NH(CH₂)₃OMe |
| -O(CH₂)₂OPr | | -S(CH₂)₂OH | | -NH(CH₂)₂SMe |
| -O(CH₂)₃OMe | | -S(CH₂)₃OH | | -NH(CH₂)₃SMe |
| -O(CH₂)₃OEt | | -S(CH₂)₄OH | | -NH(CH₂)₃Me |
| -O(CH₂)₄OMe | | -S(CH₂)₂OMe | | -NH(CH₂)₃CF₃ |
| -O(CH₂)₂SMe | | -S(CH₂)₃OMe | | -NMe(CH₂)₃OMe |
| -O(CH₂)₂SEt | | -S(CH₂)₂SMe | | -NMe(CH₂)₃CH₃ |
| -O(CH₂)₃SMe | | -S(CH₂)₃SMe | | -CH₂COOMe |
| -O(CH₂)₂SO₂Me | | -S(CH₂)3Me | | -(CH₂)₂COOMe |
| | | | | -CF₃ |

**Table 5**

| | | |
|---|---|---|
| 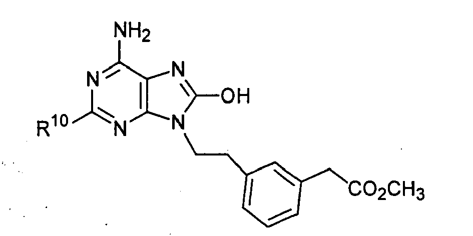 | | |

| -R¹⁰ | | -R¹⁰ |
|---|---|---|
| 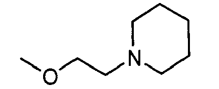 | | 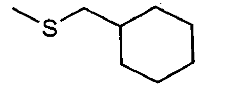 |
| 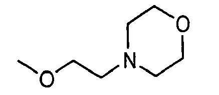 | | 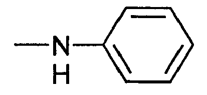 |
| 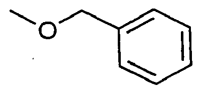 | | 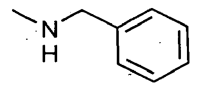 |
| 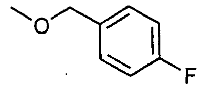 | | 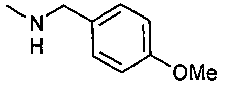 |
| 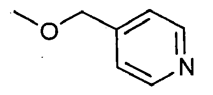 | | 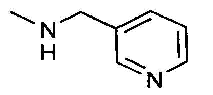 |
| 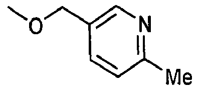 | | 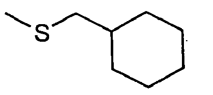 |
| 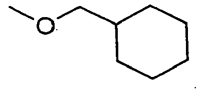 | | 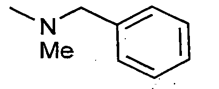 |
| 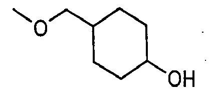 | | 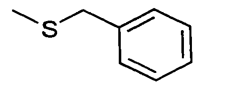 |
| 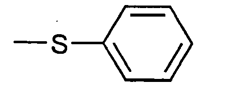 | | |

**Table 6**

| | | | | |
|---|---|---|---|---|
| 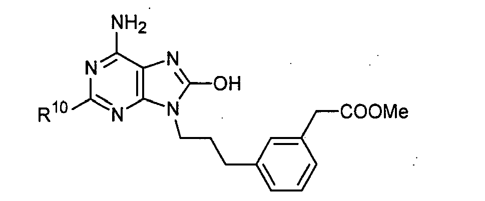 | | | | |

| -R¹⁰ | | -R¹⁰ | | -R¹⁰ |
|---|---|---|---|---|
| -O(CH₂)₂OH | | -O(CH₂)₂SO₂Et | | -S(CH₂)₃CF₃ |
| -O(CH₂)₃OH | | -O(CH₂)₃SO₂Me | | -NH(CH₂)₂OH |
| -O(CH₂)₄OH | | -O(CH₂)₂NMe₂ | | -NH(CH₂)₃OH |
| -O(CH₂)₅OH | | -O(CH₂)₃NMe₂ | | -NH(CH₂)₄OH |
| -O(CH₂)₂OMe | | -O(CH₂)₃CF₃ | | -N(CH₂)₂OMe |
| -O(CH₂)₂OEt | | -O(CH₂)₂CF₂CF₃ | | -NH(CH₂)₃OMe |
| -O(CH₂)₂OPr | | -S(CH₂)₂OH | | -NH(CH₂)₂SMe |
| -O(CH₂)₃OMe | | -S(CH₂)₃OH | | -NH(CH₂)₃SMe |
| -O(CH₂)₃OEt | | -S(CH₂)₄OH | | -NH(CH₂)₃Me |
| -O(CH₂)₄OMe | | -S(CH₂)₂OMe | | -NH(CH₂)₃CF₃ |
| -O(CH₂)₂SMe | | -S(CH₂)₃OMe | | -NMe(CH₂)₃OMe |
| -O(CH₂)₂SEt | | -S(CH₂)₂SMe | | -NMe(CH₂)₃CH₃ |
| -O(CH₂)₃SMe | | -S(CH₂)₃SMe | | -CH₂COOMe |
| -O(CH₂)₂SO₂Me | | -S(CH₂)₃Me | | -(CH₂)₂COOMe |
| | | | | -CF₃ |

**Table 7**

| | | |
|---|---|---|
| 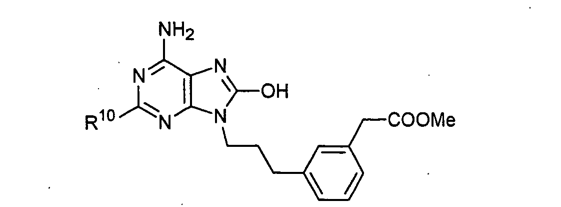 | | |

| -R¹⁰ | | -R¹⁰ |
|---|---|---|
| 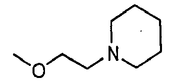 | | 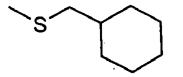 |
| 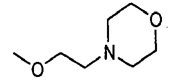 | | 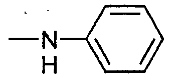 |
| 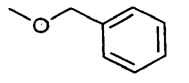 | | 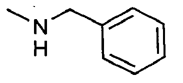 |
| 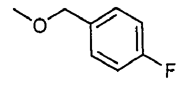 | | 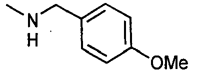 |
| 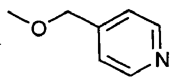 | | 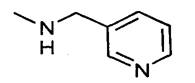 |
| 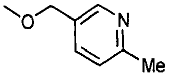 | | 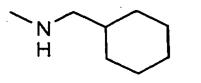 |
| 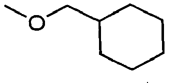 | | 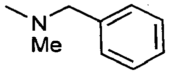 |
| 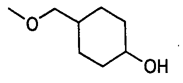 | | 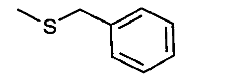 |
| 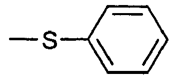 | | |

**Table 8**

| | | | | |
|---|---|---|---|---|
| 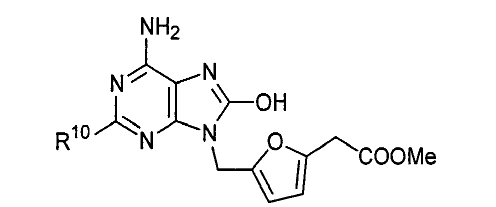 | | | | |

| -R¹⁰ | | -R¹⁰ | | -R¹⁰ |
|---|---|---|---|---|
| -O(CH₂)2OH | | -O(CH₂)₂SO₂Et | | -S(CH₂)₃CF₃ |
| -O(CH₂)₃OH | | -O(CH₂)₃SO₂Me | | -NH(CH₂)₂OH |
| -O(CH₂)₄OH | | -O(CH₂)₂NMe₂ | | -NH(CH₂)₃OH |
| -O(CH₂)₅OH | | -O(CH₂)₃NMe₂ | | -NH(CH₂)₄OH |
| -O(CH₂)₂OMe | | -O(CH₂)₃CF₃ | | -N(CH₂)₂OMe |
| -O(CH₂)₂OEt | | -O(CH₂)₂CF₂CF₃ | | -NH(CH₂)₃OMe |
| -O(CH₂)₂OPr | | -S(CH₂)₂OH | | -NH(CH₂)₂SMe |
| -O(CH₂)₃OMe | | -S(CH₂)₃OH | | -NH(CH₂)₃SMe |
| -O(CH₂)₃OEt | | -S(CH₂)₄OH | | -NH(CH₂)₃Me |
| -O(CH₂)₄OMe | | -S(CH₂)₂OMe | | -NH(CH₂)₃CF₃ |
| -O(CH₂)₂SMe | | -S(CH₂)₃OMe | | -NMe(CH₂)₃OMe |
| -O(CH₂)₂SEt | | -S(CH₂)₂SMe | | -NMe(CH₂)₃CH₃ |
| -O(CH₂)₃SMe | | -S(CH₂)₃SMe | | -CH₂COOMe |
| -O(CH₂)₂SO₂Me | | -S(CH₂)₃Me | | -(CH₂)₂COOMe |
| | | | | -CF₃ |

**Table 9**

| | | |
|---|---|---|
| 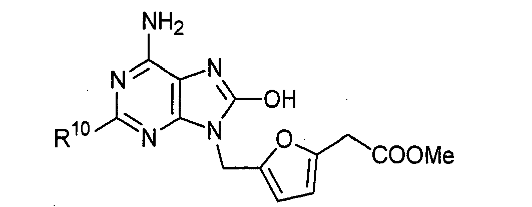 | | |

| -R¹⁰ | | -R¹⁰ |
|---|---|---|
| 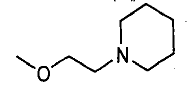 | | 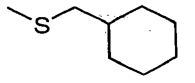 |
| 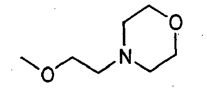 | | 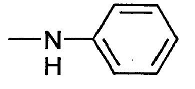 |
| 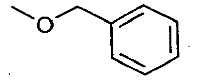 | | 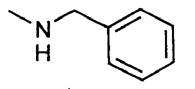 |
| 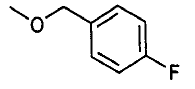 | | 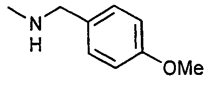 |
| 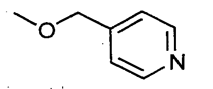 | | 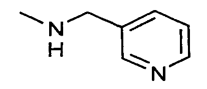 |
| 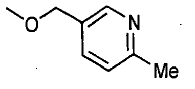 | | 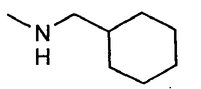 |
| 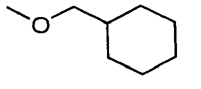 | | 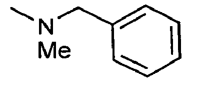 |
| 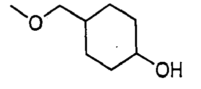 | | 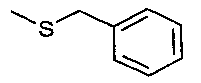 |
| 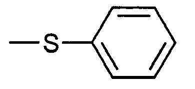 | | |

**Table 10**

| | | | | | |
|---|---|---|---|---|---|
| 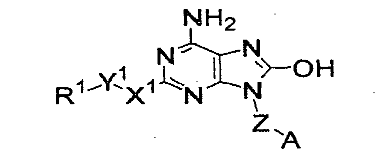 | | | | | |

| R¹-Y¹-X¹- | -Z-A | R¹-Y¹-X¹- | -Z-A | R¹-Y¹-X¹- | -Z-A |
|---|---|---|---|---|---|
| 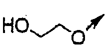 | 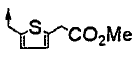 | 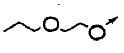 | 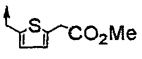 | 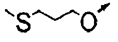 | 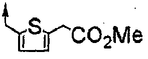 |
| 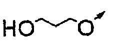 | 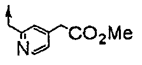 | 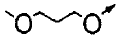 | 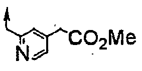 | 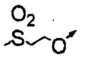 | 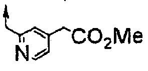 |
| 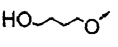 | 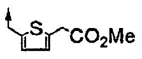 | 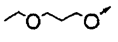 | 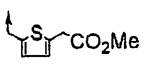 | 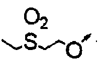 | 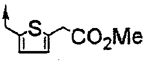 |
| 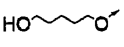 | 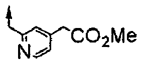 | 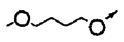 | 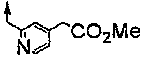 | 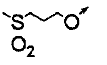 | 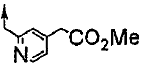 |
| 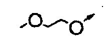 | 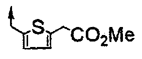 | 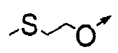 | 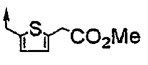 | 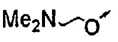 | 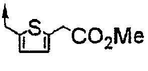 |
| 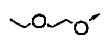 | 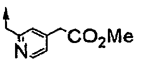 | 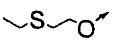 | 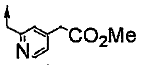 | 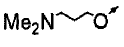 | 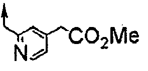 |

**Table 11**

| R¹-Y¹-X¹- | -Z-A | R¹-Y¹-X¹- | -Z-A | R¹-Y¹-X¹- | -Z-A |
|---|---|---|---|---|---|
| 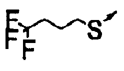 | 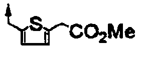 | 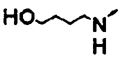 | 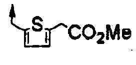 | 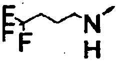 | 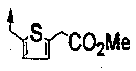 |
| 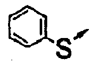 | 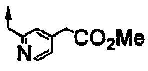 | 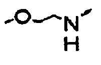 | 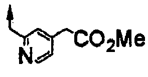 | 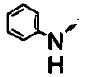 | 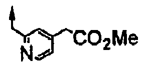 |
| 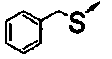 | 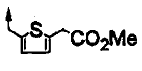 | 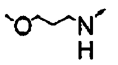 | 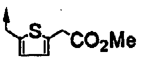 | 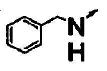 | 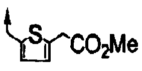 |
| 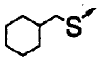 | 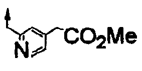 | 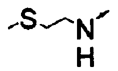 | 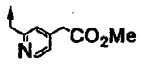 | 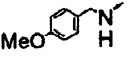 | 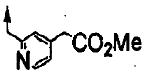 |
| 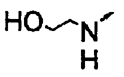 | 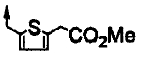 | 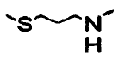 | 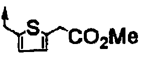 | 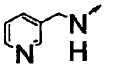 | 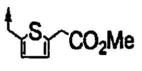 |
| 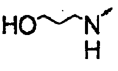 | 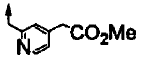 | 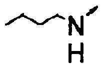 | 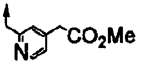 | 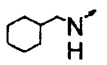 | 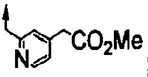 |

**Table 12**

| R¹-Y¹-X¹- | -Z-A | R¹-Y¹-X¹ | -Z-A | R¹-Y¹-X¹- | -Z-A |
|---|---|---|---|---|---|
| 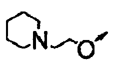 | 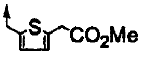 | 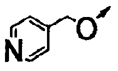 | 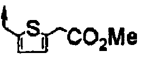 | 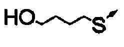 | 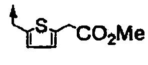 |
| 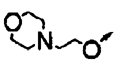 | 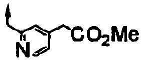 | 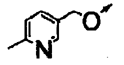 | 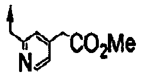 | 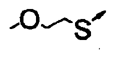 | 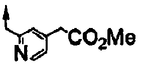 |
| 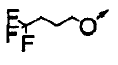 | 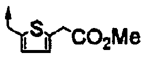 | 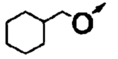 | 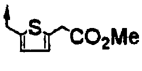 | 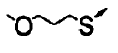 | 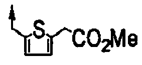 |
| 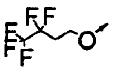 | 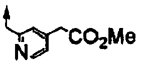 | 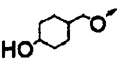 | 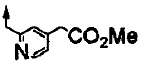 | 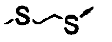 | 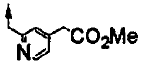 |
| 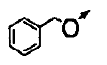 | 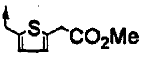 | 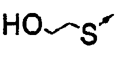 | 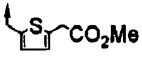 | 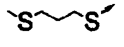 | 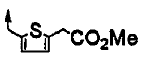 |
| 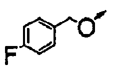 | 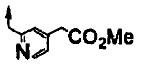 | 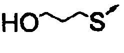 | 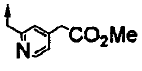 | 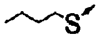 | 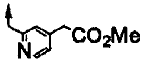 |

**Table 13**

| R¹-Y¹-X¹- | -Z-A |
|---|---|
| 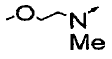 | 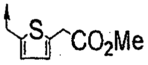 |
| 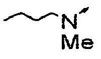 | 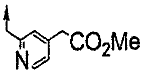 |
| 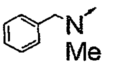 | 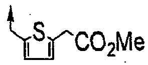 |
| 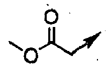 | 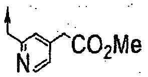 |
| 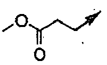 | 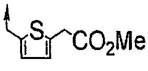 |
| 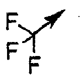 | 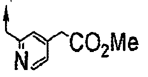 |

**Table 14**

| R¹-Y¹-X¹- | -Z-A | | R¹-Y¹-X¹- | -Z-A | | R¹-Y¹-X¹- | -Z-A |
|---|---|---|---|---|---|---|---|
| 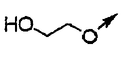 | 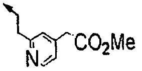 | | 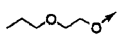 | 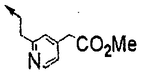 | | 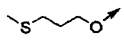 | 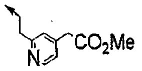 |
| 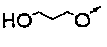 | 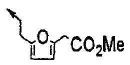 | | 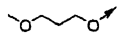 | 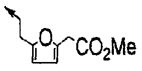 | | 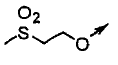 | 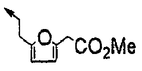 |
| 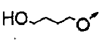 | 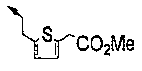 | | 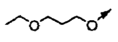 | 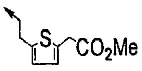 | | 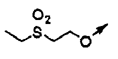 | 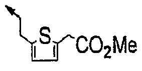 |
| 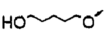 | 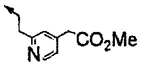 | | 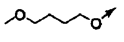 | 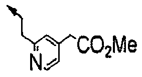 | | 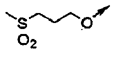 | 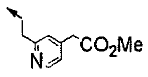 |
| 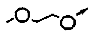 | 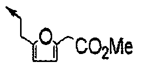 | | 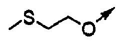 | 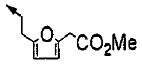 | | 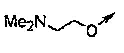 | 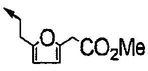 |
| 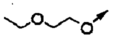 | 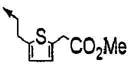 | | 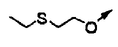 | 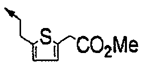 | | 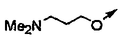 | 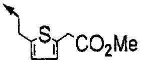 |

**Table 15**

| R¹-Y¹-X¹- | -Z-A | | R¹-Y¹-X¹- | -Z-A | | R¹-Y¹-X¹- | -Z-A |
|---|---|---|---|---|---|---|---|
| 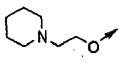 | 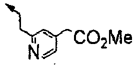 | | 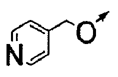 | 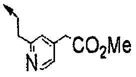 | | 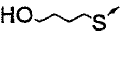 | 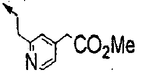 |
| 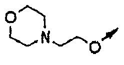 | 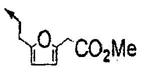 | | 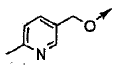 | 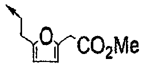 | | 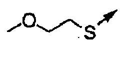 | 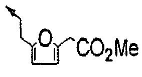 |
| 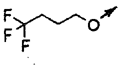 | 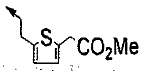 | | 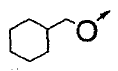 | 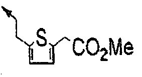 | | 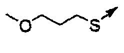 | 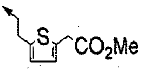 |
| 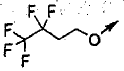 | 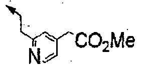 | | 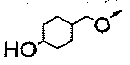 | 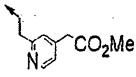 | | 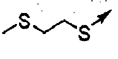 | 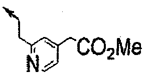 |
| 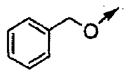 | 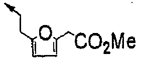 | | 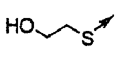 | 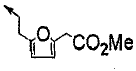 | | 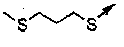 | 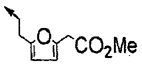 |
| 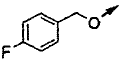 | 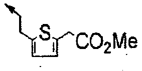 | | 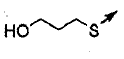 | 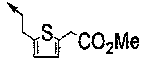 | | 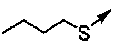 | 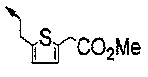 |

**Table 16**

| R¹-Y¹-X¹- | -Z-A | | R¹-Y¹-X¹- | -Z-A | | R¹-Y¹-X¹- | -Z-A |
|---|---|---|---|---|---|---|---|
| 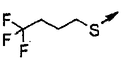 | 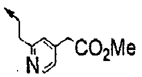 | | 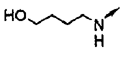 | 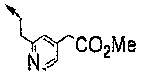 | | 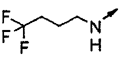 | 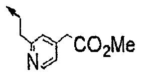 |
| 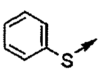 | 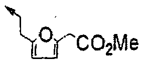 | | 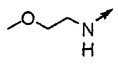 | 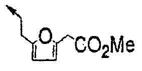 | | 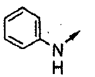 | 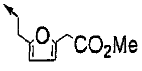 |
| 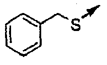 | 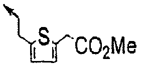 | | 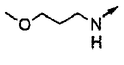 | 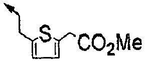 | | 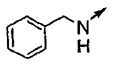 | 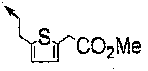 |
| 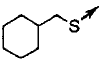 | 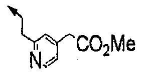 | | 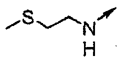 | 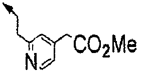 | | 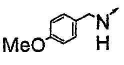 | 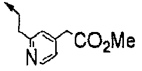 |
| 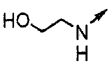 | 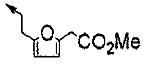 | | 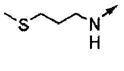 | 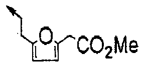 | | 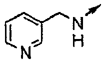 | 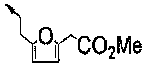 |
| 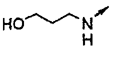 | 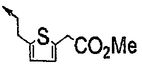 | | 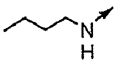 | 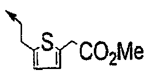 | | 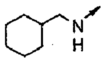 | 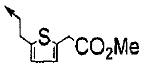 |

**Table 17**

| R¹-Y¹-X¹- | -Z-A |
|---|---|
| 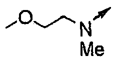 | 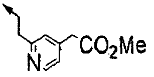 |
| 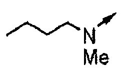 | 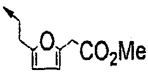 |
| 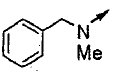 | 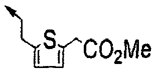 |
| 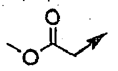 | 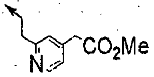 |
| 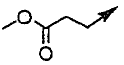 | 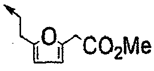 |
| 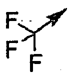 | 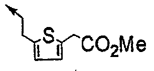 |

**Table 18**

| R¹-Y¹-X¹- | -Z-A | | R¹-Y¹-X¹- | -Z-A | | R¹-Y¹-X¹- | -Z-A |
|---|---|---|---|---|---|---|---|
| 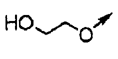 | 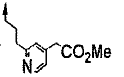 | | 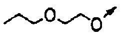 | 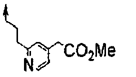 | | 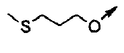 | 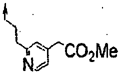 |
| 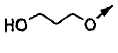 | 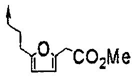 | | 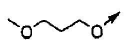 | 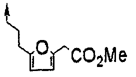 | | 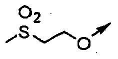 | 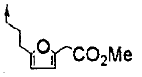 |
| 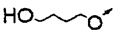 | 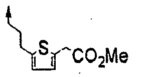 | | 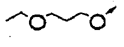 | 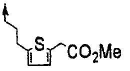 | | 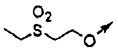 | 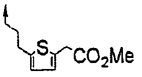 |
| 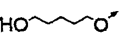 | 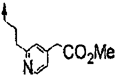 | | 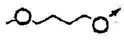 | 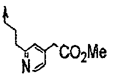 | | 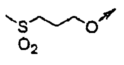 | 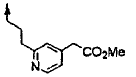 |
| 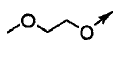 | 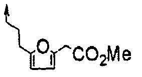 | | 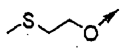 | 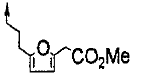 | | 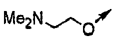 | 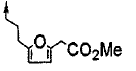 |
| 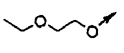 | 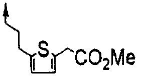 | | 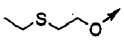 | 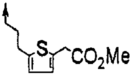 | | 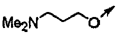 | 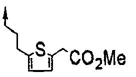 |

**Table 19**

| R¹-Y¹-X¹- | -Z-A | | R¹-Y¹-X¹- | -Z-A | | R¹-Y¹-X¹- | -Z-A |
|---|---|---|---|---|---|---|---|
| 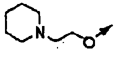 | 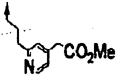 | | 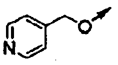 | 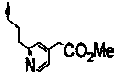 | | 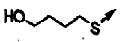 | 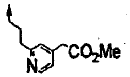 |
| 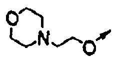 | 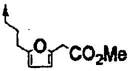 | | 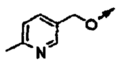 | 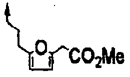 | | 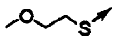 | 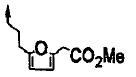 |
| 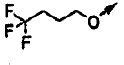 | 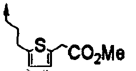 | | 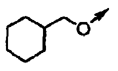 | 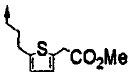 | | 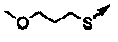 | 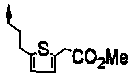 |
| 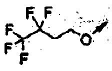 | 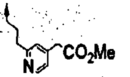 | | 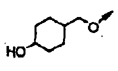 | 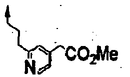 | | 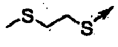 | 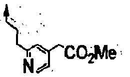 |
| 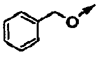 | 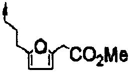 | | 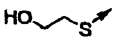 | 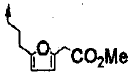 | | 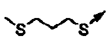 | 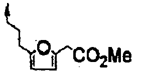 |
| 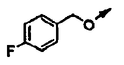 | 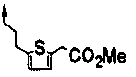 | | 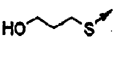 | 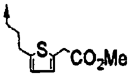 | | 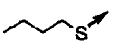 | 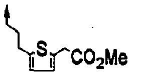 |

**Table 20**

| R¹-Y¹-X¹- | -Z-A | | R¹-Y¹-X¹- | -Z-A | | R¹-Y¹-X¹- | -Z-A |
|---|---|---|---|---|---|---|---|
| 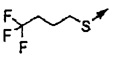 | 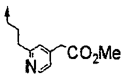 | | 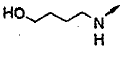 | 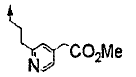 | | 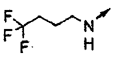 | 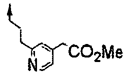 |
| 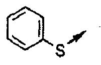 | 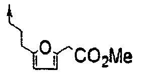 | | 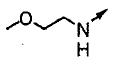 | 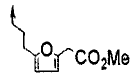 | | 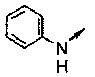 | 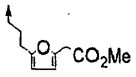 |
| 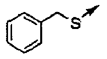 | 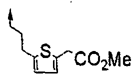 | | 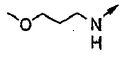 | 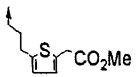 | | 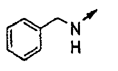 | 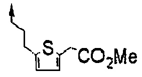 |
| 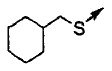 | 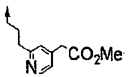 | | 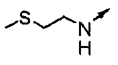 | 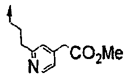 | | 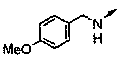 | 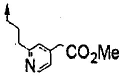 |
| 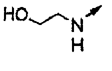 | 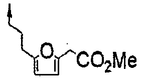 | | 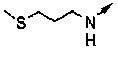 | 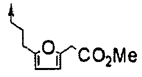 | | 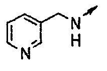 | 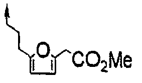 |
| 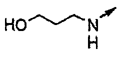 | 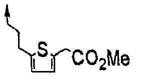 | | 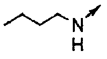 | 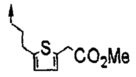 | | 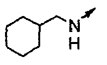 | 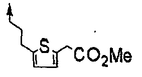 |

**Table 21**

| R¹-Y¹-X¹- | -Z-A |
|---|---|
| 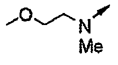 | 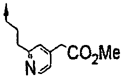 |
| 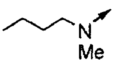 | 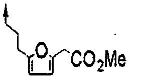 |
| 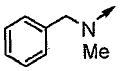 | 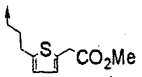 |
| 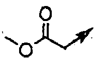 | 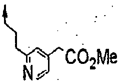 |
| 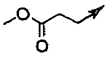 | 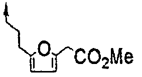 |
| 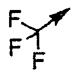 | 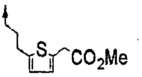 |

**Table 22**

| R¹-Y¹-X¹- | -Z-A | | R¹-Y¹-X¹- | -Z-A | | R¹-Y¹-X¹- | -Z-A |
|---|---|---|---|---|---|---|---|
| 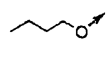 | 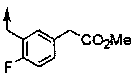 | | 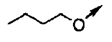 | 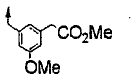 | | 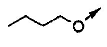 | 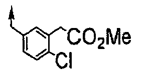 |
| 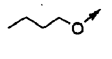 | 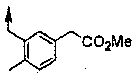 | | 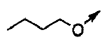 | 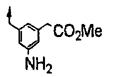 | | 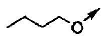 | 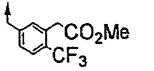 |
| 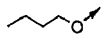 | 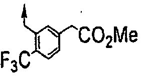 | | 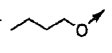 | 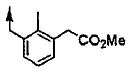 | | 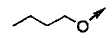 | 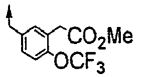 |
| 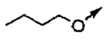 | 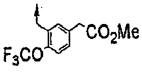 | | 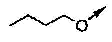 | 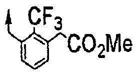 | | 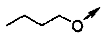 | 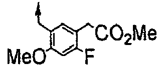 |
| 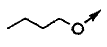 | 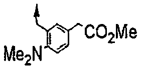 | | 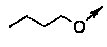 | 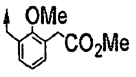 | | 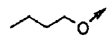 | 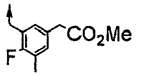 |
| 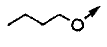 | 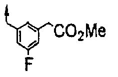 | | 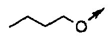 | 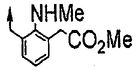 | | 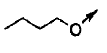 | 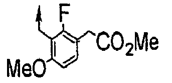 |

**Table 23**

| R¹-Y¹-X¹- | Z-A- | | R¹-Y¹-X¹- | Z-A- | | R¹-Y¹-X¹- | Z-A- |
|---|---|---|---|---|---|---|---|
| 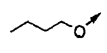 | 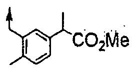 | | 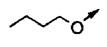 | 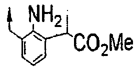 | | 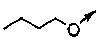 | 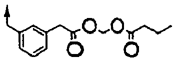 |
| 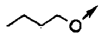 | 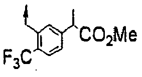 | | 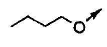 | 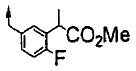 | | 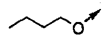 | 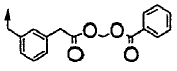 |
| 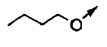 | 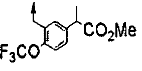 | | 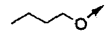 | 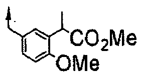 | | 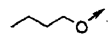 | 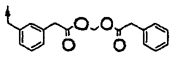 |
| 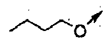 | 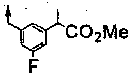 | | 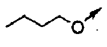 | 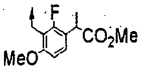 | | 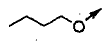 | 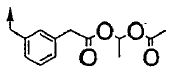 |
| 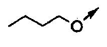 | 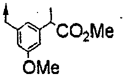 | | 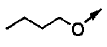 | 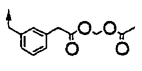 | | 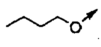 | 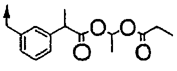 |
| 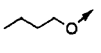 | 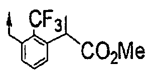 | | 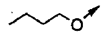 | 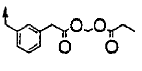 | | 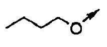 | 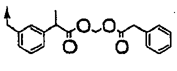 |

**Table 24**

| R¹-Y¹-X¹- | -Z-A | | R¹-Y¹-X¹- | -Z-A | | R¹-Y¹-X¹- | -Z-A |
|---|---|---|---|---|---|---|---|
| 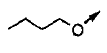 | 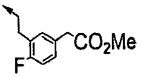 | | 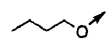 | 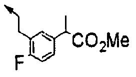 | | 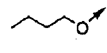 | 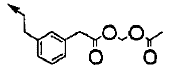 |
| 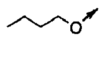 | 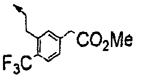 | | 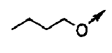 | 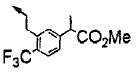 | | 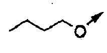 | 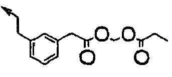 |
| 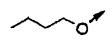 | 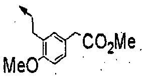 | | 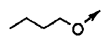 | 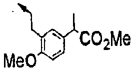 | | 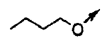 | 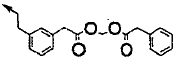 |
| 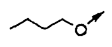 | 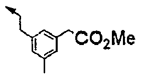 | | 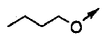 | 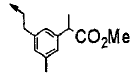 | | 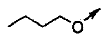 | 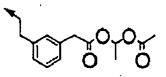 |
| 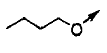 | 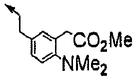 | | 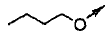 | 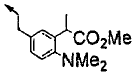 | | 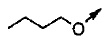 | 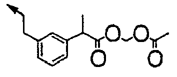 |
| 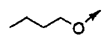 | 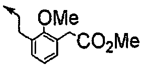 | | 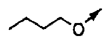 | 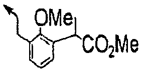 | | 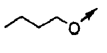 | 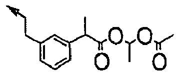 |

**Table 25**

| R¹-Y¹-X¹- | -Z-A | | R¹-Y¹-X¹- | -Z-A | | R¹-Y¹-X¹- | -Z-A |
|---|---|---|---|---|---|---|---|
| 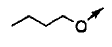 | 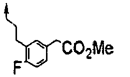 | | 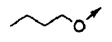 | 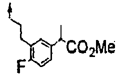 | | 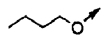 | 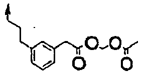 |
| 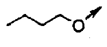 | 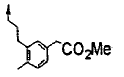 | | 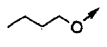 | 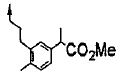 | | 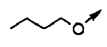 | 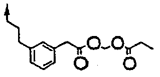 |
| 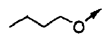 | 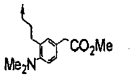 | | 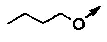 | 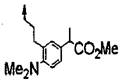 | | 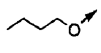 | 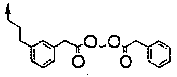 |
| 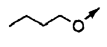 | 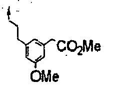 | | 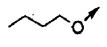 | 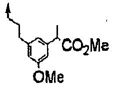 | | 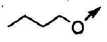 | 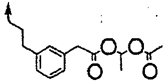 |
| 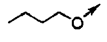 | 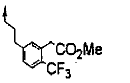 | | 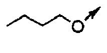 | 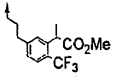 | | 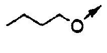 | 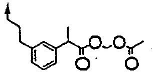 |
| 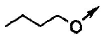 | 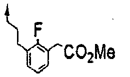 | | 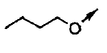 | 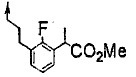 | | 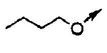 | 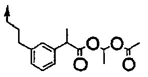 |

**Table 26**

| R¹-Y¹-X¹- | -Z-A | | R¹-Y¹-X¹- | -Z-A |
|---|---|---|---|---|
| 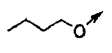 | 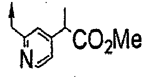 | | 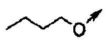 | 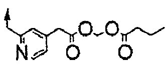 |
| 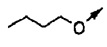 | 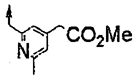 | | 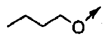 | 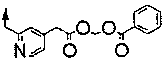 |
| 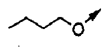 | 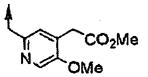 | | 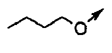 | 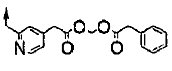 |
| 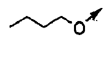 | 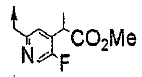 | | 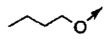 | 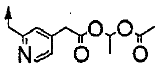 |
| 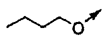 | 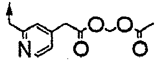 | | 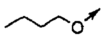 | 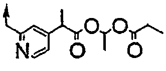 |
| 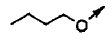 | 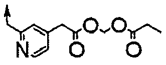 | | 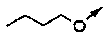 | 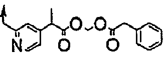 |

**Table 27**

| R¹-Y¹-X¹- | -Z-A | | R¹-Y¹-X¹- | -Z-A | | R¹-Y¹-X¹- | Z-A- |
|---|---|---|---|---|---|---|---|
| 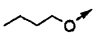 | 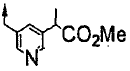 | | 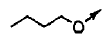 | 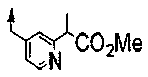 | | 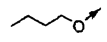 | 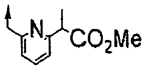 |
| 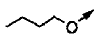 | 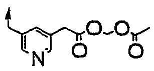 | | 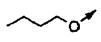 | 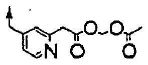 | | 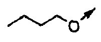 | 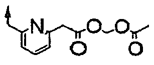 |
| 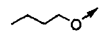 | 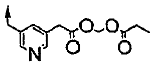 | | 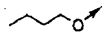 | 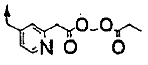 | | 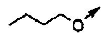 | 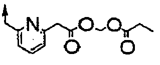 |
| 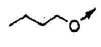 | 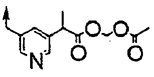 | | 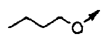 | 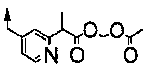 | | 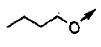 | 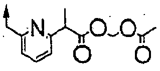 |
| 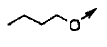 | 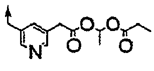 | | 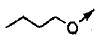 | 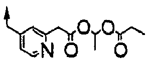 | | 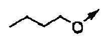 | 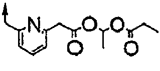 |
| 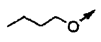 | 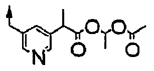 | | 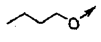 | 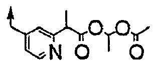 | | 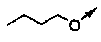 | 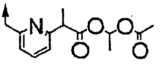 |

**Table 28**

| | | | | |
|---|---|---|---|---|
| 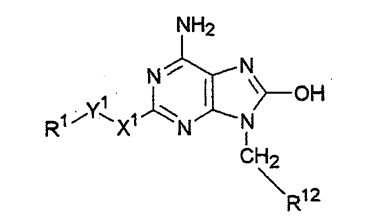 | | | | |

| R¹-Y¹-X¹- | R¹²- | | R¹-Y¹-X¹- | R¹²- |
|---|---|---|---|---|
| 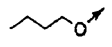 | 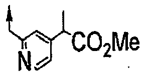 | | 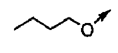 | 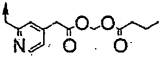 |
| 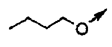 | 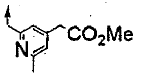 | | 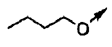 | 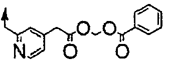 |
| 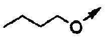 | 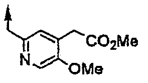 | | 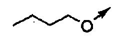 | 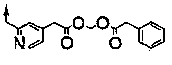 |
| 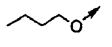 | 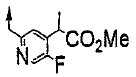 | | 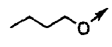 | 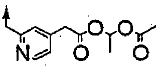 |
| 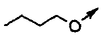 | 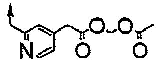 | | 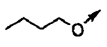 | 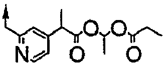 |
| 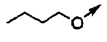 | 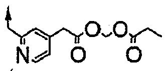 | | 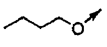 | 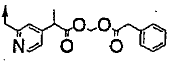 |

**Table 29**

| R¹-Y¹-X¹- | R¹²- | | R¹-Y¹-X¹- | R¹²- | | R¹-Y¹-X¹- | R¹²- |
|---|---|---|---|---|---|---|---|
| 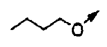 | 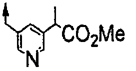 | | 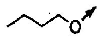 | 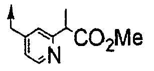 | | 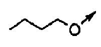 | 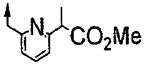 |
| 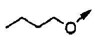 | 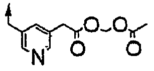 | | 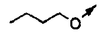 | 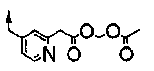 | | 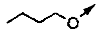 | 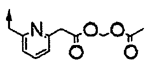 |
| 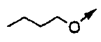 | 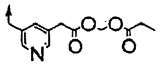 | | 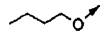 | 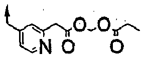 | | 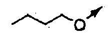 | 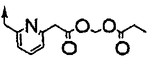 |
| 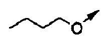 | 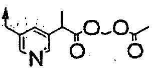 | | 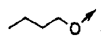 | 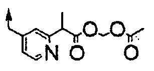 | | 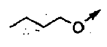 | 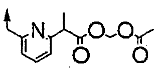 |
| 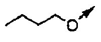 | 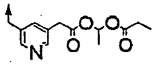 | | 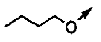 | 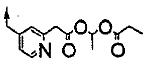 | | 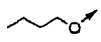 | 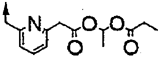 |
| 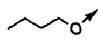 | 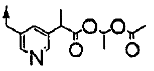 | | 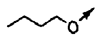 | 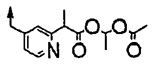 | | 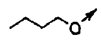 | 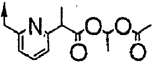 |

**Table 30**

| | | | | |
|---|---|---|---|---|
| 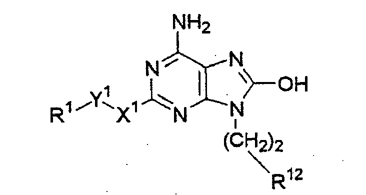 | | | | |

| R¹-Y¹-X¹- | R¹²- | | R¹-Y¹-X¹- | R¹²- |
|---|---|---|---|---|
| 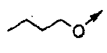 | 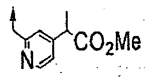 | | 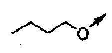 | 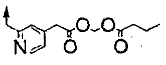 |
| 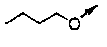 | 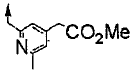 | | 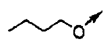 | 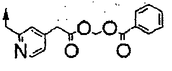 |
| 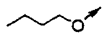 | 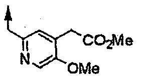 | | 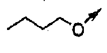 | 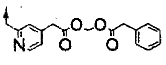 |
| 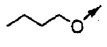 | 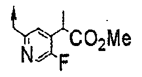 | | 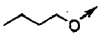 | 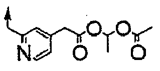 |
| 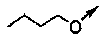 | 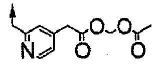 | | 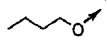 | 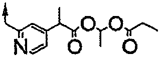 |
| 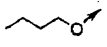 | 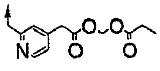 | | 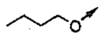 | 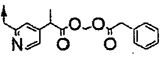 |

**Table 31**

| R¹-Y¹-X¹- | R¹²- | | R¹-Y¹-X¹- | R¹²- | | R¹-Y¹-X¹- | R¹²- |
|---|---|---|---|---|---|---|---|
| 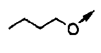 | 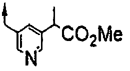 | | 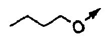 | 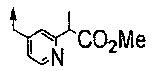 | | 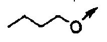 | 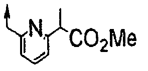 |
| 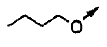 | 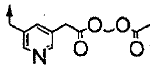 | | 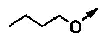 | 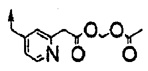 | | 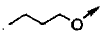 | 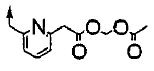 |
| 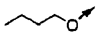 | 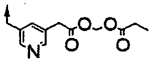 | | 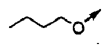 | 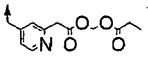 | | 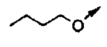 | 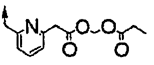 |
| 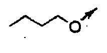 | 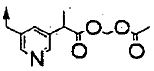 | | 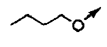 | 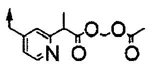 | | 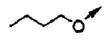 | 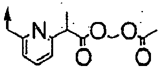 |
| 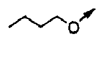 | 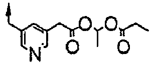 | | 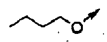 | 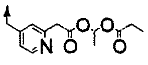 | | 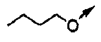 | 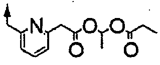 |
| 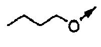 | 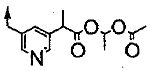 | | 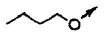 | 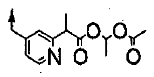 | | 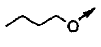 | 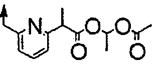 |

**Table 32**

| | | | | | | | |
|---|---|---|---|---|---|---|---|
| 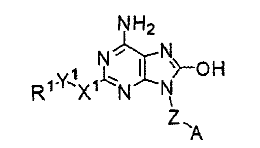 | | | | | | | |

| R¹-Y¹-X¹- | Z-A- | | R¹-Y¹-X¹- | Z-A- | | R¹-Y¹-X¹- | Z-A- |
|---|---|---|---|---|---|---|---|
| 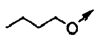 | 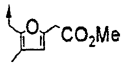 | | 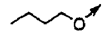 | 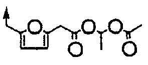 | | 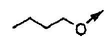 | 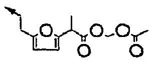 |
| 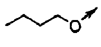 | 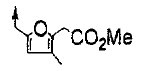 | | 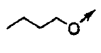 | 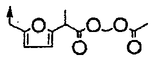 | | 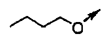 | 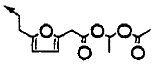 |
| 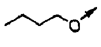 | 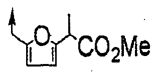 | | 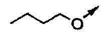 | 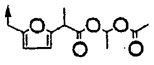 | | 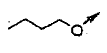 | 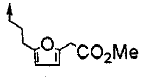 |
| 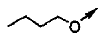 | 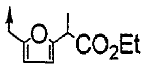 | | 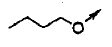 | 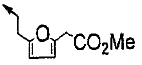 | | 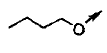 | 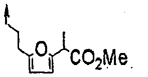 |
| 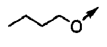 | 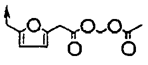 | | 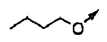 | 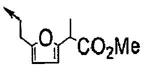 | | 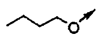 | 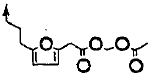 |
| 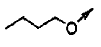 | 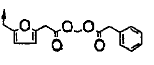 | | 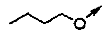 | 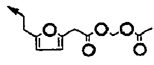 | | 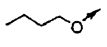 | 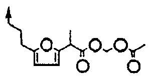 |

**Table 33**

| R¹-Y¹-X¹- | Z-A- | | R¹-Y¹-X¹- | Z-A- | | R¹-Y¹-X¹- | Z-A- |
|---|---|---|---|---|---|---|---|
| 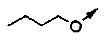 | 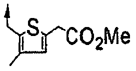 | | 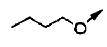 | 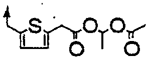 | | 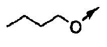 | 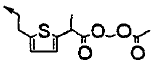 |
| 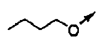 | 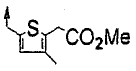 | | 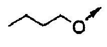 | 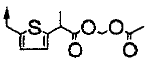 | | 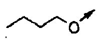 | 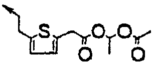 |
| 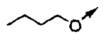 | 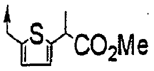 | | 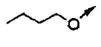 | 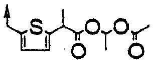 | | 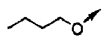 | 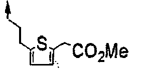 |
| 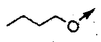 | 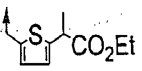 | | 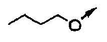 | 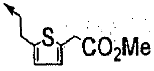 | | 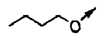 | 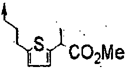 |
| 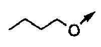 | 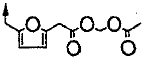 | | 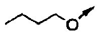 | 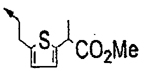 | | 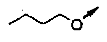 | 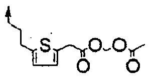 |
| 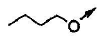 | 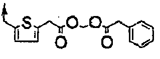 | | 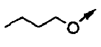 | 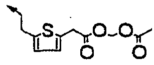 | | 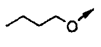 | 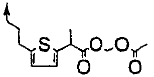 |

**Table 34**

| R¹-Y¹-X¹- | -Z-A | | R¹-Y¹-X¹- | -Z-A | | R¹-Y¹-X¹- | -Z-A |
|---|---|---|---|---|---|---|---|
| 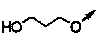 | 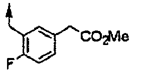 | | 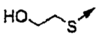 | 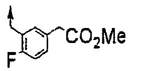 | | 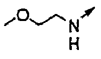 | 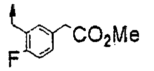 |
| 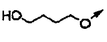 | 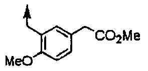 | | 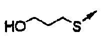 | 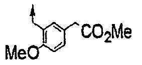 | | 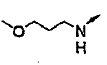 | 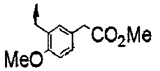 |
| 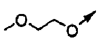 | 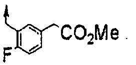 | | 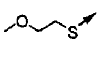 | 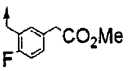 | | 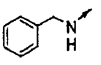 | 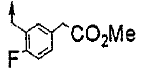 |
| 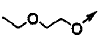 | 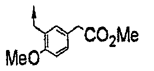 | | 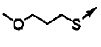 | 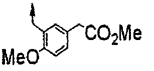 | | 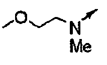 | 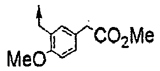 |
| 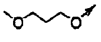 | 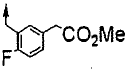 | | 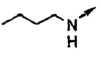 | 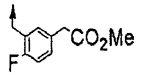 | | 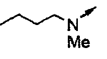 | 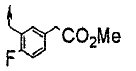 |
| 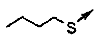 | 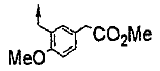 | | 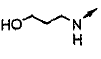 | 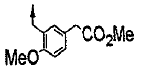 | | 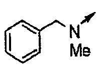 | 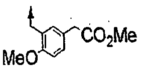 |

**Table 35**

| R¹-Y¹-X¹- | -Z-A | | R¹-Y¹-X¹- | -Z-A | | R¹-Y¹-X¹- | -Z-A |
|---|---|---|---|---|---|---|---|
| 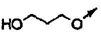 | 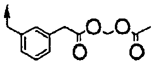 | | 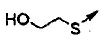 | 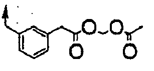 | | 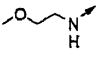 | 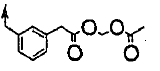 |
| 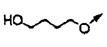 | 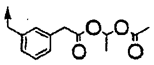 | | 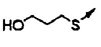 | 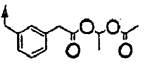 | | 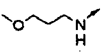 | 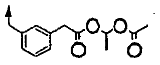 |
| 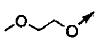 | 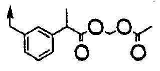 | | 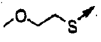 | 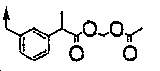 | | 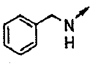 | 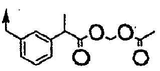 |
| 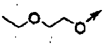 | 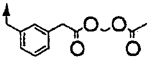 | | 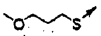 | 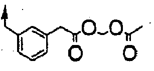 | | 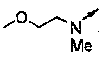 | 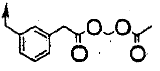 |
| 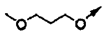 | 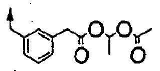 | | 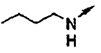 | 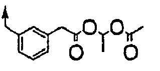 | | 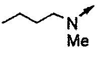 | 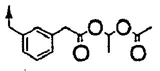 |
| 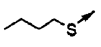 | 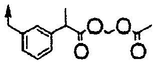 | | 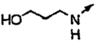 | 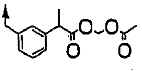 | | 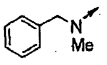 | 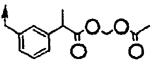 |

**Table 36**

| R¹-Y¹-X¹- | -Z-A | | R¹-Y¹-X¹- | -Z-A | | R¹-Y¹-X¹- | -Z-A |
|---|---|---|---|---|---|---|---|
| 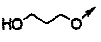 | 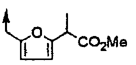 | | 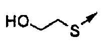 | 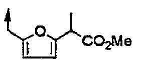 | | 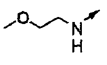 | 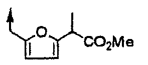 |
| 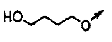 | 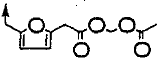 | | 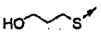 | 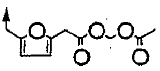 | | 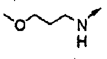 | 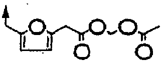 |
| 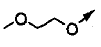 | 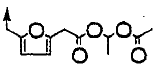 | | 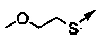 | 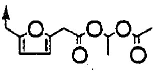 | | 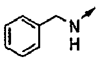 | 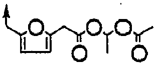 |
| 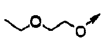 | 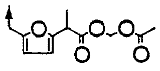 | | 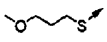 | 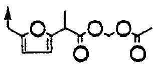 | | 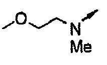 | 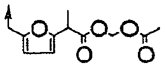 |
| 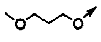 | 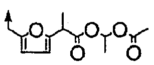 | | 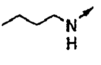 | 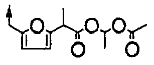 | | 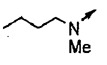 | 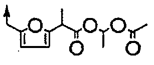 |
| 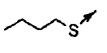 | 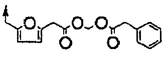 | | 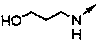 | 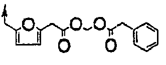 | | 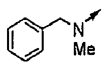 | 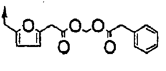 |

**Table 37**

| R¹-Y¹-X¹- | -Z-A | | R¹-Y¹-X¹- | -Z-A | | R¹-Y¹-X¹- | -Z-A |
|---|---|---|---|---|---|---|---|
| 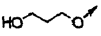 | 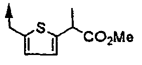 | | 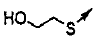 | 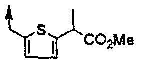 | | 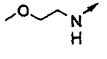 | 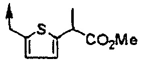 |
| 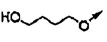 | 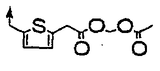 | | 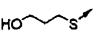 | 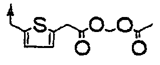 | | 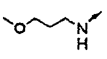 | 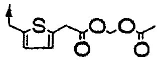 |
| 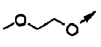 | 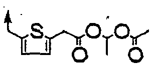 | | 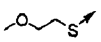 | 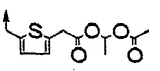 | | 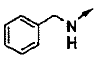 | 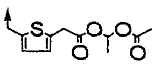 |
| 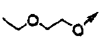 | 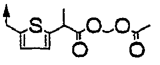 | | 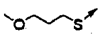 | 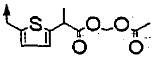 | | 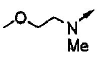 | 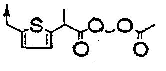 |
| 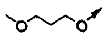 | 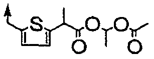 | | 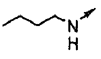 | 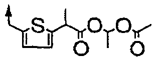 | | 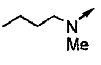 | 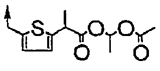 |
| 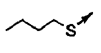 | 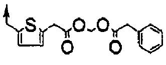 | | 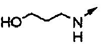 | 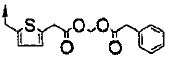 | | 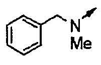 | 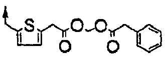 |

**Table 38**

| | | | | | | | |
|---|---|---|---|---|---|---|---|
| 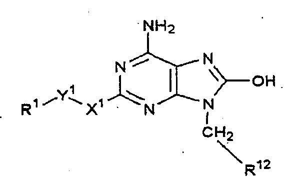 | | | | | | | |

| R¹-Y¹-X¹- | -Z-A | | R¹-Y¹-X¹- | -Z-A | | R¹-Y¹-X¹- | -Z-A |
|---|---|---|---|---|---|---|---|
| 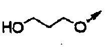 | 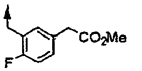 | | 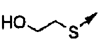 | 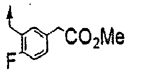 | | 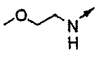 | 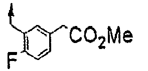 |
| 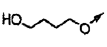 | 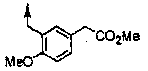 | | 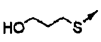 | 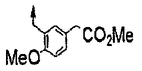 | | 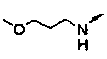 | 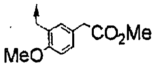 |
| 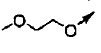 | 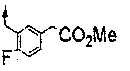 | | 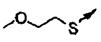 | 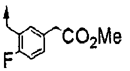 | | 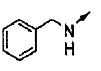 | 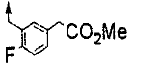 |
| 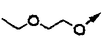 | 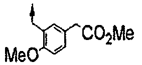 | | 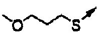 | 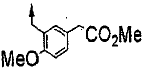 | | 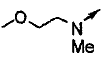 | 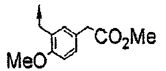 |
| 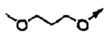 | 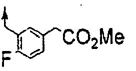 | | 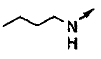 | 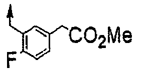 | | 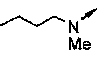 | 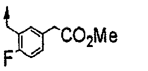 |
| 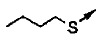 | 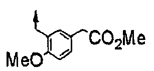 | | 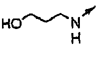 | 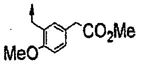 | | 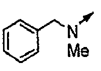 | 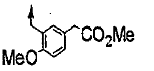 |

**Table 39**

| R¹-Y¹-X¹- | -Z-A | | R¹-Y¹-X¹- | -Z-A | | R¹-Y¹-X¹- | -Z-A |
|---|---|---|---|---|---|---|---|
| 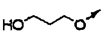 | 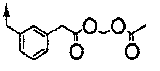 | | 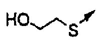 | 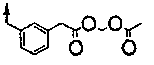 | | 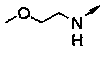 | 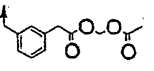 |
| 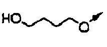 | 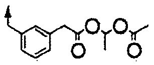 | | 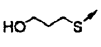 | 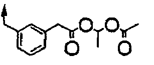 | | 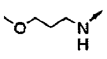 | 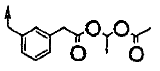 |
| 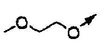 | 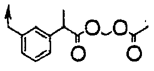 | | 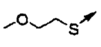 | 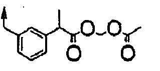 | | 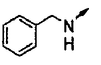 | 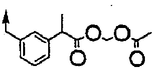 |
| 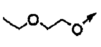 | 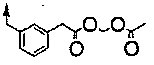 | | 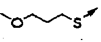 | 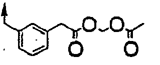 | | 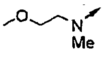 | 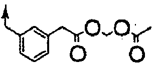 |
| 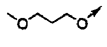 | 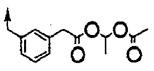 | | 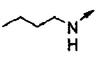 | 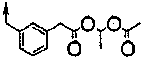 | | 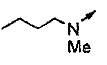 | 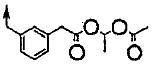 |
| 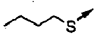 | 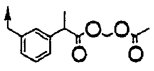 | | 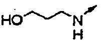 | 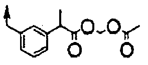 | | 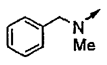 | 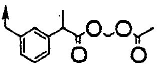 |

**Table 40**

| R¹-Y¹-X¹- | -Z-A | | R¹-Y¹-X¹- | -Z-A | | R¹-Y¹-X¹- | -Z-A |
|---|---|---|---|---|---|---|---|
| 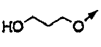 | 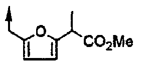 | | 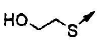 | 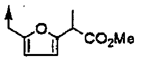 | | 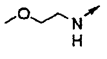 | 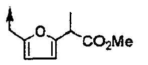 |
| 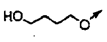 | 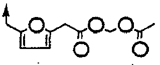 | | 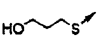 | 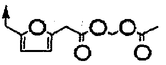 | | 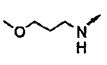 | 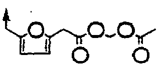 |
| 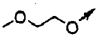 | 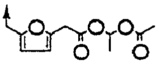 | | 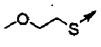 | 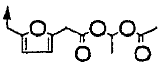 | | 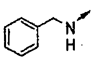 | 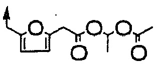 |
| 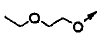 | 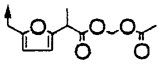 | | 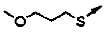 | 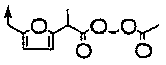 | | 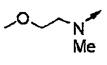 | 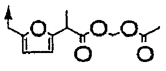 |
| 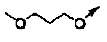 | 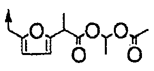 | | 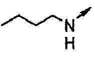 | 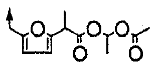 | | 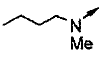 | 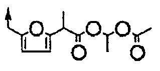 |
| 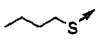 | 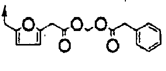 | | 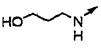 | 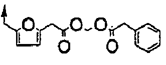 | | 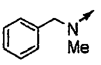 | 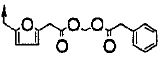 |

**Table 41**

| R¹Y¹-X¹- | -Z-A | | R¹-Y¹-X¹- | -Z-A | | R¹-Y¹-X¹- | -Z-A |
|---|---|---|---|---|---|---|---|
| 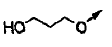 | 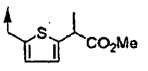 | | 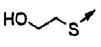 | 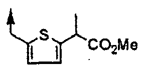 | | 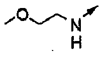 | 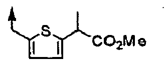 |
| 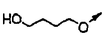 | 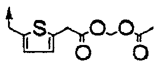 | | 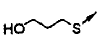 | 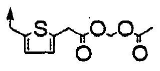 | | 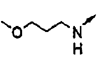 | 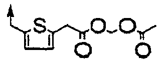 |
| 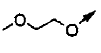 | 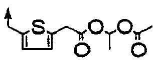 | | 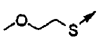 | 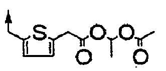 | | 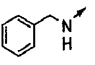 | 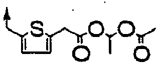 |
| 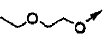 | 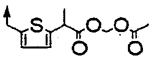 | | 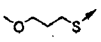 | 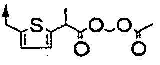 | | 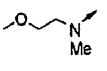 | 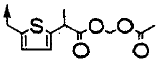 |
| 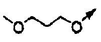 | 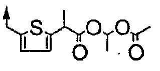 | | 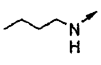 | 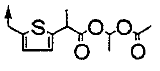 | | 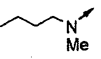 | 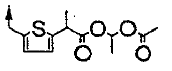 |
| 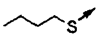 | 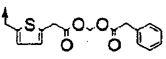 | | 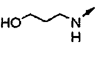 | 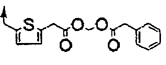 | | 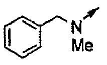 | 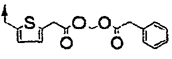 |

**Table 42**

| | | | | | | | |
|---|---|---|---|---|---|---|---|
| 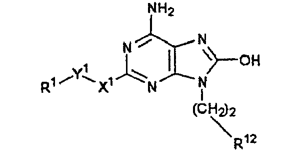 | | | | | | | |

| R¹-Y¹-X¹- | -Z-A | | R¹-Y¹-X¹- | -Z-A | | R¹-Y¹-X¹- | -Z-A |
|---|---|---|---|---|---|---|---|
| 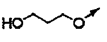 | 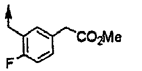 | | 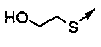 | 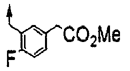 | | 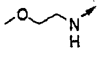 | 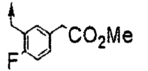 |
| 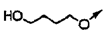 | 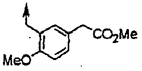 | | 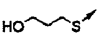 | 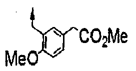 | | 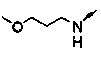 | 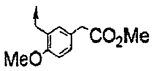 |
| 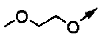 | 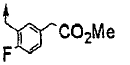 | | 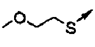 | 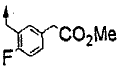 | | 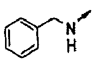 | 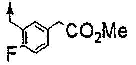 |
| 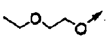 | 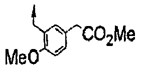 | | 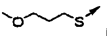 | 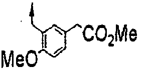 | | 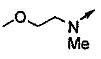 | 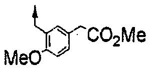 |
| 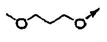 | 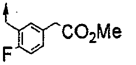 | | 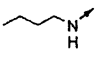 | 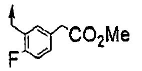 | | 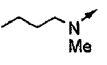 | 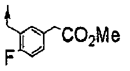 |
| 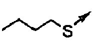 | 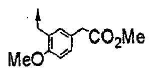 | | 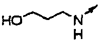 | 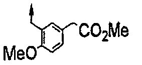 | | 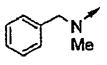 | 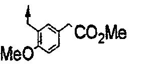 |

**Table 43**

| R¹-Y¹-X¹- | -Z-A | | R¹-Y¹-X¹- | -Z-A | | R¹-Y¹-X¹- | -Z-A |
|---|---|---|---|---|---|---|---|
| 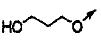 | 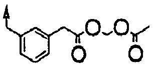 | | 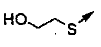 | 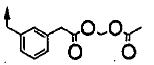 | | 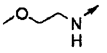 | 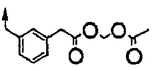 |
| 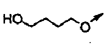 | 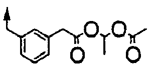 | | 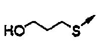 | 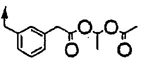 | | 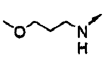 | 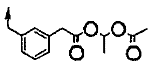 |
| 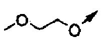 | 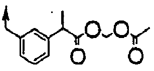 | | 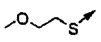 | 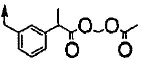 | | 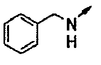 | 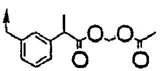 |
| 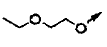 | 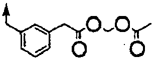 | | 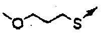 | 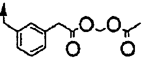 | | 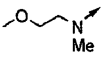 | 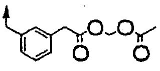 |
| 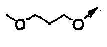 | 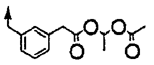 | | 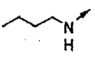 | 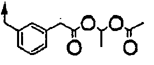 | | 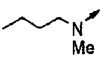 | 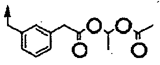 |
| 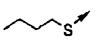 | 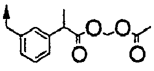 | | 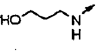 | 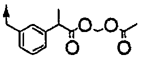 | | 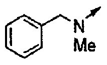 | 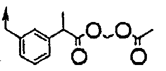 |

**Table 44**

| R¹-Y¹-X¹- | -Z-A | | R¹-Y¹-X¹- | -Z-A | | R¹-Y¹-X¹- | -Z-A |
|---|---|---|---|---|---|---|---|
| 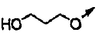 | 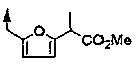 | | 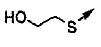 | 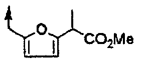 | | 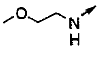 | 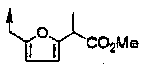 |
| 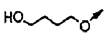 | 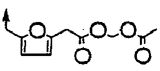 | | 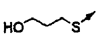 | 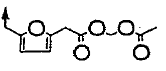 | | 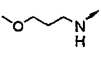 | 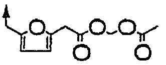 |
| 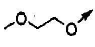 | 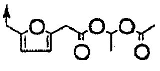 | | 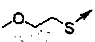 | 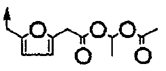 | | 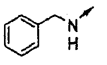 | 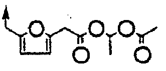 |
| 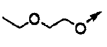 | 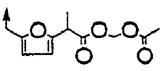 | | 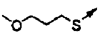 | 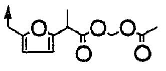 | | 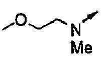 | 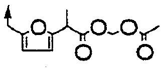 |
| 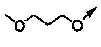 | 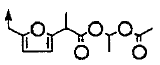 | | 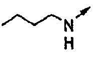 | 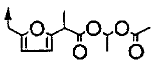 | | 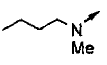 | 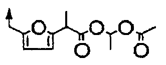 |
| 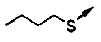 | 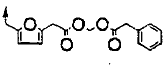 | | 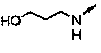 | 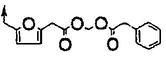 | | 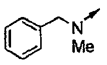 | 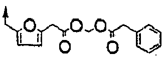 |

**Table 45**

| R¹-Y¹-X¹- | -Z-A | | R¹-Y¹-X¹- | -Z-A | | R¹-Y¹-X¹- | -Z-A |
|---|---|---|---|---|---|---|---|
| 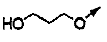 | 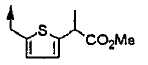 | | 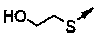 | 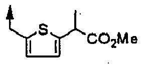 | | 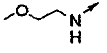 | 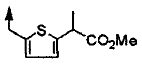 |
| 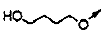 | 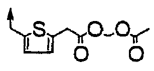 | | 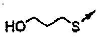 | 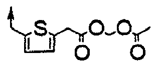 | | 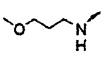 | 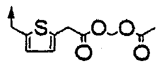 |
| 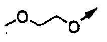 | 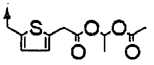 | | 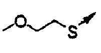 | 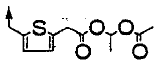 | | 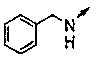 | 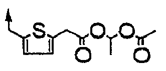 |
| 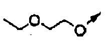 | 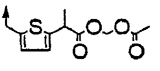 | | 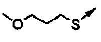 | 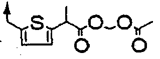 | | 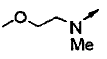 | 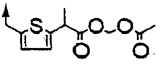 |
| 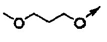 | 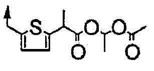 | | 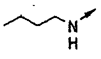 | 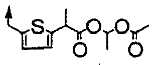 | | 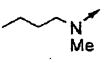 | 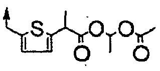 |
| 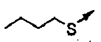 | 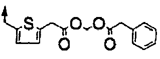 | | 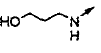 | 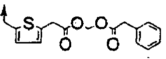 | | 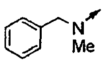 | 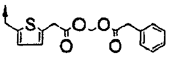 |

**Table 46**

| R¹-Y¹-X¹ | Z-A | | R¹-Y¹-X¹ | Z-A | | R¹-Y¹-X¹ | Z-A |
|---|---|---|---|---|---|---|---|
| 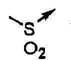 | 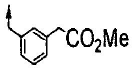 | | 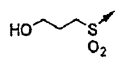 | 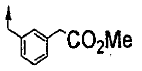 | | 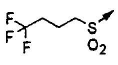 | 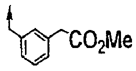 |
| 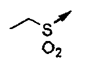 | 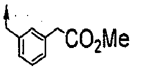 | | 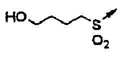 | 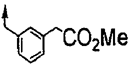 | | 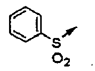 | 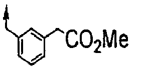 |
| 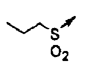 | 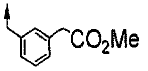 | | 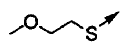 | 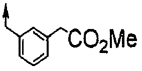 | | 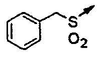 | 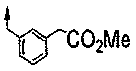 |
| 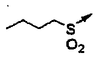 | 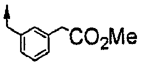 | | 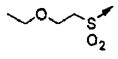 | 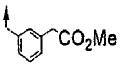 | | 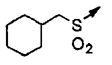 | 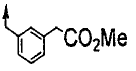 |
| 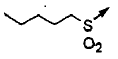 | 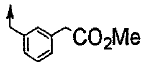 | | 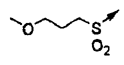 | 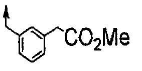 | | 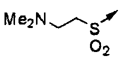 | 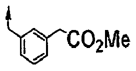 |
| 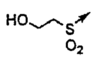 | 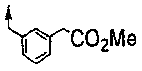 | | 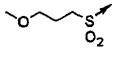 | 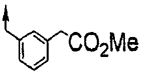 | | 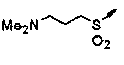 | 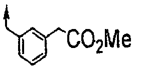 |

**Table 47**

| R¹-Y¹-X¹ | Z-A | | R¹-Y¹-X¹ | Z-A | | R¹-Y¹-X¹ | Z-A |
|---|---|---|---|---|---|---|---|
| 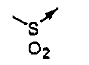 | 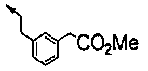 | | 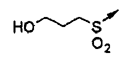 | 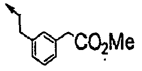 | | 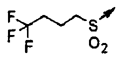 | 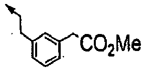 |
| 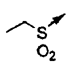 | 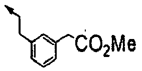 | | 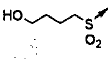 | 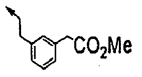 | | 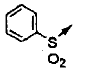 | 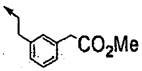 |
| 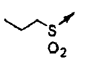 | 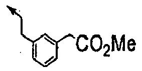 | | 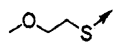 | 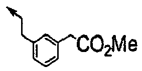 | | 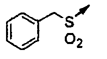 | 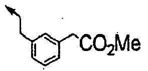 |
| 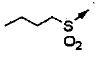 | 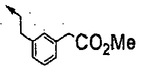 | | 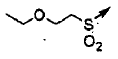 | 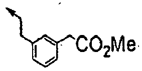 | | 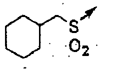 | 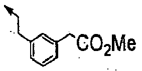 |
| 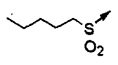 | 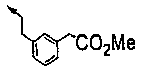 | | 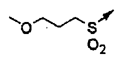 | 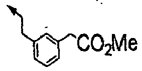 | | 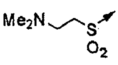 | 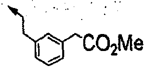 |
| 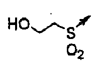 | 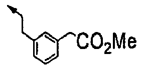 | | 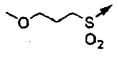 | 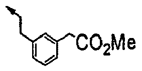 | | 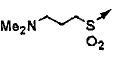 | 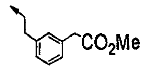 |

**Table 48**

| R¹-Y¹-X¹ | Z-A | | R¹-Y¹-X¹ | Z-A | | R¹-Y¹-X¹ | Z-A |
|---|---|---|---|---|---|---|---|
| 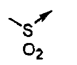 | 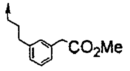 | | 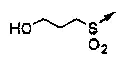 | 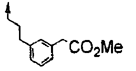 | | 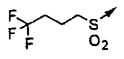 | 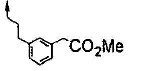 |
| 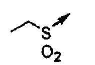 | 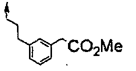 | | 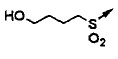 | 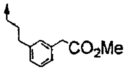 | | 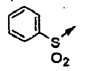 | 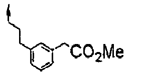 |
| 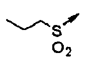 | 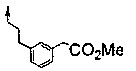 | | 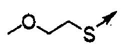 | 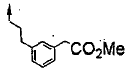 | | 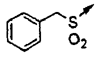 | 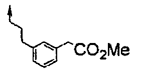 |
| 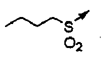 | 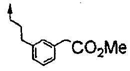 | | 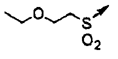 | 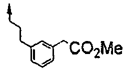 | | 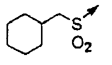 | 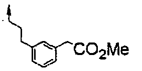 |
| 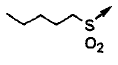 | 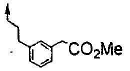 | | 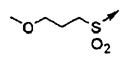 | 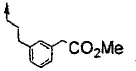 | | 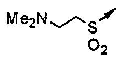 | 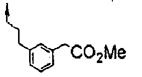 |
| 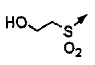 | 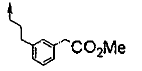 | | 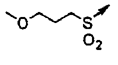 | 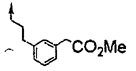 | | 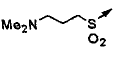 | 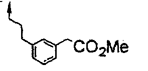 |

**Table 49**

| R¹-Y¹-X¹ | Z-A | | R¹-Y¹-X¹ | Z-A | | R¹-Y¹-X¹ | Z-A |
|---|---|---|---|---|---|---|---|
| 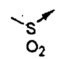 | 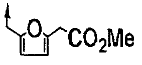 | | 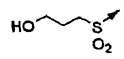 | 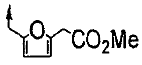 | | 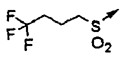 | 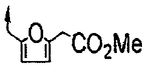 |
| 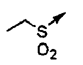 | 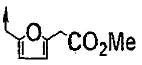 | | 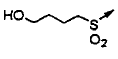 | 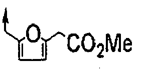 | | 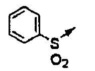 | 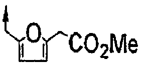 |
| 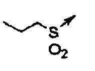 | 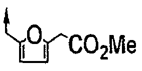 | | 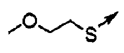 | 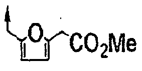 | | 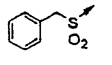 | 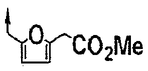 |
| 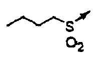 | 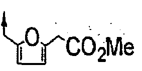 | | 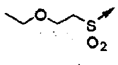 | 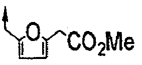 | | 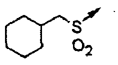 | 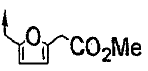 |
| 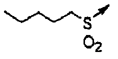 | 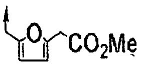 | | 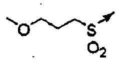 | 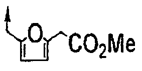 | | 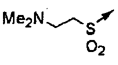 | 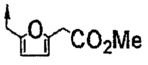 |
| 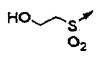 | 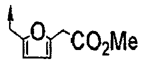 | | 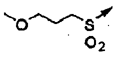 | 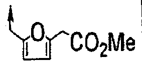 | | 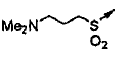 | 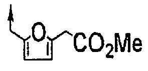 |

**Table 50**

| R¹-Y¹-X¹ | Z-A | | R¹-Y¹-X¹ | Z-A | | R¹-Y¹-X¹ | Z-A |
|---|---|---|---|---|---|---|---|
| 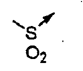 | 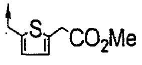 | | 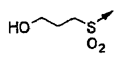 | 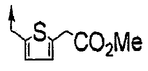 | | 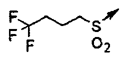 | 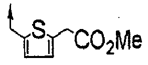 |
| 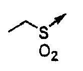 | 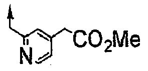 | | 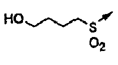 | 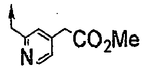 | | 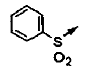 | 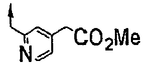 |
| 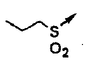 | 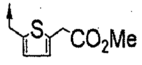 | | 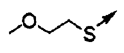 | 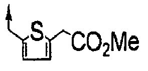 | | 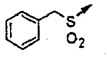 | 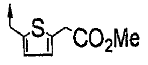 |
| 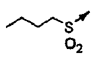 | 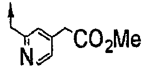 | | 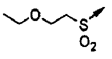 | 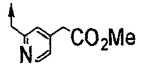 | | 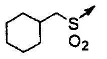 | 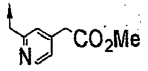 |
| 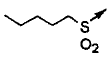 | 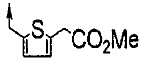 | | 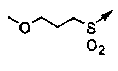 | 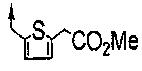 | | 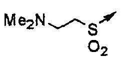 | 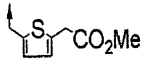 |
| 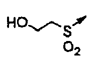 | 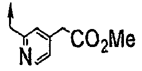 | | 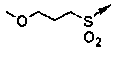 | 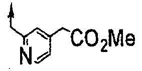 | | 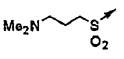 | 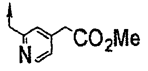 |

**Table 51**

| R¹-Y¹-X¹ | Z-A | | R¹-Y¹-X¹ | Z-A | | R¹-Y¹-X¹ | Z-A |
|---|---|---|---|---|---|---|---|
| 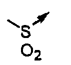 | 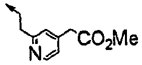 | | 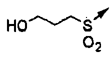 | 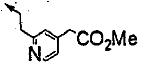 | | 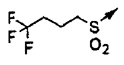 | 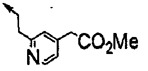 |
| 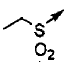 | 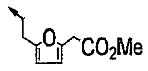 | | 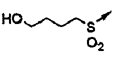 | 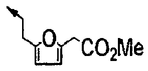 | | 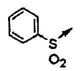 | 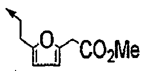 |
| 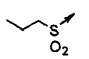 | 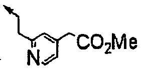 | | 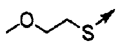 | 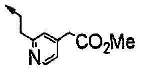 | | 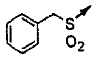 | 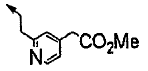 |
| 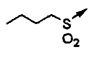 | 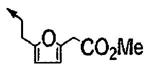 | | 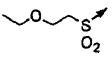 | 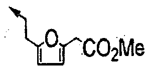 | | 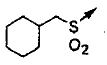 | 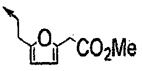 |
| 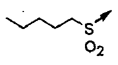 | 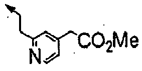 | | 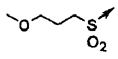 | 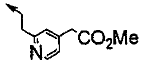 | | 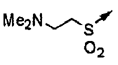 | 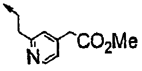 |
| 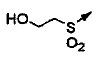 | 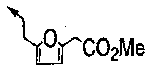 | | 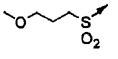 | 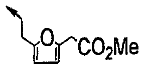 | | 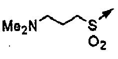 | 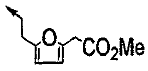 |

**Table 52**

| R¹-Y¹-X¹ | Z-A | | R¹-Y¹-X¹ | Z-A | | R¹-Y¹-X¹ | Z-A |
|---|---|---|---|---|---|---|---|
| 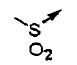 | 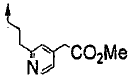 | | 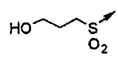 | 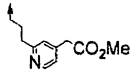 | | 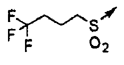 | 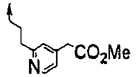 |
| 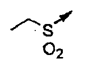 | 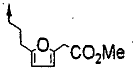 | | 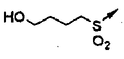 | 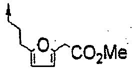 | | 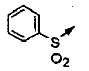 | 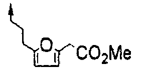 |
| 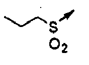 | 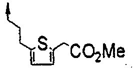 | | 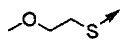 | 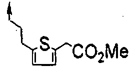 | | 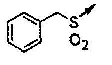 | 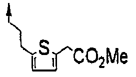 |
| 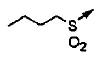 | 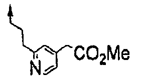 | | 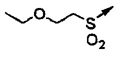 | 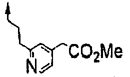 | | 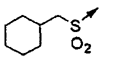 | 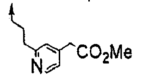 |
| 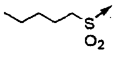 | 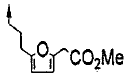 | | 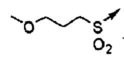 | 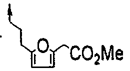 | | 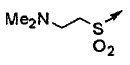 | 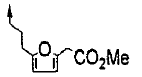 |
| 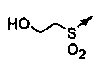 | 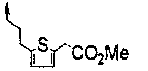 | | 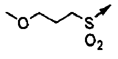 | 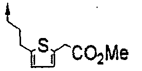 | | 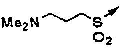 | 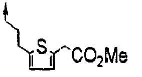 |

**Table 53**

| R¹-Y¹-X¹ | Z-A | | R¹-Y¹-X¹ | Z-A | | R¹-Y¹-X¹ | Z-A |
|---|---|---|---|---|---|---|---|
| 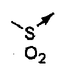 | 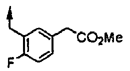 | | 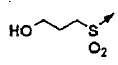 | 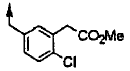 | | 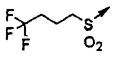 | 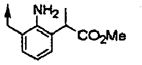 |
| 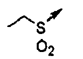 | 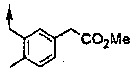 | | 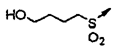 | 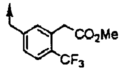 | | 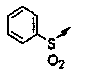 | 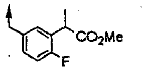 |
| 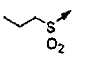 | 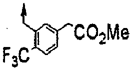 | | 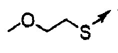 | 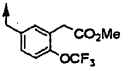 | | 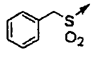 | 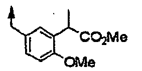 |
| 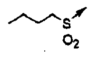 | 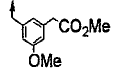 | | 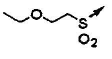 | 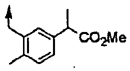 | | 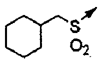 | 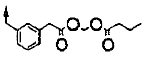 |
| 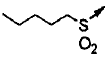 | 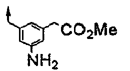 | | 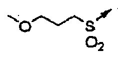 | 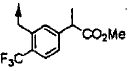 | | 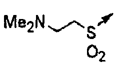 | 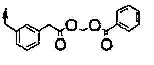 |
| 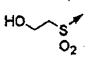 | 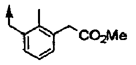 | | 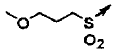 | 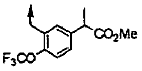 | | 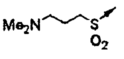 | 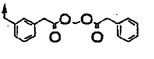 |

**Table 54**

| R¹-Y¹-X¹ | Z-A | | R¹-Y¹-X¹ | Z-A | | R¹-Y¹-X¹ | Z-A |
|---|---|---|---|---|---|---|---|
| 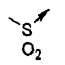 | 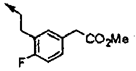 | | 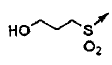 | 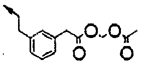 | | 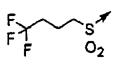 | 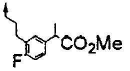 |
| 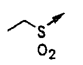 | 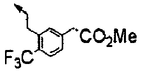 | | 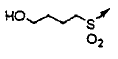 | 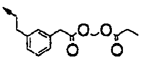 | | 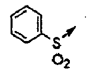 | 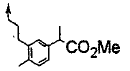 |
| 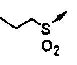 | 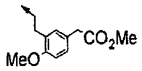 | | 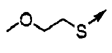 | 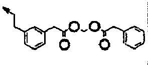 | | 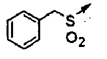 | 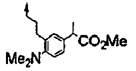 |
| 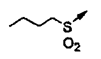 | 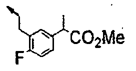 | | 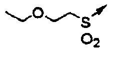 | 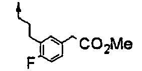 | | 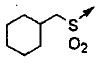 | 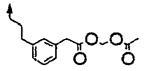 |
| 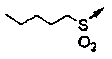 | 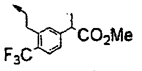 | | 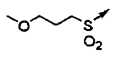 | 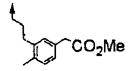 | | 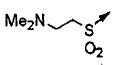 | 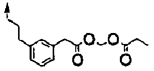 |
| 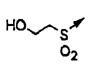 | 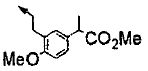 | | 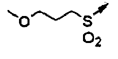 | 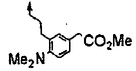 | | 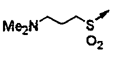 | 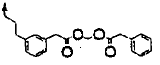 |

**Table 55**

| R¹-Y¹-X¹- | Z-A- | | R¹-Y¹-X¹- | Z-A- | | R¹-Y¹-X¹- | Z-A- |
|---|---|---|---|---|---|---|---|
| 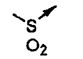 | 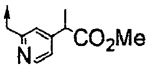 | | 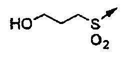 | 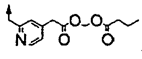 | | 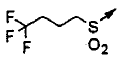 | 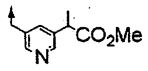 |
| 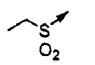 | 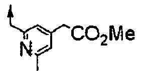 | | 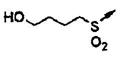 | 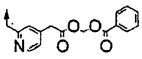 | | 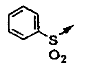 | 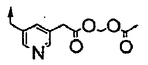 |
| 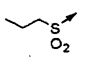 | 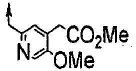 | | 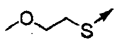 | 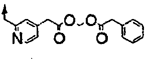 | | 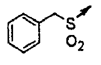 | 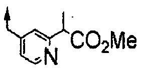 |
| 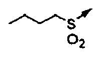 | 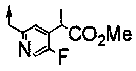 | | 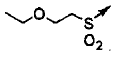 | 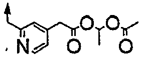 | | 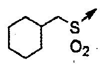 | 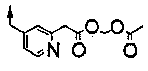 |
| 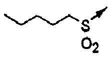 | 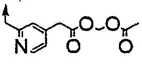 | | 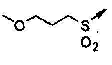 | 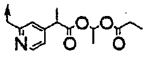 | | 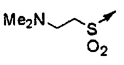 | 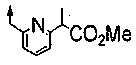 |
| 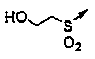 | 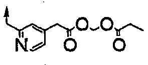 | | 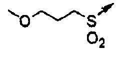 | 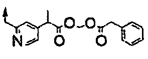 | | 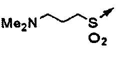 | 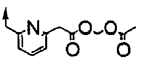 |

**Table 56**

| R¹-Y¹-X¹- | Z-A- | | R¹-Y¹-X¹- | Z-A- | | R¹-Y¹-X¹- | Z-A- |
|---|---|---|---|---|---|---|---|
| 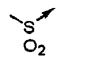 | 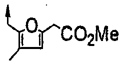 | | 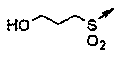 | 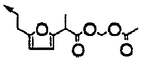 | | 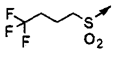 | 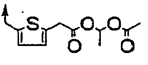 |
| 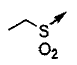 | 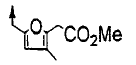 | | 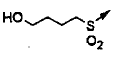 | 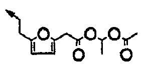 | | 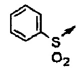 | 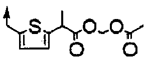 |
| 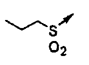 | 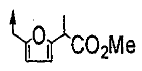 | | 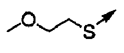 | 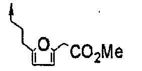 | | 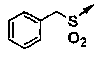 | 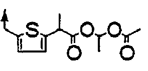 |
| 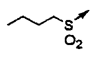 | 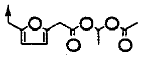 | | 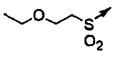 | 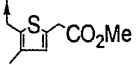 | | 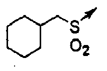 | 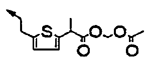 |
| 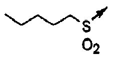 | 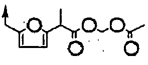 | | 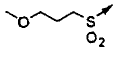 | 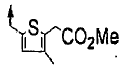 | | 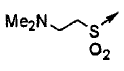 | 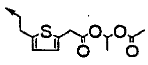 |
| 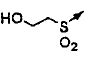 | 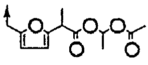 | | 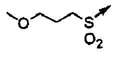 | 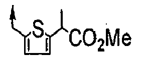 | | 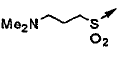 | 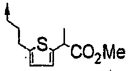 |

### Example

The present invention is further explained below in details referring to Examples, Comparison Examples and Reference Examples, but the present invention is not limited thereto. In the following examples, chemical structures are for convenience shown in a form of 8-hydroxy type and it is not differentiated from 8-oxo type.

### Example 1

### 8-Hydroxy-2-(3-hydroxypropylthio)-9-(3-methoxycarbonylmethylbenzyl)adenine

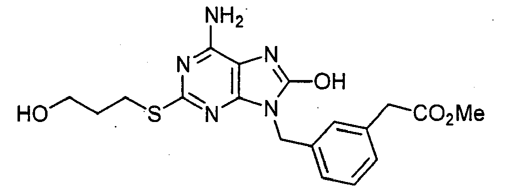

The titled compound was prepared by the same procedure as described in Reference example 4, as a white solid. Yield: 97%.
¹H NMR(DMSO-d₆)δ 10.11(1H, s), 7.22(4H, m), 6.58(2H, brs), 4.86(2H, s), 4.51(1H, t, J = 5.2 Hz), 3.65(2H, s), 3.59(3H, s), 3.48(2H, m), 3.05(2H, t. J = 6.9 Hz), 1.78(2H, m).

### Example 2

### 8-Hydroxy-2-(4-hydroxybutylthio)-9-(3-methoxycarbonylmethylbenzyl) adenine

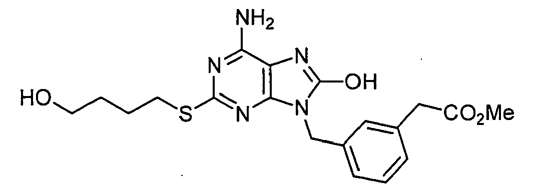

The titled compound as a white solid was prepared by the same procedure as described in Reference example 4. Yield: 24%.
¹H NMR(DMSO-d₆)δ 10.08(1H, s), 7.20(4H, m), 6.50(2H, brs), 4.85(2H, s), 4.38(1H, t, J = 5.1 Hz), 3.64(2H, s), 3.58(3H, s), 3.37(2H, m), 3.01(2H, t, J = 6.8 Hz), 1.64(2H, m), 1.50(2H, m).

### Example 3

### 8-Hydroxy-2-(2-methoxyethylthio)-9-(3-methoxycarbonylmethylbenzyl) adenine

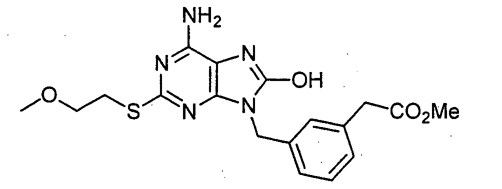

The titled compound as a white solid was prepared by the same procedure as described in Reference example 4. Yield: 84%.
¹H NMR(DMSO-d₆)δ 10.12(1H, s), 7.21(4H, m), 6.56(2H, brs), 4.86(2H, s), 3.66(2H, s), 3.59(3H, s), 3.52(2H, t, J = 6.6 Hz), 3.22(3H, s), 3.20(2H, t, J = 6.6 Hz).

### Example 4

### 8-Hydroxy-2-(3-hydroxypropoxy)-9-(3-methoxycarbonylmethylbenzyl) adenine

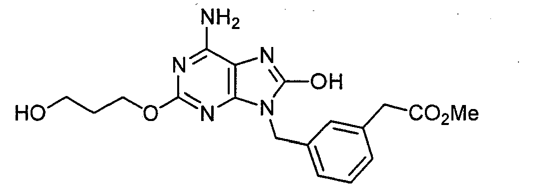

8-Bromo-2-(3-hydroxypropoxy)-9-(3-methoxycarbonylmethylbenzyl)adenine (0.43g, 0.96mmol) which was prepared in Reference example 5 was suspended in a mixture of an aqueous 5N sodium hydroxide solution (8ml) and methanol (5ml) and the mixture was stirred at 100°C for 9 hours. After neutralizing with 12N hydrochloric acid and concentrating, thereto were added methanol (30ml) and concentrated sulfuric acid (3ml). After refluxing for 5 hours the mixture was neutralized with an aqueous saturated sodium bicarbonate solution, extracted with chloroform, dried over anhydrous magnesium sulfate and concentrated. The residue was purified by column chromatography (SiO₂ 50g, elute: CHCl₃/MeOH=100/0~20/1) to give the titled compound as a white solid (0.29g, 2.41mmol). Yield: 78%.
¹H NMR(DMSO-d₆)δ9.96(1H, brs), 7.27(1H, t, J = 7.6 Hz), 7.20(1H, s), 7.16(2H, m), 6.46(2H, brs), 4.83(2H, s), 4.49(1H, t, J = 5.1 Hz), 4.19(2H, t, J = 6.5 Hz), 3.65(2H, s), 3.59(3H, s), 3.50(2H, q, J = 6.2 Hz), 1.79(2H, qui, J=6.4Hz).

### Example 5

### 8-Hydroxy-2-(2-hydroxyethoxy)-9-(3-methoxycarbonylmethylbenzyl)adenine

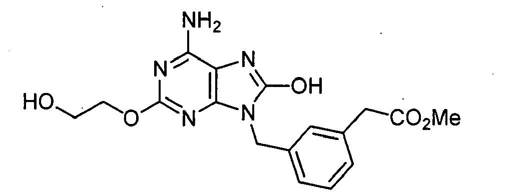

The titled compound as a white solid was prepared by the same procedure as described in Example 4. Yield: 83%.
¹H NMR(DMSO-d₆)δ 9.97(1H, s), 7.27(1H, t, J = 7.6 Hz), 7.20(3H, m), 6.47(2H, s), 4.83(2H, s), 4.79(1H, t, J = 5.6 Hz), 4.15(2H, t, J = 4.9 Hz), 3.64(4H, m), 3.59(3H, s).

### Example 6

### 8-Hydroxy-2-(4-hydroxybutoxy)-9-(3-methoxycarbonylmethylbenzyl) adenine

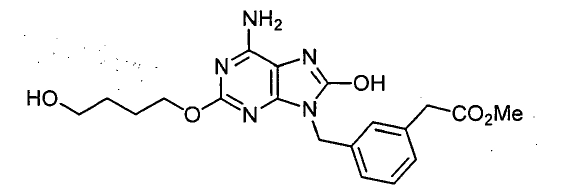

Using 2-chloro-9-(3-methoxycarbonylmethylbenzyl)adenine which was prepared in Reference example 1, the procedures of Reference example 4, Reference example 2 and Example 4 were carried out in this order to give the titled compound as a white solid. Yield: 21%.
¹H NMR(DMSO-d₆)δ 9.96(1H, brs), 7.27(1H, t, J = 7.6 Hz), 7.20(3H, m), 6.45(2H, m), 4.83(2H, s), 4.42(1H, t, J = 5.2 Hz), 4.14(2H, t, J = 6.6 Hz), 3.65(2H, s), 3.58(3H, s), 3.41(2H, q, J = 6.4 Hz), 1.67(2H, qui, J = 6.7 Hz), 1.49(2H, qui, J = 6.7 Hz).

### Example 7

### 8-Hydroxy-9-(3-methoxycarbonylmethylbenzyl)-2-(4,4,4-trifluorobutoxy)adenine

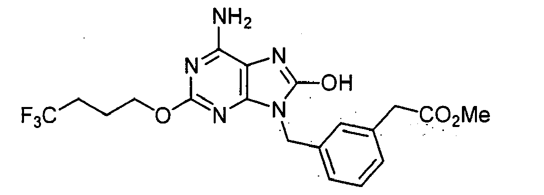

The titled compound was prepared by the same procedure as described in Example 4, as a white solid. Yield: 82%.
¹H NMR(DMSO-d₆)δ 9.97(1H, brs), 7.27(1H, t, J = 7.6 Hz), 7.20(1H, s), 7.16(2H, m), 6.49(2H, brs), 4.84(2H, s), 4.20(2H, t, J = 6.3 Hz), 3.64(2H, s), 3.58(3H, s), 2.35(2H, m), 1.88(2H, m).

### Example 8

### 8-Hydroxy-9-(3-methoxycarbonylmethylbenzyl)-2-[N-(2-methoxyethyl) amino] adenine

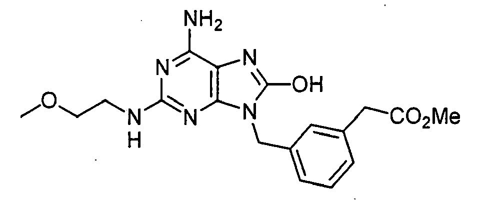

The titled compound as a white solid was prepared by the same procedure as described in Example 4. Yield: 54%.
¹H NMR(DMSO-d₆)δ 9.65(1H, s), 7.26(1H, t, J = 7.6 Hz), 7.18(3H, m), 6.15(1H, t, J = 5.5 Hz), 6.05(2H, brs), 4.78(2H, s), 3.64(2H, s), 3.59(3H, s), 3.37(4H, m), 3.22(3H, s).

### Example 9

### 2-Butoxy-8-hydroxy-9-[2-(3-methoxycarbonylmethylphenyl)ethyl]adenine

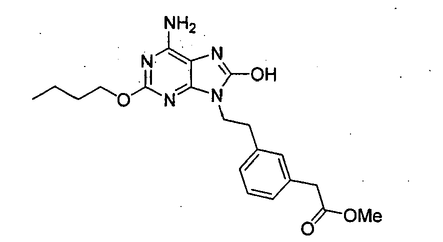

The titled compound was prepared by the same procedure as described in Example 4. Yield: 84%.
¹H NMR(DMSO-d₆)δ 9.85(1H, brs), 7.24-7.20(1H, m), 7.10(1H, s), 7.10-7.08(2H, m), 6.41(2H, brs), 4.14(2H, t, J = 6.6 Hz), 3.88(2H, t, J = 7.6 Hz), 3.62(2H, s), 3.59(3H, s), 2.96(2H, t, J = 7.6 Hz), 1.65(2H, tt, J = 7.5 Hz, 6.6 Hz), 1.39(2H, tq, J = 7.5 Hz, 7.4 Hz), 0.92(3H, t, J = 7.4 Hz).

### Example 10

### 2-Butoxy-8-hydroxy-9-[3-(3-methoxycarbonylmethylphenyl)propyl]adenine

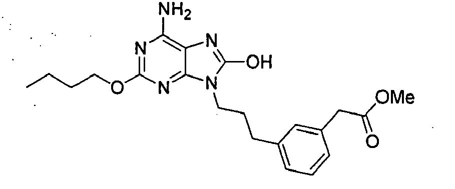

Using 8-bromo-2-butoxy-9-[3-(3-cyanomethylphenyl)propyl]adenine which is prepared in Reference example 29, the same procedure as in Example 4 was carried out to give the titled compound. Yield: 88%.
¹H NMR(DMSO-d₆)δ 9.84(1H, brs), 7.21(1H, dd, J = 7.5, 7.5 Hz), 7.12-7.05(3H, m), 6.40(2H, brs), 4.31(2H, t, J = 6.6 Hz), 3.70(2H, t, J = 7.0 Hz), 3.62(2H, s), 3.59(3H, s), 2.57(2H, t, J = 7.7 Hz), 1.94(2H, tt, J = 7.7 Hz, 7.0 Hz), 1.63(2H, tt, J = 7.8 Hz, 6.6 Hz), 1.37(2H, tq, J = 7.8 Hz, 7.4 Hz), 0.91(3H,t,J=7.4Hz).

### Example 11

### 2-(2,3-Dihydroxy-1-propoxy)-8-hydroxy-9-(3-methoxycarbonylmethylbenzyl)adenine

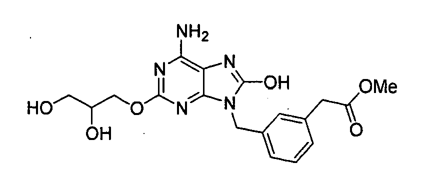

The titled compound was prepared by the same procedure as described in Example 4. Yield: 46%.
¹H NMR(DMSO-d₆)δ 9.96(1H, brs), 7.27(1H, dd, J = 7.6, 7.5 Hz), 7.20-7.14(3H, m), 6.47(2H, brs), 4.87(1H, d, J = 5.2 Hz), 4.84(2H, s), 4.61(1H, t, J = 5.6 Hz), 4.16(1H, dd, J = 10.9, 4.4 Hz), 4.03(1H, dd, J = 10.9, 6.4 Hz), 3.76-3.72(1H, m), 3.65(2H, s), 3.59(3H, s), 3.39(2H, dd, J = 5.6, 5.6 Hz).

### Example 12

### 2-(2-Ethoxyethoxy)-8-hydroxy-9-(3-methoxycarbonylmethylbenzyl)adenine

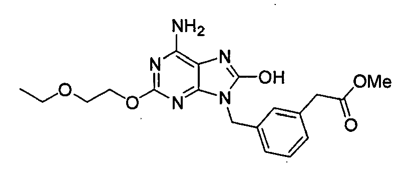

The titled compound was prepared by the same procedure as described in Example 4. Yield: 79%.
¹H NMR(DMSO-d₆)δ 9.97(1H, s), 7.27(1H, dd, J = 7.6, 7.5 Hz), 7.20-7.14(3H, m), 6.47(2H, brs), 4.83(2H, s), 4.24(2H, t, J = 4.8 Hz), 3.65(2H, s), 3.61(2H, t, J = 4.8 Hz), 3.58(3H, s), 3.45(2H, q, J = 7.0 Hz), 1.10(3H, t, J = 7.0 Hz).

### Example 13

### 2-Cyclohexylmethoxy-8-hydroxy-9-(3-methoxycarbonylmethylbenzyl)adenine

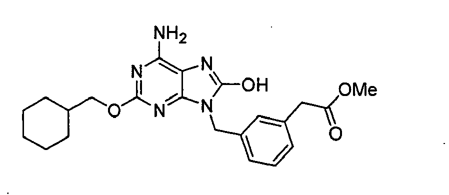

The titled compound was prepared by the same procedure as described in Example 4. Yield: 85%.
¹H NMR(DMSO-d₆)δ 9.94(1H, s), 7.27(1H, dd, J = 7.7, 7.5 Hz), 7.20-7.15(3H, m), 6.45(2H, brs), 4.83(2H, s), 3.95(2H, d, J = 6.4 Hz), 3.64(2H, s), 3.58(3H, s), 1.75-1.61(6H, m), 1.23-1.11(3H, m), 0.99-0.93(2H, m).

### Example 14

### 2-Benzyloxy-8-hydroxy-9-(3-methoxycarbonylmethylbenzyl)adenine

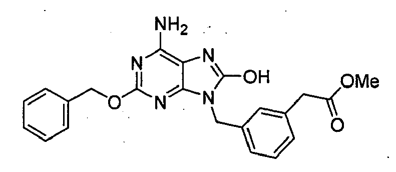

The titled compound was prepared by the same procedure as described in Example 4. Yield: 59%
¹H NMR(DMSO-d₆)δ 10.01(1H, brs), 7.42-7.40(2H, m), 7.36-7.16(5H, m), 7.16-7.14(2H, m), 6.53(2H, brs), 5.24(2H, s), 4.83(2H, s), 3.62(2H, s), 3.57(3H, s).

### Example 15

### 8-Hydroxy-2-(2-methoxycarbonylethyl)-9-(3-methoxycarbonylmethylbenzyl)adenine

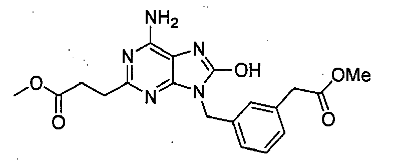

The titled compound was prepared by the same procedure as described in Example 4. Yield: 50%
¹H NMR(DMSO-d₆)δ 10.15(1H, brs), 7.26(1H, dd, J = 7.6, 7.6 Hz), 7.23(1H, s), 7.18-7.14(2H, m), 6.39(2H, brs), 4.85(2H, s), 3.65(2H, s), 3.59(3H, s), 3.53(3H, s), 2.87(2H, t, J = 7.2 Hz), 2.70(2H, t, J = 7.2 Hz).

### Example 16

### 2-Butoxy-8-hydroxy-9-{(5-methoxycarbonylmethyl-2-thienyl)methyl}adenine

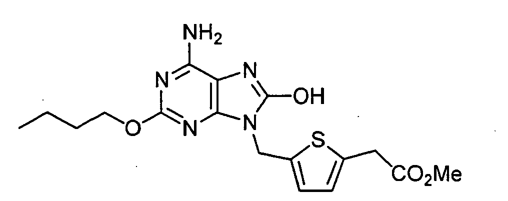

Using 2-butoxy-8-hydroxy-9-{(5-hydroxymethyl-2-thienyl)methyl}adenine which was prepared in Reference example 44, the procedures of Reference example 18, Reference example 19 and Reference example 20 were carried out in this order to give the titled compound as a white solid. Yield: 49%
¹H NMR(DMSO-d₆)δ 9.95(1H, s), 6.90(1H, d, J = 3.5 Hz), 6.78(1H; d, J = 3.5 Hz), 6.46(2H, brs), 4.94(2H, s), 4.17(2H, t, J = 6.6 Hz), 3.85(2H, s), 3.61(3H, s), 1.65(2H, 5, J = 6.6 Hz), 1.38(2H, 6, J = 7.4 Hz), 0.92(3H, t, J = 7.3Hz).

### Example 17

### 2-Butoxy-8-hydroxy-9-{(3-methoxycarbonylmethyl-4-pyridyl)methyl}adenine

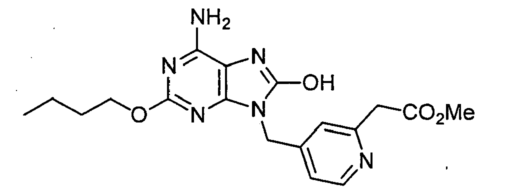

The titled compound as a white solid was prepared by the same procedure as described in Example 16. Yield: 19%
¹H NMR(DMSO-d₆)δ 10.03(1H, brs), 8.42(1H, d, J = 5.0 Hz), 7.20(1H, s), 7.12(1H, dd, J = 1.4 Hz, 5.1 Hz), 6.52(2H, brs), 4.88(2H, s), 4.10(2H, t, J = 6.6 Hz), 3.82(2H, s), 3.59(3H, s), 1.59(2H, 5, J = 6.6 Hz), 1.35(2H, 6, J = 7.3 Hz), 0.88(3H, t, J = 7.3 Hz).

### Example 18

### 2-Butoxy-8-hydroxy-9-{(6-methoxycarbonylmethyl-2-pyridyl)methyl}adenine

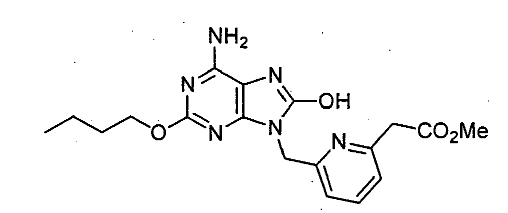

The titled compound as a white solid was prepared by the same procedure as described in Reference example 20. Yield: 34%.
¹H NMR(DMSO-d₆)δ 9.99(1H, brs), 7.70(1H, t, J = 7.8 Hz), 7.24(1H, d, J = 7.6 Hz), 6.96(1H, d, J = 7.7 Hz), 6.47(2H, brs), 4.91(2H, s), 4.14(2H, t, J = 6.6 Hz), 3.81(2H, s), 3.58(3H, s), 1.57(2H, 5, J = 6.6 Hz), 1.37(2H, 6, J = 7.4 Hz), 0.85(3H, t, J = 7.3 Hz).

### Example 19

### 2-Butoxy-8-hydroxy-9-{(4-methoxycarbonylmethyl-2-pyridyl)methyl}adenine

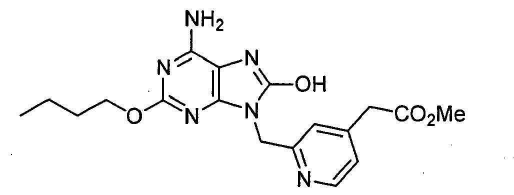

Using 8-bromo-2-butoxyadenine (525mg, 1.83mmol) which was prepared in Reference example 54, alkylation was carried out in the same manner as described in Reference example 1, and then the same procedure as described in Reference example 3 was carried out to give the titled compound as a white solid. Yield 14%
¹H NMR(DMSO-d₆)δ 9.94(1H, s), 8.39(1H, d, J = 5.0 Hz), 7.18(1H, d, J = 5.0 Hz), 7.14(1H, s), 6.45(2H, brs), 4.94(2H, s), 4.07(2H, t, J = 6.6 Hz), 3.73(2H, s), 3.60(3H, s), 1.57(2H, 5, J = 6.6 Hz), 1.33(2H, 6, J = 6.8 Hz), 0.87(3H, t, J = 7.3 Hz).

### Example 20

### 2-Butoxy-8-hydroxy-9-[(2-methoxy-5-methoxycarbonylmethyl) benzyl] adenine

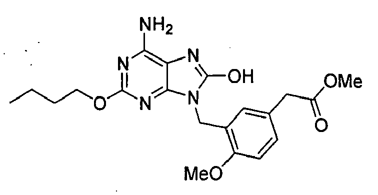

The titled compound as a white solid was prepared by the same procedure as described in Example 10. Yield: 93%
¹H NMR(DMSO-d₆)δ 10.00(1H, brs), 7.13(1H, d, J = 8.4 Hz), 6.97(1H, d, J = 8.4 Hz), 6.67(1H, s), 6.47(2H, brs), 4.80(2H, s), 4.08(2H, t, J = 6.6 Hz), 3.83(3H, s), 3.53(3H, s), 3.50(2H, s), 1.59(2H, tt, J = 7.5 Hz, 6.6 Hz), 1.33(2H, tq, J = 7.5 Hz, 7.4 Hz), 0.87(3H, t, J = 7.4 Hz).

### Example 21

### 2-Butoxy-9-[(4-fluoro-3-methoxycarbonylmethyl)benzyl]-8-hydroxyadenine

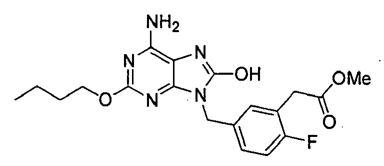

The titled compound as a white solid was prepared by the same procedure as described in Example 10. Yield: 93%
¹H NMR(DMSO-d₆)δ 9.96(1H, s), 7.29-7.23(2H, m), 7.14(1H, dd, J = 9.7, 8.4 Hz), 6.46(2H, brs), 4.82(2H, s), 4.14(2H, t, J = 6.6 Hz), 3.70(2H, s), 3.60(3H, s), 1.62(2H, tt, J = 7.5 Hz, 6.6 Hz), 1.37(2H, tq, J = 7.5 Hz, 7.4 Hz), 0.90(3H, t, J = 7.4 Hz).

### Example 22

### 2-Butoxy-8-hydroxy-9-[(4-methoxy-3-methoxycarbonylmethyl)benzyl]adenine

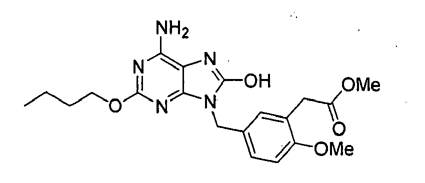

Using methyl 3-bromomethyl-6-methoxyphenylacetate which was prepared in Reference example 75 and 2-butoxyadenine, the procedures of Reference example 1, Reference example 2 and Example 4 were carried out in this order to give the titled compound as a white solid.
¹H NMR(CDCl₃)δ 9.91(1H, s), 7.20(1H, d, J = 8.4 Hz), 7.15(1H, s), 6.91(1H, d, J = 8.4 Hz), 6.42(2H, brs), 4.75(2H, s), 4.15(2H, t, J = 6.4 Hz), 3.70(3H, s), 3.55(3H, s),3.53(2H, s), 1.62(2H, 5, J = 6.8 Hz), 1.37(2H, 6, J = 7.5 Hz), 0.90(3H, t, J = 7.3 Hz).

### Example 23

### Interferon inducing activity in spleen cells (in vitro)

Spleen was removed from CD(SD)IGS rat (male; 8-10 weeks old). A suspension of spleen cells of 1x10⁷ cells/ml was prepared by using non serum MEN broth, and each 0.1ml thereof was poured in each well of 96-well microplate. The test sample diluted with the same broth (containing 0.2% DMSO) in each 0.1ml was poured in the well and incubated in 5% CO₂ incubator at 37°C for 24 hours. The culture broth was centrifuged to give a supernatant of the incubation. The interferon activity in the supernatant of the broth was quantitatively measured by the partially-improved bioassay method described in A. Armstrong, Methods in Enzymology 78, 381-7. Namely after mouse fibroblast L929 in 4 x 10⁴ cells/50µl was cultured in a 96-well culture plate for 7 hours, thereto was added 50µl of the diluted culture supernatant and the mixture was further cultured for 17 hours. After the cultured broth in each well was removed, each 100µl of vesicular stomatitis virus was added to each well and the effect of the cell denaturation 44 hours after the virus infection was confirmed by the neutral red stain. In Table 57, an interferon inducing activity (minimum effective concentration) on each compound was shown.

**Table 57**

| Compound | Minimum effective concentration (nM) | Compound | Minimum effective concentration (nM) |
|---|---|---|---|
| Example 1 | 10 | Comparative example 1 | > 1000 |
| Example 2 | 10 | Comparative example 2 | 1000 |
| Example 4 | 30 | Comparative example 4 | > 1000 |
| Example 5 | 100 | Comparative example 5 | > 1000 |
| Example 9 | 30 | Comparative example 9 | 300 |
| Example 10 | 10 | Comparative example 10 | 100 |
| Example 11 | 100 | Example 11 | >1000 |
| Example 12 | 10 | Example 12 | >1000 |
| Example 13 | 30 | Example 13 | 100 |
| Example 15 | 3 | Example 15 | 1000 |
| Example 16 | 1 | Example 16 | 3 |
| Example 18 | 3 | Example 18 | 10 |
| Example 19 | 3 | Example 19 | 30 |
| Example 20 | 0.1 | Example 20 | 10 |
| Example 21 | 1 | Example 21 | 30 |
| Example 22 | 3 | Example 22 | 10 |

### Example 24

### Metabolic stability test using human plasma

Plasma was prepared from fresh human blood and the test compound (containing 1% DMSO) of the final concentration of 1µM was added thereto.

After a metabolic reaction by plasma esterase was conducted at 37°C for 15 minutes, the test compound was extracted with ethyl acetate, and quantitatively analyzed by reverse phase HPLC. The metabolic stability of the test compound was shown by the residual amount (%) per the concentration of pre-metabolization as 100%. The result was shown in Table 58.

**Table 58**

| Compound | Residual rate (%) |
|---|---|
| Example 1 | <1 |
| Example 2 | < 1 |
| Example 4 | 3.2 |
| Example 5 | 5.8 |
| Example 9 | 7.9 |
| Example 10 | < 1 |
| Example 11 | 20.9 |

### Example 25

### Metabolic stability test on rat liver S9

The reaction using liver S9 of rat was conducted on a 96-well plate by using a screening robot by Tecan Company. S9 solution was prepared by adding 250mM Kpi (pH 7.4) 20ml and deionized water 20ml to 10ml of liver S9 of rat, a Cofactor solution was prepared by dissolving NADPH 220mg in deionized water 40.5ml (Final 6mM), and IS (Internal Standard) solution was prepared by adding IS solution (1mM DMSO solution) 300µl to acetonitrile 30ml (100 times dilution). The test compound (1µM DMSO solution) was dissolved in an incubator at 37°C. After each 35µL was poured in a 96-well plate (24 samples/plate), plates (sample plates, 96-well plates for dilution, each Deep well plates for the reaction and the recovery, plates for extraction of a solid phase) and reagents (S9 solution, Cofactor solution, IS (Internal Standard) solution, Stop solution, acetonitrile for elution) were set to the specified position in the booth of the robot, and the reaction started (the concentration of the test compounds was 1µM). Incubation was conducted under shaking at 37°C, the solid phase was extracted (at the same time, the internal standard for analysis was added). To the recovered samples 200µL/well was added 50µL of acetonitrile per each well, and to 2 plates of FALCON Deep well were poured 100µL/well of the solution per well. By subjecting to the LC/MS analysis, the chromatography of the test compound and the internal standard were described and the peak area was calculated. And then, the stability (residual rate after reaction) was calculated. The result was shown in Table 59.

**Table 59**

| Compound | Residual rate (%) |
|---|---|
| Example 4 | 8 |
| Example 9 | 0 |
| Example 10 | 1 |
| Example 12 | 0 |
| Example 13 | 11 |
| Example 15 | 0 |
| Example 16 | 0 |
| Example 17 | 3 |
| Example 18 | 0 |
| Example 19 | 0 |
| Example 20 | 0 |
| Example 21 | 1 |
| Example 22 | 2 |

### Example 26

### 2-Butylthio-8-hydroxy-9-(3-methoxycarbonylmethylbenzyl)adenine

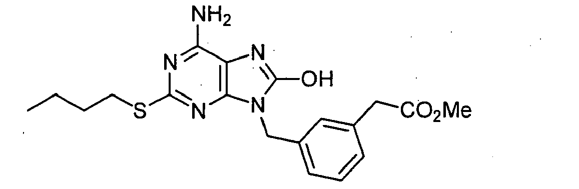

Using 2-chloro-8-hydroxy-9-(3-methoxycarbonylmethylbenzyl)adenine which was prepared in Reference example 3, the same procedure as in Reference example 4 was carried out to give the titled compound as a white solid. Yield: 67%.
¹H NMR(DMSO-d₆)δ 10.10(1H, s), 7.21(4H, m), 6.51(2H, brs), 4.86(2H, s), 3.64(2H, s), 3.59(3H, s), 3.01(2H, t, J = 7.2 Hz), 1.59(2H, m), 1.36(2H, m), 0.86(3H, t, J = 7.4 Hz).

### Example 27

### 2-[3-(Ethylsulfonyl)propoxy]-8-hydroxy-9-(3-methoxycarbonylmethylbenzyl)adenine

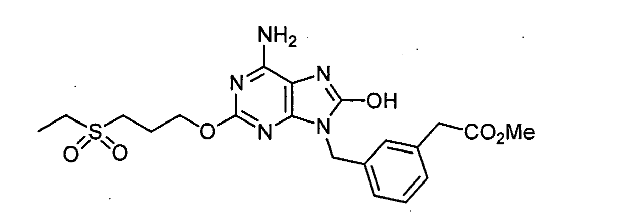

Using 2-[3-(ethylsulfonyl)propoxy]-9-(3-methoxycarbonylmethylbenzyl)adenine which was prepared in Reference example 111, the same procedures as in Reference example 2 and Reference example 3 were carried out in this order to give the titled compound as a white solid. Yield: 71%.
¹H NMR(DMSO-d₆)δ 9.99(1H, s), 7.21(4H, m), 6.51(2H, brs), 4.84(2H, s), 4.26(2H, t, J = 6.2 Hz), 3.66(2H, s), 3.59(3H, s), 3.18(2H, m), 3.13(2H, q, J = 7.4 Hz), 2.09(2H, m), 1.21(3H, t, J = 7.4 Hz).

### Example 28

### 8-Hydroxy-9-(3-methoxycarbonylmethylbenzyl)-2-[3-(methylsulfonyl)propoxy]adenine

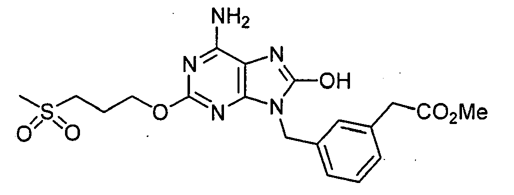

Using 2-chloro-9-(3-methoxycarbonylmethylbenzyl)adenine which was prepared in Reference example 1, the same procedure as in Example 27 was carried out to give the titled compound as a white solid. Yield: 14%
¹H NMR(DMSO-d₆)δ 9.98(1H, s), 7.21(4H, m), 6.43(2H, brs), 4.84(2H, s), 4.25(2H, t, J = 6.2 Hz), 3.65(2H, s), 3.59(3H, s), 3.22(2H, m), 3.00(3H, s), 2.09(2H, m).

### Example 29

### 8-Hydroxy-9-(3-methoxycarbonylmethylbenzyl)-2-(4-pyridylmethylamino)adenine

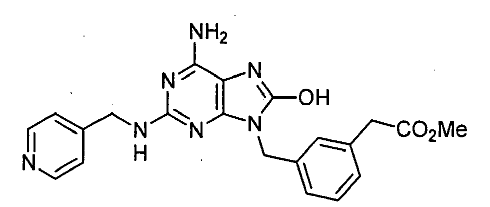

Using 2-chloro-8-hydroxy-9-(3-methoxycarbonylmethylbenzyl)adenine which was prepared in Reference example 3, the same procedure as in Reference example 11 was carried out to give the titled compound as a white solid. Yield: 8%
¹H NMR(DMSO-d₆)δ 9.70(1H, s), 8.42(2H, d, J = 4.5 Hz), 7.27(2H, d, J = 4.5 Hz), 7.18(4H, m), 6.98(1H, t, J = 6.3 Hz), 6.10(2H, brs), 4.75(2H, s), 4.41(2H, d, J = 6.3 Hz), 4.25(2H, t, J = 6.2 Hz).

### Example 30

### 8-Hydroxy-9-(3-methoxycarbonylmethylbenzyl)-2-[2-methoxyethyl(N-methyl)amino]adenine

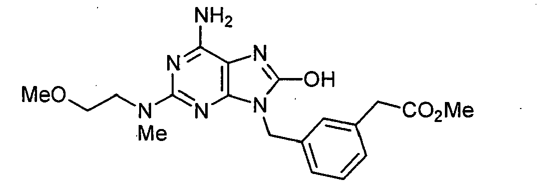

Using 9-(3-methoxycarbonylmethylbenzyl)-2-[2-methoxyethyl(N-methyl)amino]adenine which was prepared in Reference example 113, the same procedures as in Reference example 2 and Reference example 3 were carried out in this order to give the titled compound as a white solid.
Yield: 41 %
¹H NMR(DMSO-d₆)δ 9.67(1H, s), 7.23(4H, m), 6.09(2H, brs), 4.79(2H, s), 3.66(2H, s), 3.65(2H, d, J = 6.2 Hz), 3.59(3H, s), 3.46(2H, t, J = 6.2 Hz), 3.22(3H, s), 3.02(3H, s).

### Example 31

### 2-Benzylamino-8-hydroxy-9-(3-methoxycarbonylmethylbenzyl)adenine

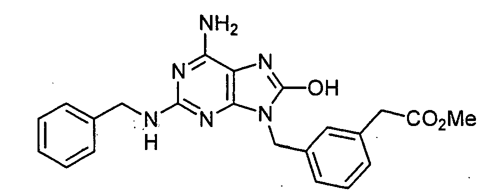

Using 9-(3-carboxylmethylbenzyl)-2-chloroadenine which was prepared in Reference example 112, the same procedures as in Reference example 75, Reference example 2 and Reference example 3 were conducted in this order to give the titled compound as a white solid. Yield: 3%
¹H NMR(DMSO-d₆)δ 9.66(1H, s), 7.22(4H, m), 6.82(1H, t, J = 6.4 Hz), 6.04(2H,brs), 4.77(2H, s), 4.39(2H, d, J = 6.4 Hz), 3.61(2H, s), 3.58(3H, s).

### Example 32

### 8-Hydroxy-9-(3-methoxycarbonylmethylbenzyl)-2-[(4-pyridylmethyl) oxy] adenine

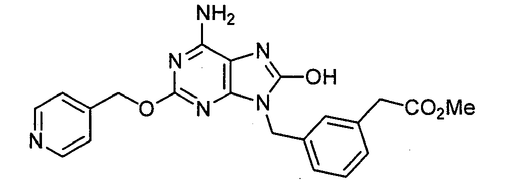

2-Chloro-9-(3-methoxycarbonylmethylbenzyl)adenine which was prepared in Reference example 1, the same procedure as in Reference example 4, Reference example 2 and Reference example 3 were carried out in this order to give the titled compound as a white solid. Yield: 31%
¹H NMR(DMSO-d₆)δ 10.03(1H, s), 8.52(2H, d, J = 4.5 Hz), 7.36(2H, d, J = 4.5 Hz), 7.20(4H, m), 6.56(2H, brs), 5.31(2H, s), 4.83(2H, s), 3.63(2H, s), 3.57(3H, s).

### Example 33

### 2-Ethoxy-8-hydroxy-9-(3-methoxycarbonylmethylbenzyl)adenine

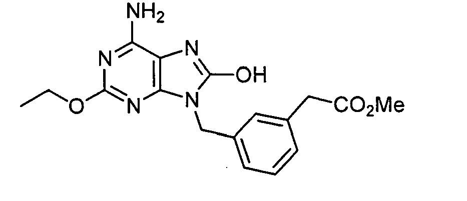

Using 2-chloro-9-(3-methoxycarbonylmethylbenzyl)adenine which was prepared in Reference example 1, the same procedures as in Reference example 4, Reference example 2 and Reference example 3 were carried out in this order to give the titled compound as a white solid. Yield 56%
¹H NMR(DMSO-d₆)δ 9.96(1H, s), 7.28(1H, t, J = 7.5 Hz), 7.21-7.15(3H, m), 6.46(2H, brs), 4.83(2H, s), 4.19(2H, q, J = 7.0Hz), 3.65(2H, s), 3.59(3H, s), 1.25(3H, t, J = 7.0Hz).

### Example 34

### 8-Hydroxy-9-(3-methoxycarbonylmethylbenzyl)-2-propoxyadenine

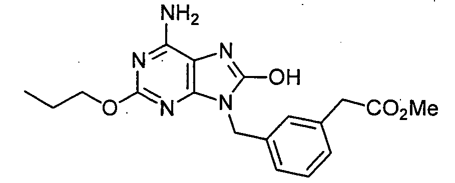

Using 2-chloro-9-(3-methoxycarbonylmethylbenzyl)adenine which was prepared in Reference example 1, the same procedures as in Reference example 4, Reference example 2 and Reference example 3 were conducted in this order to give the titled compound as a white solid. Yield: 57%
¹H NMR(DMSO-d₆)δ 9.96(1H, s), 7.29(1H, t, J = 7.5 Hz), 7.21-7.15(3H, m), 6.47(2H, brs), 4.84(2H, s), 4.09(2H, t, J = 6.7Hz), 3.65(2H, s), 3.59(3H, s), 1.70-1.61(2H, m), 0.93(3H, t, J = 7.4Hz).

### Example 35

### 8-Hydroxy-9-(3-methoxycarbonylmethylbenzyl)-2-pentoxyadenine

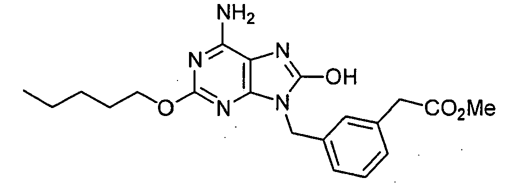

Using 2-chloro-9-(3-methoxycarbonylmethylbenzyl)adenine which was prepared in Reference example 1, the same procedures as in Reference example 4, Reference example 2 and Reference example 3 were conducted in this order to give the titled compound as a white solid. Yield: 33%
¹H NMR(DMSO-d₆)δ 9.96(1H, s), 7.28(1H, t, J = 7.5 Hz), 7.21-7.15(3H, m), 6.46(2H, brs), 4.83(2H, s), 4.13(2H, t, J = 6.6Hz), 3.65(2H, s), 3.59(3H, s), 1.66-1.62(2H, m), 1.34-1.29(2H, m), 0.88(3H, t, J = 7.0Hz).

### Example 36

### 2-Butoxy-8-hydroxy-9-{3-[(4-dimethylaminobutoxy)carbonylmethyl]benzyl}adenine

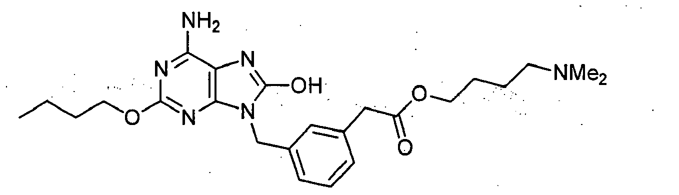

2-Butoxy-8-hydroxy-9-(3-carboxymethylbenzyl)adenine (88mg, 0.24mmol)which was prepared in Reference example 76 was suspended in DMF (10ml). Thereto were added at 0°C 4-dimethylaminobutanol (0.16 ml, 1.18mmol), 1-hydroxybenzotriazole (0.16g, 1.18mmol) and 1-(3-dimethylaminopropyl)-3-ethylcarbodiimide hydrochloride (0.23g, 1.18 mmol) in this order and the mixture was stirred at room temperature for 6 hours. After removal of the solvent by an evaporator, an aqueous saturated sodium bicarbonate solution was added to this residue and the resulted precipitate was filtered, followed by washing with water to give the titled compound as a white solid (73mg, 0.16mmol). Yield: 65%
¹H NMR(DMSO-d₆)δ 9.97(1H, brs), 7.20(4H, m), 6.45(2H, brs), 4.82(2H, s), 4.14(2H, t, J = 6.6 Hz), 4.00(2H, t, J = 6.6 Hz), 3.62(2H, s), 2.11(2H, t, J = 7.0 Hz), 2.04(6H, s), 1.62(2H, m), 1.51 (2H, m), 1.36(4H, m), 0.90(3H, t, J = 7.4 Hz).

### Example 37

### 2-Ethoxy-8-hydroXy-9-{3-[(4-dimethylaminobutoxy)carbonylmethyl]benzyl}adenine

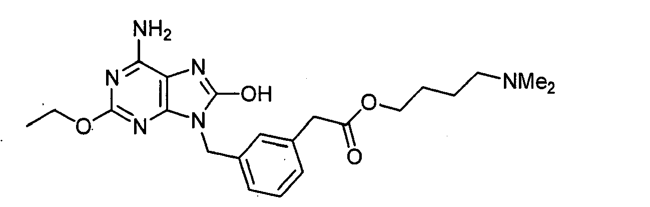

Using 2-ethoxy-8-hydroxy-9-(3-methoxycarbonylmethylbenzyl)adenine which was prepared in Example 33, the same procedures as in Comparative example 1 and Example 36 were carried out in this order to give the titled compound as a white solid.
Yield: 43%
¹H NMR(DMSO-d₆)δ 9.97(1H, s), 7.27(1H, t, J = 7.5 Hz), 7.21-7.15(3H, m), 6.46(2H, brs), 4.83(2H, s), 4.19(2H, q, J = 7.0Hz), 4.00(2H, t, J = 6.6Hz), 3.63(2H, s), 2.12(2H, t, J = 7.2Hz), 2.05(6H, s), 1.52(2H, q, J = 6.6Hz), 1.34(2H, q, J = 7.2Hz), 1.25(3H, t, J = 7.0Hz).

### Example 38

### 2-Butoxy-8-hydroxy-9-{3-[(2-dimethylaminoethoxy)carbonylmethyl]benzyl}adenine

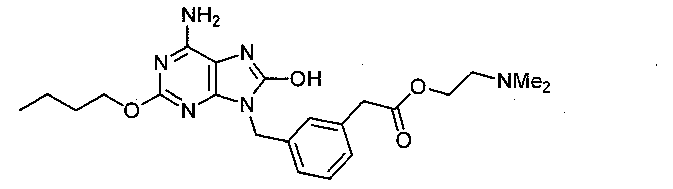

Using 2-butoxy-8-hydroxy-9-(3-carboxymethylbenzyl)adenine which was prepared in Reference example 76, the same procedure as in Example 36 was carried out to give the titled compound as a pale yellow solid.
Yield: 65%
¹H NMR(DMSO-d₆)δ 7.20(4H, m), 6.63(2H, brs), 4.83(2H, s), 4.13(2H, t, J = 6.6 Hz), 4.07(2H, t, J = 5.8 Hz), 3.62(2H, s), 2.41(2H, t, J = 5.8 Hz), 2.10(6H, s), 1.63(2H, tt, J = 7.5 Hz, 6.6 Hz), 1.38(2H, tq, J = 7.5 Hz, 7.4 Hz), 0.90(3H, t, J = 7.4 Hz).

### Example 39

### 2-Butoxy-8-hydroxy-9-{3-[(3-dimethylaminopropoxy)carbonylmethyl]benzyl}adenine

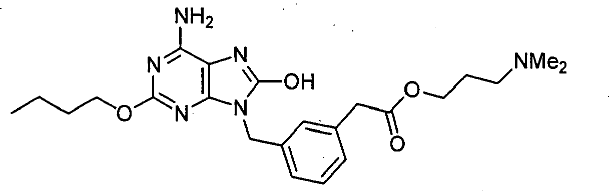

Using 2-butoxy-8-hydroxy-9-(3-carboxymethylbenzyl)adenine which was prepared in Reference example 76, the same procedure as in Example 36 was carried out to give the titled compound as a white solid. Yield 93% ¹H NMR(DMSO-d₆)δ 7.20(4H, m), 6.62(2H, brs), 4.82(2H, s), 4.12(2H, t, J = 6.6 Hz), 4.01(2H, t, J = 6.6 Hz), 3.61(2H, s), 2.14(2H, t, J = 7.0 Hz), 2.04(6H, s), 1.63(4H, m), 1.38(2H, m), 0.90(3H, t, J = 7.3 Hz).

### Example 40

### 2-Butoxy-8-hydroxy-9-{3-[(6-dimethylaminohexanoxy)carbonylmethyl]benzyl}adenine

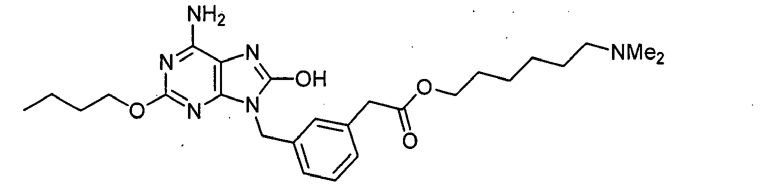

Using 2-butoxy-8-hydroxy-9-(3-carboxymethylbenzyl)adenine which was prepared in Reference example 76, the same procedure as in Example 36 was carried out to give the titled compound as a white solid. Yield: 49%.
¹H NMR(DMSO-d₆)δ 9.96(1H, brs), 7.21(4H, m), 6.46(2H, brs), 4.82(2H, s), 4.13(2H, t, J = 6.6 Hz), 3.98(2H, t, J = 6.6 Hz), 3.62(2H, s), 2.11(2H, t, J = 7.3 Hz), 2.07(6H, s), 1.64(2H, m), 1.52(2H, m), 1.36(4H, m), 1.21(4H, m), 0.90(3H, t, J = 7.3 Hz).

### Example 41

### 2-Butoxy-8-hydroxy-9-{3-[(3-diethylaminopropoxy)carbonylmethyl]benzyl}adenine

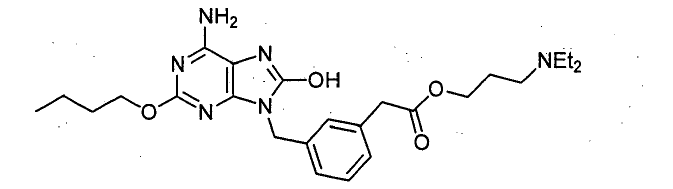

Using 2-butoxy-8-hydroxy-9-(3-carboxymethylbenzyl)adenine which was prepared in Reference example 76, the same procedure as in Example 36 was carried out to give the titled compound as a white solid. Yield: 72%
¹H NMR(DMSO-d₆)δ 9.96(1H, brs), 7.21(4H, m), 6.45(2H, brs), 4.82(2H, s), 4.13(2H, t, J = 6.6 Hz), 4.02(2H, t, J = 6.4 Hz), 3.61(2H, s), 2.34(4H, q, J = 7.1 Hz), 2.29(2H, t, J = 6.9 Hz), 1.60(4H, m), 1.37(2H, m), 0.90(3H, t, J = 7.3 Hz), 0.85(6H, t, J = 7.1 Hz).

### Example 42

### 2-Butoxy-8-hydroxy-9-{3-[(2-morpholinoethoxy)carbonylmethyl]benzyl}adenine

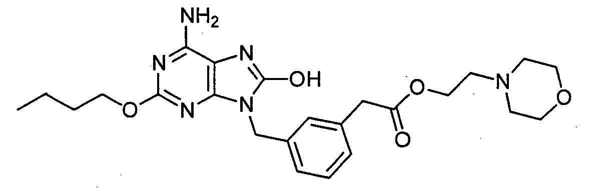

Using 2-butoxy-8-hydroxy-9-(3-carboxymethylbenzyl)adenine which was prepared in Reference example 76, the same procedure as in Example 36 was carried out to give the titled compound as a white solid. Yield: 73%
¹H NMR(DMSO-d₆)δ 9.99(1H, brs), 7.21(4H, m), 6.39(2H, brs), 4.83(2H, s), 4.14(2H, t, J = 6.6 Hz), 4.11(2H, t, J = 5.8 Hz), 3.63(2H, s), 3.48(4H, t, J = 4.6 Hz), 2.46(2H, t, J = 5.8 Hz), 2.30(4H, t, J = 4.6 Hz), 1.63(2H, m), 1.35(2H, m), 0.90(3H, t, J = 7.3 Hz).

### Example 43

### 2-Butoxy-8-hydroxy-9-{3-[(2-piperidinoethoxy)carbonylmethyl]benzyl}adenine

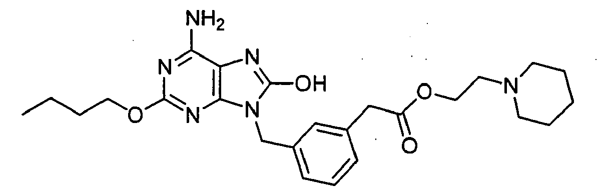

Using 2-butoxy-8-hydroxy-9-(3-carboxymethylbenzyl)adenine which was prepared in Reference example 76, the same procedure as in Example 36 was carried out to give the titled compound as a white solid. Yield: 71%
¹H NMR(DMSO-d₆)δ 9.94(1H, s), 7.21(4H, m), 6.44(2H, brs), 4.83(2H, s), 4.14(2H, t, J = 6.6 Hz), 4.08(2H, t, J = 5.9 Hz), 3.62(2H, s), 2.42(2H, t, J = 5.9 Hz), 2.26(4H, m), 1.62(2H, m), 1.38(8H, m), 0.90(3H, t, J = 7.3 Hz).

### Example 44

### 2-Butoxy-8-hydroxy-9-{3-[(2,2,2-trifluoroethoxy)carbonylmethyl]benzyl}adenine

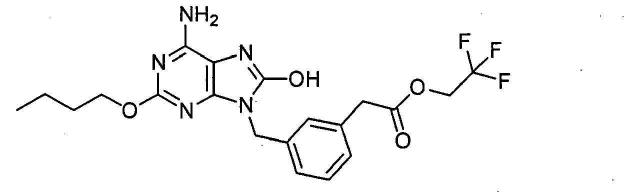

Using 2-butoxy-8-hydroxy-9-(3-carboxymethylbenzyl)adenine which was prepared in Reference example 76, the same procedure as in Example 36 was carried out to give the titled compound as a white solid. Yield: 80%.
¹H NMR(DMSO-d₆)δ 9.98(1H, brs), 7.22(4H, m), 6.46(2H, brs), 4.83(2H, s), 4.74(2H, q, J = 9.1 Hz), 4.13(2H, t, J = 6.6 Hz), 3.80(2H, s), 1.62(2H, tt, J = 7.5 Hz, 6.6 Hz), 1.38(2H, tq, J = 7.5 Hz, 7.3 Hz), 0.90(3H, t, J = 7.3 Hz).

### Example 45

### 2-Butoxy-8-hydroxy-9-{3-[(2-hydroxyethoxy)carbonylmethyl]benzyl}adenine

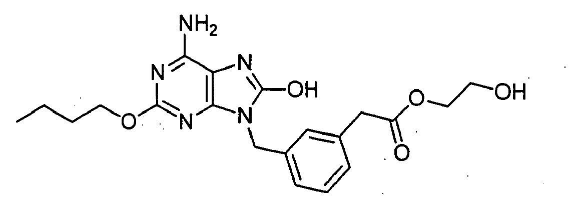

Using 2-butoxy-8-hydroxy-9-(3-carboxymethylbenzyl)adenine which was prepared in Reference example 76, the same procedure as in Example 36 was carried out to give the titled compound as a white solid. Yield: 68%.
¹H NMR(DMSO-d₆)δ 9.98(1H, brs), 7.21(4H, m), 6.46(2H, brs), 4.83(3H, brs), 4.13(2H, t, J = 6.6 Hz), 4.03(2H, t, J = 5.1 Hz), 3.65(2H, s), 3.55(2H, m), 1.62(2H, tt, J = 7.5 Hz, 6.6 Hz), 1.37(2H, tq, J = 7.5 Hz, 7.4 Hz), 0.90(3H, t, J = 7.3 Hz).

### Example 46

### 2-Butoxy-8-hydroxy-9-{3-[(2,3-dihydroxypropoxy)carbonylmethyl]benzyl}adenine

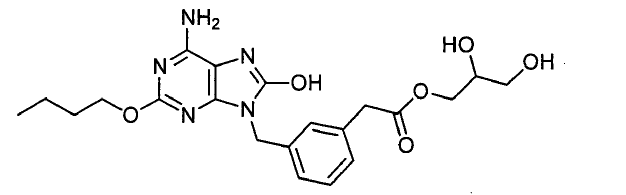

Using 2-butoxy-8-hydroxy-9-(3-carboxymethylbenzyl)adenine which was prepared in Reference example 76, the same procedure as in Example 36 was carried out to give the titled compound as a white solid. Yield: 38%
¹H NMR(DMSO-d₆)δ 9.96(1H, brs), 7.22(4H, m), 6.46(2H, brs), 4.91(1H, d, J = 5.2 Hz), 4.83(2H, s), 4.64(1H, m), 4.13(2H, t, J = 6.6 Hz), 4.05(1H, dd, J = 11.1, 4.1 Hz), 3.91(1H, dd, J = 11.1, 6.6 Hz), 3.65(2H, s), 3.63(1H, m), 3.32(2H, m), 1.62(2H, m), 1.37(2H, m), 0.90(3H, t, J = 7.3 Hz).

### Example 47

### 2-Butoxy-8-hydroxy-9-{5-[(4-dimethylaminobutoxy)carbonylmethyl]-2-methoxybenzyl}adenine

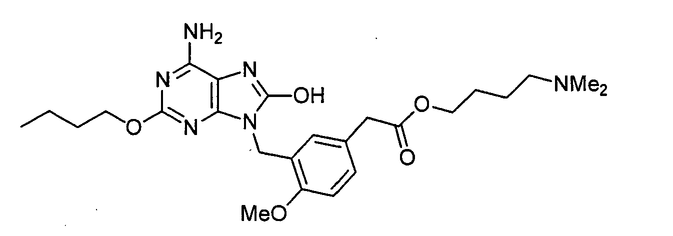

Using 2-butoxy-8-hydroxy-9-(5-carboxymethyl-2-methoxybenzyl)adenine which was prepared in Comparative examples 2 to 20, the same procedure as in Example 36 was carried out to give the titled compound as a white solid. Yield: 60%
¹H NMR(DMSO-d₆)δ 9.97(1H, brs), 7.12(1H, d, J = 8.4 Hz), 6.96(1H, d, J = 8.4 Hz), 6.69(1H, s), 6.48(2H, brs), 4.79(2H, s), 4.08(2H, t, J = 6.6 Hz), 3.92(2H, t, J = 6.6 Hz), 3.82(3H, s), 3.48(2H, s), 2.11(2H, t, J = 7.0 Hz), 2.05(6H, s), 1.58(2H, m), 1.46(2H, m), 1.33(4H, m), 0.87(3H, t, J = 7.3 Hz).

### Example 48

### 8-Hydroxy-2-(4-hydroxybutylthio)-9-{3-[(2-hydroxyethoxy)carbonylmethyl]benzyl}adenine

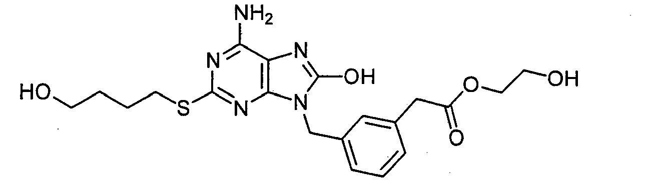

Using 9-(3-carboxymethylbenzyl)-8-hydroxy-2-(4-hydroxybutylthio)adenine which was prepared in Comparative example 2, the same procedure as in Example 36 was carried out to give the titled compound as a white solid. Yield: 13%
¹H NMR(DMSO-d₆)δ 10.11(1H, brs), 7.22(4H, m), 6.53(2H, brs), 4.85(2H, s), 4.83(1H, t, J = 5.2 Hz), 4.40(1H, t, J = 5.2 Hz), 4.03(2H, t, J = 5.2 Hz), 3.65(2H, s), 3.55(2H, dt. J = 5.2, 5.2 Hz), 3.38(2H, dt, J = 5.2, 5.2 Hz), 3.02(2H, t, J = 5.2 Hz), 1.62(2H, m), 1.50(2H, m).

### Example 49

### 8-Hydroxy-9-{3-[(4-dimethylaminobutoxy)carbonylmethyl]benzyl}-2-[(4-pyridylmethyl)oxy]adenine

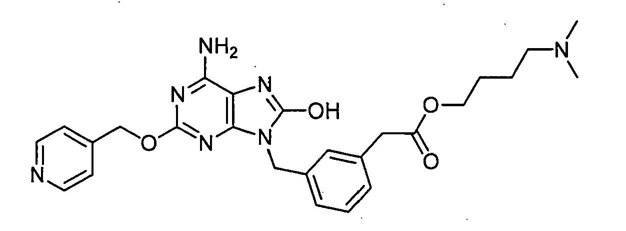

Using 8-hydroxy-9-(3-methoxycarbonylmethylbenzyl)-2-[(4-pyridylmethyl)oxy]adenine which was prepared in Example 32, the same procedures as in Comparative example 1 and Example 36 were conducted in this order to give the titled compound as a white solid. Yield: 25%
¹H NMR(DMSO-d₆)δ 10.01(1H, brs), 8.52(2H, dd, J = 4.4, 1.6 Hz), 7.36(2H, d, J = 4.5 Hz), 7.20(4H, m), 6.55(2H, brs), 5.31(2H, s), 4.82(2H, s), 3.98(2H, t, J = 6.6 Hz), 3.61(2H, s), 2.11(2H, t, J = 7.1 Hz), 2.04(6H, s), 1.50(2H, qui, J = 7.3 Hz), 1.32(2H, qui, J = 7.3 Hz).

### Example 50

### 2-[2-(4-Bromophenyloxy)ethoxy]-8-hydroxy-9-(3-methoxycarbonylmethylbenzyl)adenine

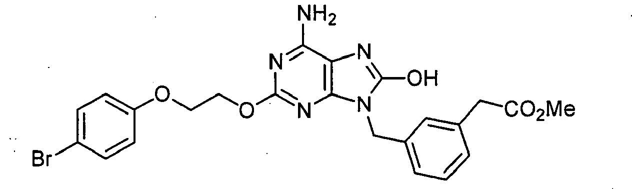

Using 8-bromo-2-[2-(3-bromophenyloxy)ethoxy]-9-(3-methoxycarbonylmethylbenzyl)adenine which was prepared in Reference example 78, the same procedure as in Reference example 3 was carried out to give the titled compound as a white solid. Yield: 98%
¹H NMR(DMSO-d₆)δ 10.16(1H, s), 7.46-7.43(2H, m), 7.27(1H, t, J = 7.6Hz), 7.21-7.15(3H, m), 6.95-6.91(2H, m), 6.59(2H, brs), 4.84(2H, s), 4.46(2H, t, J = 4.6 Hz), 4.24(2H, t, J = 4.6 Hz), 3.65(2H, s), 3.58(3H, s).

### Example 51

### 8-Hydroxy-9-(3-methoxycarbonylmethylbenzyl)-2-(2-phenyloxyethoxy) adenine

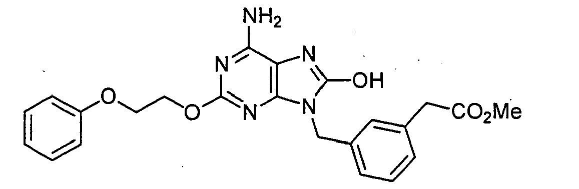

2-[2-(4-Bromophenyloxy)ethoxy]-8-hydroxy-9-(3-methoxycarbonylmethylbenzyl)adenine (200mg, 0.378mmol) which was prepared in Example 50 was dissolved in a mixture of chloroform (20ml), methanol (20ml) and 4N-hydrochloric acid-methanol (1ml), and thereto was added Pd/C (200mg). The mixture was stirred under a hydrogen atmosphere for 30 minutes. After removal of the catalyst and the solvent, the residue was recrystallized from methanol/hexane to give the titled compound as a white solid (109mg, 0.243mmol). Yield: 64%.
¹H NMR(DMSO-d₆)δ 10.16(1H, s), 7.31-7.26(3H, m), 7.22-7.16(3H, m), 6.96-6.92(3H, m), 6.65(2H, brs), 4.85(2H, s), 4.50(2H, t, J = 4.6 Hz), 4.25(2H, t, J = 4.6 Hz), 3.65(2H, s), 3.58(3H, s).

### Example 52

### 2-(3-Aminopropoxy)-8-hydroxy-9-(3-methoxycarbonylmethylbenzyl)adenine

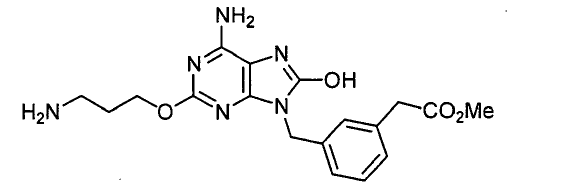

8-Hydroxy-9-(3-methoxycarbonylmethylbenzyl)-2-[3-phthalimidopropoxy]adenine (1.47g, 2.60mmol)which was prepared in Reference example 79 was dissolved in methanol (20ml)and thereto was added an aqueous 5N-sodium hydroxide solution (30ml). The solution was refluxed at 100°C for 30 minutes and then neutralized with hydrochloric acid under ice-cooling. After removal of the solvent, to the residue were added ethanol (40ml) and hydrazine solution. The solution was refluxed for 12 hours and the solvent was removed. To the residue were added methanol (40ml) and concentrated sulfuric acid (0.5ml) and the mixture was refluxed for 1 hour. After neutralizing with 28% aqueous ammonia solution under ice-cooling, the solvent was removed and the residue was extracted with chloroform/ethanol (3:1). The organic layer was dried over anhydrous magnesium sulfate and dried to give the titled compound as a white solid (0.89g, 2.30mmol). Yield: 89%
¹H NMR(DMSO-d₆)δ9.96(1H, s), 7.28(1H, t, J = 7.6 Hz), 7.21-7.15(3H, m), 6.45(2H, brs), 4.84(2H, s), 4.20(2H, t, J = 6.4 Hz), 3.65(2H, s), 3.59(3H, s), 2.64(2H, t, 6.7Hz), 1.75-1.69(2H, m).

### Example 53

### 2-[3-(N-Acetylamino)propoxy]-8-hydroxy-9-(3-methoxycarbonylmethylbenzyl)adenine

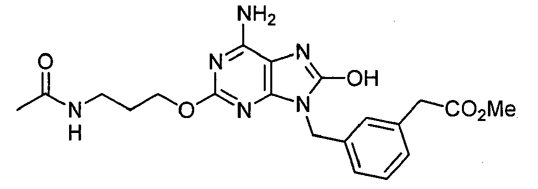

2-(3-Aminopropoxy)-8-hydroxy-9-(3-methoxycarbonylmethylbenzyl)adenine (80mg, 0.207mmol) which was prepared in Example 52 was dissolved in dimethylformamide (3ml). To the solution were added acetic anhydride (23µl, 0.248mmol), triethylamine (43µl, 0.311mmol) and 4-dimethylaminopyridine (5mg, 0.041mmo1)and the mixture was stirred at room temperature for 1.5 hours. After removal of the solvent the residue was extracted with chloroform, dried over anhydrous magnesium sulfate, and concentrated. The residue was purified by column chromatography (SiO₂ 2.4g, elute: CHCl₃/MeOH (30:1) to give the titled compound as a white solid (56mg, 0.131 mmol). Yield: 56%
¹H NMR(DMSO-d₆)δ9.99(1H, brs), 7.88(1H, t, 5,0Hz), 7.28(1H, t, J = 7.6 Hz), 7.21-7.10(3H, m), 6.48(2H, brs), 4.84(2H, s), 4.15(2H, t, J = 6.4Hz), 3.65(2H, s), 3.59(3H, s), 3.13(2H, m), 1.79(3H, s), 1.78-1.63(2H, m).

### Example 54

### 8-Hydroxy-2-[3-(N-methanesulfonylamino)propoxy]-9-(3-methoxycarbonylmethylbenzyl) adenine

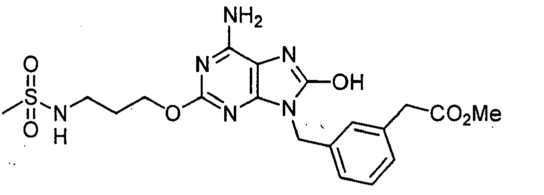

2-(3-Aminopropoxy)-8-hydroxy-9-(3-methoxycarbonylmethylbenzyl)adenine 80mg (0.207mmol) which was prepared in Example 52 was dissolved in dimethylformamide (3ml). Thereto were added methanesulfonyl chloride (19µl, 0.248mmol), triethylamine (43µl, 0.311mmol) and 4-dimethylaminopyridine (5mg, 0.041mmol) and the mixture was stirred at 50°C for 3 hours. After removal of the solvent, the residue was extracted with chloroform. The organic layer was dried over anhydrous magnesium sulfate, concentrated and the residue was purified by column chromatography (SiO₂ 2.4g, elute: CHCl₃/MeOH (20:1) to give the titled compound as a white solid (53mg, 0.131 mmol). Yield: 55%.
¹H NMR(DMSO-d₆)δ9.98(1H, brs), 7.28(1H, t, 7.6Hz), 7.21-7.15(3H, m), 7.04(1H, t, J = 5.8Hz), 6.49(2H, brs), 4.84(2H, s), 4.19(2H, t, J = 6.2Hz), 3.65(2H, s), 3.59(3H, s), 3.06(2H, m), 2.88(3H, s), 1.88-1.83(2H, m).

### Example 55

### 8-Hydroxy-2-[3-(N-methoxycarbonylamino)propoxy]-9-(3-methoxycarbonylmethylbenzyl) adenine

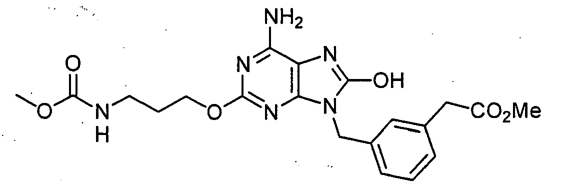

2-(3-Aminopropoxy)-8-hydroxy-9-(3-methoxycarbonylmethylbenzyl)adenine (100mg, 0.259mmol) which was prepared in Example 52 was dissolved in dimethylformamide (3ml). Thereto were added methyl chloroformate (20µl, 0.259mmol), triethylamine (36µl, 0.259mmol), 4-dimethylaminopyridine (6.4mg, 0.052mmol), and the mixture was stirred at room temperature for 3 hours. After removal of the solvent, the residue was extracted with chloroform. The organic layer was dried over anhydrous magnesium sulfate, concentrated and the residue was purified by column chromatography (SiO₂ 3.0g, elute: CHCl₃/MeOH (50:1) to give the titled compound as a white solid (61mg, 0.131mmol).
Yield: 53%
¹H NMR(DMSO-d₆)δ9.98(1H, brs), 7.28(1H, t, 7.6Hz), 7.21-7.15(4H, m), 6.47(2H, brs), 4.84(2H, s), 4.15(2H, t, J = 6.2Hz), 3.65(2H, s), 3.59(3H, s), 3.51(3H, s), 3.09(2H, m), 2.88(3H, s), 1.88-1.83(2H, m).

### Example 56

### 8-Hydroxy-9-(3-methoxycarbonylmethylbenzyl)-2-(3-ureidopropoxy)adenine

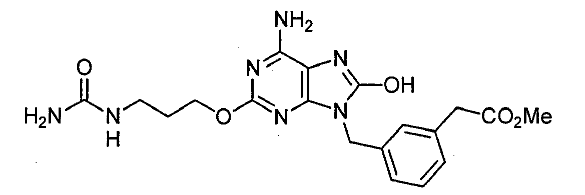

To a solution of 2-(3-aminopropoxy)-8-hydroxy-9-(3-methoxycarbonylmethylbenzyl)adenine (80mg, 0.207mmol) which was prepared in Example 52 in dimethylformamide (3ml) was added trimethylsilylisocyanate (28µl, 0.207mmol) and the mixture was stirred at room temperature for 20 hours. After removal of the solvent, the residue was extracted with chloroform. The organic layer was dried over anhydrous magnesium sulfate, concentrated and the residue was purified by column chromatography (SiO₂ 2.4g, elute: CHCl₃/MeOH/28% aqueous ammonia (50:5: 1) to give the titled compound as a white solid (14mg, 0.024mmol). Yield: 14%
¹H NMR(DMSO-d₆)δ9.92(1H, brs), 7.28(1H, t, 7.6Hz), 7.21-7.15(3H, m), 6.48(2H, brs), 6.00(1H, t, J = 5.8Hz), 5.40(2H, brs,), 4.84(2H, s), 4.15(2H, t, J = 6.4Hz), 3.66(2H, s), 3.59(3H, s), 3.06(2H, m), 1.78-1.73(2H, m).

### Example 57

### 2-(2-Diethylaminoethoxy)-8-hydroxy-9-(3-methoxycarbonylmethylbenzyl) adenine

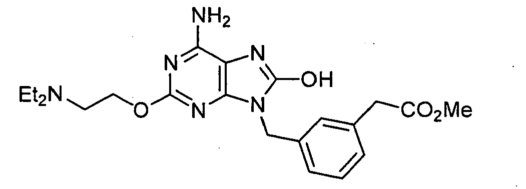

2-(2-Diethylaminoethoxy-8-iodo-9-tetrahydropyranyladenine (350mg, 0.76mmol) which was prepared in Reference example 83 was dissolved in methanol (30ml). Thereto was dropped at 0°C sulfuric acid (0.05ml, 0.91mmol), and the solution was stirred for 2 hours. After neutralization with an aqueous saturated sodium bicarbonate solution, the solution was centrated in vacuo to dryness. Then the same procedures as in Reference example 1 and Reference example 3 were carried out in this order to give the titled compound as a white solid (51 mg). Yield 16%
¹H NMR(DMSO-d₆)δ 9.96(1H, s), 7.18(4H, m), 6.47(2H, brs), 4.83(2H, s), 4.18(2H, t, J = 6.4 Hz), 3.64(2H, s), 3.37(3H, s), 2.67(2H, m), 2.50(4H, m), 0.94(6H, t, J = 7.1 Hz).

### Example 58

### 8-Hydroxy-9-(3-methoxycarbonylmethylbenzyl)-2-trifluoromethyladenine

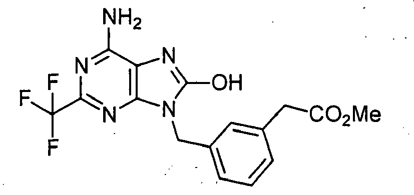

Using 2-trifluoromethyladenine formate (0.48g, 1.92mmol) which was prepared in Reference example 86, the same procedures as in Reference example 1, Reference example 2 and Reference example 3 were carried out in this order to give the titled compound as a white solid.
Yield: 41%.
¹H NMR (DMSO-d₆)δ 10.64(1H, brs), 7.28(1H, dd, J = 7.6 Hz), 7.20(1H, s), 7.16(2H, d, J = 7.6 Hz), 7.02(2H, brs), 4.93(2H, s), 3.64(2H, s), 3.58(3H, s).

### Example 59

### 2-Butyl-8-hydroxy-9-(3-methoxycarbonylmethylbenzyl)adenine

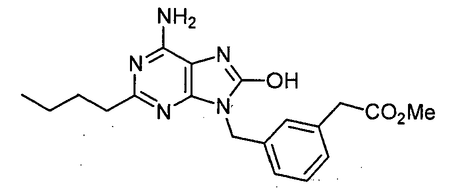

Using 2-butyl-6-hydroxypurine which was prepared in Reference example 87, the same procedures as in Reference example 84, Reference example 85, Reference example 1, Reference example 2 and Reference example 3 were carried out in this order to give the titled compound as a white solid. Yield: 8%.
¹H NMR(DMSO-d₆)δ 10.11(1H, brs), 7.22(4H, m), 6.35(2H, brs), 4.88(2H, s), 3.63(2H, s), 3.58(3H, s), 2.56(2H, t, J = 7.6 Hz), 1.64(2H, tt, J = 7.6 Hz, 7.5 Hz), 1.28(2H, tq, J = 7.5 Hz, 7.3 Hz), 0.87(3H, t, J = 7.3 Hz).

### Example 60

### 8-Hydroxy-9-(3-methoxycarbonylmethylbenzyl)-2-pentyladenine

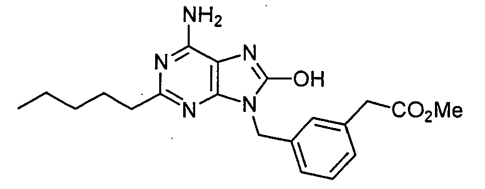

Using 1-benzyl-4-aminoimidazole-5-caboxamide and ethyl caproate, the same procedures as in Reference example 83, Reference example 84, Reference example 85, Reference example 1, Reference example 2 and Reference example 3 were carried out in this order to give the titled compound as a white solid.
¹H NMR(DMSO-d₆)δ 10.10(1H, brs), 7.21(4H, m), 6.36(2H, brs), 4.87(2H, s), 3.63(2H, s), 3.58(3H, s), 2.55(2H, m), 1.65(2H, tt, J = 7.6 Hz, 7.5 Hz), 1.26(4H, m), 0.84(3H, t, J = 7.1 Hz).

### Example 61

### 8-Hydroxy-9-(3-methoxycarbonylmethylbenzyl)-2-(3-methoxypropyl)adenine

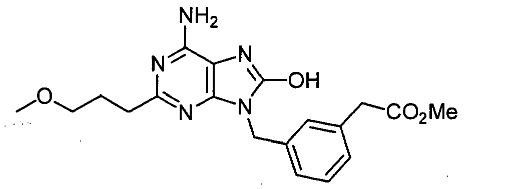

Using 4-aminoimidazole-5-caboxamide hydrochloride 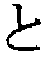 methyl 4-methoxybutyrate, the same procedures as in Reference example 83, Reference example 84, Reference example 85, Reference example 1, Reference example 2 and Reference example 3 were carried out in this order to give the titled compound as a white solid. Yield: 7%.
¹H NMR(DMSO-d₆)δ 10.40(1H, brs), 7.26(1H, dd, 7.6, 7.6 Hz), 7.21(1H, s), 7.16(2H, m), 6.44(2H, brs), 4.87(2H, s), 3.64(2H, s), 3.58(3H, s), 3.30(2H, m), 3.19(3H, s), 2.59(2H, t, J = 7.6 Hz), 1.88(2H, m).

### Example 62

### 2-Ethoxymethyl-8-hydroxy-9-(3-methoxycarbonylmethylbenzyl)adenine

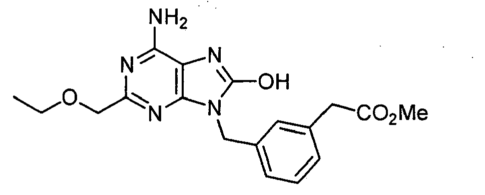

Using 1-benzyl-4-aminoimidazole-5-caboxamide and ethyl ethoxyacetate, the same procedures as in Reference example 83, Reference example 84, Reference example 85, Reference example 86, Reference example 1, Reference example 2 and Reference example 3 were carried out in this order to give the titled compound as a white solid. Yield: 6%
¹H NMR(DMSO-d₆)δ 10.28(1H, brs), 7.26(1H, dd, J = 7.6, 7.6 Hz), 7.19(1H, s), 7.15(2H, m), 6.51(2H, brs), 4.90(2H, s), 4.28(2H, s), 3.64(2H, s), 3.58(3H, s), 3.48(2H, q, J = 7.0 Hz), 1.10(3H, q, J = 7.1 Hz).

### Example 63

### 2-Ethoxymethyl-8-hydroxy-9-{3-[(4-dimethylaminobutoxy)carbonylmethyl]benzyl}adenine

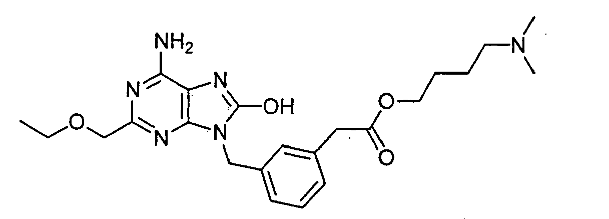

Using 2-ethoxymethyl-8-hydroxy-9-(3-methoxycarbonylmethylbenzyl) adenine which was prepared in Example 61, the same procedures as in Comparative example 1 and Example 36 were carried out in this order to give the titled compound as a white solid.
¹H NMR(DMSO-d₆)δ 10.23(1H, brs), 7.26(1H, t, J = 7.6 Hz), 7.20(3H, m), 6.49(2H, brs), 4.90(2H, s), 4.29(2H, s), 4.00(2H, t, J = 6.5 Hz), 3.62(2H, s), 3.49(2H, q, J = 7.0 Hz), 2.22(2H, m), 2.13(6H, s), 1.52(2H, qui, J = 6.6 Hz), 1.37(2H, qui, J = 7.0 Hz), 1.10(3H, t, J = 7.0 Hz).

### Example 64

### 2-Cyclopentyl-8-hydroxy-9-(3-methoxycarbonylmethylbenzyl)adenine

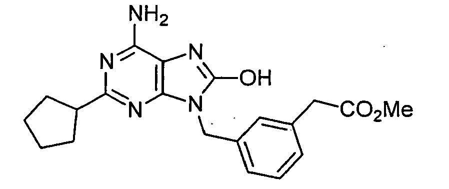

Using 8-bromo-2-cyclopentyl-9-{3-(methoxycarbonylmethyl)benzyl}adenine which was prepared in Reference example 92, the same procedure as in Reference example 3 was carried out to give the titled compound as a white solid. Yield: 70%
¹H NMR(DMSO-d₆)δ 10.09(1H, brs), 7.27(1H, dd, J = 7.6 Hz), 7.25(1H, s), 7.19(1H, d, J = 7.6 Hz), 7.15(1H, d, J = 7.6 Hz), 6.32(2H, brs), 4.86(2H, s), 3.63(2H, s), 3.58(3H, s), 3.00(1H, quin, J = 8.2 Hz), 1.91-1.83(2H, m), 1.83-1.76(2H, m), 1.76-1.66(2H, m), 1.62-1.53(2H, m).

### Example 65

### 8-Hydroxy-2-(3-hydroxypropyl)-9-(3-methoxycarbonylmethylbenzyl)adenine

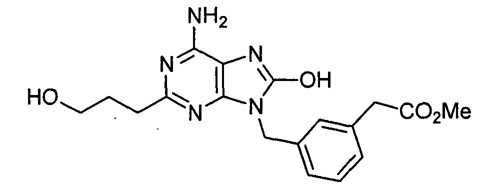

Using 8-bromo-2-(3-hydroxypropyl)-9-(3-methoxycarbonylmethylbenzyl)adenine which was prepared in Reference example 95, the same procedure as in Reference example 3 was carried out to give the titled compound as a yellow oil. Yield: 62%
¹H NMR(DMSO-d₆)δ10.02(1H, brs), 7.26(1H, dd, J = 7.6, 7.6 Hz), 7.21(1H; s), 7.16(1H, d, J = 7.6 Hz), 7.14(1H, d, J = 7.6 Hz), 6.36(2H, brs), 4.87(2H, s), 4.42(1H, t, J = 5.2 Hz), 3.64(2H, s), 3.58(3H, s), 3.42(2H, dt, J = 6.6, 5.2 Hz), 2.59(2H, t, J = 7.5 Hz),(2H, tt, J = 7.5, 6.6 Hz).

### Example 66

### 2-(4-Fluorobenzyl)-8-hydroxy-9-(3-methoxycarbonylmethylbenzyl)adenine

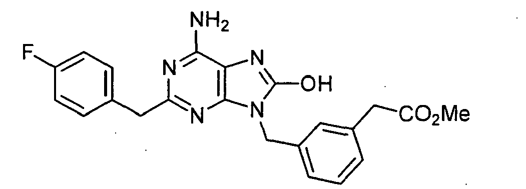

Using 2-iodo-9-(3-methoxycarbonylmethylbenzyl)adenine which was prepared in Reference example 90, the same procedures as in Reference example 91, Reference example 2 and Reference example 3 were carried out in this order to give the titled compound as a white solid.
Yield: 19%
¹H NMR(DMSO-d₆)δ 10.26(1H, brs), 7.29(3H, m), 7.17(3H, m), 7.05(2H, m), 6.45(2H, brs), 4.87(2H, s), 3.88(2H, s), 3.62(2H, s), 3.58(3H, s).

### Example 67

### 8-Hydroxy-9-(3-methoxycarbonylmethylbenzyl)-2-(2-pyridylmethoxy) adenine

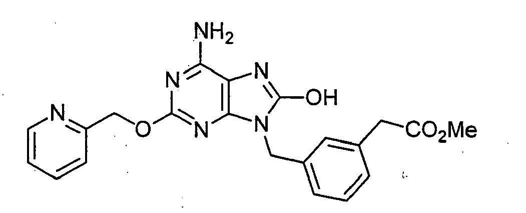

Using 8-bromo-9-(3-methoxycarbonylmethylbenzyl)-2-(2-pyridylmethoxy)adenine which was prepared in Reference example 98, the same procedure as in Reference example 3 was carried out to give the titled compound as a white solid. Yield: 78%
¹H NMR(DMSO-d₆)δ10.00(1H, brs), 8.53(1H, d, J = 4.8 Hz), 7.77(1H, dd, J = 7.8, 7.4 Hz), 7.39(1H, d, J = 7.8 Hz), 7.31(1H, dd, J = 7.4, 4.8 Hz), 7.23(1H, dd, J = 7.6, 7.6 Hz), 7.19(1H, s), 7.15(1H, d, J = 7.6 Hz), 7.12(1H, d, J = 7.6 Hz), 6.54(2H, brs), 5.33(2H, s), 4.82(2H, s), 3.63(2H, s), 3.58(3H, s).

### Example 68

### 8-Hydroxy-9-(3-methoxycarbonylmethylbenzyl)-2-(3-pyridylmethoxy)adenine

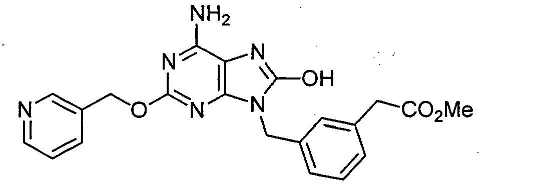

Using 8-bromo-9-(3-methoxycarbonylmethylbenzyl)-2-(3-pyridylmethoxy)adenine which was prepared in Reference example 100, the same procedure as in Reference example 3 was carried out to give the titled compound as a white solid. Yield: 99%
¹H NMR(DMSO-d₆)δ10.04(1H, brs), 8.65(1H, s), 8.51(1H, d, J = 4.8 Hz), 7.82(1H, d, J = 7.5 Hz), 7.37(1H, dd, J = 7.5, 4.8 Hz), 7.26(1H, dd, J = 7.6, 7.6 Hz), 7.24-7.13(3H, m), 6.57(2H, brs), 5.28(2H, s), 4.84(2H, s), 3.65(2H, s), 3.57(3H, s).

### Example 69

### 8-Hydroxy-9-(3-methoxycarbonylmethylbenzyl)-2-(3-morpholinopropoxy)adenine

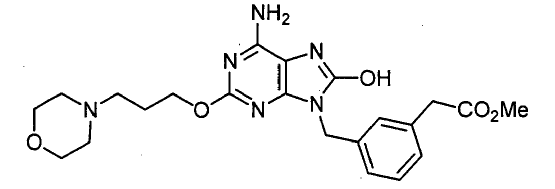

Using 8-bromo-9-(3-methoxycarbonylmethylbenzyl)-2-(3-morpholinopropoxy)-8-tetrahydropyranyladenine which was prepared in Reference example 103, the same procedure as in Reference example 3 was carried out to give the titled compound as a white solid. Yield: 66%
¹H NMR(DMSO-d₆)δ 9.96(1H, s), 7.27(1H, dd, J = 7.7, 7.4 Hz), 7.17(3H, m), 6.46(2H, brs), 4.83(2H, s), 4.17(2H, t, J = 6.5 Hz), 3.64(2H, s), 3.58(3H, s), 3.54(4H, t, J = 4.5 Hz), 2.35(6H, m), 1.82(2H, m).

### Example 70

### 8-Hydroxy-9-(3-methoxycarbonylmethylbenzyl)-2-{2-(phenylsulfanyl)ethoxy}adenine

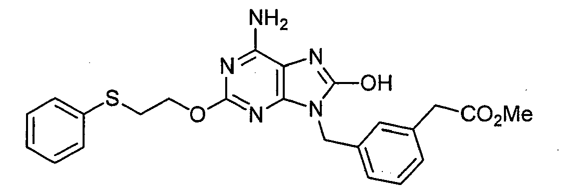

To 8-bromo-9-(3-methoxycarbonylmethylbenzyl)-2-{2-(phenylsulfanyl)ethoxy}adenine which was prepared in Reference example 108 was added 10% hydrochloric acid methanol solution (5ml) and the solution was stirred at room temperature for 12 hours. After neutralization with an aqueous saturated sodium hydrogencarbonate solution, thereto was added water. The resulted white precipitate was filtered and purified by silica gel column chromatography to give the titled compound as a white solid (108mg, 0.23mmol). Yield: 72%
¹H NMR(DMSO-d₆)δ10.12(1H, brs), 7.40-7.35(2H, m), 7.31-7.27(2H, m), 7.27-7.22(1H, m), 7.20-7.14(4H, m), 6.58(2H, brs), 4.82(2H, s), 4.30(2H, t, J = 6.9 Hz), 3.63(2H, s), 3.58(3H, s), 3.31(2H, t, J = 6.9 Hz).

### Example 71

### 8-Hydroxy-9-(3-methoxycarbonylmethylbenzyl)-2-(2-methylsulfanylethoxy) adenine

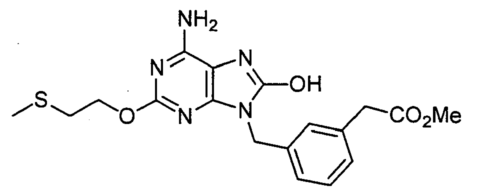

After sodium (198mg, 8.6mmol) was dissolved in 2-(methylsulfanyl)ethanol (10ml), thereto was added 2-chloro-8-hydroxy-9-(3-methoxycarbonylmethylbenzyl)adenine (300mg, 0.86mmol) which was prepared in Reference example 3, and the mixture was stirred in an autoclave at 130°C for 2.5 hours and at 150°C for 8 hours. After removal of the solvent, to the residue were added methanol (30 ml) and concentrated sulfuric acid and the mixture (pH = 2) was stirred under heating at 80°C. After 30 minutes, the mixture was neutralized with aqueous ammonia and concentrated. To the residue was added water and the insoluble solid was taken by filtration. The solid was purified by silica gel column chromatography to give the titled compound as a white solid (101mg, 0.25mmol). Yield: 30%
¹H NMR(DMSO-d₆)δ 9.98(1H, brs), 7.27(1H, dd, J = 7.6, 7.6 Hz), 7.20-7.16(3H, m), 6.50(2H, brs), 4.83(2H, s), 4.30(2H, t, J = 6.9 Hz), 3.65(2H, s), 3.59(3H, s), 2.77(2H, t, J = 6.9 Hz), 2.10(3H, s).

### Example 72

### 8-Hydroxy-9-(3-methoxycarbonylmethylbenzyl)-2-phenylsulfanyladenine

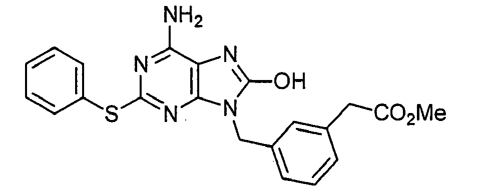

Using 2-chloro-8-hydroxy-9-(3-methoxycarbonylmethylbenzyl)adenine which was prepared in Reference example 3, the same procedure as in Reference example 4 was carried out to give the titled compound as a white solid. Yield: 58%
¹H NMR(DMSO-d₆)δ 10.11(1H, brs), 7.58-7.53(2H, m), 7.44-7.40(3H, m), 7.22(1H, dd, J = 7.6, 7.6 Hz), 7.15(1H, d, J = 7.6 Hz), 7.05(1H, s), 7.00(1H, d, J = 7.6 Hz), 6.54(2H, brs), 4.65(2H, s), 3.61(2H, s), 3.59(3H, s).

### Example 73

### 8-Hydroxy-9-(3-methoxycarbonylmethylbenzyl)-2-(tetrahydrofuran-2-ylmethoxy) adenine

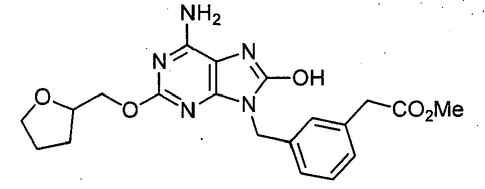

Using 8-bromo-9-(3-methoxycarbonylmethylbenzyl)-2-(tetrahydrofuran-2-ylmethoxy)adenine which was prepared in Reference example 109, the same procedure as in Reference example 3 was carried out to give the titled compound as a white solid. Yield: 80%
¹H NMR(DMSO-d₆)δ9.96(1H, brs), 7.27(1H, dd, J = 7.6, 7.6 Hz), 7.20(1H, s), 7.17(1H, d, J = 7.6 Hz), 7.15(1H, d, J = 7.6 Hz), 6.48(2H, brs), 4.82(2H, s), 4.14-4.09(2H, m), 4.09-4.01(1H, m), 3.77-3.71(1H, m), 3.64(2H, s), 3.68-3.61(1H, m), 3.58(3H, s), 1.97-1.90(1H, m), 1.89-182(2H, m), 1.65-1.58(1H, m).

### Comparative example 1

### 9-(3-Carboxymethylbenzyl)-8-hydroxy-2-(3-hydroxypropylthio)adenine

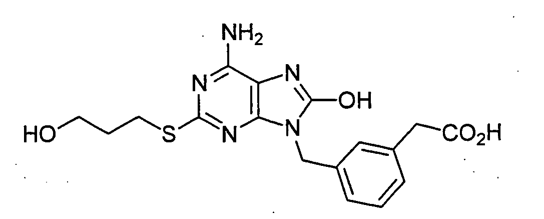

8-Hydroxy-2-(3-hydroxypropylthio)-9-(3-methoxycarbonylmethylbenzyl)adenine (50mg, 0.124mmol) which was prepared in Example 1 was added to a mixture of 1N sodium hydroxide solution (10ml) and methanol (10ml), followed by stirring at room temperature for 2 hours. After neutralization with concentrated hydrochloric acid, methanol was removed. The resulted precipitate solid was taken by filtration to give the titled compound as a white solid (47 mg, 0.121 mmol). Yield: 97%
¹H NMR(DMSO-d₆)δ 7.18(4H, m), 6.82(2H, brs), 4.83(2H, s), 3.49(2H, t, J = 6.3 Hz), 3.34(2H, s), 3.06(2H, t, J = 6.9 Hz), 1.78(2H, m).

### Comparative example 2

### 9-(3-Carboxymethylbenzyl)-8-hydroxy-2-(4-hydroxybutylthio)adenine

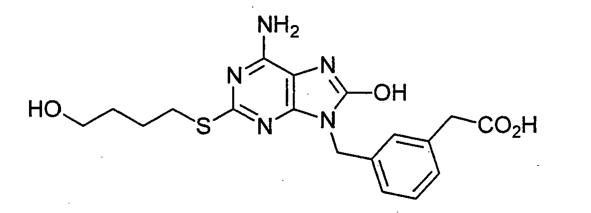

The titled compound as a white solid was prepared in the same method as in Comparative example 1. Yield: 70%
¹H NMR(DMSO-d₆)δ 12.46(1H, brs), 10.12(1H, s), 7.24(4H, m), 6.52(2H, brs), 4.89(2H, s), 3.52(2H, s), 3.39(2H, t, J = 6.4 Hz), 3.02(2H, t, J = 7.2 Hz), 1.65(2H, m), 1.52(2H, m).

### Comparative example 3

### 9-(3-Carboxymethylbenzyl)-8-hydroxy-2-(2-methoxyethylthio)adenine

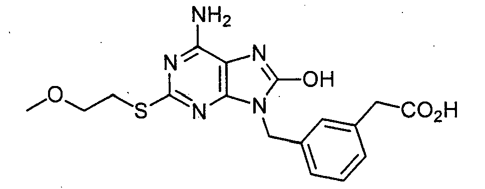

The titled compound as a white solid was prepared in the same method as in Comparative example 1. Yield: 32%
¹H NMR(DMSO-d₆)δ 7.01(4H, m), 6.56(2H, brs), 4.73(2H, s), 3.41(2H, t, J = 6.7 Hz), 3.21(2H, s), 3.14(3H, s)3.08(2H, t, J = 6.7 Hz).

### Comparative example 4

### 9-(3-Carboxymethylbenzyl)-8-hydroxy-2-(3-hydroxypropoxy)adenine

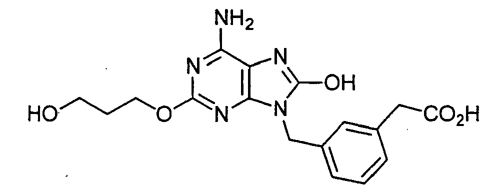

The titled compound as a white solid was prepared in the same method as in Comparative example 1. Yield: 82%
¹H NMR(DMSO-d₆)δ 12.29(1H, brs), 9.96(1H, brs), 7.26(1H, t, J = 7.6 Hz), 7.20(1H, s), 7.16(2H, m), 6.46(2H, brs), 4.83(2H, s), 4.50(1H, brs), 4.20(2H, t, J = 6.5 Hz), 3.51(4H, m), 1.79(2H, qui, J = 6.4 Hz).

### Comparative example 5

### 9-(3-Carboxymethylbenzyl)-8-hydroxy-2-(2-hydroxyethoxy)adenine

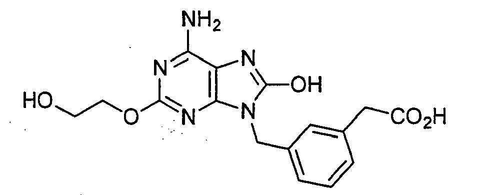

The titled compound as a white solid was prepared in the same method as in Comparative example 1. Yield: 70%
¹H NMR(DMSO-d₆)δ 10.03(1H, s), 7.26(1H, t, J = 7.8 Hz), 7.18(3H, m), 6.48(2H, s), 4.83(2H, s), 4.15(2H, t, J = 4.9 Hz), 3.64(2H, t, J = 5.0 Hz), 3.53(2H, s).

### Comparative example 6

### 9-(3-Carboxymethylbenzyl)-8-hydroxy-2-(4-hydroxybutoxy)adenine

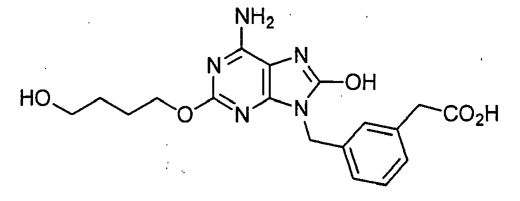

The titled compound as a white solid was prepared in the same method as in Comparative example 1. Yield: 62%
¹H NMR(DMSO-d₆)δ 10.37(1H, brs), 7.27(2H, m), 7.12(2H, m), 6.55(2H, m), 4.81 (2H, s), 4.15(2H, t, J = 6.6 Hz), 3.39(4H, m), 1.67(2H, qui, J = 6.8 Hz), 1.49(2H, qui, J = 6.7 Hz).

### Comparative example 7

### 9-(3-Carboxymethylbenzyl)-8-hydroxy-2-(4,4,4-trifluorobutoxy)adenine

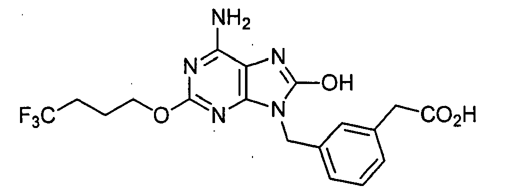

The titled compound as a white solid was prepared in the same method as in Comparative example 1. Yield: 88%
¹H NMR(DMSO-d₆)δ12.37(1H, brs), 10.00(1H, brs), 7.26(1H, t, J = 7.8 Hz), 7.21(1H, s), 7.16(2H, m), 6.50(2H, brs), 4.84(2H, s), 4.20(2H, t, J = 6.3 Hz), 3.52(2H, s), 2.36(2H, m), 1.88(2H, m).

### Comparative example 8

### 9-(3-Carboxymethylbenzyl)-8-hydroxy-2-[N-(2-methoxyethyl)amino]adenine

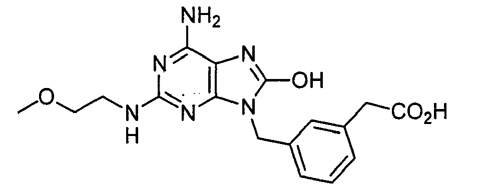

The titled compound as a white solid was prepared in the same method as in Comparative example 1. Yield: 84%
¹H NMR(DMSO-d₆)δ9.72(1H, s), 7.25(1H, t, J = 7.6 Hz), 7.18(3H, m), 6.14(1H, t, J = 5.1 Hz), 6.07(2H, brs), 4.78(2H, s), 3.52(2H, s), 3.37(4H, m), 3.22(3H, s).

### Comparative example 9

### 2-Butoxy-9-[2-(3-carboxymethylphenyl)ethyl]-8-hydroxyadenine

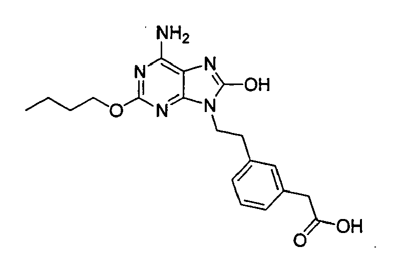

The titled compound was prepared in the same method as in Comparative example 1. Yield: 87%
¹H NMR(DMSO-d₆)δ 12.27(1H, brs), 9.91(1H, s), 7.21(1H, dd, J = 7.5, 7.5 Hz), 7.11(1H, s), 7.10-7.05(2H, m), 6.42(2H, brs), 4.15(2H, t, J = 6.6 Hz), 3.87(2H, t, J = 7.6 Hz), 3.50(2H, s), 2.95(2H, t, J = 7.6 Hz), 1.66(2H, tt, J = 7.5 Hz, 6.6 Hz), 1.39(2H, tq, J = 7.5 Hz, 7.4 Hz), 0.92(3H, t, J = 7.4 Hz).

### Comparative example 10

### 2-Butoxy-9-[3-(3-carboxymethylphenyl)propyl]-8-hydroxyadenine

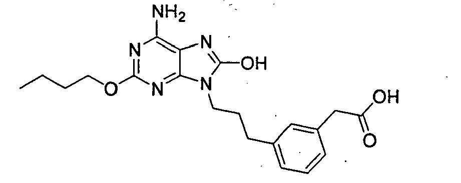

The titled compound was prepared in the same method as in Comparative example 1. Yield: 80%
¹H NMR(DMSO-d₆)δ 12.32(1H, brs), 10.09(1H, brs), 7.18(1H, dd, J = 7.9, 7.8 Hz), 7.08-7.04(3H, m), 6.46(2H, brs), 4.13(2H, t, J = 6.6 Hz), 3.70(2H, t, J = 7.0 Hz), 3.47(2H, s), 2.56(2H, t, J = 7.7 Hz), 1.94(2H, tt, J = 7.7 Hz, 7.0 Hz), 1.63(2H, tt, J = 7.5 Hz, 6.6 Hz), 1.38(2H, tq, J = 7.5 Hz, 7.4 Hz), 0.91(3H, t, J = 7.4 Hz).

### Comparative example 11

### 9-(3-Carboxymethylbenzyl)-2-(2, 3-dihydroxy-1-propoxy)-8-hydroxyadenine

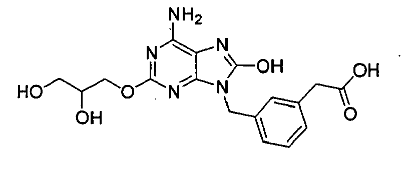

The titled compound was prepared in the same method as in Comparative example 1. Yield: 76%
¹H NMR(DMSO-d₆)δ 9.99(1H, brs), 7.26(1H, dd, J = 8.0, 7.1 Hz), 7.20(1H, s), 7.16-7.13(2H, m), 6.47(2H, brs), 5.00(1H, brs), 4.91(2H, s), 4.16(1H, dd, J = 10.9, 4.4 Hz), 4.03(1H, dd, J = 10.9, 6.4 Hz), 3.76-3.70(1H, m), 3.52(2H, s), 3.39(2H, d, J = 5.6 Hz).

### Comparative example 12

### 9-(3-Carboxymethylbenzyl)-2-(2-ethoxyethoxy)-8-hydroxyadenine

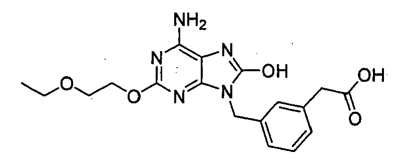

The titled compound was prepared in the same method as in Comparative example 1. Yield: 89%
¹H NMR(DMSO-d₆)δ 12.31(1H, brs), 9.97(1H, s), 7.26(1H, dd, J = 7.6, 7.5 Hz), 7.20(1H, s), 7.16-7.14(2H, m), 6.47(2H, brs), 4.83(2H, s), 4.25(2H, t, J = 4.8 Hz), 3.63(2H, t, J = 4.8 Hz), 3.53(2H, s), 3.45(2H, q, J = 7.0 Hz), 1.10(3H, t, J = 7.0 Hz).

### Comparative example 13

### 9-(3-Carboxymethylbenzyl)-2-cyclohexylmethoxy-8-hydroxyadenine

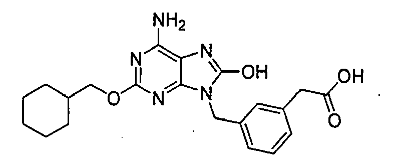

The titled compound was prepared in the same method as in Comparative example 1. Yield: 90%
¹H NMR(DMSO-d₆)δ 10.27(1H, brs), 7.23(1H, dd, J = 7.5, 7.5 Hz), 7.19-7.10(3H, m), 6.56(2H, brs), 4.81(2H, s), 3.94(2H, d, J = 6.2 Hz), 3.48(2H, s), 1.74-1.61(6H, m), 1.23-1.14(3H, m), 1.02-0.94(2H, m).

### Comparative example 14

### 2-Benzyloxy-9-(3-carboxymethylbenzyl)-8-hydroxyadenine

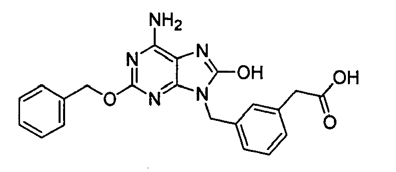

The titled compound was prepared in the same method as in Comparative example 1. Yield: 100%
¹H NMR(DMSO-d₆)δ 12.40(1H, brs), 10.29(1H, brs), 7.42-7.40(2H, m), 7.36-7.20(5H, m), 7.15-7.11(2H, m), 6.61(2H, brs), 5.24(2H, s), 4.83(2H, s), 3.49(2H, s).

### Comparative example 15

### 2-(2-Carboxyethyl)-9-(3-carboxymethylbenzyl)-8-hydroxyadenine

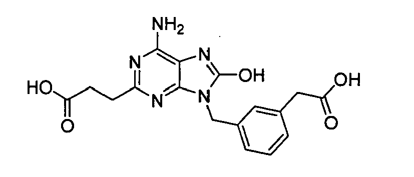

The titled compound was prepared in the same method as in Comparative example 1. Yield: 79%
¹H NMR(DMSO-d₆)δ 12.50(2H, brs), 10.10(1H, s), 7.25(1H, dd, J = 7.6, 7.4 Hz), 7.25(1H, s), 7.18(1H, d, 7.6 Hz), 7.16(1H, d, 7.4 Hz), 6.29(2H, brs), 4.86(2H, s), 3.52(2H, s), 2.83(2H, t, J = 7.2 Hz), 2.64(2H, t, J = 7.2 Hz).

### Comparative example 16

### 2-Butoxy-9-{(5-carboxylmethyl-2-thienyl)methyl}-8-hydroxyadenine

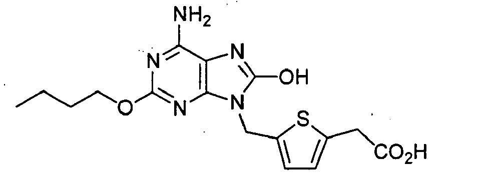

The titled compound as a white solid was prepared in the same method as in Comparative example 1. Yield: 96%
¹H NMR(DMSO-d₆)δ 12.47(1H, brs), 9.94(1H, s), 6.89(1H, d, J = 3.4 Hz), 6.75(1H, d, J = 3.5 Hz), 6.45(2H, brs), 4.94(2H, s), 4.17(2H, t, J = 6.6 Hz), 3.72(2H, s), 1.65(2H, 5, J = 6.6 Hz), 1.38(2H, 6, J = 7.5 Hz), 0.92(3H, t, J = 7.3Hz).

### Comparative example 18

### 2-Butoxy-9-{(6-carboxylmethyl-2-pyridyl)methyl}-8-hydroxyadenine

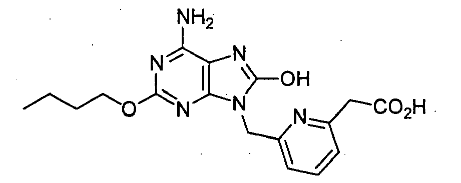

The titled compound as a white solid was prepared in the same method as in Comparative example 1. Yield: 68%
¹H NMR(DMSO-d₆)δ 12.39(1H, brs), 9.96(1H, brs), 7.62(1H, t, J = 7.7 Hz), 7.17(1H, d, J = 7.6 Hz), 6.85(1H, d, J = 7.7 Hz), 6.43(2H, brs), 4.85(2H, s), 4.01(2H, t, J = 6.6 Hz), 3.651(2H, s), 1.51(2H, 5, J = 6.6 Hz), 1.26(2H, 6, J = 7.3 Hz), 0.80(3H, t, J = 7.3 Hz).

### Comparative example 19

### 2-Butoxy-9-{(4-carboxylmethyl-2-pyridyl)methyl}-8-hydroxyadenine

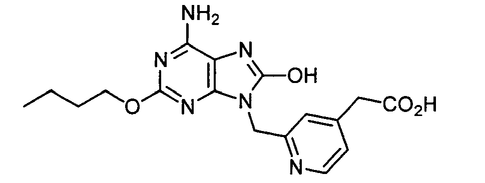

The titled compound as a white solid was prepared in the same method as in Comparative example 1. Yield: 58%
¹H NMR(DMSO-d₆)δ 10.77(1H, brs), 8.28(1H, d, J = 5.0 Hz), 7.13(1H, d, J = 4.9 Hz), 7.04(1H, s), 6.69(2H, brs), 4.91(2H, s), 4.07(2H, t, J = 6.6 Hz), 3.28(2H, s), 1.57(2H, 5, J = 6.6 Hz), 1.33(2H, 6, J = 7.4 Hz), 0.87(3H, t, J = 7.3 Hz).

### Comparative example 20

### 2-Butoxy-9-(5-carboxymethyl-2-methoxy)benzyl-8-hydroxyadenine

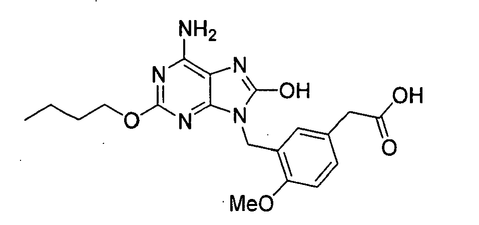

The titled compound as a white solid was prepared in the same method as in Comparative example 1. Yield: 88%
¹H NMR(DMSO-d₆)δ 12.25(1H, brs), 10.17(1H, brs), 7.12(1H, d, J = 8.4 Hz), 6.96(1H, d, J = 8.4 Hz), 6.68(1H, s), 6.51(2H, brs), 4.80(2H, s), 4.08(2H, t, J = 6.6 Hz), 3.82(3H, s), 3.36(2H, s), 1.58(2H, tt, J = 7.5 Hz, 6.6 Hz), 1.33(2H, tq, J = 7.5 Hz, 7.4 Hz), 0.87(3H, t, J = 7.4 Hz).

### Comparative example 21

### 2-Butoxy-9-(3-carboxymethyl-4-fluoro)benzyl-8-hydroxyadenine

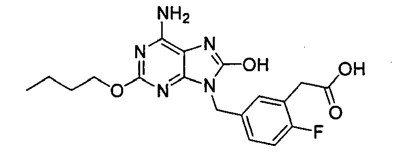

The titled compound as a white solid was prepared in the same method as in Comparative example 1. Yield: 92%
¹H NMR(DMSO-d₆)δ 12.51(1H, brs), 10.12(1H, s), 7.27(1H, dd, J = 7.2, 2.1 Hz), 7.22(1H, m), 7.11(1H, dd, J = 9.7, 8.5 Hz), 6.50(2H, brs), 4.81(2H, s), 4.14(2H, t, J = 6.6 Hz), 3.56(2H, s), 1.63(2H, tt, J = 7.5 Hz, 6.6 Hz), 1.37(2H, tq, J = 7.5 Hz, 7.4 Hz), 0.90(3H, t, J = 7.4 Hz).

### Comparative example 22

### 2-Butoxy-9-(3-carboxymethyl-4-methoxy)benzyl-8-hydroxyadenine

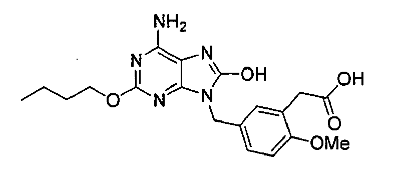

The titled compound as a white solid was prepared in the same method as in Comparative example 1. Yield: 89%
¹H NMR(DMSO-d₆)δ 12.12(1H, brs), 9.95(1H, s), 7.18(1H, d, J = 8.2 Hz), 7.14(1H, s), 6.90(2H, d, J = 8.4), 6.44(2H, brs), 4.75(2H, s), 4.14(2H, t, J = 6.6 Hz), 3.71(3H, s), 3.43(2H, s), 1.62(2H, 5, J = 7.0 Hz), 1.37(2H, 6, J = 7.5 Hz), 0.90(3H, t, J = 7.4 Hz).

### Comparative example 23

### 9-(3-Carboxymethylbenzyl)-2-ethoxy-8-hydroxyadenine

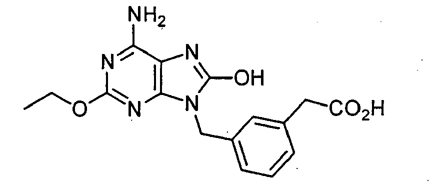

Using 2-ethoxy-8-hydroxy-9-(3-methoxycarbonylmethylbenzyl)adenine which was prepared in Example 33), the same procedure as in Comparative example 1 was carried out to give the titled compound as a white solid. Yield: 79%
¹H NMR(DMSO-d₆)δ 10.60(1H, brs), 7.25-7.20(2H, m), 7.15-7.11(2H, m), 6.63(2H, brs), 4.82(2H, s), 4.19(2H, q, J = 7.0 Hz), 3.45(2H, s), 1.25(3H, t, J = 7.0 Hz).

### Comparative example 24

### 9-(3-Carboxymethylbenzyl)-8-hydroxy-2-propoxyadenine

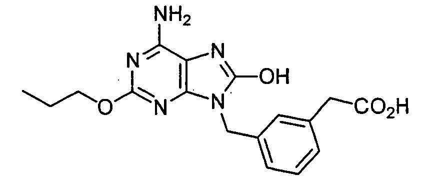

Using 8-hydroxy-9-(3-methoxycarbonylmethylbenzyl)-2-propoxyadenine which was prepared in Example 34, the same procedure as in Comparative example 1 was carried out to give the titled compound as a white solid. Yield: 56%
¹H NMR(DMSO-d₆)δ12.33(1H, brs), 9.96(1H, s), 7.30-7.25(2H, m), 7.21-7.15(2H, m), 6.46(2H, brs), 4.92(2H, s), 4.10(2H, t, J = 6.7Hz), 3.59(2H, s), 1.70-1.62(2H, m), 0.93(3H, t, J = 7.4Hz).

### Comparative example 25

### 9-(3-Carboxymethylbenzyl)-8-hydroxy-2-pentoxyadenine

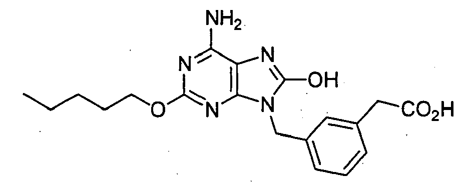

Using 8-hydroxy-9-(3-methoxycarbonylmethylbenzyl)-2-pentoxyadenine which was prepared in Example 35, the same procedure as in Comparative example 1 was carried out to give the titled compound as a white solid. Yield: 79%
¹H NMR(DMSO-d₆)δ12.33(1H, brs), 9.96(1H, s), 7.26(1H, t, J = 7.5 Hz), 7.21-7.12(3H, m), 6.46(2H, brs), 4.84(2H, s), 4.14(2H, t, J = 6.6Hz), 3.53(2H, s), 1.68-1.61(2H, m), 1.34-1.29(2H, m), 0.88(3H, t, J = 7.0Hz).

### Comparative example 26

### 9-(3-Carboxymethylbenzyl)-8-hydroxy-2-(2-phenyloxyethoxy)adenine

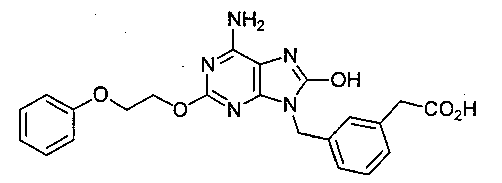

Using 8-hydroxy-9-(3-methoxycarbonylmethylbenzyl)-2-(2-phenyloxyethoxy)adenine which was prepared in Example 51, the same procedure as in Comparative example 1 was carried out to give the titled compound as a white solid. Yield: 72%
¹H NMR(DMSO-d₆)δ 12.31(1H, brs), 10.07(1H, s), 7.31-7.15(6H, m), 6.96-6.92(3H, m), 6.53(2H, brs), 4.84(2H, s), 4.48(2H, t, J = 4.6Hz), 4.25(2H, t, J = 4.6Hz), 3.52(2H, s).

### Comparative example 27

### 2-[3-(N-Acetylamino)propoxy]-9-(3-carboxymethylbenzyl)-8-hydroxyadenine

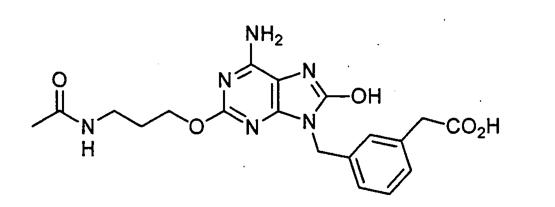

Using 2-[3-(N-acetylamino)propoxy]-8-hydroxy-9-(3-methoxycarbonylmethylbenzyl)adenine which was prepared in Example 53, the same procedure as in Comparative example 1 was carried out to give the titled compound as a white solid. Yield: 69%
¹H NMR(DMSO-d₆)δ12.28(1H, brs), 9.98(1H, brs), 7.90(1H, t, J = 5,1Hz), 7.27(1H, t, J = 7.6 Hz), 7.21-7.10(3H, m), 6.47(2H, brs), 4.84(2H, s), 4.15(2H, t, J = 6.3 Hz), 3.53(2H, s), 3.13(2H, m), 1.78(3H, s), 1.79-1.73(2H, m).

### Comparative example 28

### 9-(3-Carboxymethylbenzyl)-8-hydroxy-2-[3-(N-methanesulfonylamino)propoxy] adenine

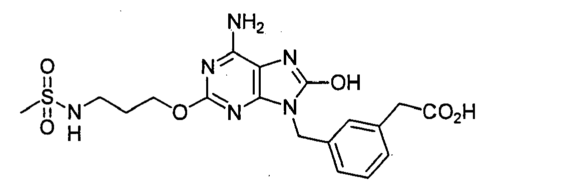

Using 8-hydroxy-2-[3-(N-methanesulfonyllamino)propoxy]-9-(3-methoxycarbonylmethylbenzyl)adenine which was prepared in Example 54, the same procedure as in Comparative example 1 was carried out to give the titled compound as a white solid. Yield: 99%
¹H NMR(DMSO-d₆)δ12.29(1H brs), 9.98(1H, brs), 7.28(1H, m), 7.21-7.15(3H, m), 7.04(1H, t, J = 5.8Hz), 6.49(2H, brs), 4.84(2H, s), 4.20(2H, t, J = 6.2Hz), 3.53(2H, s), 3.06(2H, m), 2.88(3H, s), 1.88-1.84(2H, m).

### Comparative example 29

### 9-(3-Carboxymethylbenzyl)-2-cyclopentyl-8-hydroxyaderune

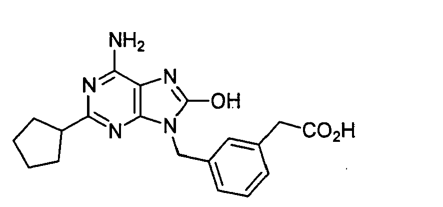

Using 2-cyclopentyl-8-hydroxy-9-(3-methoxycarbonylmethylbenzyl)adenine which was prepared in Example 64, the same procedure as in Comparative example 1 was carried out to give the titled compound as a white solid. Yield: 83%
¹H NMR(DMSO-d₆)δ12.32(1H, brs), 10.30(1H, brs), 7.25(1H, dd, J = 7.6, 7.6 Hz), 7.25(1H, s), 7.18(1H, d, J = 7.6 Hz), 7.14(1H, d, J = 7.6 Hz), 6.46(2H, brs), 4.86(2H, s), 3.57(2H, s), 3.01(1H, quin, J = 8.2 Hz), 1.93-1.84(2H, m), 1.84-1.77(2H, m), 1.77-1.67(2H, m), 1.62-1.52(2H, m).

### Comparative example 30

### 9-(3-Carboxymethylbenzyl)-8-hydroxy-2-(3-hydroxypropane-1-yl)adenine

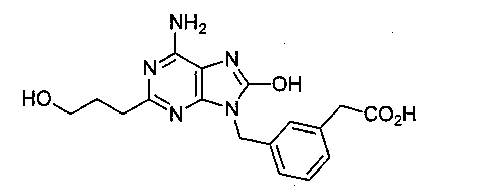

Using 8-hydroxy-2-(3-hydroxypropyl)-9-(3-methoxycarbonylmethylbenzyl)adenine which was prepared in Example 65, the same procedure as in Comparative example 1 was carried out to give the titled compound as a yellow solid. Yield: 71%
¹H NMR(DMSO-d₆)δ12.40(1H, brs), 10.11(1H, brs), 7.25(1H, dd, J = 7.6, 7.6 Hz), 7.21(1H, s), 7.40(2H, d, J =7.6 Hz), 6.36(2H, brs), 4.87(2H, s), 3.52(2H, s), 3.41(2H, t, J = 6.5 Hz), 2.59(2H, t, J = 7.6 Hz), 1.80(2H, tt, J = 7.6, 6.5 Hz).

### Comparative example 31

### 9-(3-Carboxymethylbenzyl)-8-hydroxy-2-(2-pyridylmethoxy)adenine

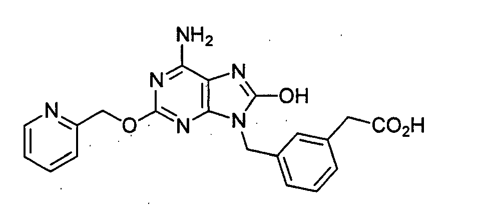

Using 8-hydroxy-9-(3-methoxycarbonylmethylbenzyl)-2-(2-pyridylmethoxy)adenine which was prepared in Example 68, the same procedure as in Comparative example 1 was carried out to give the titled compound as a white solid. Yield: 87%
¹H NMR(DMSO-d₆)δ12.57(1H, brs), 10.09(1H, brs), 8.58(1H, d, J = 4.8 Hz), 7.88(1H, dd, J = 7.8, 7.4 Hz), 7.31(1H, d, J = 7.8 Hz), 7.41(1H, dd, J = 7.4, 4.8 Hz), 7.22(1H, dd, J = 7.6, 7.6 Hz), 7.18(1H, s), 7.14(1H, d, J = 7.6 Hz), 7.09(1H, d, J = 7.6 Hz), 6.60(2H, brs), 5.37(2H, s), 4.83(2H, s), 3.52(2H, s).

### Comparative example 32

### 9-(3-Carboxymethylbenzyl)-8-hydroxy-2-(3-pyridylmethoxy)adenine

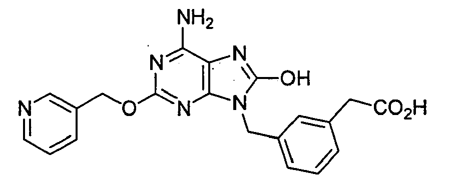

Using 8-hydroxy-9-(3-methoxycarbonylmethylbenzyl)-2-(3-pyridylmethoxy)adenine which was prepared in Example 68, the same procedure as in Comparative example 1 was carried out to give the titled compound as a white solid. Yield: 87%
¹H NMR(DMSO-d₆)δ12.84(1H, brs), 10.06(1H, brs), 8.75(1H, s), 8.60(1H, d, J = 4.8 Hz), 8.03(1H, d, J = 7.5 Hz), 7.54(1H, dd, J = 7.5, 4.8 Hz), 7.27(1H, dd, J = 7.6, 7.6 Hz), 7.21(1H, s), 7.14(1H, d, J = 7.6 Hz), 7.12(1H, d, J = 7.6 Hz), 6.59(2H, brs), 5.32(2H, s), 4.83(2H, s), 3.53(2H, s).

### Comparative example 33

### 9-(3-Carboxymethylbenzyl)-8-hydroxy-2-(2-phenylsulfanylethoxy)adenine

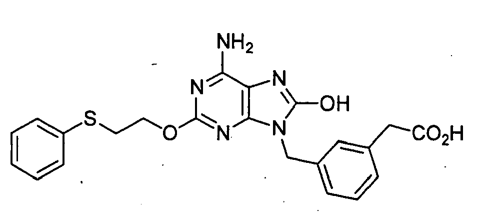

Using 8-hydroxy-9-(3-methoxycarbonylmethylbenzyl)-2-{2-(phenylsulfanyl)ethoxy}adenine which was prepared in Example 70, the same procedure as in Comparative example 1 was carried out to give the titled compound as a white solid. Yield: 76%
¹H NMR(DMSO-d₆)δ13.00(1H, brs), 10.78(1H, brs), 7.40-7.37(2H, m), 7.33-7.28(2H, m), 7.20-7.13(3H, m), 7.12(1H, d, J = 7.6 Hz), 7.07(1H, d, J = 7.5 Hz), 6.82(2H, brs), 4.79(2H, s), 4.29(2H, t, J = 6.9 Hz), 3.40(2H, s), 3.30(2H, t,J=6.9Hz).

### Comparative example 34

### 9-(3-Carboxymethylbenzyl)-8-hydroxy-2-(tetrahydrofuran-2-ylmethoxy) adenine

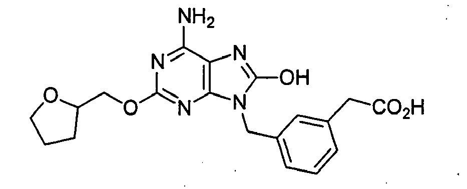

Using 8-hydroxy-9-(3-methoxycarbonylmethylbenzyl)-2-(tetrahydrofuran-2-ylmethoxy)adenine which was prepared in Example 73, the same procedure as in Comparative example 1 was carried out to give the titled compound as a white solid. Yield: 35%
¹H NMR(DMSO-d₆)δ12.80(1H, brs), 10.10(1H, brs), 7.25(1H, dd, J = 7.6, 7.6 Hz), 7.19(1H, s), 7.13(2H, d, J = 7.6 Hz), 6.52(2H, brs), 4.81(2H, s), 4.14-4.08(2H, m), 4.13-4.16(1H, m), 3.78-3.71(1H, m), 3.67-3.61(1H, m), 3.50(2H, s), 1.96-1.89(1H, m), 1.86-1.80(2H, m), 1.65-1.58(1H, m).

### Reference example 1

### 2-Chloro-9-(3-methoxycarbonylmethylbenzyl)adenine

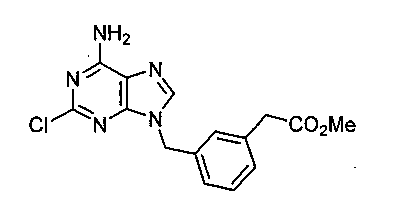

2-Chloroadenine (1.70g, 10.0mmol) and potassium carbonate (9.67 g, 70.0 mmol) were added to DMF (35ml) and the solution was stirred at 60°C for 1.5 hours. After being cooled, methyl 3-bromomethylphenylacetate (3.16g, 13.0 mmol) was added thereto and the mixture was stirred at room temperature for 1.5 hours. After removal of the solvent, thereto was added chloroform (50ml) and the precipitated solid was taken by filtration and washed with water to give the titled compound as a pale yellow solid (2.13g, 6.41mmol). Yield: 64%
¹H NMR(DMSO-d₆)δ 8.24(1H, s), 7.80(2H, brs), 7.31(1H, dd, J = 7.6, 7.6 Hz), 7.19(1H, d, 7.6 Hz), 7.18(1H, s), 7.14(1H, d, 7.6 Hz), 5.32(2H, s), 3.66(2H, s), 3.59(3H, s).

### Reference example 2

### 8-Bromo-2-chloro-9-(3-methoxycarbonylmethylbenzyl)adenine

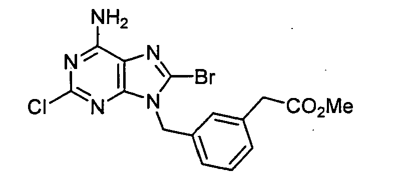

2-Chloro-9-(3-methoxycarbonylmethylbenzyl)adenine (2.95 g, 36.0 mmol) which was prepared in Reference example 1 (2.00g, 6.03mmol) and sodium acetate were added to chloroform (100ml) and thereto was dropped bromine (4.79g, 30.0mmol), followed by stirring at room temperature for 5 hours. To the reaction mixture was added water and the mixture was extracted with chloroform. The organic layer was washed with an aqueous saturated sodium bicarbonate solution, an aqueous saturated sodium hydrogen sulfite solution and saturated brine in this order, dried over anhydrous magnesium sulfate and concentrated to give the titled compound as a brown solid (1.78g, 4.34mmol). Yield: 72%
¹H NMR(CDCl₃)δ 7.32(1H, dd, J = 8.0, 7.6 Hz), 7.26-7.19(3H, m), 5.72(2H, brs), 5.34(2H, s), 3.70(3H, s), 3.61(2H, s).

### Reference example 3

### 2-Chloro-8-hydroxy-9-(3-methoxycarbonylmethylbenzyl)adenine

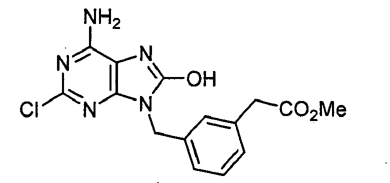

8-Bromo-2-chloro-9-(3-methoxycarbonylmethylbenzyl)adenine (1.78g, 4.34mmol) which was prepared in Reference example 2 was suspended in a mixture of 1N sodium hydroxide solution (150ml) and methanol (150ml), and the suspension was stirred at 100°C for 30 minutes. After neutralizing with 12N hydrochloric acid, the solvent was removed and to the residue were added methanol (50ml) and concentrated sulfuric acid (2.45g, 25.0mmol), followed by refluxing for 1 hour. After neutralizing with an aqueous saturated sodium bicarbonate solution, the solution was extracted with chloroform and the organic layer was dried over anhydrous magnesium sulfate and concentrated. The residue was purified by column chromatography (SiO₂ 90.0g, elute: CHCl₃/MeOH=100/0~50/1) to give the titled compound as a white solid (0.84g, 2.41mmol). Yield: 56%
¹H NMR(DMSO-d₆)δ 10.37(1H, brs), 7.29(1H, dd, J = 8.0, 4.8 Hz), 7.18-7.12(3H, m), 6.91(2H, brs), 4.88(2H, s), 3.65(2H, s), 3.58(3H, s).

### Reference example 4

### 2-(3-Hydroxypropoxy)-9-(3-methoxycarbonylmethylbenzyl)adenine

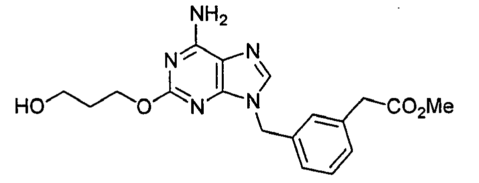

After sodium (0.43g, 18.70mmol) was dissolved in 1,3-dipropanol (15ml), thereto was added 2-chloro-9-(3-methoxycarbonylmethylbenzyl)adenine (0.53g, 1.60 mmol) which was prepared in Reference example 1 and the mixture was stirred at 100°C for 5 hours. After neutralizing with 12N hydrochloric acid, the solvent was removed and to the residue were added methanol (100ml) and concentrated sulfuric acid (5ml), followed by refluxing for 5 hours. After neutralizing with an aqueous saturated sodium bicarbonate solution, the solution was extracted with chloroform, dried over anhydrous magnesium sulfate and concentrated. The residue was purified by column chromatography (SiO₂ 20g, elute: CHCl₃/MeOH=100/0~30/1) to give the titled compound as a pale yellow solid (0.39g, 1.05mmol). Yield: 66%
¹H NMR(DMSO-d₆)δ8.02(1H, s), 7.29(1H, t, J = 7.6 Hz), 7.20(5H, m), 5.24(2H, s), 4.51(1H, t, J = 5.2 Hz), 4.26(2H, t, J = 6.5 Hz), 3.65(2H, s), 3.58(3H, s), 3.52(2H, q, J = 5.2 Hz), 1.82(2H, qui, J = 6.4 Hz).

### Reference example 5

### 8-Bromo-2-(3-hydroxypropoxy)-9-(3-methoxycarbonylmethylbenzyl)adenine

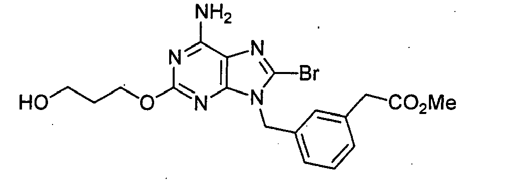

The titled compound as a brown solid was prepared in the same method as in Reference example 2. Yield: 94%
¹H NMR(DMSO-d₆)δ7.43(2H, brs), 7.29(1H, t, J = 7.6 Hz), 7.19(1H, d, J = 7.6 Hz), 7.18(1H, s), 7.09(1H, d, J = 7.8 Hz), 5.34(2H, s), 4.51(1H, t, J = 5.1 Hz), 4.26(2H, t, J = 6.5 Hz), 3.65(2H, s), 3.58(3H, s), 3.52(2H, q, J = 5.3 Hz), 1.81 (2H, qui, J = 6.4 Hz).

### Reference example 6

### 2-(2-Hydroxyethoxy)-9-(3-methoxycarbonylmethylbenzyl)adenine

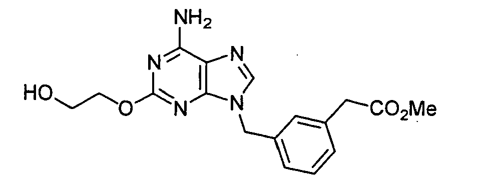

The titled compound as a white solid was prepared in the same method as in Reference example 4. Yield: 94%
¹H NMR(DMSO-d₆)δ8.22(1H, s), 7.65(2H, s), 7.30(1H, t, J = 7.6 Hz), 7.26(1H, s), 7.20(2H, m), 5.28(2H, s), 4.29(2H, t, J = 5.0 Hz), 3.69(2H, t, J = 5.3 Hz), 3.66(2H, s), 3.61(3H, s).

### Reference example 7

### 8-Bromo-2-(2-hydroxyethoxy)-9-(3-methoxycarbonylmethylbenzyl)adenine

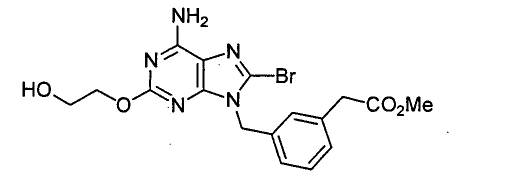

The titled compound as a white solid was prepared in the same method as in Reference example 2. Yield: 63%
¹H NMR(DMSO-d₆)δ7.44(2H, brs), 7.30(1H, t, J = 7.6 Hz), 7.19(1H, t, J = 7.6 Hz), 7.18(1H, s), 7.11(1H, t, J = 7.7 Hz), 5.23(2H, s), 4.81(1H, t, J = 5.6 Hz), 4.22(2H, t, J = 5.4 Hz), 3.67(4H, m), 3.58(3H, s).

### Reference example 8

### 9-(3-Methoxycarbonylmethylbenzyl)-2-(4,4,4-trifluorobutoxy)adenine

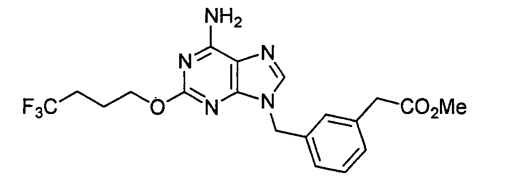

The titled compound as a yellow solid was prepared in the same method as in Reference example 4. Yield: 73%
¹H NMR(DMSO-d₆)δ8.04(1H, s), 7.24(6H, m), 5.24(2H, s), 4.27(2H, t, J = 6.3 Hz), 3.65(2H, s), 3.58(3H, s), 2.37(2H, m), 1.91(2H, m).

### Reference example 9

### 8-Bromo-9-(3-ethoxycarbonylmethylbenzyl)-2-(4,4,4-trifluorobutoxy) adenine

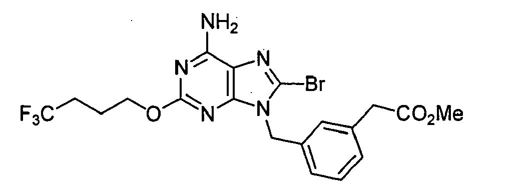

The titled compound as a white solid was prepared in the same method as in Reference example 2. Yield: 94%
¹H NMR(DMSO-d₆)δ7.50(2H, brs), 7.30(1H, t, J = 7.9 Hz), 7.19(1H, d, J = 7.6 Hz), 7.18(1H, s), 7.10(1H, d, J = 7.7 Hz), 5.24(2H, s), 4.27(2H, t, J = 6.3 Hz), 3.65(2H, s), 3.58(3H, s), 2.37(2H, m), 1.91(2H, m).

### Reference example 10

### 9-(3-Carboxymethylbenzyl)-2-chroloadenine

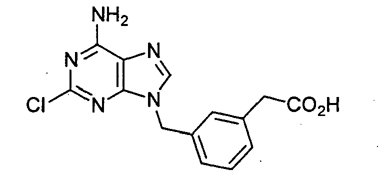

2-Chloro-9-(3-methoxycarbonylmethylbenzyl)adenine (600mg, 1.81mmol) which was prepared in Reference example 1 was dissolved in a mixture of 1N sodium hydroxide solution (18ml) and methanol (8ml) and the solution was stirred at room temperature for 6 hours. After neutralizing with 12N hydrochloric acid, the solvent was removed and to the residue was added water. The precipitated solid was taken by filtration to give the titled compound as a white solid (560mg, 1.76mmol).
Yield: 97%
¹H NMR(DMSO-d₆)δ8.24(1H, s), 7.79(2H, brs), 7.19(4H, m), 5.31(2H, s), 3.53(2H, s).

### Reference example 11

### 9-(3-Methoxycarbonylmethylbenzyl)-2-[N-(2-methoxyethyl)amino]adenine

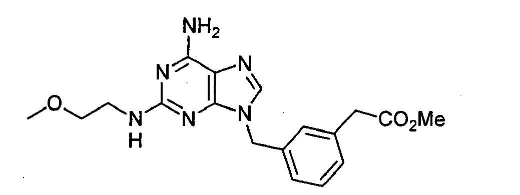

9-(3-Carboxymethylbenzyl)-2-chloroadenine (0.100g, 0.32mmol) which was prepared in Reference example 10 was added to 2-methoxyethylamine (3ml, 34.5mmol) and the mixture was stirred at 150°C for 4 hours in an autoclave. After removal of the solvent, thereto were added methanol (1ml) and concentrated sulfuric acid (0.2ml), and the mixture was refluxed for 2 hours. After neutralizing with an aqueous saturated sodium bicarbonate solution, the solution was extracted with chloroform, dried over anhydrous magnesium sulfate and concentrated. The residue was purified by column chromatography (SiO₂ 7.0g, elute: CHCl₃/MeOH=100/0~50/1) to give the titled compound as a brown oil (69 mg, 0.19 mmol). Yield: 59%
¹H NMR(DMSO-d₆)δ7.79(1H, s), 7.28(1H, t, J = 7.6 Hz), 7.22(1H, s), 7.28(2H, m), 6.69(2H, brs), 6.16(1H, brm), 5.16(2H, s), 3.64(2H, s), 3.52(4H, m), 3.23(3H, s).

### Reference example 12

### 8-Bromo-9-(3-methoxycarbonylmethylbenzyl)-2-[N-(2-methoxyethyl) amino] adenine

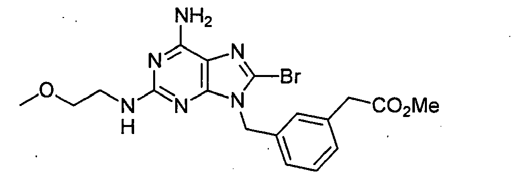

The titled compound as a brown solid was prepared in the same method as in Reference example 2. Yield: 88%
¹H NMR(DMSO-d₆)δ7.29(1H, t, J = 7.6 Hz), 7.18(3H, m), 6.91(2H, brs), 6.36(1H, brm), 5.16(2H, s), 3.65(2H, s), 3.58(3H, s), 3.41(4H, m), 3.23(3H, s).

### Reference example 13

### Methyl 3-vinylbenzoate

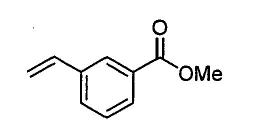

To a solution of 3-vinylbenzoic acid (3.0g, 20mmol) in DMF (15ml) were added methyl iodide (3.7ml, 60mmol) and potassium carbonate (4.1g, 30mmol) and the solution was stirred at room temperature for 2 hours. After confirming disappearance of the starting material by TLC, to the solution was added water and the solution was extracted with ethyl acetate (30 ml×3). The organic layer was concentrated to give the titled compound a colorless liquid (3.0g, 18.6 mmol). Yield: 92%
¹H NMR(CDCl₃)δ 8.08(1H, s), 7.93(1H, d, J = 7.7 Hz), 7.59(1H, d, J = 7.7 Hz), 7.39(1H, dd, J = 7.7, 7.7 Hz), 6.75(1H, dd, J = 17.6, 10.9 Hz), 5.83(1H, d, J = 17.6 Hz), 5.32(1H, d, J = 10.9 Hz), 3.93(3H, s).

### Reference example 14

### Methyl 3-(2-hydroxyethylbenzoate

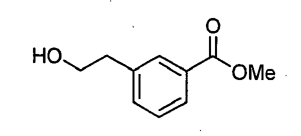

To methyl 3-vinylbenzoate (3.0g, 18.6mmol) which was prepared in Reference example 13 was dropped 9-BBN (0.5M THF solution) (50ml, 25mmol), and the mixture was stirred at room temperature for 15 hours. Then thereto were added at 0°C water (14ml) and 30% hydrgen peroxide solution (14ml) and the mixture was stirred at room temperature for 2 hours. After addition of aqueous 2N sodium hydroxide solution (3.75ml), the mixture was stirred for 2 hours. Then the reaction was quenched by adding at 0°C an aqueous saturated sodium thiosulfate solution, and the reaction mixture was extracted with ethyl acetate (30 ml×3). The organic layer was concentrated and the residue was purified by column chromatography (SiO₂ 110g, elute: Hex/AcOEt=2/1) to give the titled compound as a colorless liquid (2.8g, 15.4 mmol). Yield: 83%
¹H NMR(CDCl₃)δ 7.92(1H, s), 7.91(1H, d, J = 6.6 Hz), 7.46-7.37(2H, m), 3.93(3H, s), 3.90(2H, t, J = 6.5 Hz), 2.93(2H, t, J = 6.5 Hz), 1.50(1H, brs). Reference example 15

### Methyl 3-(2-methanesulfonyloxyethyl)benzoate

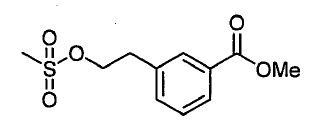

To a solution of methyl 3-(2-hydroxyethylbenzoate (2.8g, 15.4 mmol) which was prepared in Reference example 14 in THF (30ml) were added at 0°C methanesulfonyl chloride (1.4ml, 18.5 mmol) and triethylamine (2.6ml, 18.5mmol), and the solution was stirred at room temperature for 30 minutes. Thereto was added water and the mixture was extracted with ethyl acetate (30 ml×3). The organic layer was concentrated and the residue was purified by column chromatography (SiO₂ 90g, elute: Hex/AcOEt=3/1) to give the titled compound as a colorless liquid (3.4g, 13.1mmol). Yield: 85%
¹H NMR(CDCl₃)δ 7.96-7.94(1H, m), 7.92(1H, s), 7.45-7.41(2H, m), 4.44(2H, t, J = 6.8 Hz), 3.93(3H, s), 3.12(2H, t, J = 6.8 Hz), 2.89(3H, s).

### Reference example 16

### 2-Butoxy-9-[2-(3-methoxycarbonylphenyl)ethyl]adenine

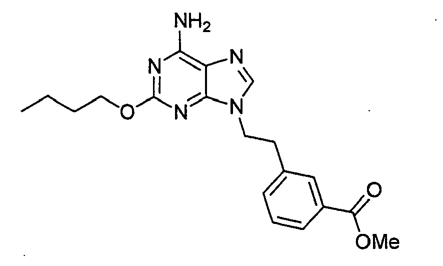

The titled compound as a white solid was prepared in the same method as in Reference example 1. Yield: 81%
¹H NMR(CDCl₃)δ 7.91(1H, d, J = 7.7 Hz), 7.85(1H, s), 7.33(1H, dd, J = 7.7, 7.6 Hz), 7.28(1H, s), 7.20(1H, d, J = 7.6 Hz), 5.59(2H, brs), 4.37(2H, t, J = 7.0 Hz), 4.33(2H, t, J = 6.6 Hz), 3.92(3H, s), 3.22(2H, t, J = 7.0 Hz), 1.80(2H, tt, J = 7.4 Hz, 6.6 Hz), 1.52(2H, tq, J = 7.4 Hz, 7.4 Hz), 0.99(3H, t, J = 7.4 Hz).

### Reference example 17

### 2-Butoxy-9-[2-(3-hydroxymethylphenyl)ethyl]adenine

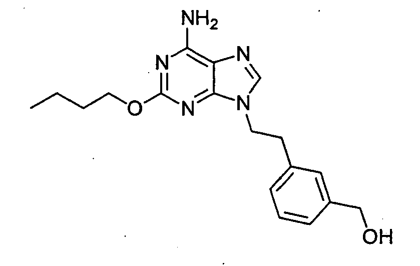

Aluminum lithium hydride (65mg, 1.71mmol) was added to THF (10ml) and thereto was dropped on an ice bath a solution of 2-butoxy-9-[2-(3-methoxycarbonylphenyl)ethyl]adenine (0.40g, 1.08mmo1) which was prepared in Reference example 16 in THF (20ml), followed by stirring at room temperature for 2 hours. To the mixture on an ice bath were dropped water (0.07ml), 1N sodium hydroxide solution (0.3ml), and water (0.3 ml) in this order. After filtration over celite, the filtrate was concentrated and the resulting crude crystals were recrystallized from chloroform/hexane to give the titled compound as a white solid (0.25g, 0.74mmol). Yield: 68%
¹H NMR(CDCl₃)δ 7.27-7.22(3H, m), 7.03(1H, s), 7.03-7.01(1H, m), 5.56(2H, brs), 4.64(2H, s), 4.34(2H, t, J = 6.9 Hz), 4.34(2H, t, J = 6.6 Hz), 3.15(2H, t, J = 6.9 Hz), 1.84-1.77(2H, m), 1.53(2H, tq, J = 7.4 Hz, 7.4 Hz), 0.99(3H, t, J = 7.4 Hz).

### Reference example 18

### 2-Butoxy-9-[2-(3-chloromethylphenyl)ethyl]adenine

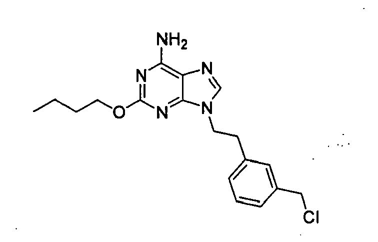

To a solution of 2-butoxy-9-[2-(3-hydroxymethylphenyl)ethyl]adenine (0.25g, 0.72mmol) which was prepared in Reference example 17 in chloroform (7.5ml) was added thionyl chloride (0.26ml, 3.6mmol), followed by relaxing for 1.5hours. After being cooled, the solution was neutralized with 5% aqueous sodium bicarbonate solution, and extracted with chloroform (30ml×3). The organic layer was concentrated to give the titled compound as a pale yellow liquid (0.25 g, 0.70 mmol). Yield: 97%
¹H NMR(CDCl₃)δ 7.28-7.23(3H, m), 7.12(1H, s), 7.03-7.00(1H, m), 5.76(2H, brs), 4.53(2H, s), 4.34(2H, t, J = 7.0 Hz), 4.34(2H, t, J = 6.6 Hz), 3.16(2H, t, J = 7.0 Hz), 1.80(2H, tt, J = 7.4 Hz, 6.6 Hz), 1.52(2H, tq, J = 7.4 Hz, 7.4 Hz), 0.99(3H, t, J = 7.4 Hz).

### Reference example 19

### 2-Butoxy-9-[2-(3-cyanomethylphenyl)ethyl] adenine

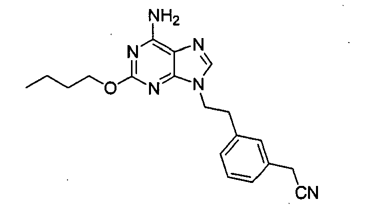

To a solution of 2-butoxy-9-[2-(3-chloromethylphenyl)ethyl]adenine (0.25g, 0.70mmol) which was prepared in Reference example 18 in DMF (7ml) was added sodium cyanate (0.10g, 2.1mmol) and the mixture was stirred at room temperature for 6 hours. After neutralizing with 1N hydrochloric acid (1.4ml), the solution was concentrated by an evaporator to remove DMF. The residue was extracted with chloroform (30 ml×3), and the extract was concentrated. The crude crystals were recrystallized from chloroform /hexane to give the titled compound as a white solid (0.20g, 0.59 mmol). Yield: 84%
¹H NMR(CDCl₃)δ 7.52-7.26(2H, m), 7.19(1H, d, J = 8.2 Hz), 7.05-6.99(2H, m), 5.50(2H, brs), 4.34(2H, t, J = 7.0 Hz), 4.34(2H, t, J = 6.6 Hz), 3.70(2H, s), 3.17(2H, t, J = 7.0 Hz), 1.84-1.76(2H, m), 1.57-1.47(2H, m), 0.99(3H, t, J = 7.4 Hz).

### Reference example 20

### 2-Butoxy-9-[2-(3-methoxycarbonylmethylphenyl)ethyl]adenine

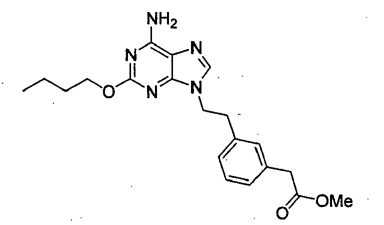

To 2-butoxy-9-[2-(3-cyanomethylphenyl)ethyl]adenine (0.20g, 0.57mmol) which was prepared in Reference example 19 were added methanol (6ml) and 5N sodium hydroxide solution (6ml) and the solution was refluxed for 3 hours. After neutralizing with concentrated hydrochloric acid at 0°C, the precipitated white solid was taken by filtration, washed with water and dried in vacuo for 12 hours. Thereto were added methanol (6ml) and concentrated sulfuric acid (0.2ml), and the solution was refluxed for 1 hour. After neutralizing with an aqueous 5% sodium bicarbonate solution, the precipitated solid was taken by filtration and washed with water to give the titled compound as a white solid (0.18g, 0.46mmol). Yield:81%
¹H NMR(CDCl₃)δ 7.29(1H, s), 7.24(1H, dd, J = 7.6, 7.6 Hz), 7.15(1H, d, J = 7.6 Hz), 7.03(1H, s), 6.97(1H, d, J = 7.6 Hz), 5.79(2H, brs), 4.34(2H, t, J = 6.7 Hz), 4.34(2H, t, J = 6.7 Hz), 3.69(3H, s), 3.58(2H, s), 3.14(2H, t, J = 7.0 Hz), 1.80(2H, tt, J = 7.5 Hz, 6.7 Hz), 1.52(2H, tq, J = 7.5 Hz, 7.4 Hz), 0.99(3H, t, J = 7.4 Hz).

### Reference example 21

### 8-Bromo-2-butoxy-9-[2-(3-methoxycarbonylmethylphenyl)ethyl]adenine

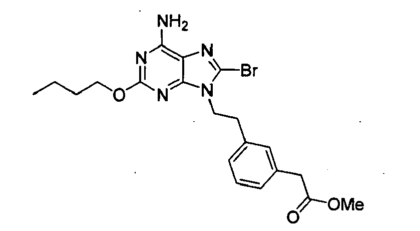

The titled compound was prepared in the same method as in Reference example 2. Yield: 88%
¹H NMR(CDCl₃)δ 7.25(1H, dd, J = 7.7, 7.6 Hz), 7.15(1H, d, J = 7.7 Hz), 7.07(1H, s), 7.05(1H, d, J = 7.6 Hz), 5.57(2H, brs), 4.33(2H, t, J = 7.5 Hz), 4.31(2H, t, J = 6.6 Hz), 3.70(3H, s), 3.59(2H, s), 3.10(2H, t, J = 7.5 Hz), 1.79(2H, tt, J = 7.6 Hz, 6.6 Hz), 1.52(2H, tq, J = 7.6 Hz, 7.4 Hz), 0.99(3H, t, J=7.4Hz).

### Reference example 22

### tert-Butyl 3-allylbenzoate

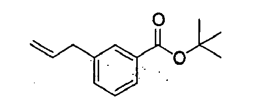

To a solution of isopropylmagnesium bromide (0.76M THF solution, 26ml, 20mmol) in THF (16ml) was dropped at 0°C butyllithium (1.59M hexane solution, 25ml, 40mmol), and the mixture was stirred for 15 minutes, followed by stirring for 20 minutes at -78°C. Thereto was dropped a solution of tert-butyl 3-bromobenzoate (2.0g, 8.0mmol) in THF (16ml) and the solution was stirred at -78°C for 30minutes. Thereto were added aryl bromide (2.8ml, 32mmol) and copper (I)cyanide (1M THF solution, 2.4ml, 2.4mmol) and the mixture was stirred for additional 1 hour. The reaction was quenched with an aqueous saturated ammonium chloride solution and extracted with hexane (30 ml×3). The organic layer was concentrated and the residue was purified by column chromatography (SiO₂ 60 g, elute: Hex/AcOEt=300/1) to give the titled compound as a colorless liquid (1.0g, 4.6mmol). Yield: 58%
¹H NMR(CDCl₃)δ 7.84-7.82(2H, m), 7.36-7.26(2H, m), 6.00-5.92(1H, m), 5.12-5.06(2H, m), 3.43(2H, d, J = 6.7 Hz), 1.60(9H, s).

### Reference example 23

### tert-Butyl 3-(3-hydroxypropyl)benzoate

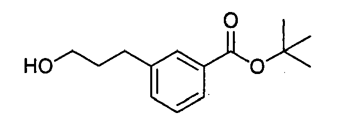

The titled compound was prepared in the same method as in Reference example 14. Yield: 60%
¹H NMR(CDCl₃)δ 7.83-7.78(2H, m), 7.38-7.31(2H, m), 3.68(2H, t, J = 6.4 Hz), 2.76(2H, t, J = 7.6 Hz), 1.91(2H, tt, J = 7.6 Hz, 6.4 Hz), 1.60(9H, s), 1.30(1H, brs).

### Reference example 24

### tert-Butyl 3-(3-methanesulfonyloxypropyl)benzoate

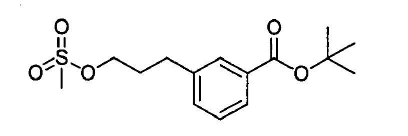

The titled compound was prepared in the same method as in Reference example 15. Yield: 100%
¹H NMR(CDCl₃)δ 7.86-7.82(1H, m), 7.82(1H, s), 7.37-7.35(2H, m), 4.23(2H, t, J = 6.3 Hz), 3.01(3H, s), 2.80(2H, t, J = 7.6 Hz), 2.10(2H, tt, J = 7.6 Hz, 6.3 Hz), 1.60(9H, s).

### Reference example 25

### 2-Butoxy-9-[3-(3-tert-butoxycarbonylphenyl)propyl]adenine

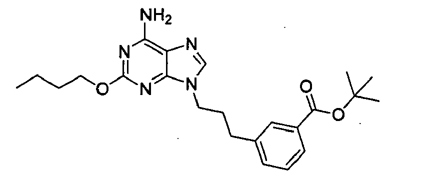

The titled compound was prepared in the same method as in Reference example 1. Yield: 72%
¹H NMR(CDCl₃)δ 7.85-7.82(1H, m), 7.80(1H, s), 7.59(1H, s), 7.34-7.32(2H, m), 5.51(2H, brs), 4.31(2H, t, J = 6.6 Hz), 4.13(2H, t, J = 7.1 Hz), 2.71(2H, t, J = 7.7 Hz), 2.26(2H, tt, J = 7.7 Hz, 7.1 Hz), 1.79(2H, tt, J = 7.6 Hz, 6.6 Hz), 1.60(9H, s), 1.52(2H, tq, J = 7.6 Hz, 7.4 Hz), 0.97(3H, t, J = 7.4 Hz).

### Reference example 26

### 2-Butoxy-9-[3-(3-hydroxymethylphenyl)propyl]adenine

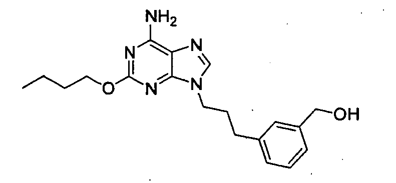

The titled compound was prepared in the same method as in Reference example 17. Yield: 97%
¹H NMR(CDCl₃)δ 7.53(1H, s), 7.25(1H, dd, J = 7.7, 7.5 Hz), 7.17(1H, d, J = 7.7 Hz), 7.14(1H, s), 7.06(1H, d, J = 7.5 Hz), 5.62(2H, brs), 4.66(2H, s), 4.31 (2H, t, J = 6.6 Hz), 4.11(2H, t, J = 7.0 Hz), 2.66(2H, t, J = 7.5 Hz), 2.56(1H, brs), 2.24(2H, tt, J = 7.5 Hz, 7.0 Hz), 1.79(2H, tt, J = 7.6 Hz, 6.6 Hz), 1.50(2H, tq, J = 7.6 Hz, 7.4 Hz), 0.97(3H, t, J = 7.4 Hz).

### Reference example 27

### 2-Butoxy-9-[3-(3-chloromethylphenyl)propyl] adenine

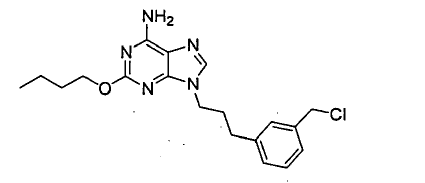

The titled compound was prepared in the same method as in Reference example 18. Yield: 100%
¹H NMR(CDCl₃)δ 7.59(1H, s), 7.28(1H, dd, J = 7.7, 7.4 Hz), 7.23(1H, d, J = 7.7 Hz), 7.19(1H, s), 7.13(1H, d, J = 7.4 Hz), 5.65(2H, brs), 4.56(2H, s), 4.32(2H, t, J = 6.6 Hz), 4.13(2H, t, J = 7.0 Hz), 2.67(2H, t, J = 7.6 Hz), 2.25(2H, tt, J = 7.6 Hz, 7.0 Hz), 1.79(2H, tt, J = 7.6 Hz, 6.6 Hz), 1.51(2H, tq, J = 7.6 Hz, 7.4 Hz), 0.97(3H, t, J = 7.4 Hz).

### Reference example 28

### 2-Butoxy-9-[3-(3-cyanomethylphenyl)propyl]adenine

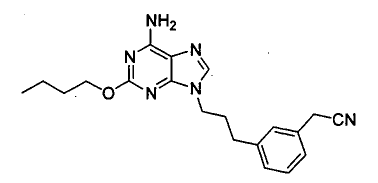

The titled compound was prepared in the same method as in Reference example 19. Yield: 85%
¹H NMR(CDCl₃)δ 7.59(1H, s), 7.29(1H, dd, J = 7.5, 7.5 Hz), 7.17-7.11(3H, m), 5.90(2H, brs), 4.32(2H, t, J = 6.6 Hz), 4.13(2H, t, J = 7.0 Hz), 3.72(2H, s), 2.67(2H, t, J = 7.6 Hz), 2.22(2H, tt, J = 7.6 Hz, 7.0 Hz), 1.78(2H, tt, J = 7.6 Hz, 6.6 Hz), 1.52(2H, tq, J = 7.6 Hz, 7.4 Hz), 0.97(3H, t, J = 7.4 Hz).

### Reference example 29

### 8-Bromo-2-butoxy-9-[3-(3-cyanomethylphenyl)propyl]adenine

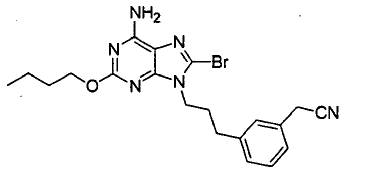

The titled compound was prepared in the same method as in Reference example 2. Yield: 85%
¹H NMR(CDCl₃)δ 7.29-7.25(1H, m), 7.15-7.13(3H, m), 5.41(2H, brs), 4.30(2H, t, J = 6.6 Hz), 4.17(2H, t, J = 7.2 Hz), 3.71(2H, s), 2.71(2H, t, J = 7.7 Hz), 2.19(2H, tt, J = 7.7 Hz, 7.2 Hz), 1.78(2H, tt, J = 7.6 Hz, 6.6 Hz), 1.52(2H, tq, J = 7.6 Hz, 7.4 Hz), 0.97(3H, t, J = 7.4 Hz).

### Reference example 30

### 2-(2,2-Dimethyl-[1,3]dioxolan-4-ylmethoxy)-9-(3-methoxycarbonylmethylbenzyl)adenine

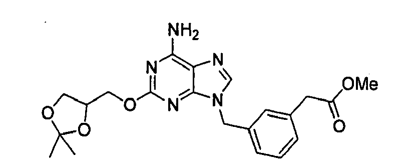

After sodium (0.69g, 30mmol) was dissolve in 2,2-dimethyl-1,3-dioxolane-4-methanol (30ml), thereto was added 2-chloro-9-(3-methoxycarbonylmethylbenzyl)adenine (1.0g, 3.0mmol) which was prepared in Reference example 1, followed by stirring at 120°C for 2 hours. After neutralizing with concentrated hydrochloric acid at 0°C, the solvent was removed. To the residue were added methanol (30ml) and concentrated sulfuric acid (2ml) and the solution was refluxed for 4 hours. After neutralizing with an aqueous 5% sodium bicarbonate solution, the solvent was removed and to the residue were added acetone (100ml) and p-toluenesulfonic acid (100mg). The solution was stirred at room temperature for 48 hours and neutralized with an aqueous 5% sodium bicarbonate solution. The solvent was removed and the residue was purified by column chromatography (SiO₂ 80g, elute: CHCl₃/MeOH=100/1) to give the titled compound as a pale yellow solid (0.80g, 1.87mmol).
Yield: 62%
¹H NMR(CDCl₃)δ 7.61(1H, s), 7.32-7.16(4H, m), 6.06(2H, brs), 5.26(2H, s), 4.52-4.47(2H, m), 4.31-4.26(1H, m), 4.16(1H, dd, J = 8.0, 6.6 Hz), 3.97-3.93(1H, m), 3.68(3H, s), 3.61(2H, s), 1.50(3H, s), 1.37(3H, s).

### Reference example 31

### 8-Bromo-2-(2,2-dimethyl-[1,3]dioxolan-4-ylmethoxy)-9-(3-methoxycarbonylmethylbenzyl) adenine

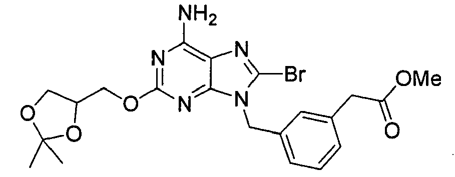

The titled compound was prepared in the same method as in Reference example 2. Yield: 82%
¹H NMR(CDCl₃)δ 7.30-7.20(4H, m), 6.30(2H, brs), 5.28(2H, s), 4.51-4.28(2H, m), 4.30(1H, dd, J = 10.5, 6.4 Hz), 4.15(1H, dd, J = 8.5, 6.4 Hz), 3.95(1H, dd, J = 8.4, 5.4 Hz), 3.68(3H, s), 3.60(2H, s), 1.48(3H, s), 1.39(3H, s).

### Reference example 32

### 2-(2-Ethoxyethoxy)-9-(3-methoxycarbonylmethylbenzyl)adenine

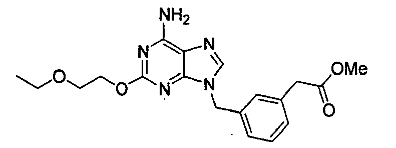

The titled compound as a brown solid was prepared in the same method as in Reference example 4. Yield: 83%
¹H NMR(CDCl₃)δ 7.62(1H, s), 7.30(1H, d, J = 7.5 Hz), 7.24-7.16(3H, m), 6.19(2H, brs), 5.26(2H, s), 4.49(2H, t, J = 5.1 Hz), 3.80(2H, t, J = 5.1 Hz), 3.68(3H, s), 3.60(2H, s), 3.59(2H, q, J = 7.0 Hz), 1.23(3H, t, J = 7.0 Hz).

### Reference example 33

### 8-Bromo-2-(2-ethoxyethoxy)-9-(3-methoxycarbonylmethylbenzyl)adenine

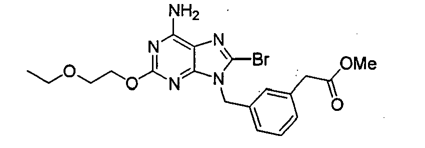

The titled compound was prepared in the same method as in Reference example 2. Yield: 90%
¹H NMR(CDCl₃)δ 7.29-7.25(2H, m), 7.22-7.20(2H, m), 6.31(2H, brs), 5.28(2H, s), 4.48(2H, t, J = 5.1 Hz), 3.79(2H, t, J = 5.1 Hz), 3.68(3H, s), 3.60(2H, s), 3.59(2H, q, J = 7.0 Hz), 1.24(3H, t, J = 7.0 Hz).

### Reference example 34

### 2-Cyclohexylmethoxy-9-(3-methoxycarbonylmethylbenzyl)adenine

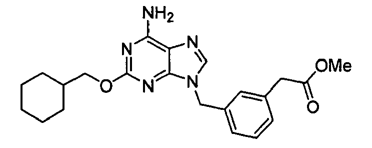

The titled compound was prepared in the same method as in Reference example 4. Yield: 39%
¹H NMR(CDCl₃)δ 7.60(1H, s), 7.31(1H, dd, J = 7.5, 7.5 Hz), 7.24(1H, d, J = 7.5 Hz), 7.23(1H, s), 7.18(1H, d, J = 7.5 Hz), 5.92(2H, brs), 5.26(2H, s), 4.19(2H, d, J = 6.4 Hz), 3.68(3H, s), 3.61(2H, s), 1.90-1.67(6H, m), 1.30-1.23(3H, m), 1.11-1.05(2H, m).

### Reference example 35

### 8-Bromo-2-cyclohexylmethoxy-9-(3-methoxycarbonylmethylbenzyl)adenine

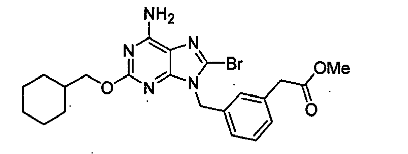

The titled compound was prepared in the same method as in Reference example 2. Yield: 90%
¹H NMR(CDCl₃)δ 7.31-7.27(2H, m), 7.24-7.21(2H, m), 5.86(2H, brs), 5.29(2H, s), 4.15(2H, d, J = 6.2 Hz), 3.68(3H, s), 3.60(2H, s), 1.90-1.67(6H, m), 1.30-1.23(3H, m), 1.11-1.04(2H, m).

### Reference example 36

### 2-Benzyloxy-9-(3-methoxycarbonylmethylbenzyl)adenine

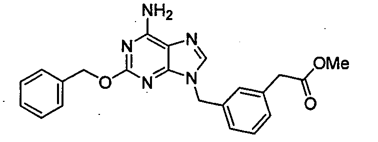

The titled compound was prepared in the same method as in Reference example 4. Yield: 72%
¹H NMR(CDCl₃)δ 7.62(1H, s), 7.49-7.46(2H, m), 7.35-7.16(7H, m), 5.98(2H, brs), 5.43(2H, s), 5.26(2H, s), 3.68(3H, s), 3.60(2H, s).

### Reference example 37

### 2-Benzyloxy-8-bromo-9-(3-methoxycarbonylmethylbenzyl)adenine)

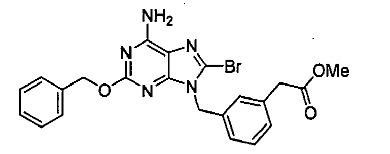

The titled compound was prepared in the same method as in Reference example 2. Yield: 89%
¹H NMR(CDCl₃)δ 7.47-7.45(2H, m), 7.36-7.17(7H, m), 5.91(2H, brs), 5.42(2H, s), 5.28(2H, s), 3.66(3H, s), 3.58(2H, s).

### Reference example 38

### 2-(2-Methoxycarbonylethyl)adenine

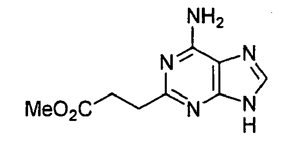

9-Benzyl-2-(2-methoxycarbonylethyl)adenine (0.29g, 0.93mmol) and 20% Pd(OH)₂/C (0.32g) were added to a mixture of isopropanol (8ml) and formic acid (8ml) and the mixture was stirred at a hydrogen atmosphere under 2 atm at 70°C for 40 hours. After filtration, the filtrate was concentrated to give the titled compound as a white solid (0.23g, 0.86mmol).
¹H NMR(DMSO-d₆)δ 12.70(1H, brs), 8.01(1H, s), 7.00(2H, brs), 3.58(3H, s), 2.91(2H, t, J = 7.1 Hz), 2.76(2H, t, J = 7.1 Hz).

### Reference example 39

### 2-(2-Methoxycarbonylethyl)-9-(3-methoxycarbonylmethylbenzyl)adenine

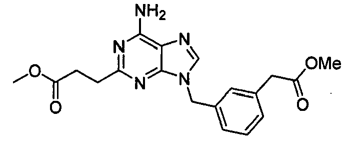

The titled compound was prepared in the same method as in Reference example 1. Yield: 77%
¹H NMR(CDCl₃)δ 7.71(1H, s), 7.31(1H, dd, J = 7.9, 7.7 Hz), 7.24-7.22(2H, m), 7.18(1H, d, J = 7.7 Hz), 5.94(2H, brs), 5.30(2H, s), 3.69(3H, s), 3.66(3H, s), 3.62(2H, s), 3.18(2H, t, J = 7.2 Hz), 2.88(2H, t, J = 7.2 Hz).

### Reference example 40

### 8-Bromo-2-(2-methoxycarbonylethyl)-9-(3-methoxycarbonylmethylbenzyl)adenine

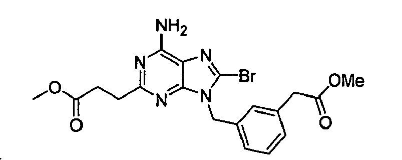

The titled compound was prepared in the same method as in Reference example 2. Yield: 85%
¹H NMR(CDCl₃)δ 7.30-7.26(2H, m), 7.23-7.21(2H, m), 6.19(2H, brs), 5.32(2H, s), 3.68(3H, s), 3.64(3H, s), 3.61(2H, s), 3.18(2H, t, J = 7.1 Hz), 2.87(2H, t, J = 7.1 Hz).

### Reference example 41

### 2-Butoxy-9-{(5-methoxycarbonyl-2-thienyl)methyl}adenine

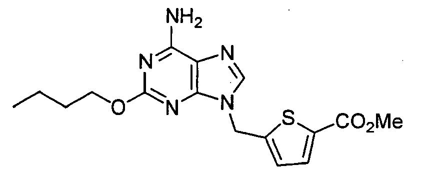

To a solution of 2-hydroxymethyl-5-methoxycarbonylthiophene (592mg, 3.44mmol), triethylamine (Et₃N) (0.70g, 6.92mmol) and 4-dimethylaminopyridine (DMAP) (84mg, 0.69mmol) in chloroform (34ml) was added on an ice bath tosyl chloride (TsCl) (1.31g, 6.87 mmol) and the mixture was stirred for 1 hour. The reaction solution was poured into a saturated sodium bicarbonate solution and the mixture was extracted with dichloromethane. The organic layer was washed with 0.5N hydrochloric acid and saturated brine, dried over anhydrous magnesium sulfate and concentrated to give the tosylated compound as a yellow oil (1.13g). 2-Butoxyadenine (0.58g, 2.84 mmol) and potassium carbonate (238mg, 1.72 mmol) were added to DMF (40 ml) and the solution was stirred at 60°C for 1 hour. After being cooled, thereto was added the tosylated compound and the mixture was stirred at room temperature for 26 hours, followed by addition of potassium carbonate (238mg, 1.72mmol) and stirring at 70°C for 4 hours. After removal of the solvent, the residue was poured into water and extracted with chloroform. The organic layer was dried over anhydrous magnesium sulfate and concentrated. The residue was purified by column chromatography (SiO₂ 40g, elute: CHCl₃/MeOH=100/1) to give the titled compound as a white solid (0.73g, 2.01 mmol). Yield: 71%
¹H NMR(DMSO-d₆)δ 8.06(1H, s), 7.67(1H, d, J = 3.7 Hz), 7.25(2H, brs), 7.19(1H, d, J = 3.7 Hz), 5.50(2H, s), 4.23(2H, t, J = 6.5 Hz), 3.77(3H, s), 1.66(2H, 5, J = 6.8 Hz), 1.39(2H, 6, J = 7.5 Hz), 0.92(3H, t, J = 7.3Hz).

### Reference example 42

### 8-Bromo-2-butoxy-9-{(5-methoxycarbonyl-2-thienyl)methyl}adenine

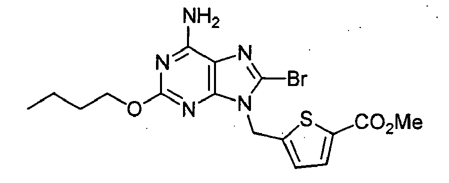

The titled compound as a yellowish white solid was prepared in the same method as in Reference example 2. Yield: 90%
¹H NMR(DMSO-d₆)δ 7.67(1H, d, J = 3.8 Hz), 7.46(2H, brs), 7.19(1H, d, J = 3.8 Hz), 5.46(2H, s), 4.23(2H, t, J = 6.5 Hz), 3.77(3H, s), 1.67(2H, 5, J = 6.6 Hz), 1.40(2H, 6, J = 7.5 Hz), 0.92(3H, t, J = 7.5 Hz).

### Reference example 43

### 2-Butoxy-8-hydroxy-9-{(5-methoxycarbonyl-2-thienyl)methyl}adenine

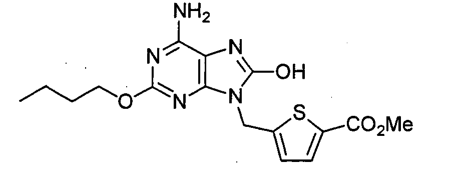

The titled compound as a white solid was prepared in the same method as in Example 4. Yield: 98% (total of 2 steps)
¹H NMR(DMSO-d₆)δ10.11(1H, brs), 7.65(1H, d, J = 3.8 Hz), 7.14(1H, d, J = 3.8 Hz), 6.53(2H, brs), 5.06(2H, s), 4.16(2H, t, J = 6.6 Hz); 3.78(3H, s), 1.63(2H, 5, J = 6.6 Hz), 1.37(2H, 6, J = 7.3 Hz), 0.90(3H, t, J = 7.3 Hz).

### Reference example 44

### 2-Butoxy-8-hydroxy-9-{(5-hydroxymethyl-2-thienyl)methyl}adenine

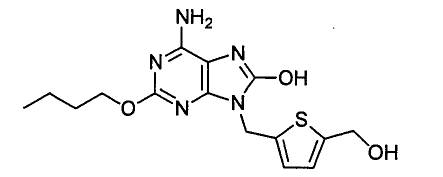

The titled compound as a white solid was prepared in the same method as in Reference example 17. Yield: 95%
¹H NMR(DMSO-d₆)δ 9.98(1H, brs), 6.89(1H, d, J = 3.5 Hz), 6.78(1H, d, J = 3.4 Hz), 6.47(2H, brs), 5.38(1H, t, J = 5.8 Hz), 4.94(2H, s), 4.51(2H, d, J = 5.6 Hz), 4.17(2H, t, J = 6.6 Hz), 1.65(2H, 5, J = 6.6 Hz), 1.38(2H, 6, J = 7.4 Hz), 0.92(3H, t, J = 7.3Hz).

### Reference example 45

### 2-Butoxy-9-{(2-methoxycarbonyl-4-pyridyl)methyl}adenine

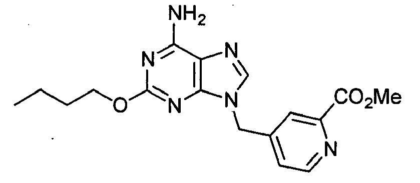

The titled compound as a brown oil was prepared in the same method as in Reference example 1. Yield: 80%
¹H NMR(DMSO-d₆)δ8.65(1H, dd, J = 0.4, 4.9 Hz), 8.09(1H, s), 7.94(1H, d, J = 0.9 Hz), 7.47(1H, dd, J = 1.5, 4.9 Hz), 7.27(2H, brs), 5.41(2H, s), 4.16(2H, t, J = 6.5 Hz), 3.85(3H, s), 1.60(2H, 5, J = 6.6 Hz), 1.35(2H, 6, J = 7.5 Hz), 0.88(3H, t, J = 7.3 Hz).

### Reference example 46

### 8-Bromo-2-butoxy-9-{(2-methoxycarbonyl-4-pyridyl)methyl}adenine

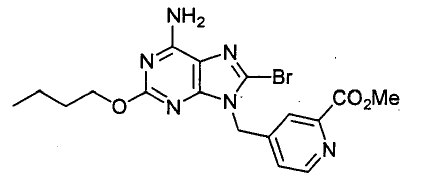

The titled compound as a pale yellow oil was prepared in the same method as in Reference example 2. Yield: 88%
¹H NMR(DMSO-d₆)δ8.67(1H, dd, J = 0.4, 5.0 Hz), 7.90(1H, d, J = 0.9 Hz), 7.49(2H, brs), 7.38(1H, dd, J = 1.7, 5.0 Hz), 5.40(2H, s), 4.18(2H, t, J = 6.6 Hz), 3.86(3H, s), 1.62(2H, 5, J = 6.6 Hz), 1.35(2H, 6, J = 7.5 Hz), 0.88(3H, t, J = 7.3 Hz).

### Reference example 47

### 2-Butoxy-8-methoxy-9-{(2-methoxycarbonyl-4-pyridyl)methyl}adenine

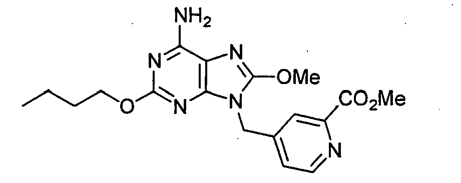

8-Bromo-2-butoxy-9-{(2-methoxycarbonyl-4-pyridyl)methyl}adenine (0.75g, 1.73mmol) which was prepared in Reference example 46 and sodium hydroxide (0.99g, 24.75mmol) were suspended in a mixture of water (6ml) and methanol (6ml), and the suspension was refluxed for 6 hours. After neutralizing with 12N hydrochloric acid, methanol was removed and the precipitated solid was taken by filtration. The solid was dissolved in a mixture of THF (100ml) and methanol (10ml) and thereto was added diazomethane ether which was prepared by a conventional method. The solution was stirred at room temperature for 2 hours. After removal of the solvent, the residue was purified by column chromatography (SiO₂ 50g, elute: CHCl₃/MeOH=200/0~30/1) to give the titled compound as a white solid (393mg, 1.01mmol). Yield: 61%
¹H NMR(DMSO-d₆)δ8.66(1H, d, J = 5.0 Hz), 7.88(1H, d, J = 0.8 Hz), 7.40(1H, dd, J = 1.6, 5.0 Hz), 6.93(2H, brs), 5.19(2H; s), 4.14(2H, t, J = 6.6 Hz), 4.03(3H, s), 3.86(3H, s), 1.60(2H, 5, J = 7.8 Hz), 1.35(2H, 6, J = 7.4 Hz), 0.88(3H, t, J = 7.3 Hz).

### Reference example 48

### 2-Butoxy-9-{(3-hydroxymethyl-4-pyridyl)methyl}-8-methoxyadenine

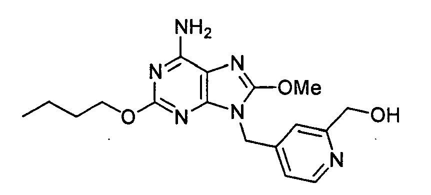

The titled compound was prepared in the same method as in Reference example 17. Yield: 78%
¹H NMR(DMSO-d₆)δ 8.42(1H, d, J = 4.9 Hz), 7.25(1H, s), 7.02(1H, d, J = 3.8 Hz), 6.91(2H, brs), 5.41(1H, t, J = 5.8 Hz), 5.09(2H, s), 4.50(2H, d, J = 5.8 Hz), 4.14(2H, t, J = 6.6 Hz), 3.59(3H, s), 1.61(2H, 5, J = 6.6 Hz), 1.38(2H, 6, J = 7.5 Hz), 0.89(3H, t, J = 7.3 Hz).

### Reference example 49

### 2-Butoxy-9-{(6-ethoxycarbonyl-2-pyridyl)methyl}adenine

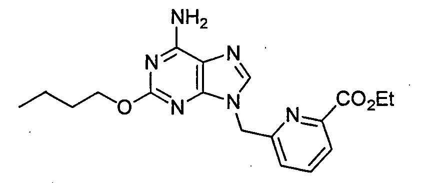

The titled compound as a colorless oil was prepared in the same method as in Reference example 1. Yield: 62%
¹H NMR(DMSO-d₆)δ8.06(1H, s), 7.94(2H, m), 7.30(1H, m), 7.26(2H, brs), 5.45(2H, s), 4.34(2H, q, J = 7.1 Hz), 4.12(2H, t, J = 6.6 Hz), 1.57(2H, 5, J = 6.6 Hz), 1.35(5H, m), 0.87(3H, t, J = 7.4 Hz).

### Reference example 50

### 8-Bromo-2-butoxy-9-{(6-ethoxycarbonyl-2-pyridyl)methyl}adenine

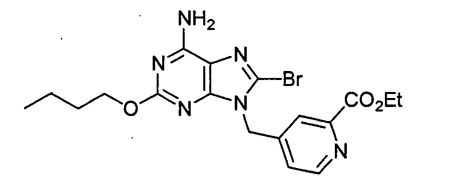

The titled compound as a yellowish red solid was prepared in the same method as in Reference example 2. Yield: 87%
¹H NMR(DMSO-d₆)δ7.96(2H, m), 7.47(2H, brs), 7.25(1H, m), 5.42(2H, s), 4.32(2H, q, J = 7.1 Hz), 4.13(2H, t, J = 6.6 Hz), 1.58(2H, 5, J = 6.6 Hz), 1.32(5H, m), 0.87(3H, t, J = 7.3 Hz).

### Reference example 51

### 2-Butoxy-9-{(6-hydroxymethyl-2-pyridyl)methyl}-8-methoxyadenine

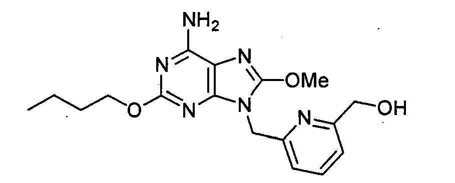

Using 8-bromo-2-butoxy-9-{(6-ethoxycarbonyl-2-pyridyl)methyl}adenine which was prepared in Reference example 50, the same procedures as in Reference example 46 and Reference example 17 were carried out in this order to give the titled compound as a pale yellow oil. Yield: 35%
¹H NMR(DMSO-d₆)δ 7.73(1H, t, J = 7.8 Hz), 7.36(1H, d, J = 7.7 Hz), 6.91(3H, m), 5.40(1H, t, J = 5.8 Hz), 5.10(2H, s), 4.50(2H, d, J = 5.8 Hz), 4.11(2H, t, J = 6.6 Hz), 4.01(3H, s), 1.59(2H, 5, J = 6.6 Hz), 1.35(2H, 6, J = 7.5 Hz), 0.88(3H, t, J = 7.3 Hz).

### Reference example 52

### 2-Butoxy-9-{(6-cyanomethyl-2-pyridyl)methyl}-8-hydroxyadenine

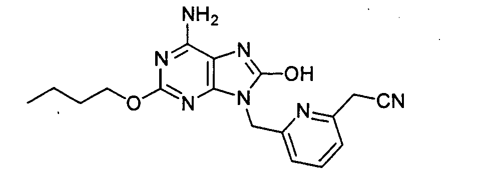

To 2-butoxy-9-{(6-hydroxymethyl-2-pyridyl)methyl}-8-methoxyadenine (0.67 mmol) which was prepared in Reference example 51 was added thionyl chloride (5ml) and the solution was refluxed with stirring for 1 hour. After the concentration, the residue was dissolved in DMF (14ml) and thereto was added sodium cyanate (164mg, 3.35mmol), followed by stirring at room temperature for 18 hours. After removal of the solvent, to the residue was added water. After neutralizing with 1N hydrochloric acid, the solution was extracted with chloroform. The extract was dried over anhydrous magnesium sulfate and concentrated. The residue was purified by column chromatography (SiO₂ 30g, elute: CHCl₃/MeOH=100/1~30/1) to give the titled compound as a yellowish red solid (133mg, 0.38 mmol). Yield: 57%
¹H NMR(DMSO-d₆)δ 10.00(1H, s), 7.78(1H, t, J = 7.8 Hz), 7.32(1H, d, J = 7.7 Hz), 7.06(1H, d, J = 7.8 Hz), 6.48(2H, s), 4.96(2H, s), 4.17(2H, s), 4.07(2H, t, J = 6.6 Hz), 1.57(2H, 5, J = 7.8 Hz), 1.32(2H, 6, J = 7.4 Hz), 0.87(3H, t, J = 7.4 Hz).

### Reference example 53

### 9-(4-Acetoxybenzyl)-2-butoxyadenine

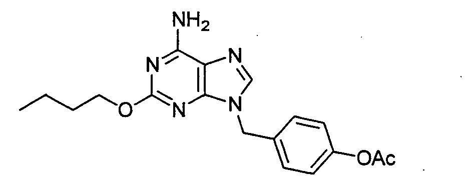

The titled compound as a pale yellow solid was prepared in the same method as in Reference example 1. Yield: 56%
¹H NMR(DMSO-d₆)δ8.04(1H, s), 7.34(2H, m), 7.20(2H, brs), 7.09(2H, m), 5.25(2H, s), 4.20(2H, t, J = 6.6 Hz), 1.65(2H, 5, J = 6.6 Hz), 1.39(2H, 6, J = 7.6 Hz), 0.91(3H, t, J = 7.4 Hz).

### Reference example 54

### 9-(4-Acetoxybenzyl)-8-bromo-2-butoxyadenine

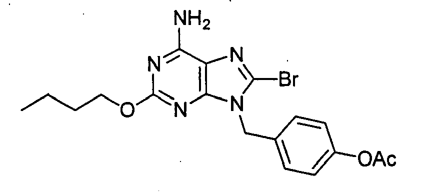

The titled compound as a yellowish red solid was prepared in the same method as in Reference example 2. Yield: 90%
¹H NMR(DMSO-d₆)δ7.39(2H, brs), 7.28(2H, d, J = 8.6 Hz), 7.19(2H, m), 5.25(2H, s), 4.21(2H, t, J = 6.6 Hz), 1.65(2H, 5, J = 6.8 Hz), 1.39(2H, 6, J = 7.6 Hz), 0.91(3H, t, J = 7.2 Hz).

### Reference example 55

### 8-Bromo-2-butoxyadenine

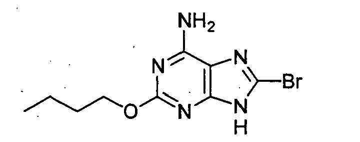

9-(4-Acetoxybenzyl)-8-bromo-2-butoxyadenine (1.04g, 2.39mmol) which was prepared in Reference example 54 was dissolved in a mixture of 1N sodium hydroxide solution (10ml) and methanol (10ml), and the solution was refluxed for 4 hours. After neutralizing with 12N hydrochloric acid, thereto was added water and the solution was extracted with chloroform. The organic layer was dried over anhydrous magnesium sulfate, concentrated and the residue was purified by column chromatography (SiO₂ 100g, elute: CHCl₃/MeOH=300/1~50/1) to give the titled compound as a pale red solid (0.56g, 1.94mmol). Yield: 81%
¹H NMR(DMSO-d₆)δ13.32(1H, brs), 7.23(2H, brs), 5.45(2H, s), 4.15(2H, q, J = 6.8 Hz), 1.64(2H, m), 1.38(2H, m), 0.92(3H, t, J = 7.2 Hz).

### Reference example 56

### Methyl 3-methyl-4-methoxybenzoate

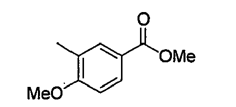

To a solution of 3-methyl-4-methoxybenzoic acid (2.9g, 17.5mmol) in methanol (50ml) was added concentrated sulfuric acid (1.5ml) and the solution was refluxed for 4 hours. After neutralizing with 5% aqueous sodium bicarbonate solution at 0°C, the precipitated solid was filtered to give the titled compound as a white solid (3.0g, 16.5mmol). Yield: 95%
¹H NMR(CDCl₃)δ 7.89(1H, d, J = 8.6 Hz), 7.83(1H, s), 6.83(1H, d, J = 8.6 Hz), 3.89(3H, s), 3.88(3H, s), 2.34(3H, s).

### Reference example 57

### Methyl 3-bromomethyl-4-methoxybenzoate

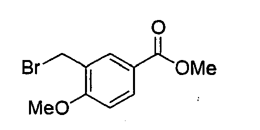

To a solution of methyl 3-methyl-4-methoxybenzoate (3.0g, 16.5mmol) which was prepared in Reference example 56 in carbon tertachloride (100ml) were added N-bromosuccinimide (2.9g, 16.5mmol) and benzoyl peroxide (0.10g), and the mixture was refluxed for 6 hours. After being cooled to 0°C, the precipitate was filtered, and to the filtrate was added a saturated aqueous sodium thiosulfate solution (1ml). The solution was stirred for 15 minutes and concentrated in vacuo. To the residue was added water and the solution was extracted with chloroform 50 ml×3). The organic layer was concentrated and the precipitated solid was filtered to give the titled compound as a white solid (3.0 g, 11.7 mmol).
Yield: 70%.
¹H NMR(CDCl₃)δ 8.04-7.99(2H, m), 6.91(1H, d, J = 8.6 Hz), 4.55(2H, s), 3.97(3H, s), 3.89(3H, s).

### Reference example 58

### 2-Butoxy-9-(2-methoxy-5-methoxycarbonyl)benzyladenine

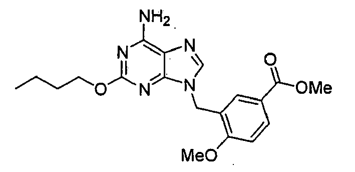

The titled compound as a white solid was prepared in the same method as in Reference example 1. Yield: 57%
¹H NMR(CDCl₃)δ 8.09(1H, s), 8.02(1H, d, J = 8.7 Hz), 7.67(1H, s), 6.92(1H, d, J = 8.7 Hz), 5.49(2H, brs), 5.27(2H, s), 4.36(2H, t, J = 6.5 Hz), 3.93(3H, s), 3.87(3H, s), 1.79(2H, tt, J = 7.6 Hz, 6.5 Hz), 1.53(2H, tq, J = 7.6 Hz, 7.4 Hz), 0.97(3H, t, J = 7.4 Hz).

### Reference example 59

### 2-Butoxy-9-(5-hydroxymethyl-2-methoxy)benzyladenine

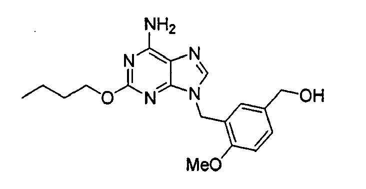

The titled compound as a white solid was prepared in the same method as in Reference example 17. Yield: 88%
¹H NMR(DMSO-d₆)δ 7.88(1H, s), 7.22-7.18(3H, m), 6.99-7.94(2H, m), 5.17(2H, s), 5.05(1H, brs), 4.33(2H, s), 4.19(2H, t, J = 6.6 Hz), 3.83(3H, s), 1.64(2H, tt, J = 7.5 Hz, 6.6 Hz), 1.40(2H, tq, J = 7.5 Hz, 7.4 Hz), 0.91(3H, t, J=7.4Hz).

### Reference example 60

### 2-Butoxy-9-(5-chloromethyl-2-methoxy)benzyladenine

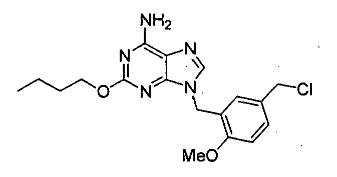

The titled compound as a white solid was prepared in the same method as in Reference example 18. Yield: 82%
¹H NMR(CDCl₃)δ 7.69(1H, s), 7.36(1H, s), 7.32(1H, d, J = 8.4 Hz), 6.87(1H, d, J = 8.4 Hz), 5.53(2H, brs), 5.25(2H, s), 4.51(2H, s), 4.36(2H, t, J = 6.6 Hz), 3.88(3H, s), 1.80(2H, tt, J = 7.6 Hz, 6.6 Hz), 1.51(2H, tq, J = 7.6 Hz, 7.4 Hz), 0.98(3H, t, J = 7.4 Hz).

### Reference example 61

### 2-Butoxy-9-(5-cyanomethyl-2-methoxy)benzyladenine

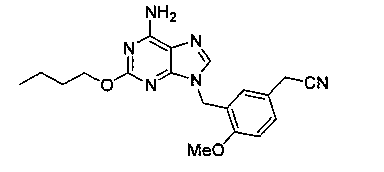

The titled compound as a white solid was prepared in the same method as in Reference example 19. Yield: 94%
¹H NMR(CDCl₃)δ 7.68(1H, s), 7.29-7.22(2H, m), 6.89(1H, d, J = 8.5 Hz), 5.50(2H, brs), 5.25(2H, s), 4.35(2H, t, J = 6.6 Hz), 3.89(3H, s), 3.64(2H, s), 1.81(2H, tt, J = 7.6 Hz, 6.6 Hz), 1.53(2H, tq, J = 7.6 Hz, 7.4 Hz), 0.98(3H, t, J = 7.4 Hz).

### Reference example 62

### 8-Bromo-2-butoxy-9-(5-cyanomethyl-2-methoxy)benzyladenine

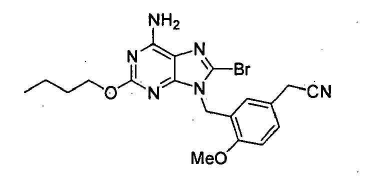

The titled compound as a white solid was prepared in the same method as in Reference example 2. Yield: 85%
¹H NMR(DMSO-d₆)δ 7.41(2H, brs), 7.26(1H, d, J = 8.5 Hz), 7.08(1H, d, J = 8.5 Hz), 6.67(1H, s), 5.18(2H, s), 4.15(2H, t, J = 6.5 Hz), 3.87(3H, s), 3.86(2H, s), 1.62(2H, tt, J = 7.4 Hz, 6.5 Hz), 1.37(2H, tq, J = 7.4 Hz, 7.4 Hz), 0.88(3H, t, J = 7.4 Hz).

### Reference example 63

### Methyl 2-fluoro-5-methylbenzoate

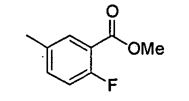

The titled compound as a colorless liquid was prepared in the same method as in Reference example 56. Yield: 98%
¹H NMR(CDCl₃)δ 7.72(1H, dd, J = 6.9, 2.2 Hz), 7.30(1H, m), 7.02(1H, dd, J = 10.6, 8.4 Hz), 3.93(3H, s), 2.35(3H, s).

### Reference example 64

### Methyl 5-bromomethyl-2-fluorobenzoate

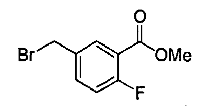

The titled compound as a white solid was prepared in the same method as in Reference example 57. Yield: 66%
¹H NMR(CDCl₃)δ 7.97(1H, dd, J = 6.7, 2.5 Hz), 7.56(1H, m), 7.13(1H, dd, J = 10.3, 8.5 Hz), 4.48(2H, s), 3.94(3H, s).

### Reference example 65

### 2-Butoxy-9-(4-fluoro-3-methoxycarbonylbenzyl)adenine

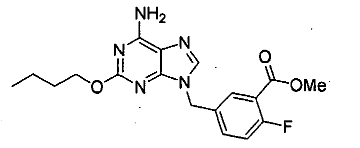

The titled compound as a white solid was prepared in the same method as in Reference example 1. Yield: 55%
¹H NMR(CDCl₃)δ 7.95(1H, dd, J = 6.7, 2.4 Hz), 7.61(1H, s), 7.48(1H, m), 7.12(1H, dd, J = 10.3, 8.6 Hz), 5.55(2H, brs), 5.27(2H, s), 4.33(2H, t, J =
6.6 Hz), 3.93(3H, s), 1.78(2H, tt, J = 7.6 Hz, 6.6 Hz), 1.51(2H, tq, J = 7.6 Hz, 7.4 Hz), 0.97(3H, t, J = 7.4 Hz).

### Reference example 66

### 2-Butoxy-9-(4-fluoro-3-hydroxymethylbenzyl)adenine

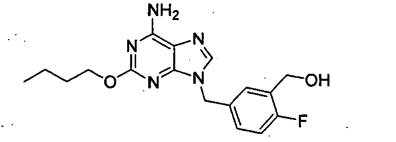

The titled compound as a white solid was prepared in the same method as in Reference example 17. Yield: 97%
¹H NMR(DMSO-d₆)δ 8.03(1H; s), 7.47(1H, dd, J = 7.1, 2.2 Hz), 7.28(1H, m), 7.11(1H, dd, J = 10.2, 8.6 Hz), 5.23(2H, s), 4.49(2H, s), 4.21(2H, t, J = 6.6 Hz), 1.65(2H, tt, J = 7.5 Hz, 6.6 Hz), 1.38(2H, tq, J = 7.5 Hz, 7.4 Hz), 0.91(3H, t, J = 7.4 Hz).

### Reference example 67

### 2-Butoxy-9-(3-chloromethyl-4-fluorobenzyl)adenine

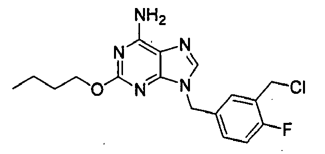

The titled compound as a pale yellow solid was prepared in the same method as in Reference example 18. Yield: 95%
¹H NMR(CDCl₃)δ 7.61(1H, s), 7.40(1H, dd, J = 6.9, 2.2 Hz), 7.25(1H, m), 7.05(1H, dd, J = 9.0, 8.8 Hz), 5.84(2H, brs), 5.25(2H, s), 4.59(2H, s), 4.33(2H, t, J = 6.6 Hz), 1.78(2H, tt, J = 7.6 Hz, 6.6 Hz), 1.50(2H, tq, J = 7.6 Hz, 7.4 Hz), 0.97(3H, t, J = 7.4 Hz).

### Reference example 68

### 2-Butoxy-9-(3-cyanomethyl-4-fluorobenzyl)adenine

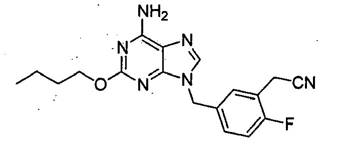

The titled compound as a white solid was prepared in the same method as in Reference example 19. Yield: 88%
¹H NMR(CDCl₃)δ 7.61(1H, s), 7.44(1H, dd, J = 7.0, 2.1 Hz), 7.29(1H, m), 7.08(1H, dd, J = 9.0, 8.8 Hz), 5.54(2H, brs), 5.26(2H, s), 4.34(2H, t, J = 6.6 Hz), 3.75(2H, s), 1.79(2H, tt, J = 7.6 Hz, 6.6 Hz), 1.51(2H, tq, J = 7.6 Hz, 7.4 Hz), 0.97(3H, t, J = 7.4 Hz).

### Reference example 69

### 8-Bromo-2-butoxy-9-(3-cyanomethyl-4-fluorobenzyl)adenine

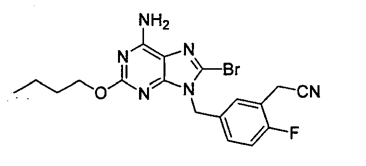

The titled compound as a white solid was prepared in the same method as in Reference example 2. Yield: 77%
¹H NMR(CDCl₃)δ 7.53(1H, dd, J = 7.0, 2.1 Hz), 7.34(1H, m), 7.06(1H, dd, J = 9.0, 8.8 Hz), 6.01(2H, brs), 5.28(2H, s), 4.34(2H, t, J = 6.6 Hz), 3.75(2H, s), 1.77(2H, tt, J = 7.6 Hz, 6.6 Hz), 1.51(2H, tq, J = 7.6 Hz, 7.4 Hz), 0.97(3H, t,J=7.4Hz).

### Reference example 70

### Methyl 2-methoxy-5-methylbenzoate

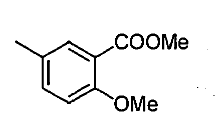

The titled compound was prepared in the same method as in Reference example 13. Yield: 88%

### Reference example 71

### 2-Methoxy-5-methylbenzyl alcohol

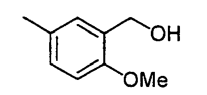

The titled compound was prepared in the same method as in Reference example 17. Yield: 82%
¹H NMR(CDCl₃)δ 7.06(1H, s), 7.04(1H, d, J = 8.3 Hz), 6.76(1H, d, J = 8.3 Hz), 4.63(2H, s), 3.81(3H, s), 2.26(3H, s).

### Reference example 72

### 2-Methoxy-5-methylbenzyl chloride

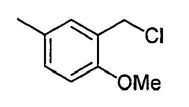

The titled compound was prepared in the same method as in Reference example 18. Yield: 100%
¹H NMR(CDCl₃)δ 7.14(1H, s), 7.08(1H, d, J = 8.3 Hz), 6.76(1H, d, J = 8.3 Hz), 4.61(2H, s), 3.831(3H, s), 2.26(3H, s).

### Reference example 73

### 2-Methoxy-5-methylphenylacetonitrile

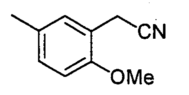

The titled compound was prepared in the same method as in Reference example 19. Yield: 73%

### Reference example 74

### Methyl 2-methoxy-5-methylphenylacetate

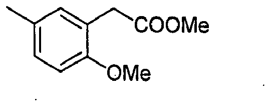

The titled compound was prepared in the same method as in Reference example 20. Yield: 73%

### Reference example 75

### Methyl 3-bromomethyl-6-methoxyphenylacetate

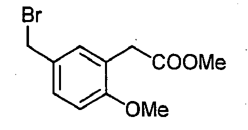

The titled compound was prepared in the same method as in Reference example 57. Yield: 70%

### Reference example 76

### 2-Butoxy-8-hydroxy-9-(3-carboxymethylbenzyl)adenine

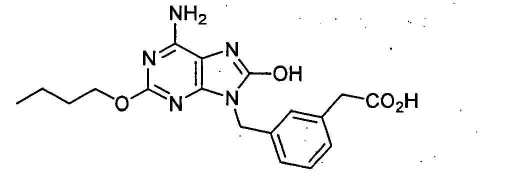

Using 2-chloro-9-(3-methoxycarbonylmethylbenzyl)adenine which was prepared in Reference example 1, the same procedures as in Reference example 4, Reference example 2, Reference example 3 and Comparative example 1 were carried out in this order to give the titled compound as a white solid.
¹H NMR(DMSO-d₆)δ12.31(1H, brs), 10.03(1H, brs), 7.22(4H, m), 6.47(2H, brs), 4.83(2H, s), 4.14(2H, t, J = 6.8 Hz), 3.50(2H, s), 1.60(2H, 5, J = 6.8 Hz), 1.38(2H, 6, J = 7.6 Hz), 0.90(3H, t, J = 7.0 Hz).

### Reference example 77

### 9-(3-Methoxycarbonylmethylbenzyl)-2-(2-phenyloxyethoxy)adenine

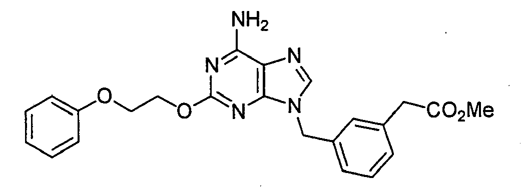

Using 2-chloro-9-(3-methoxycarbonylmethylbenzyl)adenine which was prepared in Reference example 1, the same procedure as in Reference example 4 was carried out to give the titled compound as a white solid.
Yield: 79%
¹H NMR(CDCl₃)δ 8.07(1H, s), 7.32-7.25(6H, m), 7.22(2H, t, J = 7.2Hz), 6.97-6.94(3H, m), 5.26(2H, s), 4.54(2H, t, J = 4.6Hz), 4.27(2H, t, J = 4.6Hz), 3.65(2H, s), 3.58(3H, s).

### Reference example 78

### 8-Bromo-2-[2-(4-bromophenyloxy)ethoxy]-9-(3-methoxycarbonylmethylbenzyl)adenine

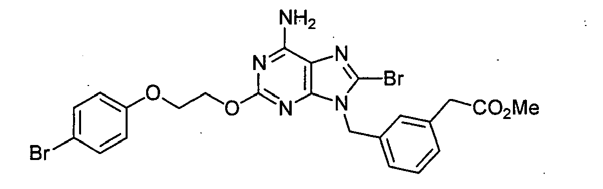

Using 9-(3-methoxycarbonylmethylbenzyl)-2-(2-phenyloxyethoxy)adenine which was prepared in Reference example 77, the same procedure as in Reference example 2 was carried out to give the titled compound as a white solid. Yield: 94%
¹H NMR(CDCl₃)δ 7.50(2H, brs), 7.47-7.42(2H, m), 7.30(1H, t, J = 7.6Hz), 7.21-7.18(2H, m), 7.12(1H, d, J = 7.6Hz), 6.95-6.92(2H, m), 5.25(2H, s), 4.53(2H, t, J = 4.6Hz), 4.27(2H, t, J = 4.6Hz), 3.66(2H, s), 3.58(3H, s).

### Reference example 79

### 8-Hydroxy-9-(3-methoxycarbonylmethylbenzyl)-2-(3-phthalimidopropoxy)adenine

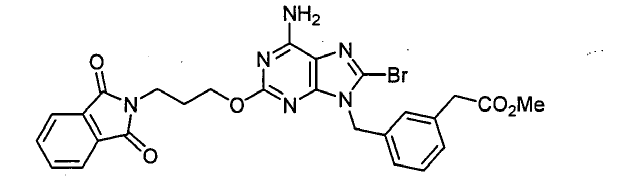

8-Bromo-2-hydroxy-9-(3-methoxycarbonylmethylbenzyl)adenine (2.0g, 5.10mmol) which was prepared in Reference example 5 was dissolved in dimethylformamide (150ml) and thereto was added potassium carbonate (1.05g, 7.65mmol). After heating at 95°C for 30minutes, thereto was added 3-bromopropylphthalimide (2.05g, 7.65mmol) and the mixture was heated at 95°C for 3.5 hours. After removal of the solvent, the residue was extracted with chloroform. The organic layer was dried over anhydrous magnesium sulfate, concentrated and the residue was purified by column chromatography (SiO₂ 120g, elute: CHCl₃/MeOH=100/1~50/1) to give the titled compound as a white solid (1.47g, 2.60 mmol). Yield: 51%.
¹H NMR(CDCl₃)δ 7.85-7.79(4H, m), 7.42(2H, brs), 7.28(1H, t, J = 7.8 Hz), 7.19-7.09(3H, m), 5.18(2H, s), 4.27(2H, t, J = 6.4Hz), 3.73(2H, t, J = 6.9Hz), 3.64(2H, s), 3.58(3H, s), 2.06-2.00(2H, m).

### Reference example 80

### 2-Chloro-9-tetrahydropyranyladenine

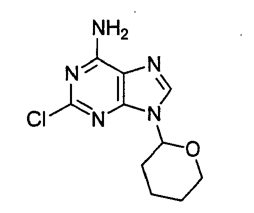

A solution of 2,6-dichloro-9-(tetrahydropyranyl)purine (55g) in 7N ammonia-methanol solution was heated at 100°C for 6 hours in a sealed flask. The reaction mixture was cooled to room temperature, allowed to stand overnight and filtered to give the titled compound (40g). Yield 80% ¹H NMR(CDCl₃)δ 8.02(1H, s), 5.94(2H, brs), 5.71(1H, dd), 4.15-4.22(1H, m), 3.75-3.82(1H, m), 1.27-2.12(6H, m).

### Reference example 81

### 2-(2-Diethylaminoethoxy-9-tetrahydropyranyladenine

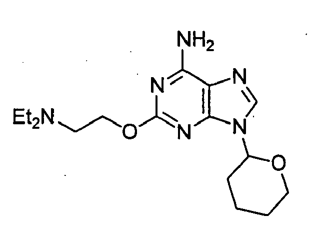

2-Diethylaminoethanol (30ml) was gradually dropped to sodium hydride (0.96g, 40mmol) under a nitrogen atmosphere at 0°C over a 15 minutes period. Then the mixture was warmed to room temperature and stirred for 20 minute. 2-Chloro-9-tetrahydropyranyladenine (1.0g, 3.9mmol) which was prepared in Reference example 80 was added thereto and the mixture was stirred at 100°C for 1.5 hours. After being cooled to 0°C and being neutralized with acetic acid, 2-diethyl amino ethanol was removed and the residue was extracted with ethyl acetate. The extracted was dried over sodium sulfate and concentrated in vacuo, To the residue was added chloroform and hexane, and the precipitated pale orange solid was filtered to give the titled compound (1.12g). Yield 85%
¹H NMR(DMSO-d₆)δ 8.11(1H, s), 7.24(2H, brs), 5.48(1H, dd, J = 11.1 Hz, 2.2 Hz), 4.25(2H, m), 3.99(1H, m), 3.63(1H, m), 2.71(2H, t, J = 6.6 Hz), 2.53(4H, q, J = 7.1 Hz), 2.22(1H, m), 1.91(2H, m), 1.70(1H, m), 1.58(2H, m), 0.97(6H, t, J = 7.1 Hz).

### Reference example 82

### 2-(2-Diethylaminoethoxy-8-iodo-9-tetrahydropyranyl)adenine

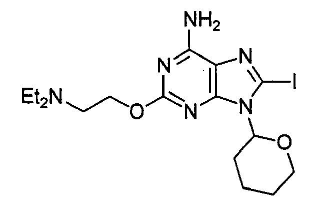

Diisopropylamine (5.9ml, 42mmol) was added to THF (50ml) under a nitrogen atmosphere and the solution was cooled to 0°C. Thereto was dropped a solution of butyllithium in 1.6M hexane solution (24ml, 38mmol) and the mixture was stirred at 0°C for 15 minutes to prepare lithium diisopropylamide (LDA). 2-(2-Diethylaminoethoxy-9-tetrahydropyranyl)adenine which was prepared in Reference example 81 (0.85g, 2.55mmol) was dissolved in THF (50ml) and cooled to -78°C. Thereto was dropped the above prepared LDA solution in a period of 15 minute, and the mixture was stirred at -78°C for 5 hours, followed by adding iodine (3.24g, 13mmol) and stirring at 0°C for 2.5 hours. The reaction was quenched by adding at 0°C an aqueous saturated sodium thiosulfate solution. After removal of THF, the residue was extracted with chloroform: ethanol (3:1). The organic layer was concentrated and the residue was purified by column chromatography (SiO₂ 70 g, elute: CHCl₃/MeOH=50/1~20/1) to give the titled compound as a brown solid (0.67g, 1.44mmol). Yield: 57%
¹H NMR(CDCl₃)δ 6.22(2H, brs), 5.53(1H, dd, J = 11.2 Hz, 2.3 Hz), 4.66(2H, t, J = 5.9 Hz), 4.17(1H, m), 3.72(1H, m), 3.23(2H, t, J = 5.9 Hz), 3.07(1H, m), 3.01(4H, q, J = 7.3 Hz), 2.11(1H, m), 1.79(4H, m), 1.29(6H, t, J = 7.3 Hz).

### Reference example 83

### 7-Benzyl-6-hydroxy-2-trifluoromethylpurine

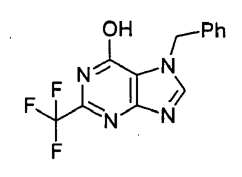

Sodium (1.06g, 46.3mmol) was completely dissolved in ethanol (100ml). Thereto were added 1-benzyl-4-aminoimidazole-5-caboxamide (2.0g, 9.25mmol) and ethyl trifluoroacetate (3.94g, 27.8mmol) in this order, and the mixture was refluxed for 4 hours. After being cooled to 0°C and being neutralized with acetic acid, the solvent was removed. To the residue was added water, and the precipitated solid was filtered and washed with methanol to give the titled compound as a white solid (2.12g, 7.21mmol). Yield: 78%
¹H NMR(DMSO-d₆)δ 13.79(1H, brs), 8.59(1H, s), 7.32(5H, m), 5.60(2H, s).

### Reference example 84

### 7-Benzyl-6-chloro-2-trifluoromethylpurine

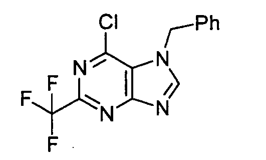

7-Benzyl-6-hydroxy-2-trifluoromethylpurine (2.0g, 6.80mmol) which was prepared in Reference example 83 was suspended in acetonitrile (200ml). Thereto were added phosphorus oxychloride (3.13g, 20.4mmol) and N,N-dimethylaniline (1.24g, 10.2mmol), and the mixture was refluxed for 5 hours. After removal of the solvent, to the residue was added a saturated sodium bicarbonate solution and the precipitated solid was filtered, washed with water and purified by silica gel column chromatography (elute:CHCl₃) to give the titled compound as a white solid (1.79g, 5.72mmo1). Yield: 84%
¹H NMR(DMSO-d₆)δ 9.21(1H, brs), 7.37(3H, m), 7.23(2H, m), 5.82(2H, s).

### Reference example 85

### 7-Benzyl-2-trifluoromethyladenine

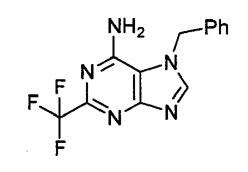

7-Benzyl-6-chloro-2-trifluoromethylpurine (1.70g, 5.43mmol) which was prepared in Reference example 84 was suspended in isopropyl alcohol (20ml) and thereto was added 28% aqueous ammonia (20ml). The mixture was heated at 120°C in an autoclave and was stirred for 2.5 hours. After being cooled to room temperature, the solution was concentrated in vacuo, the residue was extracted with chloroform: ethanol (3:1). The organic layer was concentrated and the residue was purified by column chromatography (elute: CHCl₃/MeOH=200/1~20/1) to give the titled compound as a white solid (1.19g, 4.07mmol). Yield: 75%
¹H NMR(DMSO-d₆)δ 8.62(1H, s), 7.48(2H, brs), 7.30(3H, m), 7.14(2H, m), 5.74(2H, s).

### Reference example 86

### 2-Trifluoromethyladenine formate

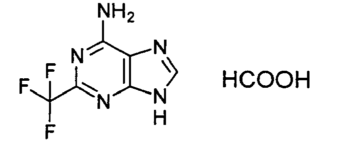

To a mixture of 7-benzyl-2-trifluoromethyladenine (0.65g, 2.22 mmol) which was prepared in Reference example 85 in ethanol (11ml) and formic acid (11ml) was added 20% Pd(OH)₂/C (3.25g), and the mixture was stirred at 70°C for 4 hours in a hydrogen atmosphere under 1.5 atm. The reaction mixture was filtered and the filtrate was concentrated to give the titled compound as a white solid (0.48g, 1.92mmol). Yield: 87%
¹H NMR(DMSO-d₆)δ 8.43(1H, s), 8.17(1H, s), 7.76(2H, brs).

### Reference example 87

### 2-Butyl-6-hydroxypurine

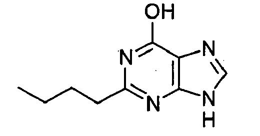

Sodium (2.92g, 127mmol) was completely dissolved in ethanol (75ml). Thereto were added 4-aminoimidazole-5-caboxamide hydrochloride (1.0g, 6.15mmol) and ethyl valerate (20.0g, 153mmol) in this order, and the solution was refluxed 20 hours with stirring. After being cooled to room temperature, thereto was added water (40ml) and the mixture was stirred for 10 minutes. After being cooled to 0°C and neutralized with concentrated hydrochloric acid, the solvent was removed.

The residue was extracted with chloroform: ethanol (3:1) and the organic layer was concentrated. The residue was recrystallized from chloroform: diethyl ether (10:1) to give the titled compound as a pale orange solid (1.16g, 6.03mmol). Yield: 98%
¹H NMR(DMSO-d₆)δ 12.06(1H, brs), 8.02(1H, s), 2.60(2H, t, J = 7.4 Hz), 1.66(2H, m), 1.32(2H, m), 0.88(3H, t, J = 7.3 Hz).

### Reference example 88

### 2-Amino-6-chloro-9-{3-(3-methoxycarbonylmethylbenzyl)purin

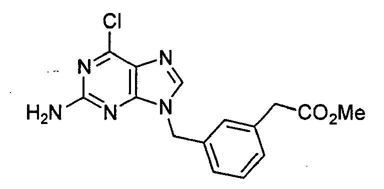

To a suspension of 2-amino-6-chloropurin (6.97g, 41.1mmol) in DMF (150 ml) were added potassium carbonate (8.52g, 61.7mmol) and 3-(methoxycarbonylmethyl)benzyl bromide (10.0g, 411mmol), and the mixture was stirred at room temperature. Three hours later the mixture was filtered over celite, the filtrate was concentrated and dried in vacuo. After adding water and extracting with chloroform, the extract was concentrated, dried, purified by silica gel column chromatography and dried in vacuo to give the titled compound as a white solid (8.4g, 25.3mmol). Yield: 62%
¹H NMR(DMSO-d₆)δ8.22(1H, s), 7.29(1H, dd, J = 7.6, 7.6 Hz), 7.18(1H, d, J = 7.6 Hz), 7.15(1H, s), 7.10(1H, d, J = 7.6 Hz), 6.96(2H, brs), 5.28(2H, s), 3.66(2H, s), 3.58(3H, s).

### Reference example 89

### 6-Chloro-2-iodo-9-(3-methoxycarbonylmethylbenzyl)purin

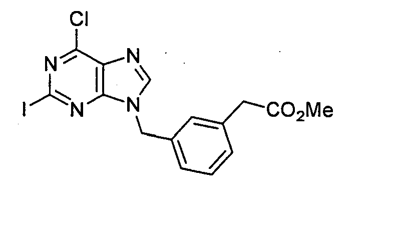

To a solution of 2-amino-6-chloro-9-(3-methoxycarbonylmethylbenzyl)purin (8.4g, 25.3mmol) which was prepared in Reference example 88 in THF (250ml) were added cupper(I) iodide (4.82g, 25.3mmol), diiodomethane (10.4ml, 129.1mmo1) and isoamylnitrite (10.2ml, 75.9mmol), and the mixture was stirred at 60°C. One and half hours later the mixture was filtered over celite, the filtrate was concentrated. After adding water and extracting with chloroform, the extract was concentrated, dried, purified by silica gel column chromatography and dried in vacuo to give the titled compound as a yellow oil (7.0g, 15.8mmol). Yield: 62%
¹H NMR(DMSO-d₆)δ8.73(1H, s), 7.32(1H, dd, J = 7.6, 7.6 Hz), 7.22-7.17(3H, m), 5.47(2H, s), 3.66(2H, s), 3.59(3H, s).

### Reference example 90

### 2-Iodo-9-(3-methoxycarbonylmethylbenzyl)adenine

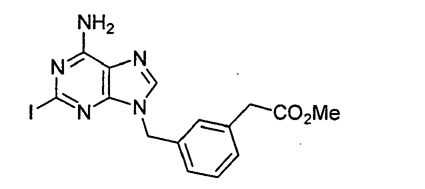

To a solution of 6-chloro-2-iodo-9-(3-methoxycarbonylmethylbenzyl)purin (7.0g, 15.8mmol) which was prepared in Reference example 89 in THF (200ml) was added 28% aqueous ammonia (20ml), and the solution was stirred at room temperature. After 75 hours, the solvent was removed. After adding water and extracting with chloroform, the extract was concentrated, dried, purified by silica gel column chromatography and dried in vacuo to give the titled compound as a white solid (4.9g, 11.6mmol). Yield: 74%
¹H NMR(DMSO-d₆)δ8.14(1H, s), 7.69(2H, brs),7.30(1H, dd, J = 7.6, 7.6 Hz), 7.19(1H, d, J = 7.6 Hz), 7.17(1H, s), 7.11(1H, d, J = 7.6 Hz), 5.30(2H, s), 3.66(2H, s), 3.59(3H, s).

### Reference example 91

### 2-Cyclopentyl-9-(3-methoxycarbonylmethylbenzyl)adenine

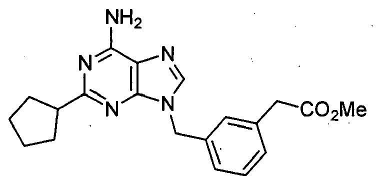

To a solution of 2-iodo-9-(3-methoxycarbonylmethylbenzyl)adenine (300mg, 0.71mmol) which was prepared in Reference example 90 in THF (2ml) were added 0.5M cyclopentyl zinc bromide (3.54mmol) in THF (7ml) and tetrakis(triphenylphosphine)palladium (44.1mg, 0.035mmol), and the mixture was stirred at room temperature over night. Thereto was added a saturated aqueous ammonium chloride solution (1ml) and the solution was stirred for 5 minutes. Then the solvent was removed and to the residue was added water. The solution was neutralized with 1N aqueous hydrochloric acid and extracted with chloroform. The extract was dried, concentrated, purified by silica gel column chromatography and dried in vacuo to give the titled compound as a white solid (250mg, 0.68mmol).
Yield: 96%
¹H NMR(DMSO-d₆)δ8.11(1H, s), 7.29(1H, dd, J = 7.6, 7.6 Hz), 7.26(1H, s), 7.23(1H, d, J = 7.6 Hz), 7.17(1H, d, J = 7.6 Hz), 7.15(2H, brs), 5.29(2H, s), 3.57(2H, s), 3.33(3H, s), 3.08(1H, quin, J = 8.2 Hz), 1.96-1.82(2H, m), 1.79-1.69(2H, m), 1.63-1.56(2H, m), 1.55-1.45(2H, m).

### Reference example 92

### 8-Bromo-2-cyclopentyl-9-(3-methoxycarbonylmethylbenzyl)adenine

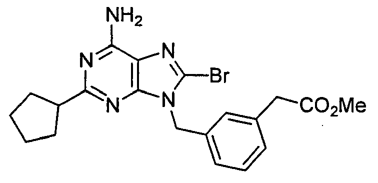

Using 2-cyclopentyl-9-(3-methoxycarbonylmethylbenzyl)adenine which was prepared in Reference example 91, the same procedure as in Reference example 2 was carried out to give the titled compound as a yellow oil. Yield: 60%
¹H NMR(CDCl₃)δ7.36(1H, s), 7.29-7.25(2H, m), 7.24-7.20(1H, m), 5.38(2H, brs), 5.34(2H, s), 3.68(3H, s), 3.59(2H, s), 3.21(1H, quin, J = 8.2 Hz), 2.10-2.01(2H, m), 2.00-1.90(2H, m), 1.89-1.78(2H, m), 1.73-1.64(2H, m).

### Reference example 93

### 2-(1,3-Dioxolan-2-yl)-9-(3-methoxycarbonylmethylbenzyl)adenine

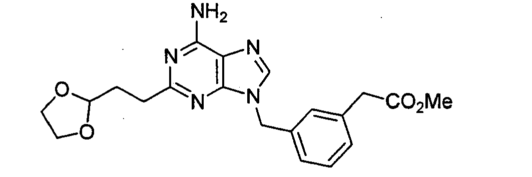

Using 2-iodo-9-(3-methoxycarbonylmethylbenzyl)adenine which was prepared in Reference example 90, the same procedure as in Reference example 91 was carried out to give the titled compound as a yellow oil.
Yield: 73%.
¹H NMR(DMSO-d₆)δ8.13(1H, s), 7.27(1H, dd, J = 7.6, 7.6 Hz), 7.24(1H, s), 7.20-7.16(2H, m), 7.13(2H, brs), 5.31(2H, s), 4.86(1H, t, J = 4.8 Hz), 3.90-3.86(2H, m), 3.77-3.72(2H, m), 3.64(2H, s), 3.58(3H, s), 2.75-2.70(2H, m), 2.04-1.98(2H, m).

### Reference example 94

### 2-(3-Hydroxypropyl)-9-(3-methoxycarbonylmethylbenzyl)adenine

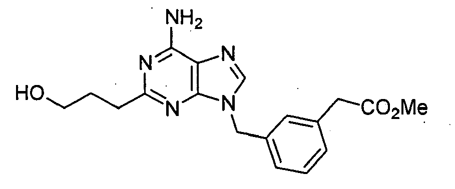

To 2-(1,3-dioxolan-2-yl)-9-(3-methoxycarbonylmethylbenzyl)adenine (340mg, 0.86mmol) which was prepared in Reference example 93 was added under ice cooling concentrated hydrochloric acid (5ml), and the solution was stirred under ice cooling for 5 minutes. Thereto was added water (10ml) and the solution was neutralized with 28% aqeous ammonia. The solution was extracted with chloroform and the extract was dried, and concentrated. The residue was dissolved in methanol (5ml) and thereto was added under ice cooling sodium borohydride (43.4mg, 1.15mmol).
The mixture was stirred at room temperature for 1.5 hours, and neutralized with 1N aqueous hydrochloric acid. After removal of the solvent, thereto was added water, and the solution was extracted with chloroform, dried and concentrated, purified by silica gel column chromatography and dried in vacuo to give the titled compound as a pale yellow solid (90mg, 0.25mmol). Yield: 30%
¹H NMR()δ7.71(1H, s), 7.32(1H, dd, J = 7.6, 7.6 Hz), 7.25(1H, d, J = 7.6 Hz), 7.23(1H, s), 7.18(1H, d, J = 7.6 Hz), 5.72(2H, brs), 5.31(2H, s), 3.74(2H, t, J = 5.8 Hz), 3.68(3H, s), 3.61(2H, s), 3.02(2H, t, J = 6.6 Hz), 2.06(2H, tt, J = 6.6, 5.8 Hz).

### Reference example 95

### 8-Bromo-2-(3-hydroxypropyl)-9-(3-methoxycarbonylmethylbenzyl)adenine

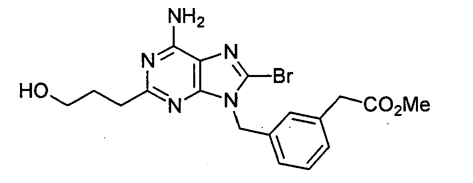

Using 2-(3-hydroxypropyl)-9-(3-methoxycarbonylmethylbenzyl)adenine which was prepared in Reference example 94, the same procedure as in Reference example 2 was carried out to give the titled compound as a yellow oil. Yield: 77%
¹H NMR(CDCl₃)δ7.29(1H, dd, J = 7.6, 7.6 Hz), 7.29(1H, s), 7.22(1H, d, J = 7.6 Hz), 7.21(1H, d, J = 7.6 Hz), 5.54(2H, brs), 5.34(2H, s), 3.73(2H, t, J = 5.8 Hz), 3.68(3H, s), 3.60(2H, s), 2.99(2H, t, J = 6.6 Hz), 2.06(2H, tt, J = 6.6, 5.8 Hz).

### Reference example 96

### [2-Chloro-9-(3-methoxycarbonylmethylbenzyl)purin-6-yl]-(tetrahydropyran-2-yl)-amine

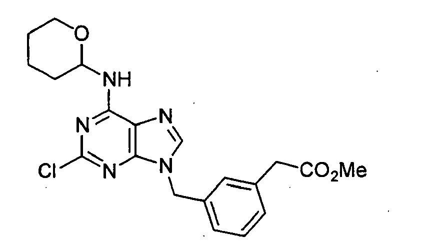

To a solution of 2-chloro-9-(3-methoxycarbonylmethylbenzyl)adenine (100mg, 0.3mmol) which was prepared in Reference example 1 in THF (3.5ml) were added 3,4-dihydro-2H-pyran (136µl, 1.5mmol) and p-toluenesufonic acid monohydrate (3mg, 0.02mmol), and the mixture was stirred at 67°C. After 5.5 hours, the mixture was neutralized with an aqueous saturated sodium hydrogencarbonate solution. After removal of the solvent, to the residue was added water (30ml) and the solution was extracted with chloroform. The organic layer was dried, and concentrated in vacuo to give the titled compound as a yellow oil (128mg, 0.3mmol). Yield: 99%
¹H NMR(DMSO-d₆)δ8.77(1H, brs), 8.33(1H, brs), 7.30(1H, dd, J = 7.6 Hz), 7.19(1H, d, J = 7.6 Hz), 7.18(1H, s), 7.13(1H, d, J = 7.6 Hz), 5.93(1H, brs), 5.35(2H, s), 3.85-3.81(1H, m), 3.66(1H, s), 3.58(3H, s), 3.54-3.49(1H, m), 1.91-1.83(1H, m), 1.82-1.73(1H, m), 1.72-1.65(1H, m), 1.64-1.52(1H, m), 1.51-1.40(2H, m).

### Reference example 97

### 9-(3-Methoxycarbonylmethylbenzyl)-2-(2-pyridylmethoxy)adenine

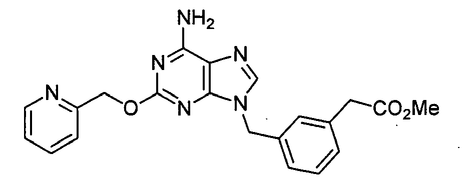

Sodium hydride (60% in oil) (365mg, 9.1mmol) was dissolved in 2-pyridylmethanol (15ml), and thereto was added [2-chloro-9-(3-methoxycarbonylmethylbenzyl)purin-6-yl]-(tetrahydropyran-2-yl)-amine (380mg, 0.91mmol) which was prepared in Reference example 95, followed by stirring at 100°C for 1.5 hours. After neutralizing with 12N hydrochloric acid, the solvent was removed, and the residue was dried in vacuo. To the residue were added methanol (20ml) and concentrated sulfuric acid (200µl), and the solution was stirred at 90°C. After 2 hours, the solution was neutralized with 28% aqueous ammonia and the solvent was removed. To the residue was added water and the solution was extracted with chloroform. The organic layer was dried, concentrated and the residue was purified by silica gel column chromatography to give the titled compound as a white solid (249mg, 0.62mmol). Yield: 67%
¹H NMR(DMSO-d₆)δ8.55(1H, d, J = 4.8 Hz), 8.06(1H, brs), 7.77(1H, dd, J = 7.6 Hz), 7.42(1H, d, J = 8.4 Hz), 7.33-7.29(1H, m), 7.25-7.21(1H, m), 7.18-7.12(1H, m), 5.40(2H, s), 5.22(2H, s), 3.63(2H, s), 3.57(3H, s).

### Reference example 98

### 8-bromo-9-(3-methoxycarbonylmethylbenzyl)-2-(2-pyridylmethoxy)adenine

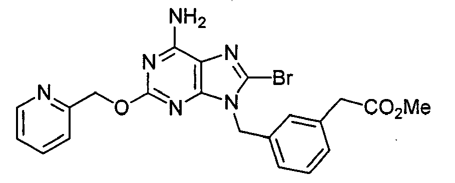

Using 9-(3-methoxycarbonylmethylbenzyl)-2-(2-pyridylmethoxy adenine which was prepared in Reference example 97, the same procedure as in Reference example 2 was carried out to give the titled compound as a white solid. Yield: 77%
¹H NMR(DMSO-d₆)δ8.54(1H, d, J = 4.8 Hz), 7.77(1H, dd, J = 7.8, 7.4 Hz), 7.53(2H, brs), 7.42(1H, d, J = 7.8 Hz), 7.31(1H, dd, J = 7.4, 4.8 Hz), 7.25(1H, dd, J = 7.6, 7.6 Hz), 7.18(1H, d, J = 7.6 Hz), 7.17(1H, s), 7.06(1H, d, J = 7.6 Hz), 5.40(2H, s), 5.22(2H, s), 3.64(2H, s), 3.57(3H, s).

### Reference example 99

### 9-(3-Methoxycarbonylmethylbenzyl)-2-(3-pyridylmethoxy)adenine

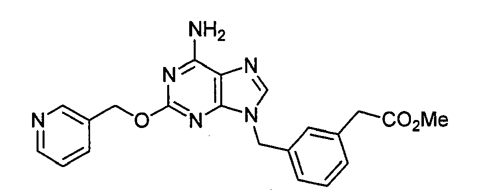

Using [2-chloro-9-(3-methoxycarbonylmethylbenzyl)purin-6-yl]-(tetrahydropyran-2-yl)-amine which was prepared in Reference example 95, the same procedure as in Reference example 97 was carried out to give the titled compound as a white solid. Yield: 50%
¹H NMR(DMSO-d₆)δ8.68(1H, s), 8.51(1H, d, J = 4.7 Hz), 8.06(1H, brs), 7.84(1H, d, J = 7.8 Hz), 7.39-7.35(1H, m), 7.32(2H, brs), 7.30-7.26(1H, m), 7.26-7.23(1H, m), 7.20-7.15(1H, m), 5.35(2H, s), 5.25(2H, s), 3.67(2H, s), 3.58(3H, s).

### Reference example 100

### 8-Bromo-9-(3-methoxycarbonylmethylbenzyl)-2-(3-pyridylmethoxy)adenine

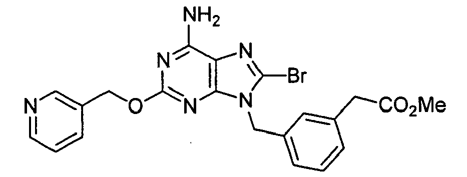

Using 9-(3-methoxycarbonylmethylbenzyl)-2-(3-pyridylmethoxy)adenine which was prepared in Reference example 99, the same procedure as in Reference example 2 was carried out to give the titled compound as a white solid. Yield: 50%
¹H NMR(CDCl₃)δ8.66(1H, s), 8.48(1H, d, J = 4.8 Hz), 7.75(1H, d, J = 7.8 Hz), 7.23-7.18(3H, m), 7.15(1H, d, J = 7.8 Hz), 7.11(1 H, d, J = 7.6 Hz), 5.49(2H, brs), 5.35(2H, s), 5.21(2H, s), 3.60(3H, s), 3.52(2H, s).

### Reference example 101

### 2-(3-Acetoxypropoxy)-8-bromo-9-(3-methoxycarbonylmethylbenzyl)adenine

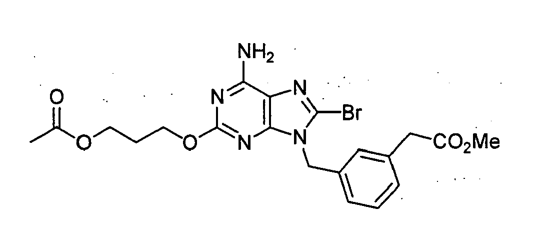

To a solution of 8-bromo-2-(3-hydroxypropoxy)-9-(3-methoxycarbonylmethylbenzyl)adenine (2.55g, 5.66mmol) which was prepared in Reference example 5 in DMF (50ml) were added acetic anhydride (2.15ml, 22.7mmol), triethylamine (3.16ml, 22.7mmol) and 4-dimethylaminopyridine (100mg), and the mixture was stirred at room temperature for 1 hour. After removal of the solvent, the residue was extracted with chloroform, and the organic layer was washed with an aqueous saturated sodium bicarbonate solution, 5% aqueous citric acid, and saturated brine in this order to give the titled compound as a pale yellow oil (2.75g, 5.59mmol). Yield: 99%
¹H NMR(DMSO-d₆)δ 7.48(2H, brs), 7.30(1H, dd, J = 7.7 Hz, 7.6 Hz), 7.19(1H, d, J = 7.6 Hz), 7.18(1H, s), 7.11(1H, d, J = 7.7 Hz), 5.24(2H, s), 4.27(2H, t, J = 6.3 Hz), 4.12(2H, t, J = 6.5 Hz), 3.63(2H, s), 3.58(3H, s), 2.01(2H, m), 2.01(3H, s).

### Reference example 102

### 8-Bromo-2-(3-hydroxypropoxy)-9-(3-methoxycarbonylmethylbenzyl)-6-(2-tetrahydropyranylamino)adenine

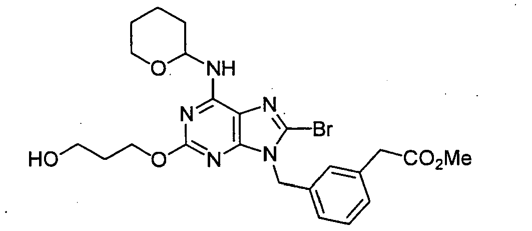

Using 2-(3-acetoxypropoxy)-8-bromo-9-(3-methoxycarbonylmethylbenzyl)adenine (2.48g, 5.04mmol) which was prepared in Reference example 101, introduction of tetrahydropyranyl moiety at 6 position was carried our in the same procedure as in Reference example 96 and the obtained compound was dissolved in a mixture of methanol (75ml) and water (35ml). Thereto was added potassium carbonate (0.26g, 1.88mmol) and the mixture was stirred at room temperature for 30 minutes. The solution was neutralized with 5% aqueous citric acid. After removal of the solvent, to the residue were added DMF (15ml), methyl iodide (0.13ml, 2.0 mmol), diisopropylethylamine (0.35ml, 2.0 mmol) and 4-dimethylaminopyridine (24mg, 0.20mmol), and the mixture was stirred at room temperature for 2.5 hours, followed by neutralization with 5% aqueous citric acid. After removal of the solvent, the residue was extracted with chloroform. The organic layer was concentrated and the residue was purified by column chromatography (elute: CHCl₃/MeOH=100/1) to give the titled compound as a pale yellow oil (1.04g, 4.07mmol). Yield: 46%
¹H NMR(DMSO-d₆)δ 8.51(1H, brs), 7.30(1H, dd, J = 7.7 Hz, 7.6 Hz), 7.19(1H, d, J = 7.6 Hz), 7.18(1H, s), 7.10(1H, d, J = 7.7 Hz), 5.36(1H, m), 5.26(2H, s), 4.53(1H, t, J = 5.0 Hz), 4.29(2H, m), 3.82(1H, m), 3.65(2H, s), 3.58(3H, s), 3.51(3H, m), 1.85(2H, m), 1.66(6H, m).

### Reference example 103

### 8-Bromo-9-(3-methoxycarbonylmethylbenzyl)-2-(3-morpholinopropoxy)-6-(2-tetrahydropyranylamino)adenine

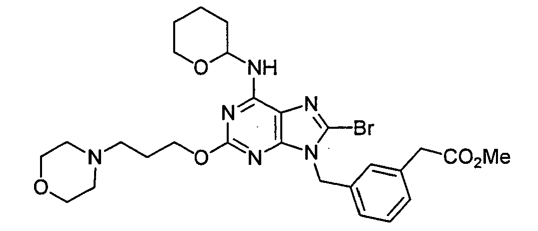

8-Bromo-2-(3-hydroxypropoxy)-9-(3-methoxycarbonylmethylbenzyl)-6-(2-tetrahydropyranylamino)adenine (165mg, 0.31mmol) which was prepared in Reference example 102 was dissolved in THF(10ml) and the solution was cooled to 0°C. Thereto were added mesyl chloride (142mg, 1.24mmol), triethylamine (125mg, 1.24mmol) and 4-dimethylaminopyridine (10mg), and the mixture was stirred at 0°C for 30 minutes. After removal of the solvent, the residue was extracted with chloroform and the organic layer was washed with an aqueous saturated sodium bicarbonate solution, 5% aqueous citric acid and saturated brine in this order. After concentration, to the residue was added morpholine (5ml) and the solution was stirred at room temperature for 6 hours. After removal of the morpholine by evaporator, the residue was extracted with chloroform. The organic layer was concentrated and the residue was purified by column chromatography (elute: CHCl₃/MeOH=300/1~50/1) to give the titled compound as a pale yellow oil (135mg, 0.22mmol). Yield: 72%
¹H NMR(DMSO-d₆)δ 8.52(1H, brs), 7.30(1H, dd, J = 7.6 Hz, 7.6 Hz), 7.19(2H, m), 7.10(1H, d, J = 7.6 Hz), 5.34(1H, m), 5.26(2H, s), 4.28(2H, m), 3.88(1H, m), 3.82(1H, m), 3.65(2H, s), 3.58(3H, s), 3.40(5H, m), 2.39(7H, m), 1.60(6H, m).

### Reference example 104

### 9-(3-Methoxycarbonylmethylbenzyl)-2-{2-(methylsulfanyl)ethoxy}adenine

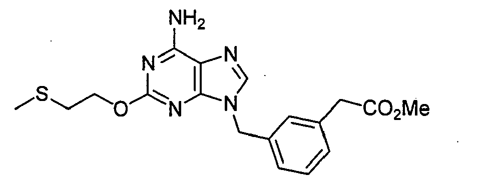

Sodium (1.00g, 45mmol) was dissolved in 2-methylsulfanyl-ethanol (30ml) and thereto was added 2-chloro-9-(3-methoxycarbonylmethylbenzyl)adenine (3.00g, 9.04mmol) which was prepared in Reference example 1. The mixture was stirred at 105°C for 2 hours and then neutralized with 12N hydrochloric acid. After removal of the solvent, to the residue was added an aqueous saturated sodium bicarbonate solution and the solution was washed with chloroform, followed by neutralization with 12N hydrochloric acid. The precipitated solid was filtered to give the titled compound as a pale yellow solid (3.20g, 8.25mmol). Yield:91%
¹H NMR(DMSO-d₆)δ8.04(1H, s), 7.32-7.27(1H, m), 7.25(2H, brs), 7.23(1H, s), 7.21-7.17(2H, m), 5.24(2H, s), 4.37(2H, t, J = 6.9 Hz), 3.65(2H, s), 3.58(3H, s), 2.80(2H, t, J = 6.9 Hz), 2.12(3H, s).

### Reference example 105

### 2-(2-(methanesulfonyl)ethoxy)-9-(3-methoxycarbonylmethylbenzyl)adenine

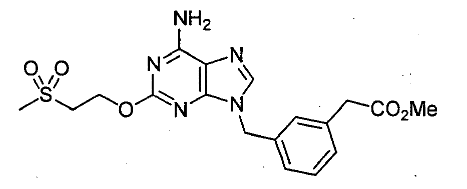

To 9-(3-methoxycarbonylmethylbenzyl)-2-{2-(methylsulfanyl)ethoxy}adenine (3.32g, 8.6mmol) which was prepared in Reference example 104 were added acetone (200ml) and 6.7% aqueous sodium hydrogencarbonate solution to prepare a suspension. Thereto was added oxone (7.9g, 12.9mmol) and the mixture was stirred at room temperature for 2.5 hours. After filtration of the reaction mixture over celite, the filtrate was concentrated and the residue was made weak acidic with 1N hydrochloric acid (pH=5). The precipitated solid was filtered to give the titled compound as a pale brown solid (3.13g, 7.46mmol. Yield: 87%
¹H NMR(DMSO-d₆)δ8.07(1H, s), 7.36(2H, brs), 7.29(1H, dd, J = 7.6 Hz), 7.26(1H, s), 7.21(1H, d, J = 7.6 Hz), 7.18(1H, d, J = 7.6 Hz), 5.26(2H, s), 4.57(2H, t, J = 5.8 Hz), 3.65(2H, s), 3.61(2H, t, J = 5.8 Hz), 3.58(3H, s), 3.04(3H, s).

### Reference example 106

### 8-Bromo-2-{2-(methanesulfonyl)ethoxy}-9-(3-methoxycarbonylmethylbenzyl) adenine

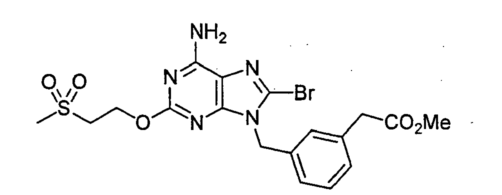

Using 2-{2-(methanesulfonyl)ethoxy}-9-(3-methoxycarbonylmethylbenzyl)adenine which was prepared in Reference example 104, the same procedure as in Reference example 2 was carried out to give the titled compound as a brown solid. Yield: 76%
¹H NMR(DMSO-d₆)δ7.57(2H, brs), 7.31(1H, dd, J = 7.8, 7.8 Hz), 7.21-7.17(2H, m), 7.13(1H, d, J = 7.8 Hz), 5.26(2H, s), 4.57(2H, t, J = 5.8 Hz), 3.66(2H, s), 3.59(2H, t, J = 5.8 Hz), 3.58(3H, s), 3.04(3H, s).

### Reference example 107

### 8-Bromo-2-hydroxy-9-(3-methoxycarbonylmethylbenzyl)adenine

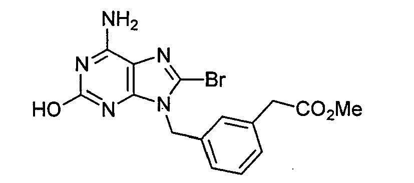

To 8-bromo-2-{2-(methanesulfonyl)ethoxy}-9-(3-methoxycarbonylmethylbenzyl)adenine (2.8g, 5.5mmol) which was prepared in Reference example 106 were added 2.5N aqueous sodium hydroxide solution (56ml) and methanol (28ml) to prepare a suspension, and the suspension was refluxed at 80°C. After 2 hours, the mixture was neutralized with concentrated hydrochloric acid and the solvent was removed. The residue was dried in vacuo and thereto were added methanol (100ml) and concentrated sulfuric acid (0.5ml), followed by stirring at 90°C. After 2 hours, the mixture was neutralized with 28% aqueous ammonia and the solvent was removed. To the residue was added water and the precipitated solid was filtered to give the titled compound as a pale brown solid (2.11g, 5.4mmol). Yield: 98%
¹H NMR(DMSO-d₆)δ10.51(1H, brs), 7.30(1H, dd, J = 7.7, 7.7 Hz), 7.18(1H, d, J = 7.7 Hz), 7.12(1H, s), 7.08(1H, d, J = 7.7 Hz), 5.11(2H, s), 3.66(2H, s), 3.59(3H, s).

### Reference example 108

### 8-Bromo-9-(3-methoxycarbonylmethylbenzyl)-2-{2-(phenylsulfanyl)ethoxy}adenine

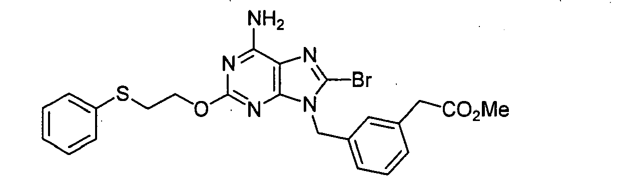

8-Bromo-2-hydroxy-9-(3-methoxycarbonylmethylbenzyl)adenine (300mg, 0.8mmol) which was prepared in Reference example 107 was suspended in DMF (20ml) and stirred at 105°C for 1 hour. Thereto was added potassium carbonate (159mg, 1.1mmol) and the solution was stirred at 105°C for 1 hour. Thereto was added 1-bromo-2-(phenylthio)ethane (173µl, 1.1mmol), and the mixture was stirred at 100°C for 3 hours, followed by being cooled to room temperature. After filtrating over celite, the filtrate was concentrated and to the residue was added water. The solution was extracted with chloroform, dried and concentrated and the residue was purified by silica gel column chromatography to give the titled compound as a white solid (189mg, 0.36mmol). Yield: 42%

### Reference example 109

### 8-Bromo-9-(3-methoxycarbonylmethylbenzyl)-2-(tetrahydrofuran-2-ylmethoxy) adenine

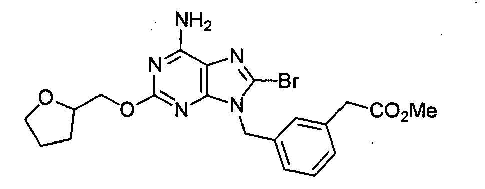

Using 8-bromo-2-hydroxy-9-(3-methoxycarbonylmethylbenzyl)adenine which was prepared in Reference example 107, the same procedures as in Reference example 108 and Reference example 2 were carried out in this order to give the titled compound as a colorless oil. Yield: 77%
¹H NMR(DMSO-d₆)δ7.46(2H, brs), 7.30(1H, dd, J = 7.6, 7.6 Hz), 7.19(1H, d, J = 7.6 Hz), 7.17(1H, s), 7.11(1H, d, J = 7.6 Hz), 5.23(2H, s), 4.18(2H, d, J = 5.2 Hz), 4.20-4.11(1H, m), 3.80-3.72(1H, m), 3.70-3.63(1H, m), 3.65(2H, s), 3.59(3H, s), 1.99-1.92(1H, m), 1.86-1.78(2H, m), 1.67-1.60(1H, m).

### Reference example 110

### 2-[3-(Ethylthio)propoxy]-9-(3-methoxycarbonylmethylbenzyl)adenine

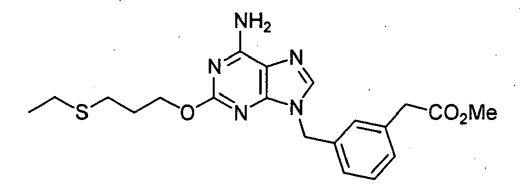

Using 2-chloro-9-(3-methoxycarbonylmethylbenzyl)adenine which was prepared in Reference example 1, the same procedure as in Reference example 4 was carried out to give the titled compound as a white solid.
Yield: 53%
¹H NMR(DMSO-d₆)δ 8.03(1H, s), 7.38-7.15(6H, m), 5.26(2H, s), 4.29(2H, t, J = 6.6 Hz), 3.65(2H, s), 3.59(3H, s), 2.61(2H, t, J = 7.0 Hz), 2.51(2H, q, J = 7.4 Hz), 1.95(2H, m), 1. 18(3H, t, J = 7.4 Hz).

### Reference example 111

### 2-[3-(Ethylsulfonyl)propoxy]-9-(3-methoxycarbonylmethylbenzyl)adenine

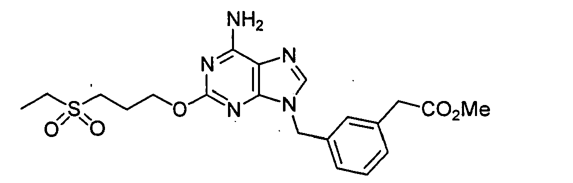

To a solution of 2-[3-(ethylthio)propoxy]-9-(3-methoxycarbonylmethylbenzyl)adenine (333mg, 0.801mmol) which was prepared in Reference example 110 in a mixture of acetone (50ml) and water (25ml) were added sodium hydrogencarbonate (673mg, 8.014mmol) and oxone (739mg, 1.202mmol), and the mixture was stirred at room temperature for 6 hours. After removal of the solvent, the residue was extracted with chloroform and the extract was dried over sodium sulfate, and concentrated in vacuo. The residue was purified by silica gel column chromatography (elute: MeOH/CHCl₃=3/100) to give the titled compound as a white solid (277mg). Yield: 77%.
¹H NMR(DMSO-d₆)δ 8.05(1H, s), 7.33-7.17(6H, m), 5.25(2H, s), 4.33(2H, t, J = 6.3 Hz), 3.66(2H, s), 3.59(3H, s), 3.20(2H, m), 3.12(2H, q, J = 7.4 Hz), 2.11(2H, m), 1.22(3H, t, J = 7.4 Hz).

### Reference example 112

### 9-(3-Carboxymethylbenzyl)-2-chloroadenine

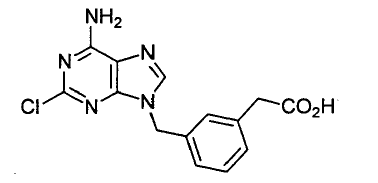

Using 2-chloro-9-(3-methoxycarbonylmethylbenzyl)adenine which was prepared in Reference example 1, the same procedure as in Comparative example 1 was carried out to give the titled compound as a white solid. Yield: quantitative.
¹H NMR(DMSO-d₆)δ 8.24(1H, s), 7.78(2H brs), 7.20(4H, m), 5.31(2H, s), 3.54(2H, s).

### Reference example 113

### 9-(3-Methoxycarbonylmethylbenzyl)-2-[2-methoxyethyl(N-methyl)amino]adenine

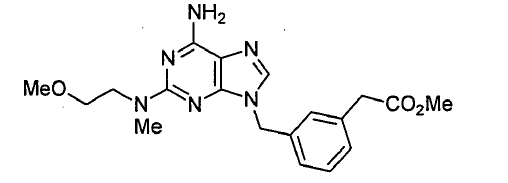

Using 9-(3-carboxylmethylbenzyl)-2-chloroadenine which was prepared in Reference example 112, the same procedure as in Reference example 11 was carried out and then esterifications were carried out using methanol and sulfuric acid to give the titled compound as a white solid.
Yield: 80%
¹H NMR(DMSO-d₆)δ 7.82(1H, s), 7.22(4H, m), 6.73(2H, brs), 5.16(2H, s), 3.71(2H, t, J = 6.1 Hz), 3.57(3H, s), 3.49(2H, d, J = 6.1 Hz), 3.23(3H, s).

### Pharmaceutical preparation 1

An aerosol solution having following formulation in 1g is prepared.

| | |
|---|---|
| Compound of Example 1 | : 0.641mg (0.06%) |
| Ethanol | : 26.816mg (2.68%) |
| 1,1,1,2-Tetrafluoroethone | : 972.543mg (97.25%) |

### Pharmaceutical preparation 2

An aerosol solution having following formulation in 1g is prepared.

| | |
|---|---|
| Compound of Example 15 | : 0.641mg (0.06%) |
| Ethanol | : 26.816mg (2.68%) |
| 1,1,1,2-Tetrafluoroethane | : 972.543mg (97.25%) |

### INDUSTRIAL APPLICABILITY

The present invention is to provide an 8-oxoadenine compound useful as a medicament for topical application which is chracterised of exhibiting its effects by a topical application and exhibiting no systemically pharmacological effects. By the present invention it becomes possible to treat or prevent diseases including allergic diseases such as asthma and atopic dermatitis, viral diseases such as herpes and cancers. Further, in a case where the compound of the present invention is externally applied (topical administration) in a form of spray, etc., systemic adverse effects caused by an interferon inducing activity is suppressed and the strong effect is exhibited in the applied region.

## Claims

1. An 8-oxoadenine compound represented by the following formula (1): 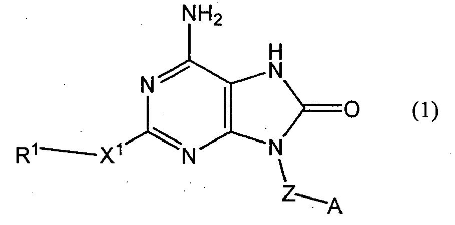 , wherein A is a group selected from the group consisting of the following formulas (2) to (8): 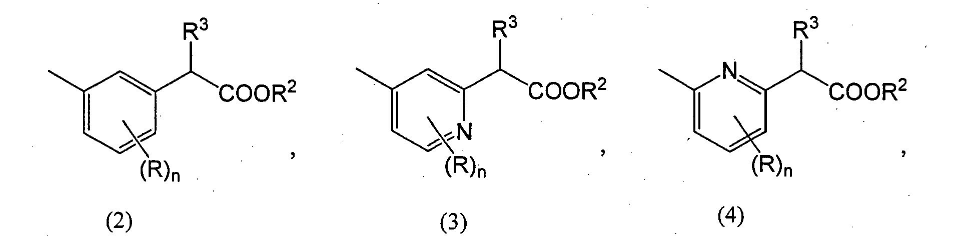 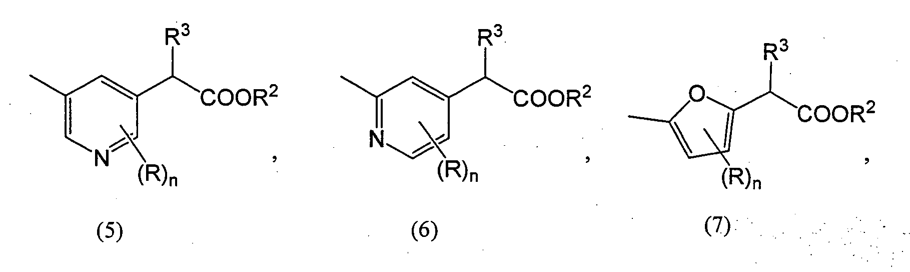 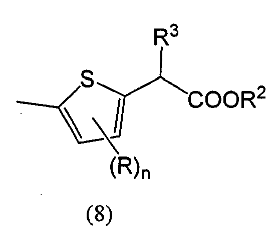 , wherein R² is hydrogen atom, or a substituted or unsubstituted alkyl group;
R³ is hydrogen atom or an alkyl group;
R is a halogen atom, a haloalkyl group, a haloalkoxy group, an alkyl group, an alkoxy group, amino group, an alkylamino group or dialkylamino group;
n is an integer of 0 to 2, and when n is 2, R_{S} may be the same or different;
X¹ is oxygen atom, sulfur atom, SO₂ NR⁴ (wherein R⁴ is hydrogen atom or an alkyl group.), or a single bond;
Z is a straight or branched chain alkylene;
R¹ is hydrogen atom, a halogen atom, a substituted or unsubstituted alkyl group, a substituted or unsubstituted alkenyl group, a substituted or unsubstituted alkynyl group, a substituted or unsubstituted aryl group, a substituted or unsubstituted heteroaryl group or a substituted or unsubstituted cycloalkyl group,
or a pharmaceutically acceptable salt thereof.

2. The 8-oxoadenine compound according to claim 1, wherein
R² is a substituted or unsubstituted C₁₋₈ alkyl group, wherein said alkyl group may be substituted by one or plural substituents which may be the same or different,
and the substituents on said alkyl group are selected from the group consisting of a halogen atom, hydroxy group, carboxy group, C₃₋₈ cycloalkyl group, an C₁₋₆ alkoxy group, an C₁₋₆ alkylthio group, a C₃₋₈ cycloalkoxy group, an C₂₋₁₀ acyloxy group, an C₁₋₆ alkylsulfonyl group, an C₁₋₆ alkylsulfinyl group, a substituted or unsubstituted carbamoyl group, a substituted or unsubstituted sulfamoyl group, a substituted or unsubstituted amino group, a substituted or unsubstituted 6 to 10 membered aryl group, a substituted or unsubstituted 5 to 10 membered heteroaryl group which contains 1 to 4 hetero atoms consisting of 0 to 2 nitrogen atoms, 0 to 1 oxygen atom and 0 to 1 sulfur atom, and a substituted or unsubstituted 4 to 7 membered saturated heterocyclic group which contains 1 to 4 hetero atoms consisting of 0 to 2 nitrogen atoms, 0 to 2 oxygen atoms and 0 to 2 sulfur atoms;
R³ is hydrogen atom or an alkyl group.
R is a halogen atom, a C₁₋₆ haloalkyl group, a C₁₋₆ haloalkoxy group, an C₁₋₆ alkyl group, an C₁₋₆ alkoxy group, amino group, an C₁₋₆ alkylamino group, or a di C₁₋₆ alkyl amino group;
n is an integer of 0 to 2, and when n is 2, Rs may be the same or different;
X¹ is oxygen atom, sulfur atom, SO₂, NR⁴ (wherein R⁴ is hydrogen atom or an C₁₋₆ alkyl group.), or a single bond;
Z is a straight or branched chain C₁₋₈ alkylene;
R¹ is hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group;
and the substituents of the said alkyl group, alkenyl group and alkynyl group are selected from the group consisting of a halogen atom, hydroxy group, carboxy group, an C₁₋₆ alkoxy group, a C₁₋₆ haloalkoxy group, an C₁₋₆ alkylthio group, an C₁₋₆ alkylsulfonyl group, an C₁₋₆ alkylsulfinyl group, an C₂₋₅ alkoxycarbonyl group, an C₂₋₁₀ acyloxy group, a substituted or unsubstituted amino group, a substituted or unsubstituted carbamoyl group, a substituted or unsubstituted sulfamoyl group, an ureido group which may be substituted by the same or different one or two alkyl groups, a substituted or unsubstituted 6 to 10 membered aryl group, a substituted or unsubstituted aryloxy group, a substituted or unsubstituted arylthio group, a substituted or unsubstituted 5 to 10 membered heteroaryl group which contains 1 to 4 hetero atom selected from 0 to 2 nitrogen atoms, 0 to 1 oxygen atom and 0 to 1 sulfur atom, a substituted or unsubstituted heteroaryloxy group, a substituted or unsubstituted heteroarylthio group, a substituted or unsubstituted C₃₋₈ cycloalkyl group, a substituted or unsubstituted C₃₋₈ cycloalkoxy group, a substituted or unsubstituted cycloalkylthio group, a substituted or unsubstituted 4 to 7 membered saturated heterocyclic group which contains 1 to 4 hetero atoms selected from 0 to 2 nitrogen atoms, 0 to 2 oxygen atoms and 0 to 2 sulfur atoms, a substituted or unsubstituted saturated heterocycle-oxy group, and a substituted or unsubstituted saturated heterocycle-thio group;
and the substituents of said amino group, carbamoyl group and sulfamoyl group are selected from the group consisting an C₁₋₆ alkyl group, an C₂₋₆ alkenyl group, an C₂₋₆ alkynyl group, an C₃₋₆ acycloalkyl group, an C₂₋₅ alkylcarbonyl group, an C₂₋₅ alkoxycarbonyl group and an C₁₋₆ alkylsulfonyl group (the above seven groups may be substituted by a halogen atom, hydroxy group or an alkoxy group, respectively.), or the two substituents may be combined together to form a substituted or unsubstituted 4 to 7 membered saturated heterocyclic group containing 1 to 4 hetero atoms selecting from 1 to 2 nitrogen atoms, 0 to 1 oxygen atom and 0 to 1 sulfur atom;
the substituents of said aryl group, aryloxy group, arylthio group, heteroaryl group, heteroaryloxy group, heteroarylthio group, cycloalkyl group, cycloalkoxy group, cycloalkylthio group, saturated heterocyclic group, saturated heterocycle-oxy group, saturated heterocycle-thio group and saturated nitrogen containing heterocyclic group are selected from the group consisting of a halogen atom, hydroxy group, carboxy group, an C₁₋₆ alkyl group, an C₁₋₆ alkoxy group, an C₂₋₅ alkylcarbonyl group, an C₂₋₅ alkoxycarbonyl group (the above four groups may be substituted by a halogen atom, hydroxy group or an alkoxy group, respectively), a C₁₋₆ haloalkyl group, a C₁₋₆ haloalkoxy group, amino group, an C₁₋₆ alkylamino group, and a di C₁₋₆ alkyl amino group, in the formula (1),
or its pharmaceutically acceptable salt.

3. The 8-oxoadenine compound or a pharmaceutically acceptable salt thereof according to claim 1 or 2, wherein R² in the formula (1) is methyl group.

4. The 8-oxoadenine compound or a pharmaceutically acceptable salt thereof according to claim 1 or 2, wherein R² in the formula (1) is a substituted C₂-₆ alkyl group.

5. The 8-oxoadenine compound or a pharmaceutically acceptable salt thereof according to claim 4, wherein R² in the formula (1) is an C₂₋₁₀ alkyl group substituted by a substituted or unsubstituted amino group.

6. The 8-oxoadenine compound or a pharmaceutically acceptable salt thereof according to any one of claims 1 to 5, wherein R³ in the formula (1) is hydrogen atom.

7. The 8-oxoadenine compound or a pharmaceutically acceptable salt thereof according to any one of claims 1 to 6, wherein Z in the formula (1) is a straight chain C₁₋₆ alkylene group.

8. The 8-oxoadenine compound or a pharmaceutically acceptable salt thereof according to any one of 1 to 7, wherein X¹ in the formula (1) is a single bond, oxygen atom or sulfur atom.

9. The 8-oxoadenine compound or a pharmaceutically acceptable salt thereof according to any one of claims 1 to 8, wherein R¹ in the formula (1) is an C₁₋₆ alkyl group which is optionally substituted by an alkoxycarbonyl group, hydroxy group or an alkoxy group.

10. The 8-oxoadenine compound or a pharmaceutically acceptable salt thereof according to claim 1, wherein X¹ in the formula (1) is a single bond, R¹ is an C₁₋₆ alkyl group which is substituted by methoxycarbonyl group.

11. A pharmaceutical composition containing the 8-oxoadenine compound or a pharmaceutically acceptable salt thereof as claimed in any one of claims 1 to 10 as an active ingredient.

12. A medicament for topical administration containing the 8-oxoadenine compound or a pharmaceutically acceptable salt thereof as clamed in any one of claims 1 to 10 as an active ingredient.

13. An immuno-modulator containing the 8-oxoadenine compound or a pharmaceutically acceptable salt thereof as claimed in any one of claims 1 to 10 as an active ingredient.

14. A therapeutic or prophylactic agent for viral diseases, cancers or allergic diseases containing the 8-oxoadenine compound or a pharmaceutically acceptable salt thereof as claimed in any one of claims 1 to 10 as an active ingredient.

15. Use of the 8-oxoadenine compound, or a pharmaceutically acceptable salt thereof as claimed in any of claim 1 to 10 as a medicament.

16. Use of the 8-oxoadenine compound, or a pharmaceutically acceptable salt thereof as claimed in any of claims 1 to 10 for manufacturing an immuno-modulator.

17. Use of the 8-oxoadenine compound, or a pharmaceutically acceptable salt thereof as claimed in any of claims 1 to 10 for manufacturing a therapeutic or prophylactic agent for viral diseases, cancers or allergic diseases.

18. A method for modulating immune response which comprises administering, to a patient an effective amount of the 8-oxoadenine compound, or a pharmaceutically acceptable salt thereof as claimed in any of claims 1 to 10.

19. A method for treating or preventing viral diseases, cancers or allergic diseases which comprises administering, to a patient an effective amount of the 8-oxoadenine compound, or a pharmaceutically acceptable salt thereof as claimed in any of claims 1 to 10.

20. A process for preparing the 8-oxoadenine compound as claimed in any of claims 1 to 10, which comprises brominating a compound represented by the formula (9): 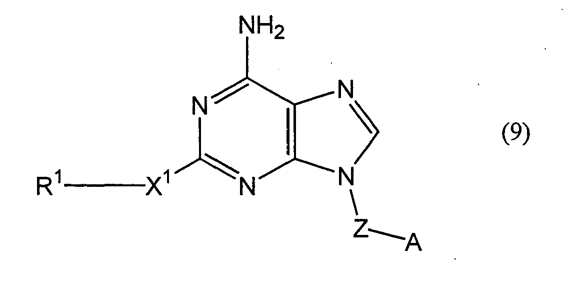 , wherein A, Z, R¹ and X¹ are the same as defined above, and hydrolyzing the resultant or reacting the resultant with a metal alkoxide and then hydrolyzing.

21. A compound represented by the formula (9): 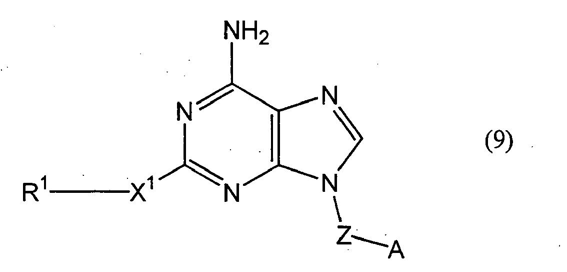 , wherein A, Z, R¹ and X¹ are the same as defined in claim 1.

22. An 8-oxoadenine compound or its pharmaceutically acceptable salt selected from the group consisting of the following compounds:
8-hydroxy-2-(3-hydroxypropyl thio)-9-(3-methoxycarbonylmethylbenzyl)adenine,
8-hydroxy-2-(4-hydroxybutylthio)-9-(3-methoxycarbonylmethylbenzyl)adenine,
8-hydroxy-2-(2-methoxyethylthio)-9-(3-methoxycarbonylmethylbenzyl) adenine,
8-hydroxy-2-(3-hydroxypropoxy)-9-(3-methoxycarbonylmethylbenzyl) adenine,
8-hydroxy-2-(2-hydroxyethoxy)-9-(3-methoxycarbonylmethylbenzyl)adenine,
8-hydroxy-2-(4-hydroxybutoxy)-9-(3-methoxycarbonylmethylbenzyl)adenine,
8-hydroxy-9-(3-methoxycarbonylmethylbenzyl)-2-(4,4,4-trifluorobutoxy)adenine,
8-hydroxy-9-(3-methoxycarbonylmethylbenzyl)-2-[N-(2-methoxyethyl)amino]adenine,
2-butoxy-8-hydroxy-9-[2-(3-methoxycarbonylmethylphenyl)ethyl] adenine,
2-butoxy-8-hydroxy-9-[3-(3-methoxycarbonylmethylphenyl)propyl]adenine,
2-(2,3-dihydroxy-1-propoxy)-8-hydroxy-9-(3-methoxycarbonylmethylbenzyl)adenine,
2-(2-ethoxyethoxy)-8-hydroxy-9-(3-methoxycarbonylmethylbenzyl)adenine,
2-cyclohexylmethoxy-8-hydroxy-9-(3-methoxycarbonylmethylbenzyl)adenine,
2-benzyloxy-8-hydroxy-9-(3-methoxycarbonylmethylbenzyl)adenine,
8-hydroxy-2-(2-methoxycarbonylethyl)-9-(3-methoxycarbonylmethylbenzyl)adenine,
2-butoxy-8-hydroxy-9-{(5-methoxycarbonylmethyl-2-thienyl) methyl}adenine,
2-butoxy-8-hydroxy-9-{(2-methoxycarbonylmethyl-4-pyridyl) methyl}adenine,
2-butoxy-8-hydroxy-9-{(6-methoxycarbonylmethyl-2-pyridyl)methyl}adenine,
2-butoxy-8-hydroxy-9-{(4-methoxycarbonylmethyl-2-pyridyl)methyl}adenine,
2-butoxy-8-hydroxy-9-[(2-methoxy-5-methoxycarbonylmethyl) benzyl] adenine,
2-butoxy-9-[(4-fluoro-3-methoxycarbonylmethyl)benzyl]-8-hydroxyadenine,
2-butoxy-8-hydroxy-9-[(4-methoxy-3-methoxycarbonylmethyl)benzyl]adenine,
2-butylthio-8-hydroxy-9-(3-methoxycarbonylmethylbenzyl)adenine,
2-[3-(ethylsulfonyl)propoxy]-8-hydroxy-9-(3-methoxycarbonylmethylbenzyl)adenine,
8-hydroxy-9-(3-methoxycarbonylmethylbenzyl)-2-[3-(methylsulfonyl)propoxy]adenine,
8-hydroxy-9-(3-methoxycarbonylmethylbenzyl)-2-(4-pyridylmethylamino) adenine,
8-hydroxy-9-(3-methoxycarbonylmethylbenzyl)-2-[2-methoxyethyl(N-methyl)amino]adenine,
2-benzylamino-8-hydroxy-9-(3-methoxycarbonylmethylbenzyl)adenine,
8-hydroxy-9-(3-methoxycarbonylmethylbenzyl)-2-[(4-pyridylmethyl)oxy]adenine,
2-ethoxy-8-hydroxy-9-(3-methoxycarbonylmethylbenzyl)adenine,
8-hydroxy-9-(3-methoxycarbonylmethylbenzyl)-2-propoxyadenine,
8-hydroxy-9-(3-methoxycarbonylmethylbenzyl)-2-pentoxyadenine,
2-butoxy-8-hydroxy-9-{3-[(4-dimethylaminobutoxy)carbonylmethyl]benzyl}adenine,
2-ethoxy-8-hydroxy-9-{3-[(4-dimethylaminobutoxy)carbonylmethyl]adenine,
2-butoxy-8-hydroxy-9-{3-[(2-dimethylaminoethoxy)carbonylmethyl]benzyl}adenine,
2-butoxy-8-hydroxy-9-{3-[(3-dimethylaminopropoxy)carbonylmethyl]benzyl}adenine,
2-butoxy-8-hydroxy-9-{3-[(6-dimethylaminohexanoxy)carbonylmethyl]benzyl}adenine,
2-butoxy-8-hydroxy-9-{3-[(3-diethylaminopropoxy)carbonylmethyl]benzyl}adenine,
2-butoxy-8-hydroxy-9-{3-[(2-morpholinoethoxy)carbonylmethyl]benzyl}adenine,
2-butoxy-8-hydroxy-9-{3-[(2-piperidinoethoxy)carbonylmethyl]benzyl}adenine,
2-butoxy-8-hydroxy-9-{3-[(2,2,2-trifluoroethoxy)carbonylmethyl]benzyl}adenine,
2-butoxy-8-hydroxy-9-{3-[(2-hydroxyethoxy)carbonylmethyl]benzyl}adenine,
2-butoxy-8-hydroxy-9-{3-[(2,3-dihydroxypropoxy)carbonylmethyl]benzyl}adenine,
2-butoxy-8-hydroxy-9-{5-[(4-dimethylaminobutoxy)carbonylmethyl]-2-methoxybenzyl}adenine,
8-hydroxy-2-(4-hydroxybutylthio)-9-{3-[(2-hydroxyethoxy)carbonylmethyl]benzyl}adenine,
8-hydroxy-9-{3-[(4-dimethylaminobutoxy)carbonylmethyl]benzyl}-2-[(4-pyridylmethyl)oxy]adenine,
2-[2-(4-bromophenyloxy)ethoxy]-8-hydroxy-9-(3-methoxycarbonylmethylbenzyl) adenine,
8-hydroxy9-(3-methoxycarbonylmethylbenzyl)-2-(2-phenyloxyethoxy)adenine,
2-(3-aminopropoxy)-8-hydroxy-9-(3-methoxycarbonylmethylbenzyl)adenine,
2-[3-(N-acetylamino)propoxy]-8-hydroxy-9-(3-methoxycarbonylmethylbenzyl)adenine,
8-hydroxy-2-[3-(N-methanesulfonylamino)propoxy]-9-(3-methoxycarbonylmethylbenzyl)adenine,
8-hydroxy-2-[3-(N-methoxycarbonylamino)propoxy]-9-(3-methoxycarbonylmethylbenzyl) adenine,
8-hydroxy-9-(3-methoxycarbonylmethylbenzyl)-2-(3-ureidopropoxy)adenine,
2-(2-diethylaminoethoxy)-8-hydroxy-9-(3-methoxycarbonylmethylbenzyl)adenine,
8-hydroxy-9-(3-methoxycarbonylmethylbenzyl)-2-trifluoromethyladenine,
2-butyl-8-hydroxy-9-(3-methoxycarbonylmethylbenzyl)adenine,
8-hydroxy-9-(3-methoxycarbonylmethylbenzyl)-2-pentyladenine,
8-hydroxy-9-(3-methoxycarbonylmethylbenzyl)-2-(3-methoxypropyl)adenine,
2-ethoxymethyl-8-hydroxy-9-(3-methoxycarbonylmethylbenzyl)adenine,
2-ethoxymethyl-8-hydroxy-9-{3-[(4-dimethylaminobutoxy)carbonylmethyl]benzyl}adenine,
2-cyclopentyl-8-hydroxy-9-(3-methoxycarbonylmethylbenzyl)adenine,
8-hydroxy-2-(3-hydroxypropyl)-9-(3-methoxycarbonylbenzyl)adenine,
2-(4-fluorobenzyl)-8-hydroxy-9-(3-methoxycarbonylmethylbenzyl)adenine,
8-hydroxy-9-(3-methoxycarbonylmethylbenzyl)-2-(2-pyridylmethoxy) adenine,
8-hydroxy-9-(3-methoxycarbonylmethylbenzyl)-2-(3-pyridylmethoxy) adenine,
8-hydroxy-9-(3-methoxycarbonylmethylbenzyl)-2-(3-morpholinopropoxy)adenine,
8-hydroxy-9-(3-methoxycarbonylmethylbenzyl)-2-{2-(phenylsulfanyl)ethoxy}adenine,
8-hydroxy-9-(3-methoxycarbonylmethylbenzyl)-2-(2-methylsulfanylethoxy)adenine,
8-hydroxy-9-(3-methoxycarbonylmethylbenzyl)-2-phenylsulfanyladenine,
8-hydroxy-9-(3-methoxycarbonylmethylbenzyl)-2-(tetrahydrofuran-2-ylmethoxy) adenine,
9-(3-carboxymethylbenzyl)-8-hydroxy-2-(3-hydroxypropylthio)adenine,
9-(3-carboxymethylbenzyl)-8-hydroxy-2-(4-hydroxybutylthio)adenine,
9-(3-carboxymethylbenzyl)-8-hydroxy-2-(2-methoxyethylthio)adenine,
9-(3-carboxymethylbenzyl)-8-hydroxy-2-(3-hydroxypropoxy)adenine,
9-(3-carboxymethylbenzyl)-8-hydroxy-2-(2-hydroxyethoxy)adenine,
9-(3-carboxymethylbenzyl)-8-hydroxy-2-(4-hydroxybutoxy)adenine,
9-(3-carboxymethylbenzyl)-8-hydroxy-2-(4,4,4-trifluorobutoxy)adenine,
9-(3-carboxymethylbenzyl)-8-hydroxy-2-[N-(2-methoxyethyl)amino]adenine,
2-butoxy-9-[2-(3-carboxymethylphenyl)ethyl]-8-hydroxyadenine,
2-butoxy-9-[3-(3-carboxymethylphenyl)propyl]-8-hydroxyadenine,
9-(3-carboxymethylbenzyl)-2-(2,3-dihydroxy-1-propoxy)-8-hydroxyadenine,
9-(3-carboxymethylbenzyl)-2-(2-ethoxyethoxy)-8-hydroxyadenine,
9-(3-carboxymethylbenzyl)-2-cyclohexylmethoxy-8-hydroxyadenine,
2-benzyloxy-9-(3-carboxymethylbenzyl)-8-hydroxyadenine,
2-(2-carboxyethyl)-9-(3-carboxymethylbenzyl)-8-hydroxyadenine,
2-butoxy 9-{(5-carboxymethyl-2-thienyl)methyl}-8-hydroxyadenine,
2-butoxy-9-{(6-carboxymethyl-2-pyridyl)methyl}-8-hydroxyadenine,
2-butoxy-9-{(4-carboxymethyl-2-pyridyl)methyl}-8-hydroxyadenine,
2-butoxy-9-(5-carboxymethyl-2-methoxy)benzyl-8-hydroxyadenine,
2-butoxy-9-(3-carboxymethyl-4-fluoro)benzyl-8-hydroxyadenine,
2-butoxy-9-(3-carboxyrnethyl-4-methoxy)benzyl-8-hydroxyadenine,
9-(3-carboxymethylbenzyl)-2-ethoxy-8-hydroxyadenine,
9-(3-carboxymethylbenzyl)-8-hydroxy-2-propoxyadenine,
9-(3-carboxymethylbenzyl)-8-hydroxy-2-pentoxyadenine,
9-(3-carboxymethylbenzyl)-8-hydroxy-2-(2-phenyloxyethoxy)adenine,
2-[3-(N-acetylamino)propoxy]-9-(3-carboxymethylbenzyl)-8-hydroxyadenine,
9-(3-carboxymethylbenzyl)-8-hydroxy-2-[3-(N-methanesulfonylamino)propoxy]adenine,
9-(3-carboxymethylbenzyl)-2-cyclopentyl-8-hydroxyadenine
9-(3-carboxymethylbenzyl)-8-hydroxy-2-(3-hydroxypropane-1-yl)adenine,
9-(3-carboxymethylbenzyl)-8-hydroxy-2-(2-pyridylmethoxy)adenine,
9-(3-carboxymethylbenzyl)-8-hydroxy-2-(3-pyridylmethoxy)adenine,
9-(3-carboxymethylbenzyl)-8-hydroxy-2-(2-phenylsulfanylethoxy)adenine,
and
9-(3-carboxymethylbenzyl)-8-hydroxy-2-(tetrahydrofuran-2-ylmethoxy) adenine.
